# EUROPEAN PATENT APPLICATION

(11) **EP 4 317 185 A2**
(43) Date of publication of application: **07.02.2024**
(21) Application number: 23200645.2
(22) Date of filing: 17.10.2018
(51) Int. Cl.: C07K 16/22

(54) **FULLY-HUMAN POST-TRANSLATIONALLY MODIFIED ANTIBODY THERAPEUTICS**

(30) Priority: 18.10.2017 US 201762574106 P; 22.12.2017 US 201762609750 P; 18.07.2018 US 201862700124 P
(62) Divisional of application: 18797410.0
(71) Applicant: REGENXBIO Inc., Rockville, MD 20850 (US)
(72) Inventor: DANOS, Olivier, New York, 10128 (US); WU, Zhuchun, North Potomac, 20878 (US); GERNER, Franz, Myersville, 21773 (US); VAN EVEREN, Sherri, Menlo Park, 94025 (US)
(74) Representative: Appleyard Lees IP LLP

(57) **Abstract**

Provided are methods and compositions for the delivery of fully human post-translationally modified therapeutic monoclonal antibodies and antigen-binding fragments thereof. The fully human post-translationally modified therapeutic monoclonal antibodies may be preferably delivered by gene therapy methods, particularly as a recombinant adeno-associated virus (rAAV) vector to the appropriate tissue. Methods of manufacture of the AAV vectors, pharmaceutical compositions and methods of treatment are also provided. In addition, provided are methods of producing therapeutic antibodies that are "biobetters" as fully human post-translationally modified. These fully human post-translationally modified therapeutic antibodies may be administered to a subject in need of treatment with the therapeutic antibody.

## Description

### 0. SEQUENCE LISTING

The instant application contains a Sequence Listing which has been submitted electronically in ASCII format and is hereby incorporated by reference in its entirety. Said ASCII copy, created on October 17, 2018, is named 26115_105004_SL.txt and is 400,185 bytes in size.

### 1. INTRODUCTION

Compositions and methods are described for the delivery of a fully human post-translationally modified (HuPTM) therapeutic monoclonal antibody ("mAb") or the HuPTM antigen-binding fragment of a therapeutic mAb-*e.g*., a fully human-glycosylated (HuGly) Fab of the therapeutic mAb-to a human subject diagnosed with a disease or condition indicated for treatment with the therapeutic mAb.

### 2. BACKGROUND OF THE INVENTION

Therapeutic mAbs have been shown to be effective in treating a number of diseases and conditions. However, because these agents are effective for only a short period of time, repeated injections for long durations are often required, thereby creating considerable treatment burden for patients.

### 3. SUMMARY OF THE INVENTION

Compositions and methods are described for the delivery of a HuPTM mAb or a HuPTM antigen-binding fragment of a therapeutic mAb (for example, a fully human-glycosylated Fab (HuGlyFab) of a therapeutic mAb) to a patient (human subject) diagnosed with a disease or condition indicated for treatment with the therapeutic mAb. Such antigen-binding fragments of therapeutic mAbs include a Fab, F(ab')₂, or scFv (single-chain variable fragment) (collectively referred to herein as "antigen-binding fragment"). "HuPTM Fab" as used herein may include other antigen binding fragments of a mAb. In an alternative embodiment, full-length mAbs can be used. Delivery may be advantageously accomplished via gene therapy-*e.g*., by administering a viral vector or other DNA expression construct encoding a therapeutic mAb or its antigen-binding fragment (or a hyperglycosylated derivative of either) to a patient (human subject) diagnosed with a condition indicated for treatment with the therapeutic mAb-to create a permanent depot in a tissue or organ of the patient that continuously supplies the HuPTM mAb or antigen-binding fragment of the therapeutic mAb, *i.e.,* a human-glycosylated transgene product, to a target tissue where the mAb or antigen-binding fragment there of exerts its therapeutic effect.

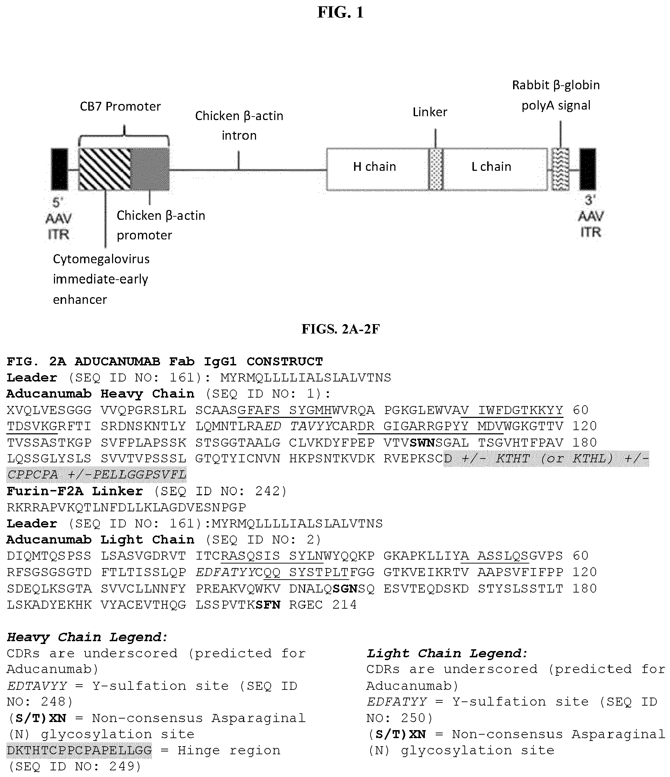

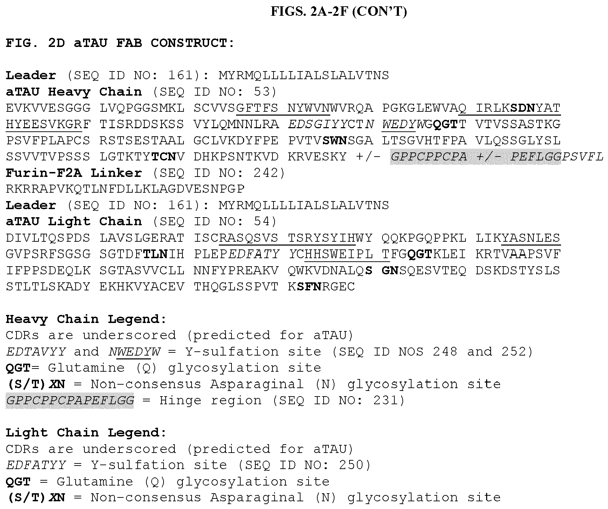

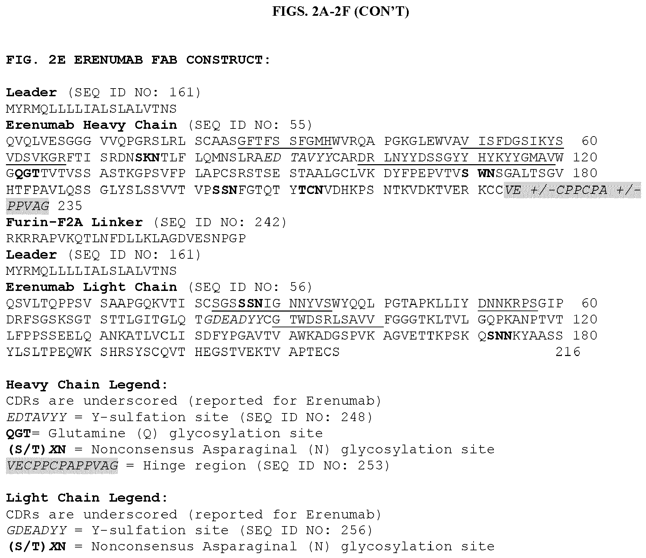

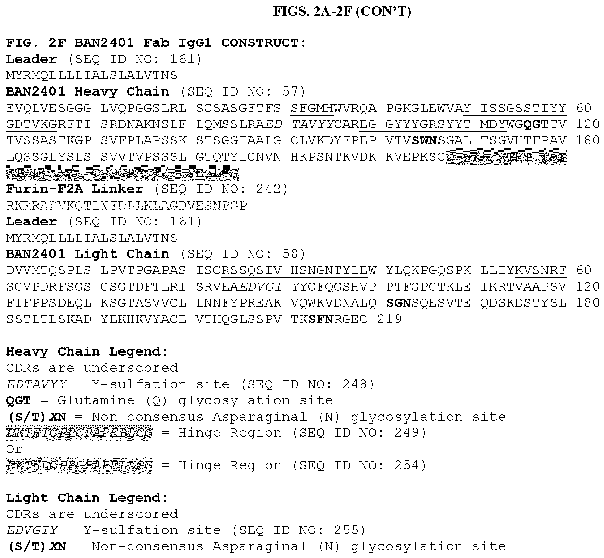

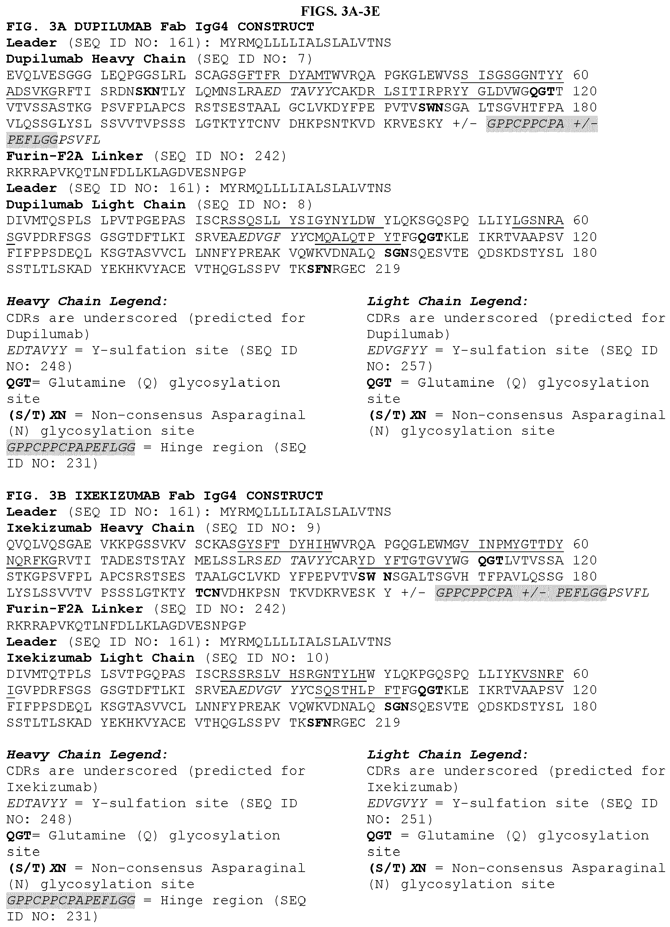

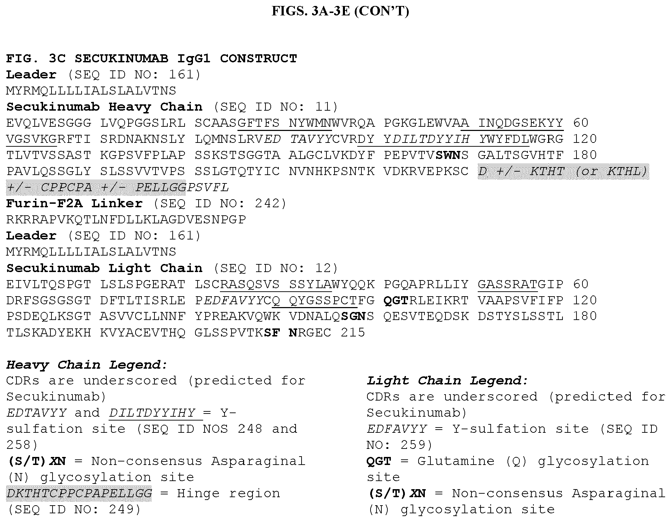

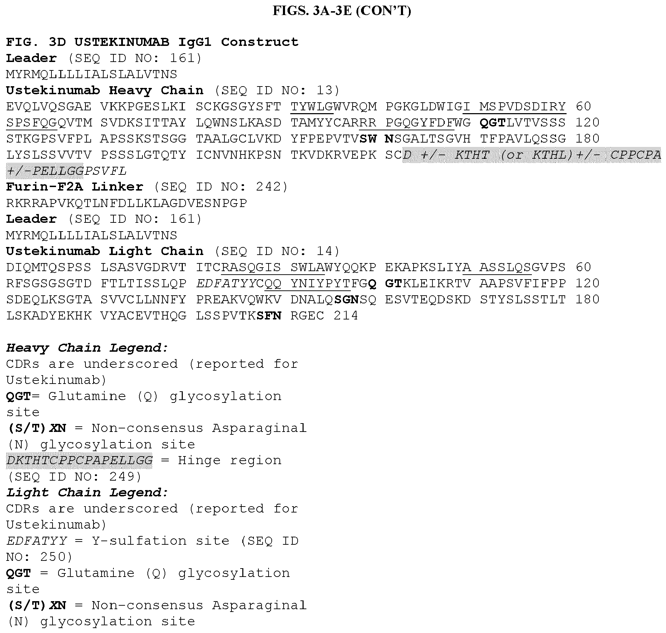

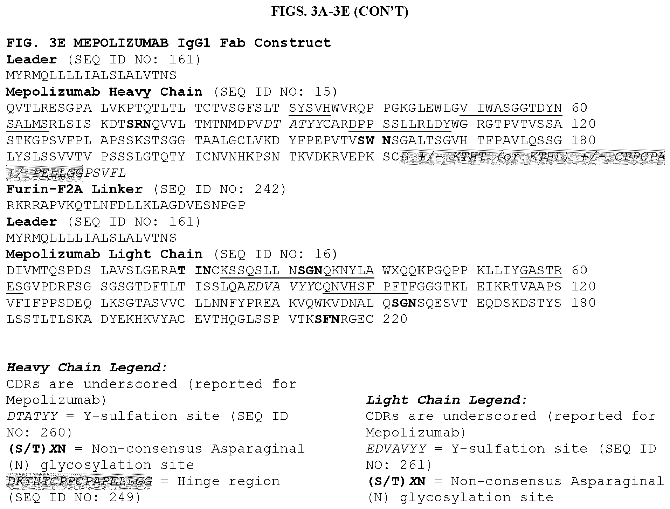

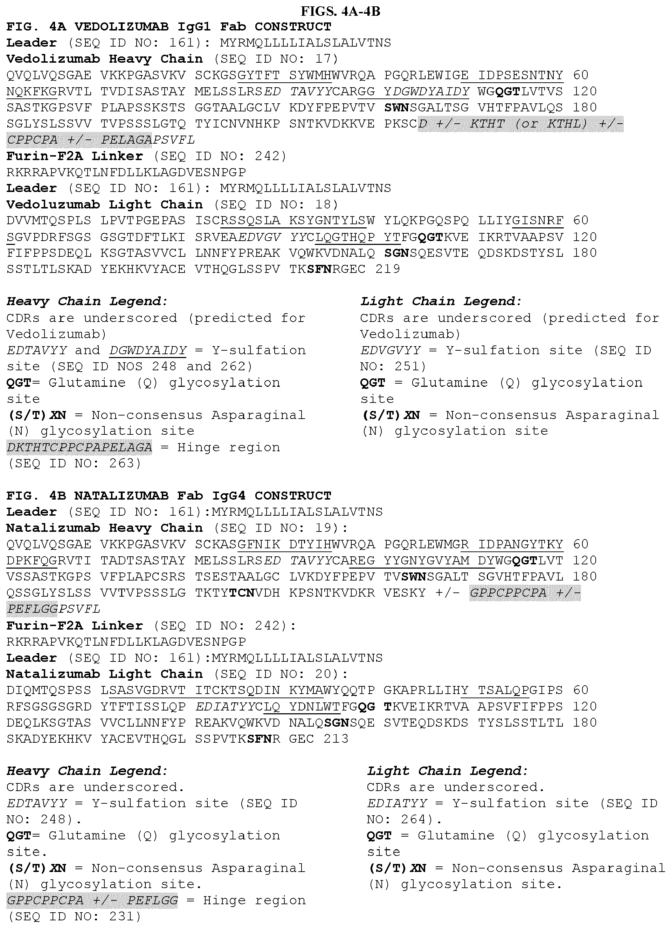

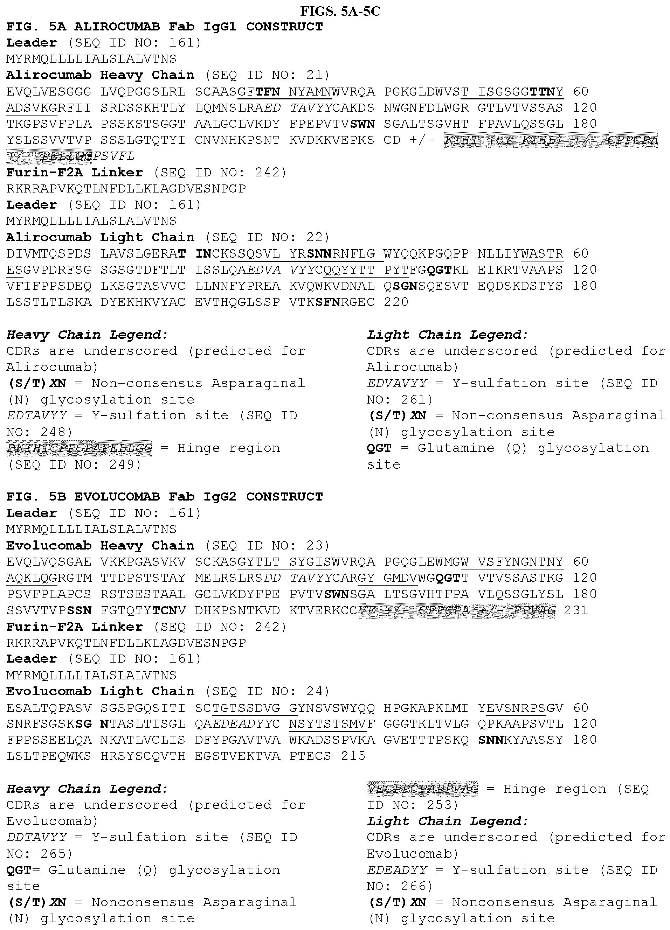

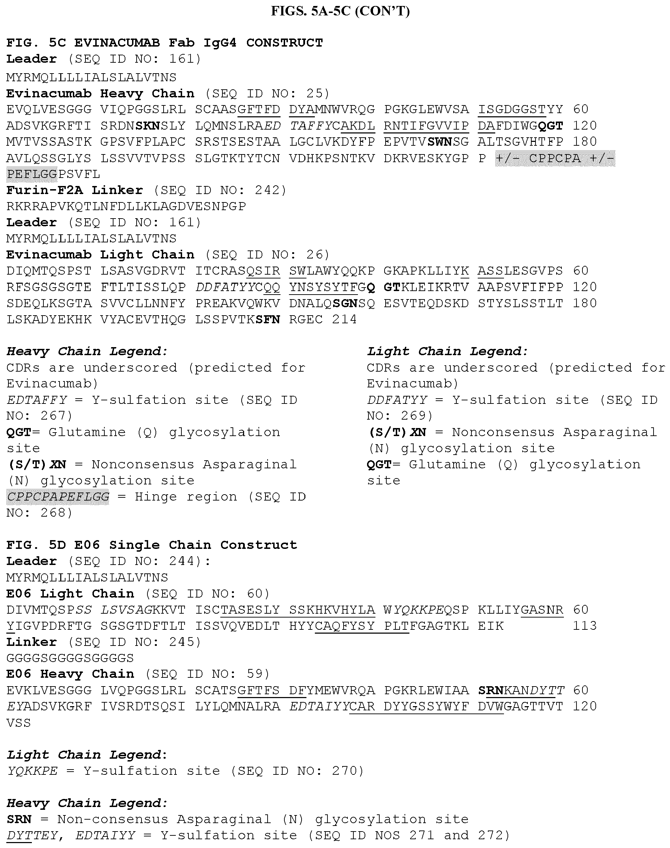

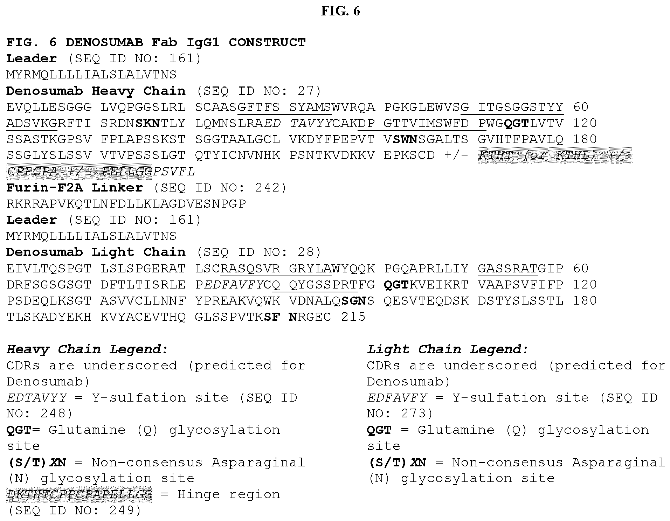

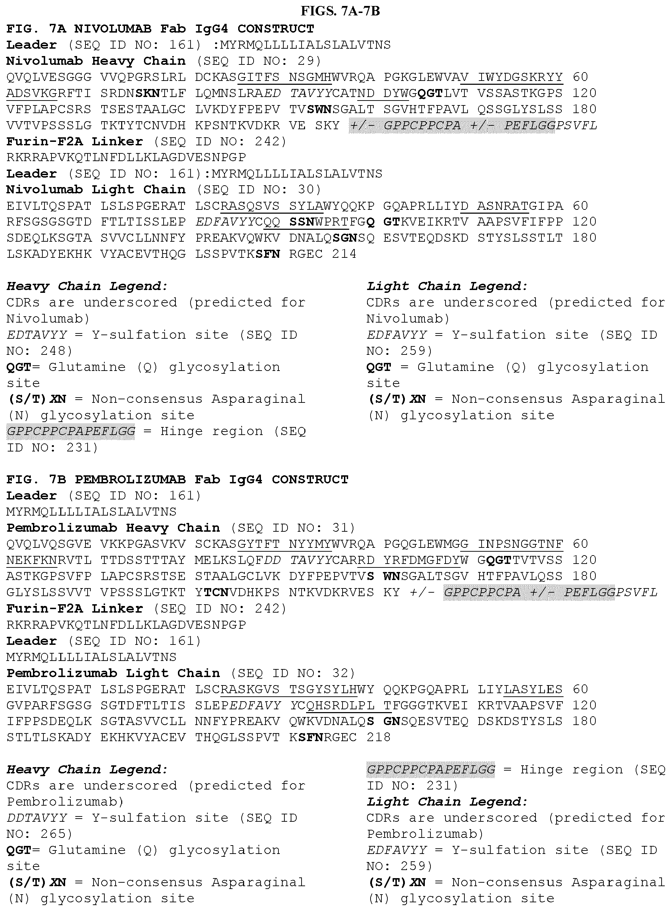

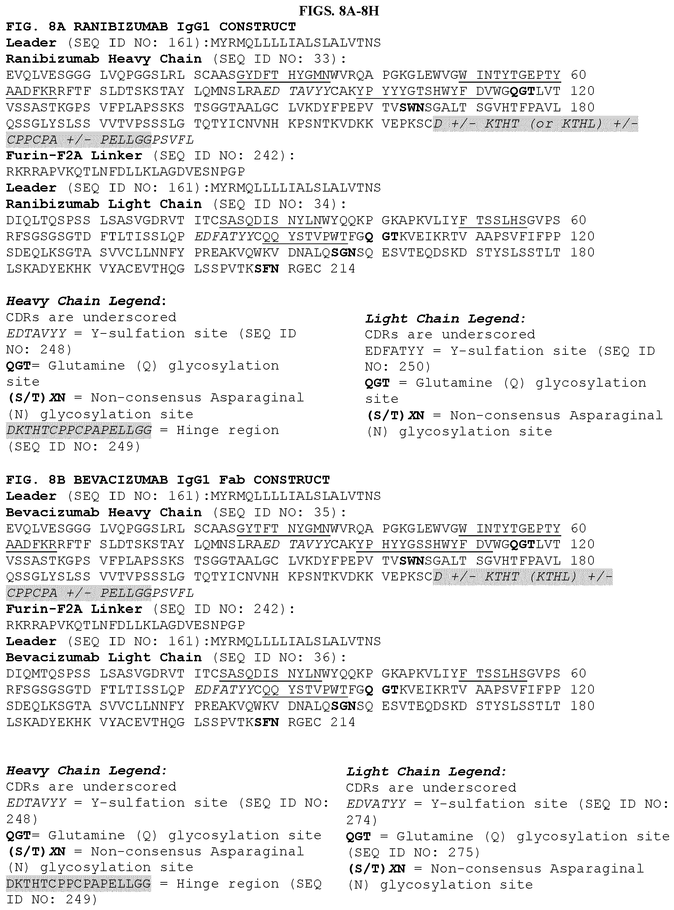

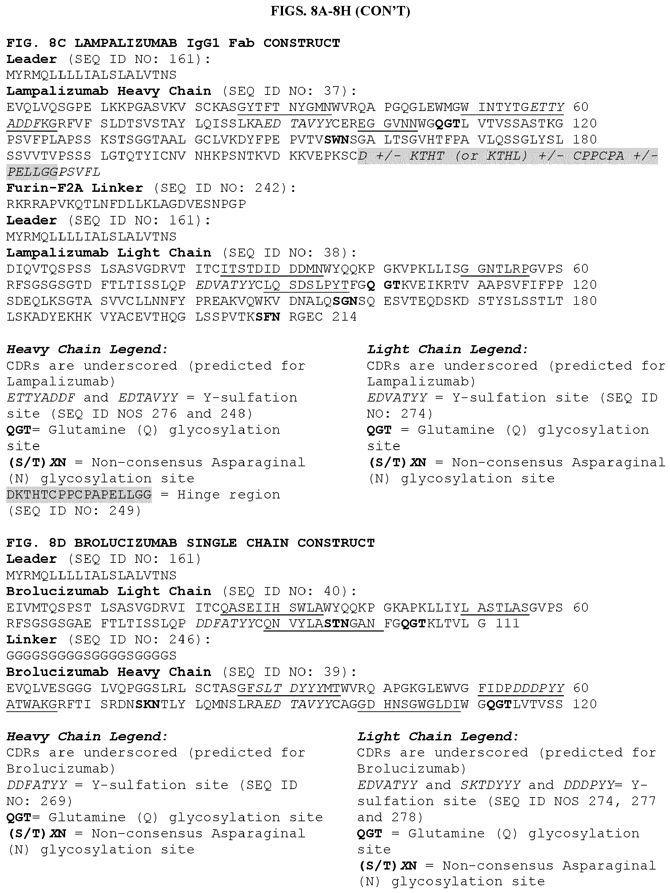

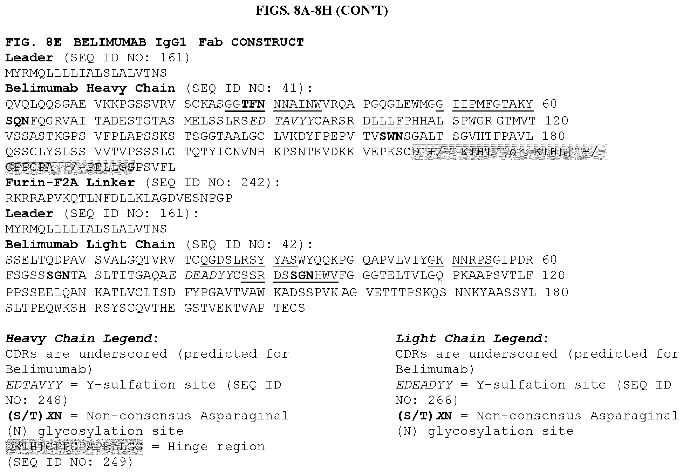

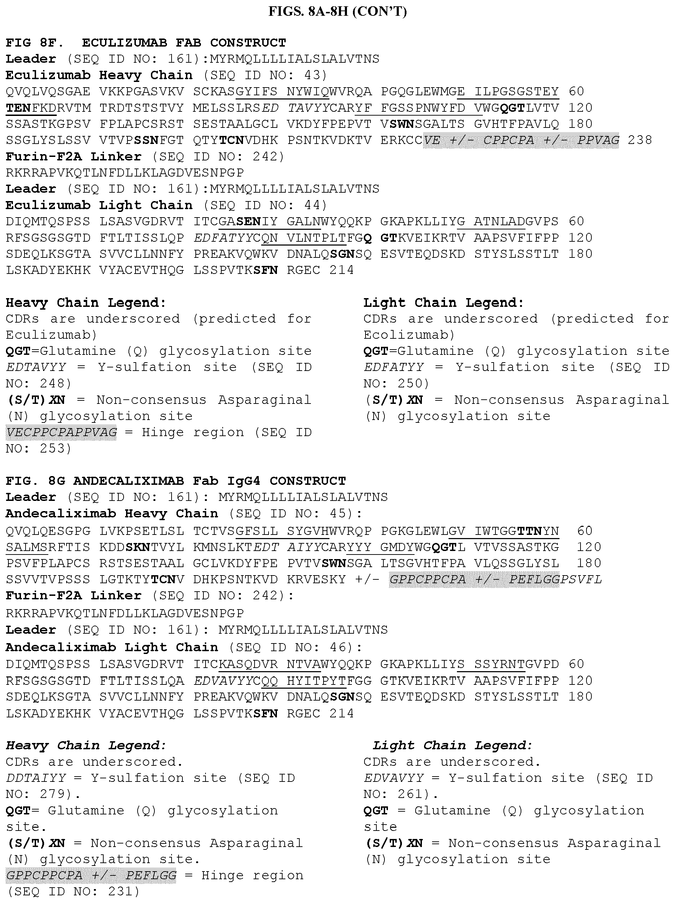

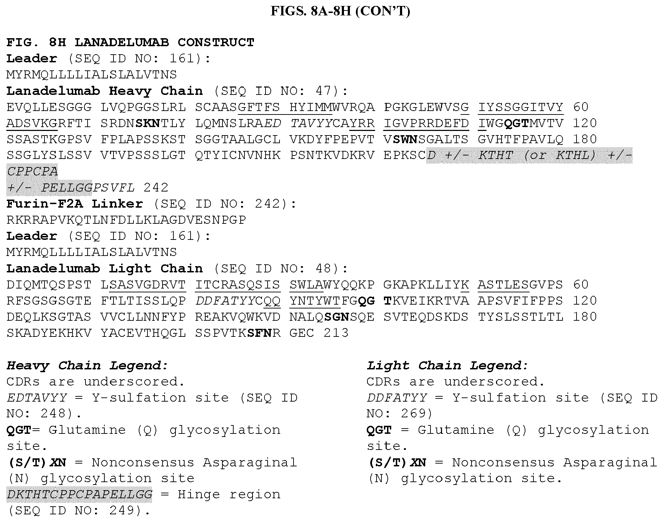

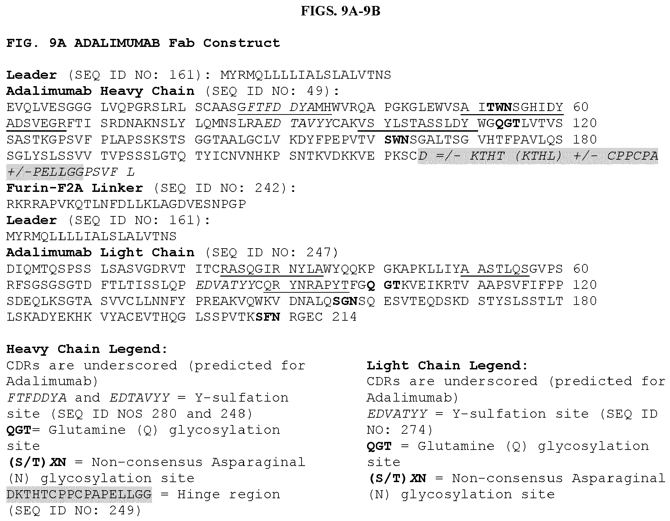

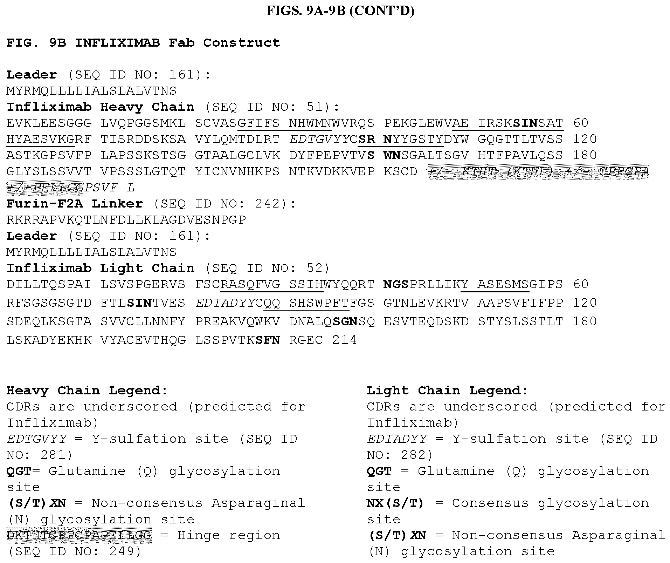

The HuPTM mAb or HuPTM antigen-binding fragment encoded by the transgene can include, but is not limited to, a full-length or an antigen-binding fragment of a therapeutic antibody that binds to:
- *Nervous System Targets, including Amyloid beta (Aβ or Abeta) peptides* derived from the amyloid precursor protein (APP) implicated in Alzheimer's disease, including but not limited to, aducanumab, crenezumab, gantenerumab, and BAN2401, indicated for treating Alzheimer's disease (see FIGS. 2A-2C and 2F); *Tau protein* implicated in tauopathies, including Alzheimer's disease, progressive supranuclear palsy, frontotemporal dementia, chronic traumatic encephalopathy, Pick's Complex, primary age-related taupothy, including but not limited to "aTAU" (see FIG. 2D) for treating tauopathies; and *CGRP receptor* implicated in migraines and cluster headaches including but not limited to erenumab (AIMOVIG^{™}) (see FIG. 2E), eptinezumab, fremanezumab, and galcanezumab for treating migraines and cluster headaches;
- *Interleukins or interleukin receptors,* including but not limited to, *IL4R,* such as dupilumab (see FIG 3A), indicated for treating atopic dermatitis; *IL17A* such as ixekizumab (TALTZ^{®}) or secukinumab (COSENTYX^{®}) (see FIGS. 3B and 3C) indicated for treating plaque psoriasis, psoriatic arthritis, and ankylosing spondylitis; *IL-*5, such as mepolizumab (NUCALA^{®}) (see FIG. 3D), indicated for treating asthma; and *IL12*/*IL23* such as ustekinumab (STELARA^{®}) (see FIG. 3E) indicated for treating psoriasis and Crohn's disease;
- *Integrin,* including but not limited to, vedolizumab (ENTYVIO^{®}), indicated for treating ulcerative colitis and Crohn's disease (see FIG. 4A) and natalizumab (anti-integrin alpha 4) for treating multiple sclerosis and Crohn's disease (see FIG. 4B);
- *Hypercholesterolemia and Cardiovascular Disease Targets, such as PCSK9,* including but not limited to, alirocumab (PRALUENT^{®}) and evolocumab (REPATHA^{®}), indicated for treating HeFH and HoFH (see FIGS. 5A and 5B); or *ANGPTL3,* including but not limited to, evinacumab (see FIG. 5C), indicated for the treatment of HoFH and severe forms of dyslipidemia and *proinflammatory proalherogenic phospholipids* including but not limited to E06-scFv for the treatment of cardiovascular disease, including atherosclerosis (see FIG. 5D);
- *RANKL,* including but not limited to, denosumab (XGEVA^{®} and PROLIA^{®}), indicated for treating osteoporosis, increasing bone mass in breast and prostate cancer patients, and preventing skeletal-related events due to bone metastasis (see FIG. 6);
- *PD-1, or PD-L1 or PD-L2,* (these antibodies sometimes referred to herein as PD-1 blockers), including but not limited to, nivolumab (OPDIVO^{®}) and pembrolizumab (KEYTRUDA^{®}), indicated for treating metastatic melanoma, lymphomas, and non-small cell lung carcinomas (see FIGS. 7A and 7B);
- *BLyS (B-lymphocyte stimulator, also known as B-cell activating factor (BAFF)),* including but not limited to, belimumab (BENLYSTA^{®}), indicated for the treatment of systemic lupus erythromatosis (SLE) (see FIG. 8E);
- *Ocular Targets,* including but not limited to, VEGF (vascular endothelial growth factor), including but not limited to, ranibizumab (LUCENTIS^{®}), bevacizumab (AVASTIN^{®}), and brolucizumab indicated for treating neovascular age-related macular degeneration (e.g., "wet AMD") (see FIGS. 8A, 8B and 8D); factor D, including but not limited to lampalizumab, for treating dry AMD (see FIG. 8C); and matrix metalloproteinase 9 (MMP9), including but not limited to andecaliximab, for treating dry AMD (FIG. 8G);
- *TNF-alpha,* including but not limited, to adalimumab (HUMIRA^{®}) and infliximab (REMICADE^{®}) indicated for treating rheumatoid arthritis, psoriatic arthritis, ankylosing spondylitis, Crohn's disease, plaque psoriasis, and ulcerative colitis (FIG. 9A for adalimumab and FIG. 9B for infliximab); and
- *Plasma Protein targets,* such as human complement proteins including but not limited to anti- C5 and C5a complement proteins, such as eculizumab (SOLIRIS^{®}) for the treatment of patients with paroxysmal nocturnal hemoglobinuria (PNH) to reduce hemolysis, or the treatment of atypical hemolytic uremic syndrome (aHUS) to inhibit complement-mediated thrombotic microangiopathy (FIG. 8F); and plasma kallikrein, including but not limited to lanadelumab for treating hereditary angioedema (see FIG 8H);
or such mAbs or antigen-binding fragments engineered to contain additional glycosylation sites on the Fab domain (e.g., see Courtois et al., 2016, mAbs 8: 99-112 which is incorporated by reference herein in its entirety for it description of derivatives of antibodies that are hyperglycosylated on the Fab domain of the full-length antibody).

The recombinant vector used for delivering the transgene includes non-replicating recombinant adeno-associated virus vectors ("rAAV"). However, other viral vectors may be used, including but not limited to lentiviral vectors; vaccinia viral vectors, or non-viral expression vectors referred to as "naked DNA" constructs. Expression of the transgene can be controlled by constitutive or tissue-specific expression control elements.

Gene therapy constructs are designed such that both the heavy and light chains are expressed. The coding sequences for the heavy and light chains can be engineered in a single construct in which the heavy and light chains are separated by a cleavable linker or IRES so that separate heavy and light chain polypeptides are expressed. In certain embodiments, the coding sequences encode for a Fab or F(ab')₂ or an scFv. In other embodiments, the constructs express an scFv in which the heavy and light chain variable domains are connected via a flexible, non-cleavable linker. In certain embodiments, the construct expresses, from the N-terminus, NH₂-V_{L}-linker-V_{H}-COOH or NH₂-V_{H}-linker-V_{L}-COOH.

Therapeutic antibodies delivered by gene therapy have several advantages over injected or infused therapeutic antibodies that dissipate over time resulting in peak and trough levels. Sustained expression of the transgene product antibody, as opposed to injecting an antibody repeatedly, allows for a more consistent level of antibody to be present at the site of action, and is less risky and more convenient for patients, since fewer injections need to be made. Furthermore, antibodies expressed from transgenes are post-translationally modified in a different manner than those that are directly injected because of the different microenvironment present during and after translation. Without being bound by any particular theory, this results in antibodies that have different diffusion, bioactivity, distribution, affinity, pharmacokinetic, and immunogenicity characteristics, such that the antibodies delivered to the site of action are "biobetters" in comparison with directly injected antibodies.

In addition, antibodies expressed from transgenes in vivo are not likely to contain degradation products associated with antibodies produced by recombinant technologies, such as protein aggregation and protein oxidation. Aggregation is an issue associated with protein production and storage due to high protein concentration, surface interaction with manufacturing equipment and containers, and purification with certain buffer systems. These conditions, which promote aggregation, do not exist in transgene expression in gene therapy. Oxidation, such as methionine, tryptophan, and histidine oxidation, is also associated with protein production and storage, and is caused by stressed cell culture conditions, metal and air contact, and impurities in buffers and excipients. The proteins expressed from transgenes in vivo may also oxidize in a stressed condition. However, humans, and many other organisms, are equipped with an antioxidation defense system, which not only reduces the oxidation stress, but sometimes also repairs and/or reverses the oxidation. Thus, proteins produced in vivo are not likely to be in an oxidized form. Both aggregation and oxidation could affect the potency, pharmacokinetics (clearance), and immunogenicity.

Pharmaceutical compositions suitable for administration to human subjects comprise a suspension of the recombinant vector in a formulation buffer comprising a physiologically compatible aqueous buffer, a surfactant and optional excipients.

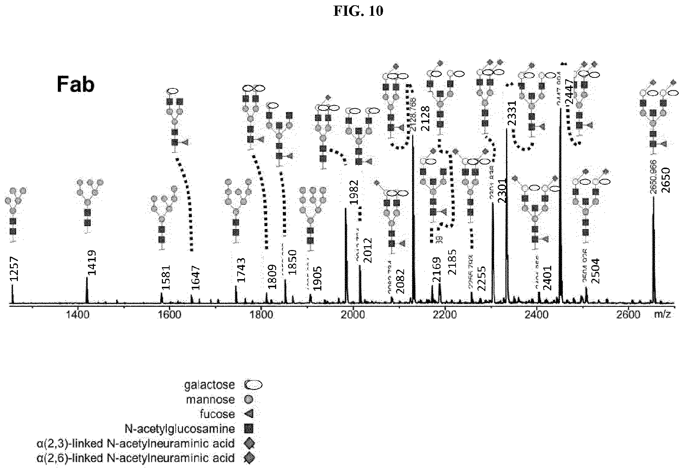

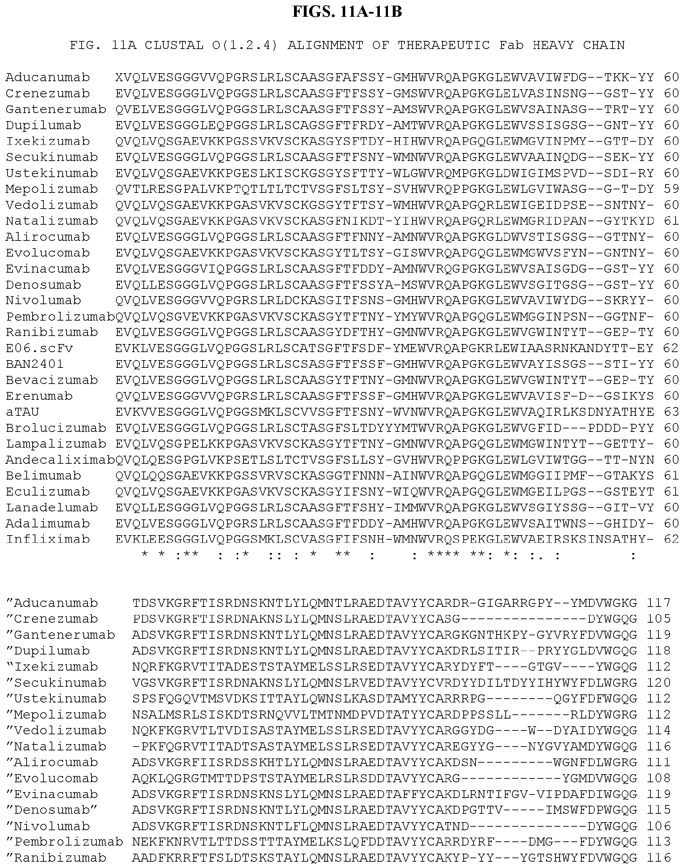

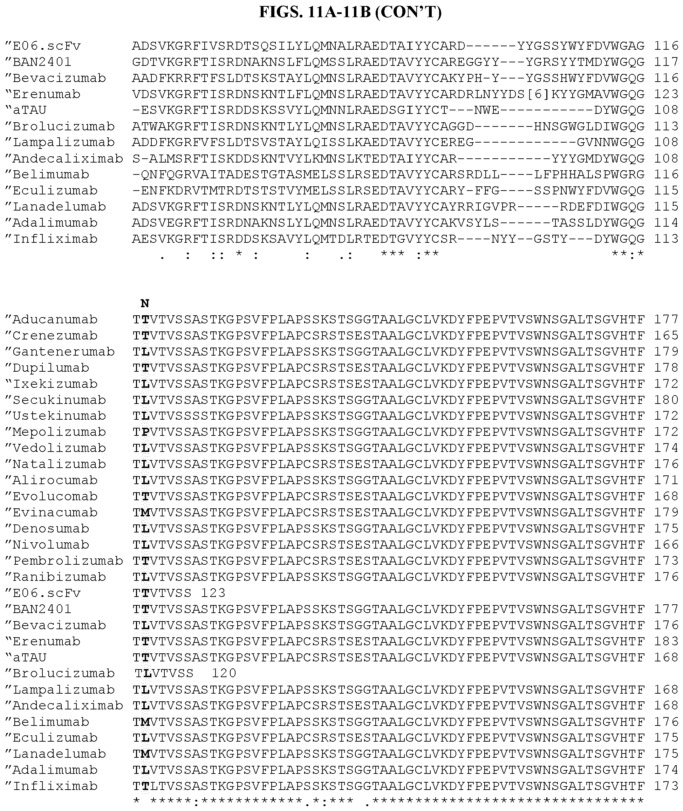

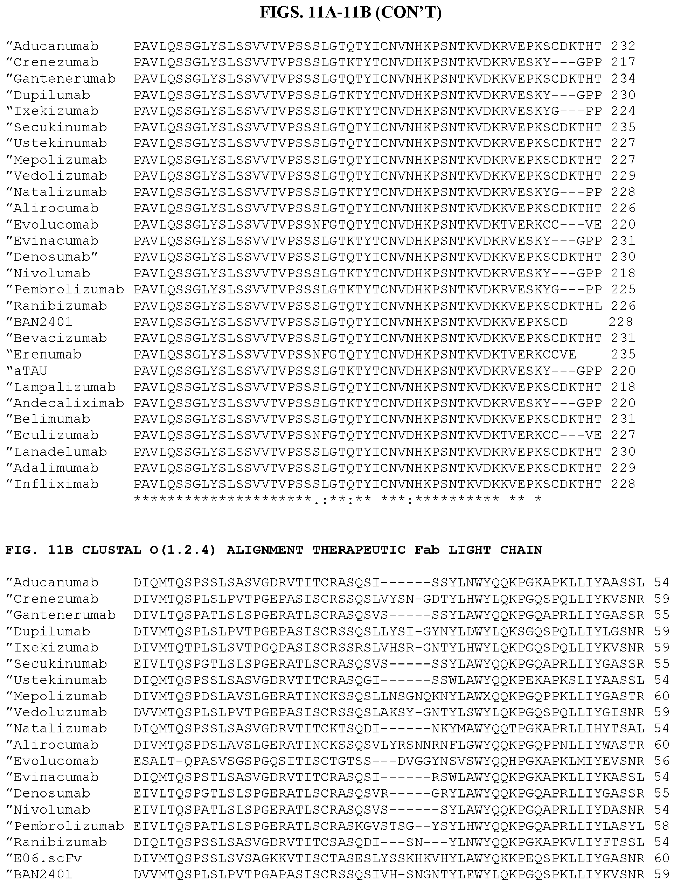

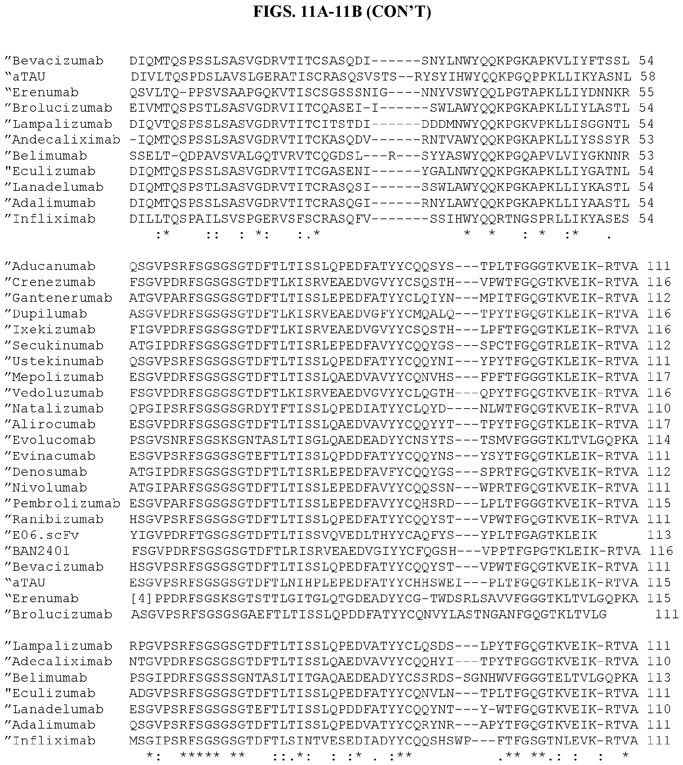

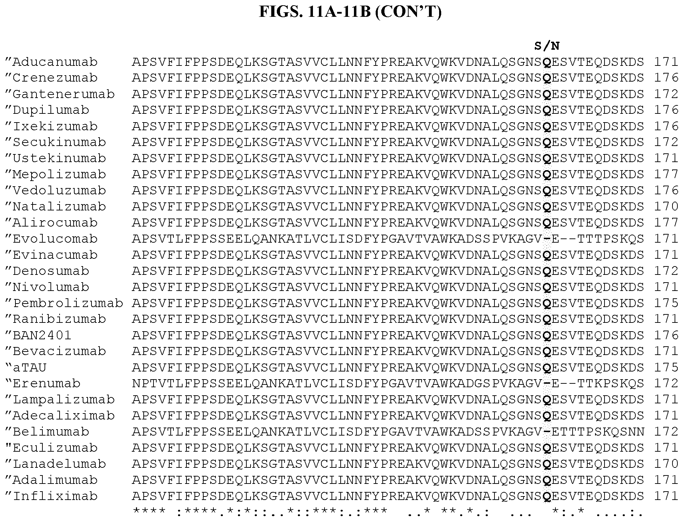

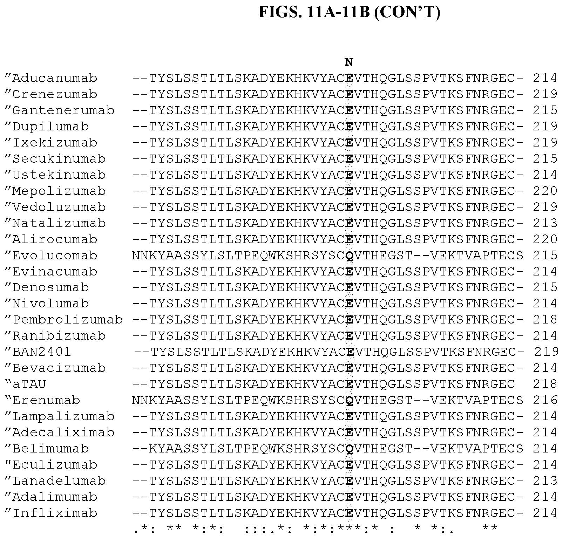

The invention is based, in part, on the following principles:
(i) The mAb therapeutics currently on the market are of the immunoglobulin G (IgG) isotypes, such as IgG1, IgG2, and IgG4, which in general have pharmacokinetic (PK) characteristics, such as slow clearance, long half-life, and limited tissue distribution. After intravenous administration, typical mAb serum PK profiles are biphasic with a rapid distribution phase and a slower elimination phase; thus, repeat administration is required to maintain doses required to treat chronic conditions. Moreover, the distribution of mAbs is generally limited to the vascular and interstitial spaces due to their large size and hydrophilicity. The extent of mAb partitioning from circulation into most tissues generally ranges from about 5-15%, except for brain where it is much lower. *(See, e.g.,* Kamath, 2016, Drug Discovery Today: Technologies 21-22: 75-83, which is incorporated by reference herein in its entirety). Continuous production of HuPTMmAbs or HuPTM Fabs *in situ* avoids repeat administrations and allows the use of Fabs, which would otherwise have too short a systemic half-life to achieve efficacy; and the methods of administration described allow direct access to target tissues, such as the brain, where the delivery of higher doses to such tissues can be achieved.
(ii) The Fab region of a number of therapeutic mAbs possesses glycosylation sites. For example, see FIGS. 2A-2F, 3A-3E, 4A-4B, 5A-5D, 6, 7A-7B, 8A-8H and 9A-9B which identify and highlight in blue and green, respectively, consensus and non-consensus asparaginal ("N") glycosylation sites as well as glutamine ("Q") residues that are glycosylation sites in the Fab region of certain therapeutic mAbs. (*See, e.g.,* Valliere-Douglass et al., 2009, J. Biol. Chem. 284: 32493-32506, and Valliere-Douglass etal., 2010, J. Biol. Chem. 285: 16012-16022, each of which is incorporated by reference in its entirety for the identification of N-linked glycosylation sites in antibodies). In addition, O-glycosylation comprises the addition of N-acetyl-galactosamine to serine or threonine residues by the enzyme. It has been demonstrated that amino acid residues present in the hinge region of antibodies can be O-glycosylated. The possibility of O-glycosylation confers another advantage to the therapeutic antibodies provided herein, as compared to, *e.g.,* antigen-binding fragments produced in *E*. *coli,* again because the *E. coli* naturally does not contain machinery equivalent to that used in human O-glycosylation. (Instead, O-glycosylation in *E. coli* has been demonstrated only when the bacteria is modified to contain specific O-glycosylation machinery. See, e.g., Farid-Moayer et al., 2007, J. Bacteriol. 189:8088-8098.) Moreover, the Fab amino acid sequence may be modified to engineer hyperglycosylated variants (*e.g*., see amino acid substitutions that can be made to engineer hyperglycosylated Fab regions of therapeutic antibodies shown in FIGS. 11A and 11B; and Courtois et al., 2016, mAbs 8: 99-112 which is incorporated by reference herein in its entirety for it description of derivatives of antibodies that are hyperglycosylated on the Fab domain of the full-length antibody).
(iii) In addition to the glycosylation sites, the Fab regions can contain tyrosine ("Y") sulfation sites in or near the CDRs; see FIGS. 2A-2F, 3A-3E, 4A-4B, 5A-5D, 6, 7A-7B, 8A-8H and 9A-9B which identify tyrosine-O-sulfation sites in the Fab region of certain therapeutic mAbs, as highlighted in yellow. (*See, e.g.,* Yang et al., 2015, Molecules 20:2138-2164 (particularly at 2154), which is incorporated by reference in its entirety for the analysis of amino acids surrounding tyrosine residues subjected to protein tyrosine sulfation). The "rules" can be summarized as follows: Y residues with E or D within +5 to -5 position of Y, and where position -1 of Y is a neutral or acidic charged amino acid - but not a basic amino acid, *e.g.,* R, K, or H that abolishes sulfation.
(iv) The glycosylation of Fab regions, such as those shown in FIGS. 2A-2F, 3A-3E, 4A-4B, 5A-5D, 6, 7A-7B, 8A-8H and 9A-9B by human cells will result in the addition of glycans that can improve stability, half-life and reduce unwanted aggregation and/or immunogenicity of the transgene product. (*See, e.g.,* Bovenkamp et al., 2016, J. Immunol. 196: 1435-1441 for a review of the emerging importance of Fab glycosylation; and FIG. 10 which identifies glycans that can be attached to HuGlyFab (adapted from Bondt et al., 2014, Mol & Cell Proteomics 13.1: 3029-2029)). The Fab and Fc portions of antibodies have been shown to have distinct glycosylation patterns, with Fab glycans being high in galactosylation, sialylation, and bisection (*e.g*., with bisecting GlcNAc) but low in fucosylation with respect to Fc glycans. (*E.g*., see Bondt et al., 2014, Mol. & Cell. Proteomics 13.11:3029-3039, incorporated by reference herein in its entirety for its disclosure of Fab-associated N-glycans).
(v) Significantly, glycans that are added to HuGlyFab of the invention are highly processed complex-type N-glycans that contain 2,6-sialic acid. Such glycans are not present in (a) therapeutic mAbs produced in *E. coli* (which are not glycosylated at all); (b) in therapeutic antibodies produced in CHO cells that do not have the 2,6-sialyltransferase required to add 2,6-sialic acid during glycosylation; or (c) in therapeutic antibodies produced in either CHO or murine cell lines that add N-Glycolylneuraminic acid ("Neu5Gc" or "NeuGc") which is not natural to humans (and potentially immunogenic), instead of N-Acetylneuraminic acid ("Neu5Ac") the predominant human sialic acid. *See, e.g.,* Dumont et al., 2015, Crit. Rev. Biotechnol. 36(6):1110-1122; Huang et al., 2006, Anal. Biochem. 349:197-207 (NeuGc is the predominant sialic acid in murine cell lines such as SP2/0 and NS0); and Song et al., 2014, Anal. Chem. 86:5661-5666, each of which is incorporated by reference herein in its entirety.
(vi) The human glycosylation pattern of the HuGlyFab of the invention should reduce immunogenicity of the transgene product and improve efficacy. Importantly, when the antigen-binding fragments, used in accordance with the methods described herein are expressed in human target cells, the need for *in vitro* production in prokaryotic host cells (*e.g., E. coli*) or eukaryotic host cells (*e.g.,* CHO cells or murine NS0 or SP2/0 cells) is circumvented. Instead, as a result of the methods described herein (*e.g*., use of human target cells to express the antigen-binding fragments), N-glycosylation sites of the antigen-binding fragments are advantageously decorated with glycans relevant to and beneficial to treatment of humans. Such an advantage is unattainable when CHO cells, murine cells, or *E. coli* are utilized in antibody/antigen-binding fragment production, because, *e.g.,* (a) CHO cells lack components needed for addition of certain glycans (*e.g*., 2,6 sialic acid and bisecting GlcNAc); (b) CHO cells and murine cells (NS0 and SP2/0 cells) add Neu5Gc as sialic acid not typical to humans instead of Neu5Ac; (c) CHO cells can also produce an immunogenic glycan, the α-Gal antigen, which reacts with anti-α-Gal antibodies present in most individuals, which at high concentrations can trigger anaphylaxis (*see, e.g.,* Bosques, 2010, Nat Biotech 28:1153-1156); and (d) *E. coli* does not naturally contain components needed for N-glycosylation.
(vii) Tyrosine-sulfation of Fab regions, such as those shown in FIGS. 2A-2F, 3A-3E, 4A-4B, 5A-5D, 6, 7A-7B, 8A-8H and 9A-9B - a robust post-translational process in many human cells - should result in transgene products with increased avidity for their molecular targets. Indeed, tyrosine-sulfation of the Fab of antibodies has been shown to dramatically increase avidity for antigen and activity. (*See, e.g.,* Loos et al., 2015, PNAS 112: 12675-12680, and Choe et al., 2003, Cell 114: 161-170). Such post-translational modifications are not present on therapeutic antibodies made in *E. coli* (a host that does not possess the enzymes required for tyrosine-sulfation), and at best are under-represented in therapeutic mAbs made in CHO cells. CHO cells are not secretory cells and have a limited capacity for post-translational tyrosine-sulfation. (*See, e.g.,* Mikkelsen & Ezban, 1991, Biochemistry 30: 1533-1537, especially discussion at p. 1537).

For the foregoing reasons, the production of HuPTM mAb or HuPTM Fab should result in a "biobetter" molecule for the treatment of disease accomplished via gene therapy - *e.g.,* by administering a viral vector or other DNA expression construct encoding a full-length or HuPTM Fab of a therapeutic mAb to a patient (human subject) diagnosed with a disease indication for that mAb, to create a permanent depot in the subject that continuously supplies the human-glycosylated, sulfated transgene product produced by the subject's transduced cells. The cDNA construct for the HuPTMmAb or HuPTM Fab should include a signal peptide that ensures proper co- and post-translational processing (glycosylation and protein sulfation) by the transduced human cells.

As an alternative, or an additional treatment to gene therapy, the full-length or HuPTM Fab can be produced in human cell lines by recombinant DNA technology, and the glycoprotein can be administered to patients.

Combination therapies involving delivery of the full-length or HuPTM Fab to the patient accompanied by administration of other available treatments are encompassed by the methods of the invention. The additional treatments may be administered before, concurrently or subsequent to the gene therapy treatment. Such additional treatments can include but are not limited to co-therapy with the therapeutic mAb.

Also provided are methods of manufacturing the viral vectors, particularly the AAV based viral vectors. In specific embodiments, provided are methods of producing recombinant AAVs comprising culturing a host cell containing an artificial genome comprising a cis expression cassette flanked by AAV ITRs, wherein the cis expression cassette comprises a transgene encoding a therapeutic antibody operably linked to expression control elements that will control expression of the transgene in human cells; a trans expression cassette lacking AAV ITRs, wherein the trans expression cassette encodes an AAV rep and capsid protein operably linked to expression control elements that drive expression of the AAV rep and capsid proteins in the host cell in culture and supply the rep and cap proteins in trans; sufficient adenovirus helper functions to permit replication and packaging of the artificial genome by the AAV capsid proteins; and recovering recombinant AAV encapsidating the artificial genome from the cell culture.

### 3.1 ILLUSTRATIVE EMBODIMENTS

### Compositions of Matter

1. A pharmaceutical composition for treating Alzheimer's disease, migraines, cluster headaches, or tauopathies including chronic traumatic encephalopathy, progressive supranuclear palsy, and frontotemporal dementia in a human subject in need thereof, comprising an adeno-associated virus (AAV) vector having:
   (a) a viral capsid that is at least 95% identical to the amino acid sequence of an AAV9 capsid (SEQ ID NO: 79) or AAVrh10 capsid (SEQ ID NO: 80); and
   (b) an artificial genome comprising an expression cassette flanked by AAV inverted terminal repeats (ITRs), wherein the expression cassette comprises a transgene encoding an anti-amyloid beta, anti-Tau, or anti-CGRPR mAb, or an antigen-binding fragment thereof, operably linked to one or more regulatory sequences that control expression of the transgene in human CNS cells;
   wherein said AAV vector is formulated for intrathecal administration to the CNS of said subject.
2. The pharmaceutical composition of paragraph 1, wherein the anti-amyloid β mAb is aducanumab, crenezumab, gantenerumab, or BAN2401 and the anti-Tau mAb is aTAU and the anti-CGRPR is erenumab, eptinezumab, fremanezumab, or galcanezumab.
3. The pharmaceutical composition of paragraphs 1 or 2, wherein the antigen-binding fragment is a Fab, a F(ab')₂, or a single chain variable domain (scFv).
4. The pharmaceutical composition of any of paragraphs 1 to 3, wherein the antigen-binding fragment comprises a heavy chain with an amino acid sequence of SEQ ID NO: 1 and a light chain with an amino acid sequence of SEQ ID NO:2; or a heavy chain with an amino acid sequence of SEQ ID NO: 3 and a light chain with an amino acid sequence of SEQ ID NO: 4; or a heavy chain with an amino acid sequence of SEQ ID NO: 5 and a light chain with an amino acid sequence of SEQ ID NO:6; or a heavy chain with an amino acid sequence of SEQ ID NO: 53 and a light chain with an amino acid sequence of SEQ ID NO:54; a heavy chain with an amino acid sequence of SEQ ID NO: 55 and a light chain with an amino acid sequence of SEQ ID NO:56; or a heavy chain with an amino acid sequence of SEQ ID NO: 57 and a light chain with an amino acid sequence of SEQ ID NO:58.
5. The pharmaceutical composition of paragraph 4, wherein the transgene comprises a nucleotide sequence of SEQ ID NO: 101 encoding the heavy chain and a nucleotide sequence of SEQ ID NO: 102 encoding the light chain; or a nucleotide sequence of SEQ ID NO: 103 encoding the heavy chain and a nucleotide sequence of SEQ ID NO: 104 encoding the light chain; or a nucleotide sequence of SEQ ID NO: 105 encoding the heavy chain and a nucleotide sequence of SEQ ID NO: 106 encoding the light chain; or a heavy chain with an nucleotide sequence of SEQ ID NO: 153 and a light chain with an nucleotide sequence of SEQ ID NO:154; a heavy chain with an nucleotide sequence of SEQ ID NO: 155 and a light chain with an nucleotide sequence of SEQ ID NO: 156; or a heavy chain with an nucleotide sequence of SEQ ID NO: 157 and a light chain with an nucleotide sequence of SEQ ID NO:158.
6. The pharmaceutical composition of any of paragraphs 1 to 4, wherein the antibody or antigen-binding fragment thereof is a hyperglycosylated mutant.
7. The pharmaceutical composition of any of paragraphs 1 to 6, wherein the transgene encodes a signal sequence at the N-terminus of the heavy chain and the light chain of said antigen-binding fragment that directs secretion and post translational modification in said human CNS cells.
8. The pharmaceutical composition of paragraph 7, wherein said signal sequence is MYRMQLLLLIALSLALVTNS (SEQ ID NO: 161) or a signal sequence from Table 1.
9. The pharmaceutical composition of any of paragraphs 1 to 8, wherein the AAV capsid is AAV9.
10. A pharmaceutical composition for treating atopic dermatitis in a human subject in need thereof, comprising an AAV vector comprising:
   (a) a viral capsid that is at least 95% identical to the amino acid sequence of an AAV8 capsid (SEQ ID NO: 78) or AAV9 capsid (SEQ ID NO: 79); and
   (b) an artificial genome comprising an expression cassette flanked by AAV ITRs wherein the expression cassette comprises a transgene encoding an anti-IL4R mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
11. The pharmaceutical composition of paragraph 10 wherein the anti-IL4R mAb is dupilumab.
12. The pharmaceutical composition of paragraphs 10 or 11, wherein the antigen-binding fragment is a Fab, a F(ab')₂, or an scFv.
13. The pharmaceutical composition of any of paragraphs 10 to 12, wherein the antigen-binding fragment comprises a heavy chain with an amino acid sequence of SEQ ID NO: 7 and a light chain with an amino acid sequence of SEQ ID NO:8.
14. The pharmaceutical composition of paragraph 13, wherein the transgene comprises a nucleotide sequence of SEQ ID NO: 107 encoding the heavy chain and a nucleotide sequence of SEQ ID NO: 108 encoding the light chain.
15. The pharmaceutical composition of any of paragraphs 10 to 13, wherein the antibody or antigen-binding fragment thereof is a hyperglycosylated mutant.
16. The pharmaceutical composition of any of paragraphs 10 to 15, wherein the transgene encodes a signal sequence at the N-terminus of the heavy chain and the light chain of said antigen-binding fragment that directs secretion and post translational modification in said human liver cells or human muscle cells.
17. The pharmaceutical composition of paragraph 16, wherein said signal sequence is selected from the signal sequences in Table 2 or 3.
18. The pharmaceutical composition of any of paragraphs 10 to 17, wherein the AAV capsid is AAV8.
19. A pharmaceutical composition for treating psoriasis, psoriatic arthritis, ankylosing spondylitis, or Crohn's disease in a human subject in need thereof, comprising an AAV vector comprising:
   (a) a viral capsid that is at least 95% identical to the amino acid sequence of an AAV8 capsid (SEQ ID NO: 78) or an AAV9 capsid (SEQ ID NO: 79); and
   (b) an artificial genome comprising an expression cassette flanked by AAV ITRs wherein the expression cassette comprises a transgene encoding an anti-IL17A mAb or anti-IL12/IL23 mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
20. The pharmaceutical composition of paragraph 19 wherein the anti-IL17A or anti-IL12/IL23 mAb is ixekizumab, secukinumab or ustekinumab.
21. The pharmaceutical composition of paragraphs 19 or 20, wherein the antigen-binding fragment is a Fab, a F(ab')₂, or an scFv.
22. The pharmaceutical composition of any of paragraphs 19 to 21, wherein the antigen-binding fragment comprises a heavy chain with an amino acid sequence of SEQ ID NO: 9 and a light chain with an amino acid sequence of SEQ ID NO: 10; or a heavy chain with an amino acid sequence of SEQ ID NO: 11 and a light chain with an amino acid sequence of SEQ ID NO: 12; or a heavy chain with an amino acid sequence of SEQ ID NO: 13 and a light chain with an amino acid sequence of SEQ ID NO:14.
23. The pharmaceutical composition of paragraph 22, wherein the transgene comprises a nucleotide sequence of SEQ ID NO: 109 encoding the heavy chain and a nucleotide sequence of SEQ ID NO: 110 encoding the light chain; or a nucleotide sequence of SEQ ID NO: 111 encoding the heavy chain and a nucleotide sequence of SEQ ID NO: 112 encoding the light chain; or a nucleotide sequence of SEQ ID NO: 113 encoding the heavy chain and a nucleotide sequence of SEQ ID NO: 114 encoding the light chain.
24. The pharmaceutical composition of any of paragraphs 19 to 22, wherein the antibody or antigen-binding fragment thereof is a hyperglycosylated mutant.
25. The pharmaceutical composition of any of paragraphs 19 to 24, wherein the transgene encodes a signal sequence at the N-terminus of the heavy chain and the light chain of said antigen-binding fragment that directs secretion and post translational modification in said human liver cells or human muscle cells.
26. The pharmaceutical composition of paragraph 25, wherein said signal sequence is selected from the signal sequences in Table 2 or 3.
27. The pharmaceutical composition of any of paragraphs 19 to 26, wherein the AAV capsid is AAV8.
28. A pharmaceutical composition for treating asthma in a human subject in need thereof, comprising an AAV vector comprising:
   (a) a viral capsid that is at least 95% identical to the amino acid sequence of an AAV8 capsid (SEQ ID NO: 78) or an AAV9 capsid (SEQ ID NO: 79); and
   (b) an artificial genome comprising an expression cassette flanked by AAV ITRs wherein the expression cassette comprises a transgene encoding an anti-IL-5 mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
29. The pharmaceutical composition of paragraph 28 wherein the anti-IL-5 mAb is mepolizumab.
30. The pharmaceutical composition of paragraphs 28 or 29, wherein the antigen-binding fragment is a Fab, a F(ab')₂, or an scFv.
31. The pharmaceutical composition of any of paragraphs 28 to 30, wherein the antigen-binding fragment comprises a heavy chain with an amino acid sequence of SEQ ID NO: 15 and a light chain with an amino acid sequence of SEQ ID NO: 16.
32. The pharmaceutical composition of paragraph 31, wherein the transgene comprises a nucleotide sequence of SEQ ID NO: 115 encoding the heavy chain and a nucleotide sequence of SEQ ID NO: 116 encoding the light chain.
33. The pharmaceutical composition of any of paragraphs 28 to 31, wherein the antibody or antigen-binding fragment thereof is a hyperglycosylated mutant.
34. The pharmaceutical composition of any of paragraphs 28 to 33, wherein the transgene encodes a signal sequence at the N-terminus of the heavy chain and the light chain of said antigen-binding fragment that directs secretion and post translational modification in said human liver cells or human muscle cells.
35. The pharmaceutical composition of paragraph 34, wherein said signal sequence is selected from the signal sequences in Table 2 or 3.
36. The pharmaceutical composition of any of paragraphs 28 to 35, wherein the AAV capsid is AAV8.
37. A pharmaceutical composition for treating multiple sclerosis, ulcerative colitis or Crohn's disease in a human subject in need thereof, comprising an AAV vector comprising:
   (a) a viral capsid that is at least 95% identical to the amino acid sequence of an AAV8 capsid (SEQ ID NO: 78), an AAV9 capsid (SEQ ID NO: 79), or an AAVrh10 capsid (SEQ ID NO: 80); and
   (b) an artificial genome comprising an expression cassette flanked by AAV ITRs wherein the expression cassette comprises a transgene encoding an anti-integrin mAb, or an antigen-binding fragment thereof, operably linked to one or more regulatory sequences that control expression of the transgene in human liver cells or human muscle cells or human CNS cells;
   wherein said AAV vector is formulated for intravenous administration to the liver or muscle of said subject or for the intrathecal administration to the CNS of said subject.
38. The pharmaceutical composition of paragraph 37, wherein the anti-integrin mAb is vedolizumab or natalizumab.
39. The pharmaceutical composition of paragraphs 37 or 38, wherein the antigen-binding fragment is a Fab, a F(ab')₂, or an scFv.
40. The pharmaceutical composition of any of paragraphs 37 to 39, wherein the antigen-binding fragment comprises a heavy chain with an amino acid sequence of SEQ ID NO: 17 and a light chain with an amino acid sequence of SEQ ID NO: 18; or a heavy chain with an amino acid sequence of SEQ ID NO: 19 and a light chain with an amino acid sequence of SEQ ID NO:20.
41. The pharmaceutical composition of paragraph 40, wherein the transgene comprises a nucleotide sequence of SEQ ID NO: 117 encoding the heavy chain and a nucleotide sequence of SEQ ID NO: 118 encoding the light chain; or a nucleotide sequence of SEQ ID NO: 119 encoding the heavy chain and a nucleotide sequence of SEQ ID NO: 120 encoding the light chain.
42. The pharmaceutical composition of any of paragraphs 37 to 41, wherein the antibody or antigen-binding fragment thereof is a hyperglycosylated mutant.
43. The pharmaceutical composition of any of paragraphs 37 to 42, wherein the transgene encodes a signal sequence at the N-terminus of the heavy chain and the light chain of said antigen-binding fragment that directs secretion and post translational modification in said human liver cells or human muscle cells.
44. The pharmaceutical composition of paragraph 43, wherein said signal sequence is selected from the signal sequences in Table 1, 2 or 3.
45. The pharmaceutical composition of any of paragraphs 37 to 44, wherein the AAV capsid is AAV8.
46. A pharmaceutical composition for treating HeFH, HoFH, dyslipidemia, cardiovascular disease including atherosclerotic cardiovascular disease (ACD), atherosclerotic plaque formation, abnormally high levels of non-HDL cholesterol and LDL, aortic stenosis, hepatic stenosis, or hypercholesterolemia in a human subject in need thereof, comprising an AAV vector comprising:
   (a) a viral capsid that is at least 95% identical to the amino acid sequence of an AAV8 capsid (SEQ ID NO: 78) or an AAV9 capsid (SEQ ID NO: 79); and
   (b) an artificial genome comprising an expression cassette flanked by AAV ITRs wherein the expression cassette comprises a transgene encoding an anti-PCSK9, anti-ANGPTL3, or anti-OxPL mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
47. The pharmaceutical composition of paragraph 46, wherein the anti-PCSK9 or anti-ANGPTL3 mAb is alirocumab, evolocumab or evinacumab or the anti-OxPL is E06.
48. The pharmaceutical composition of paragraphs 46 or 47, wherein the antigen-binding fragment is a Fab, a F(ab')₂, or an scFv.
49. The pharmaceutical composition of any of paragraphs 46 to 48, wherein the antigen-binding fragment comprises a heavy chain with an amino acid sequence of SEQ ID NO: 21 and a light chain with an amino acid sequence of SEQ ID NO: 22; or a heavy chain with an amino acid sequence of SEQ ID NO: 23 and a light chain with an amino acid sequence of SEQ ID NO:24; a heavy chain with an amino acid sequence of SEQ ID NO: 25 and a light chain with an amino acid sequence of SEQ ID NO:26; or a heavy chain with an amino acid sequence of SEQ ID NO: 59 and a light chain with an amino acid sequence of SEQ ID NO:60.
50. The pharmaceutical composition of paragraph 49, wherein the transgene comprises a nucleotide sequence of SEQ ID NO: 121 encoding the heavy chain and a nucleotide sequence of SEQ ID NO: 122 encoding the light chain; or a nucleotide sequence of SEQ ID NO: 123 encoding the heavy chain and a nucleotide sequence of SEQ ID NO: 124 encoding the light chain; a nucleotide sequence of SEQ ID NO: 125 encoding the heavy chain and a nucleotide sequence of SEQ ID NO: 126 encoding the light chain; or a nucleotide sequence of SEQ ID NO: 159 encoding the heavy chain and a nucleotide sequence of SEQ ID NO: 160 encoding the light chain .
51. The pharmaceutical composition of any of paragraphs 44 to 50, wherein the antibody or antigen-binding fragment thereof is a hyperglycosylated mutant.
52. The pharmaceutical composition of any of paragraphs 44 to 51, wherein the transgene encodes a signal sequence at the N-terminus of the heavy chain and the light chain of said antigen-binding fragment that directs secretion and post translational modification in said human liver cells or human muscle cells.
53. The pharmaceutical composition of paragraph 52, wherein said signal sequence is selected from the signal sequences in Table 2 or 3.
54. The pharmaceutical composition of any of paragraphs 44 to 53, wherein the AAV capsid is AAV8.
55. A pharmaceutical composition for treating osteoporosis in a human subject in need thereof, comprising an AAV vector comprising:
   (a) a viral capsid that is at least 95% identical to the amino acid sequence of an AAV8 capsid (SEQ ID NO: 78) or AAV9 capsid (SEQ ID NO: 79); and
   (b) an artificial genome comprising an expression cassette flanked by AAV ITRs wherein the expression cassette comprises a transgene encoding an anti-RANKL mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
56. The pharmaceutical composition of paragraph 55, wherein the anti-RANLK mAb is denosumab.
57. The pharmaceutical composition of paragraphs 55 or 56, wherein the antigen-binding fragment is a Fab, a F(ab')₂, or an scFv.
58. The pharmaceutical composition of any of paragraphs 55 to 57, wherein the antigen-binding fragment comprises a heavy chain with an amino acid sequence of SEQ ID NO: 27 and a light chain with an amino acid sequence of SEQ ID NO:28.
59. The pharmaceutical composition of paragraph 58, wherein the transgene comprises a nucleotide sequence of SEQ ID NO: 127 encoding the heavy chain and a nucleotide sequence of SEQ ID NO: 128 encoding the light chain.
60. The pharmaceutical composition of any of paragraphs 55 to 59, wherein the antibody or antigen-binding fragment thereof is a hyperglycosylated mutant.
61. The pharmaceutical composition of any of paragraphs 55 to 60, wherein the transgene encodes a signal sequence at the N-terminus of the heavy chain and the light chain of said antigen-binding fragment that directs secretion and post translational modification in said human liver cells or human muscle cells.
62. The pharmaceutical composition of paragraph 61, wherein said signal sequence is selected from the signal sequences in Table 2 or 3.
63. The pharmaceutical composition of any of paragraphs 55 to 62, wherein the AAV capsid is AAV8.
64. A pharmaceutical composition for treating metastatic melanoma, lymphoma or non-small cell lung carcinoma in a human subject in need thereof, comprising an AAV vector comprising:
   (a) a viral capsid that is at least 95% identical to the amino acid sequence of an AAV8 capsid (SEQ ID NO: 78) or AAV9 capsid (SEQ ID NO: 79); and
   (b) an artificial genome comprising an expression cassette flanked by AAV ITRs wherein the expression cassette comprises a transgene encoding a PD-1 blocker mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
65. The pharmaceutical composition of paragraph 64, wherein the PD-1 blocker mAb is nivolumab or pembrolizumab.
66. The pharmaceutical composition of paragraphs 64 or 65, wherein the antigen-binding fragment is a Fab, a F(ab')₂, or an scFv.
67. The pharmaceutical composition of any of paragraphs 64 to 66, wherein the antigen-binding fragment comprises a heavy chain with an amino acid sequence of SEQ ID NO: 29 and a light chain with an amino acid sequence of SEQ ID NO: 30; or a heavy chain with an amino acid sequence of SEQ ID NO: 31 and a light chain with an amino acid sequence of SEQ ID NO: 32.
68. The pharmaceutical composition of paragraph 67, wherein the transgene comprises a nucleotide sequence of SEQ ID NO: 129 encoding the heavy chain and a nucleotide sequence of SEQ ID NO: 130 encoding the light chain; or a nucleotide sequence of SEQ ID NO: 131 encoding the heavy chain and a nucleotide sequence of SEQ ID NO: 132 encoding the light chain.
69. The pharmaceutical composition of any of paragraphs 64 to 68, wherein the antibody or antigen-binding fragment thereof is a hyperglycosylated mutant.
70. The pharmaceutical composition of any of paragraphs 64 to 69, wherein the transgene encodes a signal sequence at the N-terminus of the heavy chain and the light chain of said antigen-binding fragment that directs secretion and post translational modification in said human liver cells or human muscle cells.
71. The pharmaceutical composition of paragraph 70, wherein said signal sequence is selected from the signal sequences in Table 2 or 3.
72. The pharmaceutical composition of any of paragraphs 64 to 71, wherein the AAV capsid is AAV8.
73. A pharmaceutical composition for treating systemic lupus erythromatosis (SLE) in a human subject in need thereof, comprising an AAV vector comprising:
   (a) a viral capsid that is at least 95% identical to the amino acid sequence of an AAV8 capsid (SEQ ID NO: 78) or an AAV9 capsid (SEQ ID NO: 79); and
   (b) an artificial genome comprising an expression cassette flanked by AAV ITRs wherein the expression cassette comprises a transgene encoding an anti-BLyS mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
74. The pharmaceutical composition of paragraph 73, wherein the anti-BLyS mAb is belimumab.
75. The pharmaceutical composition of paragraphs 73 or 74, wherein the antigen-binding fragment is a Fab, a F(ab')₂, or an scFv.
76. The pharmaceutical composition of any of paragraphs 73 to 75, wherein the antigen-binding fragment comprises a heavy chain with an amino acid sequence of SEQ ID NO: 41 and a light chain with an amino acid sequence of SEQ ID NO:42.
77. The pharmaceutical composition of paragraph 76, wherein the transgene comprises a nucleotide sequence of SEQ ID NO: 141 encoding the heavy chain and a nucleotide sequence of SEQ ID NO: 142 encoding the light chain.
78. The pharmaceutical composition of any of paragraphs 73 to 77, wherein the antibody or antigen-binding fragment thereof is a hyperglycosylated mutant.
79. The pharmaceutical composition of any of paragraphs 73 to 78, wherein the transgene encodes a signal sequence at the N-terminus of the heavy chain and the light chain of said antigen-binding fragment that directs secretion and post translational modification in said human liver cells or human muscle cells.
80. The pharmaceutical composition of paragraph 79, wherein said signal sequence is selected from the signal sequences in Table 2 or 3.
81. The pharmaceutical composition of any of paragraphs 73 to 80, wherein the AAV capsid is AAV8.
82. A pharmaceutical composition for treating ocular disorders, including age-related macular degeneration, in a human subject in need thereof, comprising an AAV vector comprising:
   (a) a viral capsid that is at least 95% identical to the amino acid sequence of an AAV8 capsid (SEQ ID NO: 78) or AAV9 capsid (SEQ ID NO: 79); and
   (b) an artificial genome comprising an expression cassette flanked by AAV ITRs wherein the expression cassette comprises a transgene encoding an anti-MMP9, anti-VEGF or anti-fD mAb, or an antigen-binding fragment thereof, operably linked to one or more regulatory sequences that control expression of the transgene in human retinal cells;
   wherein said AAV vector is formulated for subretinal, intravitreal or suprachoroidal administration to the eye of said subject.
83. The pharmaceutical composition of paragraph 82, wherein the anti-VEGF mAb is ranibizumab, bevacizumab, or brolucizumab, said anti-Fd mAb is lampalizumab or said anti-MMP9 mAb is andecaliximab.
84. The pharmaceutical composition of paragraphs 82 or 83, wherein the antigen-binding fragment is a Fab, a F(ab')₂, or an scFv.
85. The pharmaceutical composition of any of paragraphs 82 to 84, wherein the antigen-binding fragment comprises a heavy chain with an amino acid sequence of SEQ ID NO: 33 and a light chain with an amino acid sequence of SEQ ID NO:34, or a heavy chain with an amino acid sequence of SEQ ID NO: 35 and a light chain with an amino acid sequence of SEQ ID NO:36; or a heavy chain with an amino acid sequence of SEQ ID NO: 37 and a light chain with an amino acid sequence of SEQ ID NO:38; or a heavy chain with an amino acid sequence of SEQ ID NO: 39 and a light chain with an amino acid sequence of SEQ ID NO: 40; or a heavy chain with an amino acid sequence of SEQ ID NO: 45 and a light chain with an amino acid sequence of SEQ ID NO:46.
86. The pharmaceutical composition of paragraph 85, wherein the transgene comprises a nucleotide sequence of SEQ ID NO: 133 encoding the heavy chain and a nucleotide sequence of SEQ ID NO: 134 encoding the light chain; or a nucleotide sequence of SEQ ID NO: 135 encoding the heavy chain and a nucleotide sequence of SEQ ID NO: 136 encoding the light chain; or a nucleotide sequence of SEQ ID NO: 137 encoding the heavy chain and a nucleotide sequence of SEQ ID NO: 138 encoding the light chain; or a nucleotide sequence of SEQ ID NO: 139 encoding the heavy chain and a nucleotide sequence of SEQ ID NO: 140 encoding the light chain; or a nucleotide sequence of SEQ ID NO: 145 encoding the heavy chain and a nucleotide sequence of SEQ ID NO: 146 encoding the light chain.
87. The pharmaceutical composition of any of paragraphs 82 to 85, wherein the antibody or antigen-binding fragment thereof is a hyperglycosylated mutant.
88. The pharmaceutical composition of any of paragraphs 82 to 87, wherein the transgene encodes a signal sequence at the N-terminus of the heavy chain and the light chain of said antigen-binding fragment that directs secretion and post translational modification in said human retinal cells.
89. The pharmaceutical composition of paragraph 88, wherein said signal sequence is selected from the signal sequences in Table 1.
90. The pharmaceutical composition of any of paragraphs 82 to 89, wherein the AAV capsid is AAV8.
91. A pharmaceutical composition for treating rheumatoid arthritis, psoriatic arthritis, ankylosing spondylitis, Crohn's disease, plaque psoriasis, or ulcerative colitis, in a human subject in need thereof, comprising an AAV vector comprising:
   (a) a viral capsid that is at least 95% identical to the amino acid sequence of an AAV8 capsid (SEQ ID NO: 78) or AAV9 capsid (SEQ ID NO:79); and
   (b) an artificial genome comprising an expression cassette flanked by AAV ITRs wherein the expression cassette comprises a transgene encoding an anti-TNF antibody, or an antigen-binding fragment thereof, operably linked to one or more regulatory sequences that control expression of the transgene in human liver cells or human muscle cells;
   wherein said AAV vector is formulated for intravenous administration to said subject.
92. The pharmaceutical composition of paragraph 91, wherein the anti-TNF-alpha mAb is adalimumab or infliximab.
93. The pharmaceutical composition of paragraphs 91 or 92, wherein the antigen-binding fragment is a Fab, a F(ab')₂, or an scFv.
94. The pharmaceutical composition of any of paragraphs 91 to 93, wherein the antigen-binding fragment comprises a heavy chain with an amino acid sequence of SEQ ID NO: 49 and a light chain with an amino acid sequence of SEQ ID NO: 50; or a heavy chain with an amino acid sequence of SEQ ID NO: 51 and a light chain with an amino acid sequence of SEQ ID NO: 52.
95. The pharmaceutical composition of paragraph 94, wherein the transgene comprises a nucleotide sequence of SEQ ID NO: 149 encoding the heavy chain and a nucleotide sequence of SEQ ID NO: 150 encoding the light chain; or a nucleotide sequence of SEQ ID NO: 151 encoding the heavy chain and a nucleotide sequence of SEQ ID NO: 152 encoding the light chain.
96. The pharmaceutical composition of any of paragraphs 91 to 94, wherein the antibody or antigen-binding fragment thereof is a hyperglycosylated mutant.
97. The pharmaceutical composition of any of paragraphs 91 to 96, wherein the transgene encodes a signal sequence at the N-terminus of the heavy chain and the light chain of said antigen-binding fragment that directs secretion and post translational modification in said human liver cells or human muscle cells.
98. The pharmaceutical composition of paragraph 97, wherein said signal sequence is selected from the signal sequences in Table 2 or 3.
99. The pharmaceutical composition of any of paragraphs 91 to 98, wherein the AAV capsid is AAV8.
100. A pharmaceutical composition for treating paroxysmal nocturnal hemoglobinuria (PNH) or atypical hemolytic uremic syndrome (aHUS), in a human subject in need thereof, comprising an AAV vector comprising:
   (a) a viral capsid that is at least 95% identical to the amino acid sequence of an AAV8 capsid (SEQ ID NO: 78) or AAV9 capsid (SEQ ID NO: 79); and
   (b) an artificial genome comprising an expression cassette flanked by AAV ITRs wherein the expression cassette comprises a transgene encoding an anti-C5 or C5a complement protein mAb, or an antigen-binding fragment thereof, operably linked to one or more regulatory sequences that control expression of the transgene in human liver cells;
   wherein said AAV vector is formulated for intravenous administration to said subject.
101. The pharmaceutical composition of paragraph 100, wherein the anti-C5 or C5a complement protein mAb is eculizumab.
102. The pharmaceutical composition of paragraphs 100 or 101, wherein the antigen-binding fragment is a Fab, a F(ab')₂, or an scFv.
103. The pharmaceutical composition of any of paragraphs 100 to 102, wherein the antigen-binding fragment comprises a heavy chain with an amino acid sequence of SEQ ID NO: 43 and a light chain with an amino acid sequence of SEQ ID NO: 44.
104. The pharmaceutical composition of paragraph 103, wherein the transgene comprises a nucleotide sequence of SEQ ID NO: 143 encoding the heavy chain and a nucleotide sequence of SEQ ID NO: 144 encoding the light chain.
105. The pharmaceutical composition of any of paragraphs 101 to 104, wherein the antibody or antigen-binding fragment thereof is a hyperglycosylated mutant.
106. The pharmaceutical composition of any of paragraphs 100 to 105, wherein the transgene encodes a signal sequence at the N-terminus of the heavy chain and the light chain of said antigen-binding fragment that directs secretion and post translational modification in said human liver cells.
107. The pharmaceutical composition of paragraph 106, wherein said signal sequence is selected from the signal sequences in Table 3.
108. The pharmaceutical composition of any of paragraphs 101 to 107, wherein the AAV capsid is AAV8.
109. A pharmaceutical composition for treating hereditary angiodema, in a human subject in need thereof, comprising an AAV vector comprising:
   (a) a viral capsid that is at least 95% identical to the amino acid sequence of an AAV8 capsid (SEQ ID NO: 78) or AAV9 capsid (SEQ ID NO: 79); and
   (b) an artificial genome comprising an expression cassette flanked by AAV ITRs wherein the expression cassette comprises a transgene encoding an anti-plasma kallikrein mAb, or an antigen-binding fragment thereof, operably linked to one or more regulatory sequences that control expression of the transgene in human liver cells or human muscle cells;
   wherein said AAV vector is formulated for intravenous administration to said subject.
110. The pharmaceutical composition of paragraph 109, wherein the anti-plasma kallikrein mAb is lanadelumab.
111. The pharmaceutical composition of paragraphs 109 or 111, wherein the antigen-binding fragment is a Fab, a F(ab')₂, or an scFv.
112. The pharmaceutical composition of any of paragraphs 109 to 111, wherein the antigen-binding fragment comprises a heavy chain with an amino acid sequence of SEQ ID NO: 47 and a light chain with an amino acid sequence of SEQ ID NO: 48.
113. The pharmaceutical composition of paragraph 112, wherein the transgene comprises a nucleotide sequence of SEQ ID NO: 147 encoding the heavy chain and a nucleotide sequence of SEQ ID NO: 148 encoding the light chain.
114. The pharmaceutical composition of any of paragraphs 110 to 113, wherein the antibody or antigen-binding fragment thereof is a hyperglycosylated mutant.
115. The pharmaceutical composition of any of paragraphs 109 to 114, wherein the transgene encodes a signal sequence at the N-terminus of the heavy chain and the light chain of said antigen-binding fragment that directs secretion and post translational modification in said human liver cells.
116. The pharmaceutical composition of paragraph 115, wherein said signal sequence is selected from the signal sequences in Table 3.
117. The pharmaceutical composition of any of paragraphs 110 to 116, wherein the AAV capsid is AAV8.

### Method of Treatment

118. A method of treating Alzheimer's disease, migraines, cluster headaches, or tauopathies including chronic traumatic encephalopathy, progressive supranuclear palsy, and frontotemporal dementia in a human subject in need thereof, comprising delivering to the cerebrospinal fluid (CSF) of said human subject, a therapeutically effective amount of an anti-amyloid beta, anti-Tau, or anti-CGRPR mAb or antigen-binding fragment thereof, produced by human central nervous system (CNS) cells.
119. A method of treating Alzheimer's disease, migraines, cluster headaches, or tauopathies including chronic traumatic encephalopathy, progressive supranuclear palsy, and frontotemporal dementia in a human subject in need thereof, comprising:
   administering to the cisterna magna of said subject a therapeutically effective amount of a recombinant nucleotide expression vector comprising a transgene encoding an anti-amyloid beta, anti-Tau, or anti-CGRPR mAb, or an antigen-binding fragment thereof, operably linked to one or more regulatory sequences that control expression of the transgene in human CNS cells, so that a depot is formed that releases a human post-translationally modified (HuPTM) form of said mAb or antigen-binding fragment thereof.
120. The method of paragraphs 118 or 119 wherein the anti-amyloid beta mAb is aducanumab, crenezumab, gantenerumab, or BAN2401 or wherein the anti-Tau mAb is aTAU or wherein the anti-CGRPR is erenumab, eptinezumab, fremanezumab, or galcanezumab.
121. The method of any of paragraphs 118 to 120 wherein the antigen-binding fragment is a Fab, a F(ab')₂, or an scFv.
122. The method of any of paragraphs 118 to 121, wherein the antigen-binding fragment comprises a heavy chain with an amino acid sequence of SEQ ID NO: 1 and a light chain with an amino acid sequence of SEQ ID NO:2; or a heavy chain with an amino acid sequence of SEQ ID NO: 3 and a light chain with an amino acid sequence of SEQ ID NO:4; or a heavy chain with an amino acid sequence of SEQ ID NO: 5 and a light chain with an amino acid sequence of SEQ ID NO:6; or a heavy chain with an amino acid sequence of SEQ ID NO: 53 and a light chain with an amino acid sequence of SEQ ID NO:54; a heavy chain with an amino acid sequence of SEQ ID NO: 55 and a light chain with an amino acid sequence of SEQ ID NO:56; or a heavy chain with an amino acid sequence of SEQ ID NO: 57 and a light chain with an amino acid sequence of SEQ ID NO: 58.
123. The method of claim 122, wherein the transgene comprises a nucleotide sequence of SEQ ID NO: 101 encoding the heavy chain and a nucleotide sequence of SEQ ID NO: 102 encoding the light chain; or a nucleotide sequence of SEQ ID NO: 103 encoding the heavy chain and a nucleotide sequence of SEQ ID NO: 104 encoding the light chain; or a nucleotide sequence of SEQ ID NO: 105 encoding the heavy chain and a nucleotide sequence of SEQ ID NO: 106 encoding the light chain; or a heavy chain with an nucleotide sequence of SEQ ID NO: 153 and a light chain with an nucleotide sequence of SEQ ID NO:154; a heavy chain with an nucleotide sequence of SEQ ID NO: 155 and a light chain with an nucleotide sequence of SEQ ID NO: 156 or a heavy chain with an nucleotide sequence of SEQ ID NO: 157 and a light chain with an nucleotide sequence of SEQ ID NO:158.
124. The method of any of paragraphs 118 to 122, wherein the mAb or antigen-binding fragment thereof is a hyperglycosylated mutant.
125. The method of any of paragraphs 118 to 124 wherein the mAb or antigen-binding fragment thereof contains an alpha 2,6-sialylated glycan.
126. The method of any of paragraphs 118 to 125 wherein the mAb or antigen-binding fragment thereof is glycosylated but does not contain detectable NeuGc and/or α-Gal.
127. The method of any of paragraphs 118 to 126 wherein the mAb or antigen-binding fragment thereof contains a tyrosine sulfation.
128. The method of any of paragraphs 119 to 127 wherein the recombinant expression vector is AAV9 or AAVrh10.
129. The method of any of paragraphs 119 to 128 in which production of said HuPTM form of said mAb or antigen-binding fragment thereof is confirmed by transducing human CNS cells in culture with said recombinant nucleotide expression vector and expressing said mAb or antigen-binding fragment thereof.
130. A method of treating psoriasis, psoriatic arthritis, ankylosing spondylitis, or Crohn's disease in a human subject in need thereof, comprising delivering to the circulation of said human subject, a therapeutically effective amount of an anti-IL17A or anti-IL12/IL23 mAb or antigen-binding fragment thereof, produced by human liver cells or human muscle cells.
131. A method of treating psoriasis, psoriatic arthritis, ankylosing spondylitis, or Crohn's disease in a human subject in need thereof, comprising:
   administering to the liver or muscle of said subject a therapeutically effective amount of a recombinant nucleotide expression vector comprising a transgene encoding an anti-IL17A or anti-IL12/IL23 mAb, or an antigen-binding fragment thereof, operably linked to one or more regulatory sequences that control expression of the transgene in human liver cells or human muscle cells, so that a depot is formed that releases a HuPTM form of said mAb or antigen-binding fragment thereof.
132. The method of paragraph 130 or 131 wherein the anti-IL17A or anti-IL12/IL23 mAb is ixekizumab, secukinumab, or ustekinumab.
133. The method of any of paragraphs 130 to 132 wherein the antigen-binding fragment is a Fab, a F(ab')₂, or an scFv.
134. The method of any of paragraphs 130 to 133, wherein the antigen-binding fragment comprises a heavy chain with an amino acid sequence of SEQ ID NO: 9 and a light chain with an amino acid sequence of SEQ ID NO:10; or a heavy chain with an amino acid sequence of SEQ ID NO: 11 and a light chain with an amino acid sequence of SEQ ID NO: 12; or a heavy chain with an amino acid sequence of SEQ ID NO: 13 and a light chain with an amino acid sequence of SEQ ID NO: 14.
135. The method of claim 134, wherein the transgene comprises a nucleotide sequence of SEQ ID NO: 109 encoding the heavy chain and a nucleotide sequence of SEQ ID NO: 110 encoding the light chain; or a nucleotide sequence of SEQ ID NO: 111 encoding the heavy chain and a nucleotide sequence of SEQ ID NO: 112 encoding the light chain; or a nucleotide sequence of SEQ ID NO: 113 encoding the heavy chain and a nucleotide sequence of SEQ ID NO: 114 encoding the light chain.
136. The method of any of paragraphs 132 to 134, wherein the mAb or antigen-binding fragment thereof is a hyperglycosylated mutant.
137. The method of any of paragraphs 132 to 136 wherein the mAb or antigen-binding fragment thereof contains an alpha 2,6-sialylated glycan.
138. The method of any of paragraphs 132 to 137 wherein the mAb or antigen-binding fragment thereof is glycosylated but does not contain detectable NeuGc or α-Gal.
139. The method of any of paragraphs 132 to 138 wherein the mAb or antigen-binding fragment thereof contains a tyrosine sulfation.
140. The method of any of paragraphs 133 to 139 wherein the recombinant expression vector is AAV8 or AAV9.
141. The method of any of paragraphs 133 to 140 in which production of said HuPTM form of the mAb or antigen-binding fragment thereof is confirmed by transducing human liver cells or muscle cells in culture with said recombinant nucleotide expression vector and expressing said mAb or antigen-binding fragment thereof.
142. A method of treating multiple sclerosis, ulcerative colitis or Crohn's disease in a human subject in need thereof, comprising delivering to the CSF or circulation of said human subject, a therapeutically effective amount of an anti-integrin mAb or antigen-binding fragment thereof, produced by human CNS cells, human liver cells or human muscle cells.
143. A method of treating multiple sclerosis, ulcerative colitis or Crohn's disease in a human subject in need thereof, comprising:
   administering to the CNS, liver or muscle of said human subject a therapeutically effective amount of a recombinant nucleotide expression vector comprising a transgene encoding an anti-integrin mAb, or an antigen-binding fragment thereof, operably linked to one or more regulatory sequences that control expression of the transgene in human CNS cells, human liver cells or in human muscle cells, so that a depot is formed that releases a HuPTM form of said mAb or antigen-binding fragment.
144. The method of paragraphs 142 or 143 wherein the anti-integrin mAb is natalizumab or vedolizumab.
145. The method of any of paragraphs 142 to 144 wherein the antigen-binding fragment is a Fab, a F(ab')₂, or an scFv.
146. The method of any of paragraphs 142 to 145, wherein the antigen-binding fragment comprises a heavy chain with an amino acid sequence of SEQ ID NO: 17 and a light chain with an amino acid sequence of SEQ ID NO:18; or a heavy chain with an amino acid sequence of SEQ ID NO: 19 and a light chain with an amino acid sequence of SEQ ID NO:20.
147. The method of claim 146, wherein the transgene comprises a nucleotide sequence of SEQ ID NO: 117 encoding the heavy chain and a nucleotide sequence of SEQ ID NO: 118 encoding the light chain; or a nucleotide sequence of SEQ ID NO: 119 encoding the heavy chain and a nucleotide sequence of SEQ ID NO: 120 encoding the light chain.
148. The method of any of paragraphs 142 to 145, wherein the antibody or antigen-binding fragment thereof is a hyperglycosylated mutant.
149. The method of any of paragraphs 142 to 148 wherein the mAb or antigen-binding fragment thereof contains an alpha 2,6-sialylated glycan.
150. The method of any of paragraphs 142 to 149 wherein the mAb or antigen-binding fragment thereof is glycosylated but does not contain detectable NeuGc or α-Gal.
151. The method of any of paragraphs 142 to 150 wherein the mAb or antigen-binding fragment thereof contains a tyrosine sulfation.
152. The method of any of paragraphs 143 to 151 wherein the recombinant expression vector is AAV8, AAV9, or AAVrh10.
153. The method of any of paragraphs 143 to 152 in which production of the HuPTM form of the mAb or antigen-binding fragment thereof is confirmed by transducing human CNS cells, human liver cells or muscle cells in culture with said recombinant nucleotide expression vector and expressing said mAb or antigen-binding fragment thereof.
154. A method of treating atopic dermatitis in a human subject in need thereof, comprising delivering to the circulation of said human subject, a therapeutically effective amount of an anti-IL4R mAb or antigen-binding fragment thereof, produced by human liver cells or human muscle cells.
155. A method of treating atopic dermatitis in a human subject in need thereof, comprising:
   administering to the liver or muscle of said human subject, a therapeutically effective amount of a recombinant nucleotide expression vector comprising a transgene encoding an anti-IL4R mAb, or antigen-binding fragment thereof, operably linked to one or more regulatory sequences that control expression of the transgene in human liver cells or human muscle cells, so that a depot is formed that released a HuPTM form of said mAb or antigen-binding fragment thereof.
156. The method of paragraphs 154 or 155 wherein the anti-IL-4R mAb is dupilumab.
157. The method of any of paragraphs 154 to 156 wherein the antigen-binding fragment is a Fab, a F(ab')₂, or an scFv.
158. The method of any of paragraphs 154 to 157, wherein the antigen-binding fragment comprises a heavy chain with an amino acid sequence of SEQ ID NO: 7 and a light chain with an amino acid sequence of SEQ ID NO: 8.
159. The method of claim 158, wherein the transgene comprises a nucleotide sequence of SEQ ID NO: 107 encoding the heavy chain and a nucleotide sequence of SEQ ID NO: 108 encoding the light chain.
160. The method of any of paragraphs 154 to 158, wherein the mAb or antigen-binding fragment thereof is a hyperglycosylated mutant.
161. The method of any of paragraphs 154 to 160 wherein the mAb or antigen-binding fragment thereof contains an alpha 2,6-sialylated glycan.
162. The method of any of paragraphs 154 to 161 wherein the mAb or antigen-binding fragment thereof is glycosylated but does not contain detectable NeuGc or α-Gal.
163. The method of any of paragraphs 154 to 162 wherein the mAb or antigen-binding fragment thereof contains a tyrosine sulfation.
164. The method of any of paragraphs 155 to 163 wherein the recombinant expression vector is AAV8 or AAV9.
165. The method of any of paragraphs 155 to 164 in which production of the HuPTM form of said mAb or antigen-binding fragment thereof is confirmed by transducing human liver cells or muscle cells in culture with said recombinant nucleotide expression vector and expressing said mAb or antigen-binding fragment thereof.
166. A method of treating asthma in a human subject in need thereof, comprising delivering to the circulation of said human subject, a therapeutically effective amount of an anti-IL-5 mAb, or antigen-binding fragment thereof, produced by human liver cells or human muscle cells.
167. A method of treating asthma in a human subject in need thereof, comprising:
   administering to the liver or muscle of said human subject a therapeutically effective amount of a recombinant nucleotide expression vector comprising a transgene encoding an anti-IL-5 mAb, or an antigen-binding fragment thereof, operably linked to one or more regulatory sequences that control expression of the transgene in human liver cells or human muscle cells, so that a depot is formed that releases a HuPTM form of said mAb or antigen-binding fragment thereof.
168. The method of paragraphs 166 or 167 wherein the anti-IL-5 mAb is mepolizumab.
169. The method of any of paragraphs 166 to 168 wherein the antigen-binding fragment is a Fab, a F(ab')₂, or an scFv.
170. The method of any of paragraphs 166 to 169, wherein the antigen-binding fragment comprises a heavy chain with an amino acid sequence of SEQ ID NO: 15 and a light chain with an amino acid sequence of SEQ ID NO:16.
171. The method of claim 170, wherein the transgene comprises a nucleotide sequence of SEQ ID NO: 115 encoding the heavy chain and a nucleotide sequence of SEQ ID NO: 116 encoding the light chain.
172. The method of any of paragraphs 166 to 170, wherein the antibody or antigen-binding fragment thereof is a hyperglycosylated mutant.
173. The method of any of paragraphs 166 to 172 wherein the mAb or antigen-binding fragment thereof contains an alpha2,6-sialylated glycan.
174. The method of any of paragraphs 166 to 173 wherein the mAb or antigen-binding fragment thereof is glycosylated but does not contain detectable NeuGc or α-Gal.
175. The method of any of paragraphs 166 to 174 wherein the mAb or antigen-binding fragment thereof contains a tyrosine sulfation.
176. The method of any of paragraphs 167 to 175 wherein the recombinant expression vector is AAV8 or AAV9.
177. The method of any of paragraphs 167 to 176 in which production of said HuPTM form of said mAb or antigen-binding fragment thereof is confirmed by transducing human liver cells or muscle cells in culture with said recombinant nucleotide expression vector and expressing said mAb or antigen-binding fragment thereof.
178. A method of treating HeFH, HoFH, dyslipidemia, cardiovascular disease including atherosclerotic cardiovascular disease (ACD), atherosclerotic plaque formation, abnormally high levels of non-HDL cholesterol and LDL, aortic stenosis, hepatic stenosis, or hypercholesterolemia dyslipidemia in a human subject in need thereof, comprising delivering to the circulation of said human subject, a therapeutically effective amount of an anti-PCSK9, anti-ANGPTL3, anti-OxPL mAb or antigen-binding fragment thereof, produced by human liver cells or human muscle cells.
179. A method of treating HeFH, HoFH, dyslipidemia, cardiovascular disease including atherosclerotic cardiovascular disease (ACD), atherosclerotic plaque formation, abnormally high levels of non-HDL cholesterol and LDL, aortic stenosis, hepatic stenosis, or hypercholesterolemia in a human subject in need thereof, comprising:
   administering to the liver or muscle of said human subject a therapeutically effective amount of a recombinant nucleotide expression vector comprising a transgene encoding an anti-PCSK9, anti-OxPL, or anti-ANGPTL3 mAb, or an antigen-binding fragment thereof, operably linked to one or more regulatory sequences that control expression of the transgene in human liver cells or human muscle cells, so that a depot is formed that releases a HuPTM form of the mAb or antigen-binding fragment thereof.
180. The method of paragraph 178 or 179 wherein the anti-PCSK9 is alirocumab or evolocumab, or the anti-ANGPTL3 mAb is evinacumab or the anti-OxPL is E06.
181. The method of any of paragraphs 178 to 180 wherein the antigen-binding fragment is a Fab, a F(ab')₂, or an scFv.
182. The method of any of paragraphs 178 to 181, wherein the antigen-binding fragment comprises a heavy chain with an amino acid sequence of SEQ ID NO: 21 and a light chain with an amino acid sequence of SEQ ID NO:22; or a heavy chain with an amino acid sequence of SEQ ID NO: 23 and a light chain with an amino acid sequence of SEQ ID NO: 24; a heavy chain with an amino acid sequence of SEQ ID NO: 25 and a light chain with an amino acid sequence of SEQ ID NO:26; or a heavy chain with an amino acid sequence of SEQ ID NO: 59 and a light chain with an amino acid sequence of SEQ ID NO: 60.
183. The method of claim 182, wherein the transgene comprises a nucleotide sequence of SEQ ID NO: 121 encoding the heavy chain and a nucleotide sequence of SEQ ID NO: 122 encoding the light chain; or a nucleotide sequence of SEQ ID NO: 123 encoding the heavy chain and a nucleotide sequence of SEQ ID NO: 124 encoding the light chain; a nucleotide sequence of SEQ ID NO: 125 encoding the heavy chain and a nucleotide sequence of SEQ ID NO: 126 encoding the light chain; or a heavy chain with an nucleotide sequence of SEQ ID NO: 159 and a light chain with an nucleotide sequence of SEQ ID NO: 160.
184. The method of any of paragraphs 178 to 182, wherein the mAb or antigen-binding fragment thereof is a hyperglycosylated mutant.
185. The method of any of paragraphs 178 to 184 wherein the mAb or antigen-binding fragment thereof contains an alpha2,6-sialylated glycan.
186. The method of any of paragraphs 178 to 185 wherein the mAb or antigen-binding fragment thereof is glycosylated but does not contain detectable NeuGc or α-Gal.
187. The method of any of paragraphs 178 to 186 wherein the mAb or antigen-binding fragment thereof contains a tyrosine sulfation.
188. The method of any of paragraphs 179 to 187 wherein the recombinant expression vector is AAV8 or AAV9.
189. The method of any of paragraphs 179 to 188 in which production of said HuPTM form of the mAb or antigen-binding fragment thereof is confirmed by transducing human liver cells or human muscle cells in culture with said recombinant nucleotide expression vector and expressing said mAb or antigen-binding fragment thereof.
190. A method of treating osteoporosis, increasing bone mass in breast or prostate cancer patients, or preventing skeletal related events due to bone metastasis in a human subject in need thereof, comprising delivering to the circulation of said human subject, a therapeutically effective amount of an anti-RANKL mAb, or antigen-binding fragment thereof, produced by human liver cells or human muscle cells.
191. A method of treating osteoporosis, increasing bone mass in breast or prostate cancer patients, or preventing skeletal related events due to bone metastasis in a human subject in need thereof, comprising:
   administering to the liver or muscle of said human subject a therapeutically effective amount of a recombinant nucleotide expression vector comprising a transgene encoding an anti-RANKL mAb, or an antigen-binding fragment thereof, operably linked to one or more regulatory sequences that control expression of the transgene in human liver cells or human muscle cells, so that a depot is formed that releases a HuPTM form of the mAb or antigen-binding fragment thereof.
192. The method of paragraph 190 or 191 wherein the anti-RANKL mAb is denosumab.
193. The method of any of paragraphs 190 to 192 wherein the antigen-binding fragment is a Fab, a F(ab')₂, or an scFv.
194. The method of any of paragraphs 190 to 193, wherein the antigen-binding fragment comprises a heavy chain with an amino acid sequence of SEQ ID NO: 27 and a light chain with an amino acid sequence of SEQ ID NO: 28.
195. The method of claim 194, wherein the transgene comprises a nucleotide sequence of SEQ ID NO: 128 encoding the heavy chain and a nucleotide sequence of SEQ ID NO: 127 encoding the light chain.
196. The method of any of paragraphs 190 to 194, wherein the mAb or antigen-binding fragment thereof is a hyperglycosylated mutant.
197. The method of any of paragraphs 190 to 196 wherein the mAb or antigen-binding fragment thereof contains an alpha 2,6-sialylated glycan.
198. The method of any of paragraphs 190 to 197 wherein the mAb or antigen-binding fragment thereof is glycosylated but does not contain detectable NeuGc or α-Gal.
199. The method of any of paragraphs 190 to 198 wherein the mAb or antigen-binding fragment thereof contains a tyrosine sulfation.
200. The method of any of paragraphs 191 to 199 wherein the recombinant expression vector is AAV8 or AAV9.
201. The method of any of paragraphs 191 to 200 in which production of said HuPTM form of said mAb or antigen-binding fragment thereof is confirmed by transducing human liver cells or muscle cells in culture with said recombinant nucleotide expression vector and expressing said mAb or antigen-binding fragment thereof.
202. A method of treating metastatic melanoma, lymphoma, non-small cell lung carcinoma, head and neck squamous cell cancer, urothelial carcinoma, microsatellite instability-high cancer, gastric cancer, renal cell carcinoma, mismatch repair deficit metastatic colon cancer, or hepatocellular carcinoma in a human subject in need thereof, comprising delivering to the circulation of said human subject, a therapeutically effective amount of a PD-1 blocker mAb, or antigen-binding fragment thereof, produced by human liver cells or human muscle cells.
203. A method of treating metastatic melanoma, lymphoma, non-small cell lung carcinoma, head and neck squamous cell cancer, urothelial carcinoma, microsatellite instability-high cancer, gastric cancer, renal cell carcinoma, mismatch repair deficit metastatic colon cancer, or hepatocellular carcinoma in a human subject in need thereof, comprising:
   administering to the liver or muscle of said human subject a therapeutically effective amount of a recombinant nucleotide expression vector comprising a transgene encoding a PD-1 blocker mAb, or an antigen-binding fragment thereof, operably linked to one or more regulatory sequences that control expression of the transgene in human liver cells or human muscle cells, so that a depot is formed that releases a HuPTM form of said mAb or antigen-binding fragment thereof.
204. The method of paragraph 202 or 203 wherein the PD-1 blocker mAb is nivolumab or pembrolizumab.
205. The method of any of paragraphs 202 to 204 wherein the antigen-binding fragment is a Fab, a F(ab')₂, or an scFv.
206. The method of any of paragraphs 202 to 205, wherein the antigen-binding fragment comprises a heavy chain with an amino acid sequence of SEQ ID NO: 29 and a light chain with an amino acid sequence of SEQ ID NO:30; or a heavy chain with an amino acid sequence of SEQ ID NO: 31 and a light chain with an amino acid sequence of SEQ ID NO:32.
207. The method of claim 206, wherein the transgene comprises a nucleotide sequence of SEQ ID NO: 129 encoding the heavy chain and a nucleotide sequence of SEQ ID NO: 130 encoding the light chain; or a nucleotide sequence of SEQ ID NO: 131 encoding the heavy chain and a nucleotide sequence of SEQ ID NO: 132 encoding the light chain.
208. The method of any of paragraphs 202 to 206, wherein the mAb or antigen-binding fragment thereof is a hyperglycosylated mutant.
209. The method of any of paragraphs 202 to 208 wherein the mAb or antigen-binding fragment thereof contains an alpha 2,6-sialylated glycan.
210. The method of any of paragraphs 202 to 209 wherein the mAb or antigen-binding fragment thereof is glycosylated but does not contain detectable NeuGc or α-Gal.
211. The method of any of paragraphs 202 to 210 wherein the mAb or antigen-binding fragment thereof contains a tyrosine sulfation.
212. The method of any of paragraphs 203 to 211 wherein the recombinant expression vector is AAV8 or AAV9.
213. The method of any of paragraphs 203 to 212 in which production of said HuPTM form of said mAb or antigen-binding fragment thereof is confirmed by transducing human liver cells or muscle cells in culture with said recombinant nucleotide expression vector and expressing said mAb or antigen-binding fragment thereof.
214. A method of treating systemic lupus erythromatosis (SLE) in a human subject in need thereof, comprising delivering to the circulation of said human subject, a therapeutically effective amount of an anti-BLyS mAb or antigen-binding fragment thereof, produced by human liver cells or human muscle cells.
215. A method of treating SLE in a human subject in need thereof, comprising:
   administering to the liver or muscle of said subject a therapeutically effective amount of a recombinant nucleotide expression vector comprising a transgene encoding an anti-BLyS mAb, or an antigen-binding fragment thereof, operably linked to one or more regulatory sequences that control expression of the transgene in human liver cells or in human muscle cells, so that a depot is formed that releases a HuPTM form of said mAb or antigen-binding fragment thereof.
216. The method of paragraph 214 or 215 wherein the anti-BLyS mAb is belimumab.
217. The method of any of paragraphs 214 to 216 wherein the antigen-binding fragment is a Fab, a F(ab')₂, or an scFv.
218. The method of any of paragraphs 214 to 217, wherein the antigen-binding fragment comprises a heavy chain with an amino acid sequence of SEQ ID NO: 41 and a light chain with an amino acid sequence of SEQ ID NO:42.
219. The method of claim 218, wherein the transgene comprises a nucleotide sequence of SEQ ID NO: 139 encoding the heavy chain and a nucleotide sequence of SEQ ID NO: 142 encoding the light chain.
220. The method of any of paragraphs 214 to 218, wherein the mAb or antigen-binding fragment thereof is a hyperglycosylated mutant.
221. The method of any of paragraphs 214 to 220 wherein the mAb or antigen-binding fragment thereof contains an alpha2,6-sialylated glycan.
222. The method of any of paragraphs 214 to 221 wherein the mAb or antigen-binding fragment thereof is glycosylated but does not contain detectable NeuGc or α-Gal.
223. The method of any of paragraphs 214 to 222 wherein the mAb or antigen-binding fragment thereof contains a tyrosine sulfation.
224. The method of any of paragraphs 215 to 223 wherein the recombinant expression vector is AAV8 or AAV9.
225. The method of any of paragraphs 215 to 224 in which production of said HuPTM form of said mAb or antigen-binding fragment thereof is confirmed by transducing human liver cells or muscle cells in culture with said recombinant nucleotide expression vector and expressing said mAb or antigen-binding fragment thereof.
226. A method of treating an ocular disorder, including neovascular age-related macular degeneration (nAMD), dry AMD, diabetic retinopathy, diabetic macular edema (DME), central retinal vein occlusion (RVO), pathologic myopia, or polypoidal choroidal vasculopathy, in a human subject in need thereof, comprising delivering to the retina of said human subject, a therapeutically effective amount of an anti-MMP9, anti-VEGF or anti-fD mAb, or antigen-binding fragment thereof, produced by human retina cells.
227. A method of treating an ocular disorder, including nAMD, dry AMD, diabetic retinopathy, DME, RVO, pathologic myopia, or polypoidal choroidal vasculopathy, in a human subject in need thereof, comprising:
   administering subretinally, intravitreally or suprachoroidally to said human subject, a therapeutically effective amount of a recombinant nucleotide expression vector comprising a transgene encoding an anti-MMP9, anti-VEGF or anti-fD mAb, or an antigen-binding fragment thereof, operably linked to one or more regulatory sequences that control expression of the transgene in human retinal cells, so that a depot is formed that releases a HuPTM form of said mAb or antigen-binding fragment thereof.
228. The method of paragraph 226 or 227 wherein the anti-MMP9, anti-VEGF or anti-fD mAb is andecaliximab, ranibizumab, bevacizumab, brolucizumab, or lampalizumab.
229. The method of any of paragraphs 226 to 228 wherein the antigen-binding fragment is a Fab, a F(ab')₂, or an scFv.
230. The method of any of paragraphs 226 to 229, wherein the antigen-binding fragment comprises a heavy chain with an amino acid sequence of SEQ ID NO: 33 and a light chain with an amino acid sequence of SEQ ID NO: 34; or a heavy chain with an amino acid sequence of SEQ ID NO: 35 and a light chain with an amino acid sequence of SEQ ID NO: 36; or a heavy chain with an amino acid sequence of SEQ ID NO: 37 and a light chain with an amino acid sequence of SEQ ID NO:38; or a heavy chain with an amino acid sequence of SEQ ID NO: 39 and a light chain with an amino acid sequence of SEQ ID NO: 40; or a heavy chain with an amino acid sequence of SEQ ID NO: 45 and a light chain with an amino acid sequence of SEQ ID NO: 46.
231. The method of claim 230, wherein the transgene comprises a nucleotide sequence of SEQ ID NO: 133 encoding the heavy chain and a nucleotide sequence of SEQ ID NO: 134 encoding the light chain; or a nucleotide sequence of SEQ ID NO: 135 encoding the heavy chain and a nucleotide sequence of SEQ ID NO: 136 encoding the light chain; or a nucleotide sequence of SEQ ID NO: 137 encoding the heavy chain and a nucleotide sequence of SEQ ID NO: 138 encoding the light chain; or a nucleotide sequence of SEQ ID NO: 139 encoding the heavy chain and a nucleotide sequence of SEQ ID NO: 140 encoding the light chain; or a nucleotide sequence of SEQ ID NO: 145 encoding the heavy chain and a nucleotide sequence of SEQ ID NO: 146 encoding the light chain.
232. The method of any of paragraphs 226 to 230, wherein the mAb or antigen-binding fragment thereof is a hyperglycosylated mutant.
233. The method of any of paragraphs 226 to 232 wherein the mAb or antigen-binding fragment thereof contains an alpha 2,6-sialylated glycan.
234. The method of any of paragraphs 226 to 233 wherein the mAb or antigen-binding fragment thereof is glycosylated but does not contain detectable NeuGc or α-Gal.
235. The method of any of paragraphs 226 to 234 wherein the mAb or antigen-binding fragment thereof contains a tyrosine sulfation.
236. The method of any of paragraphs 227 to 235 wherein the recombinant expression vector is AAV8 or AAV9.
237. The method of any of paragraphs 227 to 236 in which production of said HuPTM form of said mAb or antigen-binding fragment thereof is confirmed by transducing human retinal cells in culture with said recombinant nucleotide expression vector and expressing said mAb or antigen-binding fragment thereof.
238. A method of treating cystic fibrosis (CF), rheumatoid arthritis (RA), UC ,CD, solid tumors, pancreatic adenocarcinoma, lung adenocarcinoma, lung squamous cell carcinoma, esophagogastric adenocarcinoma, gastric cancer, colorectal cancer, or breast cancer in a human subject in need thereof, comprising delivering to the circulation of said human subject, a therapeutically effective amount of an anti-MMP9 mAb, or antigen-binding fragment thereof, produced by human liver cells or human muscle cells.
239. A method of treating cystic fibrosis (CF), rheumatoid arthritis (RA), UC ,CD, solid tumors, pancreatic adenocarcinoma, lung adenocarcinoma, lung squamous cell carcinoma, esophagogastric adenocarcinoma, gastric cancer, colorectal cancer, or breast cancer in a human subject in need thereof, comprising:
   administering to the liver or muscle of said subject a therapeutically effective amount of a recombinant nucleotide expression vector comprising a transgene encoding an anti-MMP9 mAb, or an antigen-binding fragment thereof, operably linked to one or more regulatory sequences that control expression of the transgene in human liver cells or in human muscle cells, so that a depot is formed that releases a HuPTM form of said mAb or antigen-binding fragment thereof.
240. The method of paragraph 238 or 239 wherein the anti-MMP9 mAb is andecaliximab.
241. The method of any of paragraphs 238 to 240 wherein the antigen-binding fragment is a Fab, a F(ab')₂, or an scFv.
242. The method of any of paragraphs 238 to 241, wherein the antigen-binding fragment comprises a heavy chain with an amino acid sequence of SEQ ID NO: 45 and a light chain with an amino acid sequence of SEQ ID NO: 46.
243. The method of claim 242, wherein the transgene comprises a nucleotide sequence of SEQ ID NO: 145 encoding the heavy chain and a nucleotide sequence of SEQ ID NO: 146 encoding the light chain.
244. The method of any of paragraphs 238 to 242, wherein the mAb or antigen-binding fragment thereof is a hyperglycosylated mutant.
245. The method of any of paragraphs 238 to 244 wherein the mAb or antigen-binding fragment thereof contains an alpha 2,6-sialylated glycan.
246. The method of any of paragraphs 238 to 245 wherein the mAb or antigen-binding fragment thereof is glycosylated but does not contain detectable NeuGc or α-Gal.
247. The method of any of paragraphs 238 to 246 wherein the mAb or antigen-binding fragment thereof contains a tyrosine sulfation.
248. The method of any of paragraphs 239 to 247 wherein the recombinant expression vector is AAV8 or AAV9.
249. The method of any of paragraphs 239 to 248 in which production of said HuPTM form of said mAb or antigen-binding fragment thereof is confirmed by transducing human liver cells or muscle cells in culture with said recombinant nucleotide expression vector and expressing said mAb or antigen-binding fragment thereof.
250. A method of treating hereditary angioedema in a human subject in need thereof, comprising delivering to the circulation of said human subject, a therapeutically effective amount of an anti-kallikrein mAb, or antigen-binding fragment thereof, produced by human liver cells or human muscle cells.
251. A method of treating hereditary angioedema in a human subject in need thereof, comprising:
   administering to the liver or muscle of said subject a therapeutically effective amount of a recombinant nucleotide expression vector comprising a transgene encoding an anti-kallikrein mAb, or an antigen-binding fragment thereof, operably linked to one or more regulatory sequences that control expression of the transgene in human liver cells or in human muscle cells, so that a depot is formed that releases a HuPTM form of said mAb or antigen-binding fragment thereof.
252. The method of paragraph 250 or 251 wherein the anti-kallikrein mAb is lanadelumab.
253. The method of any of paragraphs 250 to 252 wherein the antigen-binding fragment is a Fab, a F(ab')₂, or an scFv.
254. The method of any of paragraphs 250 to 253, wherein the antigen-binding fragment comprises a heavy chain with an amino acid sequence of SEQ ID NO: 47 and a light chain with an amino acid sequence of SEQ ID NO: 48.
255. The method of claim 254, wherein the transgene comprises a nucleotide sequence of SEQ ID NO: 147 encoding the heavy chain and a nucleotide sequence of SEQ ID NO: 148 encoding the light chain.
256. The method of any of paragraphs 250 to 255, wherein the mAb or antigen-binding fragment thereof is a hyperglycosylated mutant.
257. The method of any of paragraphs 250 to 256 wherein the mAb or antigen-binding fragment thereof contains an alpha 2,6-sialylated glycan.
258. The method of any of paragraphs 250 to 257 wherein the mAb or antigen-binding fragment thereof is glycosylated but does not contain detectable NeuGc or α-Gal.
259. The method of any of paragraphs 250 to 258 wherein the mAb or antigen-binding fragment thereof contains a tyrosine sulfation.
260. The method of any of paragraphs 251 to 259 wherein the recombinant expression vector is AAV8 or AAV9.
261. The method of any of paragraphs 251 to 260 in which production of said HuPTM form of said mAb or antigen-binding fragment thereof is confirmed by transducing human liver cells or muscle cells in culture with said recombinant nucleotide expression vector and expressing said mAb or antigen-binding fragment thereof.
262. A method of treating rheumatoid arthritis, psoriatic arthritis, ankylosing spondylitis, Crohn's disease, plaque psoriasis, or ulcerative colitis in a human subject in need thereof, comprising delivering to the circulation of said human subject, a therapeutically effective amount of an anti-TNF-alpha mAb, or antigen-binding fragment thereof, produced by human liver cells or human muscle cells.
263. A method of treating rheumatoid arthritis, psoriatic arthritis, ankylosing spondylitis, Crohn's disease, plaque psoriasis, or ulcerative colitis in a human subject in need thereof, comprising:
   administering to the liver or muscle of said subject a therapeutically effective amount of a recombinant nucleotide expression vector comprising a transgene encoding an anti-TNF-alpha mAb, or an antigen-binding fragment thereof, operably linked to one or more regulatory sequences that control expression of the transgene in human liver cells or in human muscle cells, so that a depot is formed that releases a HuPTM form of said mAb or antigen-binding fragment thereof.
264. The method of paragraph 262 or 263 wherein the anti-TNF-alpha mAb is adalimumab or infliximab.
265. The method of any of paragraphs 262 to 264 wherein the antigen-binding fragment is a Fab, a F(ab')₂, or an scFv.
266. The method of any of paragraphs 262 to 265, wherein the antigen-binding fragment comprises a heavy chain with an amino acid sequence of SEQ ID NO: 49 and a light chain with an amino acid sequence of SEQ ID NO: 50; or a heavy chain with an amino acid sequence of SEQ ID NO: 51 and a light chain with an amino acid sequence of SEQ ID NO: 52.
267. The method of claim 266, wherein the transgene comprises a nucleotide sequence of SEQ ID NO: 149 encoding the heavy chain and a nucleotide sequence of SEQ ID NO: 150 encoding the light chain; a nucleotide sequence of SEQ ID NO: 151 encoding the heavy chain and a nucleotide sequence of SEQ ID NO: 152 encoding the light chain.
268. The method of any of paragraphs 262 to 266, wherein the mAb or antigen-binding fragment thereof is a hyperglycosylated mutant.
269. The method of any of paragraphs 262 to 268 wherein the mAb or antigen-binding fragment thereof contains an alpha 2,6-sialylated glycan.
270. The method of any of paragraphs 262 to 269 wherein the mAb or antigen-binding fragment thereof is glycosylated but does not contain detectable NeuGc or α-Gal.
271. The method of any of paragraphs 262 to 270 wherein the mAb or antigen-binding fragment thereof contains a tyrosine sulfation.
272. The method of any of paragraphs 263 to 271 wherein the recombinant expression vector is AAV8 or AAV9.
273. The method of any of paragraphs 263 to 272 in which production of said HuPTM form of said mAb or antigen-binding fragment thereof is confirmed by transducing human liver cells or muscle cells in culture with said recombinant nucleotide expression vector and expressing said mAb or antigen-binding fragment thereof.
274. A method of treating PNH or aHUS in a human subject in need thereof, comprising delivering to the circulation of said human subject, a therapeutically effective amount of an anti-C5 or C5a protein mAb, or antigen-binding fragment thereof, produced by human liver cells.
275. A method of treating PNH or aHUS in a human subject in need thereof, comprising:
   administering to the liver of said subject a therapeutically effective amount of a recombinant nucleotide expression vector comprising a transgene encoding an anti-C5 or C5a protein mAb, or an antigen-binding fragment thereof, operably linked to one or more regulatory sequences that control expression of the transgene in human liver cells, so that a depot is formed that releases a HuPTM form of said mAb or antigen-binding fragment thereof.
276. The method of paragraphs 274 to 275 wherein the anti-C5 or C5a protein mAb, or antigen binding fragment, is eculizumab.
277. The method of any of paragraphs 274 to 276 wherein the antigen-binding fragment is a Fab, a F(ab')₂, or an scFv.
278. The method of any of paragraphs 274 to 277, wherein the antigen-binding fragment comprises a heavy chain with an amino acid sequence of SEQ ID NO: 43 and a light chain with an amino acid sequence of SEQ ID NO: 44.
279. The method of claim 278, wherein the transgene comprises a nucleotide sequence of SEQ ID NO: 143 encoding the heavy chain and a nucleotide sequence of SEQ ID NO: 144 encoding the light chain.
280. The method of any of paragraphs 274 to 279, wherein the mAb or antigen-binding fragment thereof is a hyperglycosylated mutant.
281. The method of any of paragraphs 274 to 280 wherein the mAb or antigen-binding fragment thereof contains an alpha 2,6-sialylated glycan.
282. The method of any of paragraphs 274 to 281 wherein the mAb or antigen-binding fragment thereof is glycosylated but does not contain detectable NeuGc or α-Gal.
283. The method of any of paragraphs 274 to 282 wherein the mAb or antigen-binding fragment thereof contains a tyrosine sulfation.
284. The method of any of paragraphs 275 to 283 wherein the recombinant expression vector is AAV8 or AAV9.
285. The method of any of paragraphs 275 to 284 in which production of said HuPTM form of said mAb or antigen-binding fragment thereof is confirmed by transducing human liver cells or muscle cells in culture with said recombinant nucleotide expression vector and expressing said mAb or antigen-binding fragment thereof.

### Method of Manufacture

286. A method of producing recombinant AAVs comprising:
   (a) culturing a host cell containing:
      (i) an artificial genome comprising a cis expression cassette flanked by AAV ITRs, wherein the cis expression cassette comprises a transgene encoding a therapeutic antibody operably linked to expression control elements that will control expression of the transgene in human cells;
      (ii) a trans expression cassette lacking AAV ITRs, wherein the trans expression cassette encodes an AAV rep and capsid protein operably linked to expression control elements that drive expression of the AAV rep and capsid proteins in the host cell in culture and supply the rep and cap proteins in trans;
      (iii) sufficient adenovirus helper functions to permit replication and packaging of the artificial genome by the AAV capsid proteins; and
   (b) recovering recombinant AAV encapsidating the artificial genome from the cell culture.
287. The method of claim 286 wherein the transgene encodes a mAb or antigen binding fragment thereof that comprises the heavy and light chain variable domains of aducanumab, crenezumab, gantenerumab, BAN2401, aTAU, erenumab, eptinezumab, fremanezumab, or galcanezumab.
288. The method of claim 286 or 287 in which the AAV capsid protein is an AAV9 or AAVrh10 capsid protein.
289. The method of claim 286 wherein the transgene encodes a mAb or antigen binding fragment that comprises the heavy and light chain variable domains of ixekizumab, secukinumab or ustekinumab.
290. The method of claim 286 wherein the transgene encodes a mAb or antigen binding fragment that comprises the heavy and light chain variable domains of natalizumab or vedolizumab.
291. The method of claim 286 wherein the transgene encodes a mAb or antigen binding fragment that comprises the heavy and light chain variable domains of dupilumab
292. The method of claim 286 wherein the transgene encodes a mAb or antigen binding fragment that comprises the heavy and light chain variable domains of mepolizumab
293. The method of claim 286 wherein the transgene encodes a mAb or antigen binding fragment that comprises the heavy and light chain variable domains of alirocumab, evolocumab, evinacumab or E06.
294. The method of claim 286 wherein the transgene encodes a mAb or antigen binding fragment that comprises the heavy and light chain variable domains of denosumab.
295. The method of claim 286 wherein the transgene encodes a mAb or antigen binding fragment that comprises the heavy and light chain variable domains of nivolumab or pembrolizumab.
296. The method of claim 286 wherein the transgene encodes a mAb or antigen binding fragment that comprises the heavy and light chain variable domains of belimumab.
297. The method of claim 286 wherein the transgene encodes a mAb or antigen binding fragment that comprises the heavy and light chain variable domains of ranibizumab, bevacizumab, or lampalizumab.
298. The method of claim 286 wherein the transgene encodes a mAb or antigen binding fragment that comprises the heavy and light chain variable domains of andecaliximab.
299. The method of claim 286 wherein the transgene encodes a mAb or antigen binding fragment that comprises the heavy and light chain variable domains of lanadelumab.
300. The method of claim 286 wherein the transgene encodes a mAb or antigen binding fragment that comprises the heavy and light chain variable domains of adalimumab or infliximab
301. The method of claim 286 wherein the transgene encodes a mAb or antigen binding fragment that comprises the heavy and light chain variable domains of eculizumab.
302. The method of any of claims 286 or 289 to 301 wherein the AAV capsid protein is an AAV8 or AAV9 capsid protein.

### 4. BRIEF DESCRIPTION OF THE DRAWINGS

The patent or application file contains at least one drawing executed in color. Copies of this patent or patent application publication with color drawing(s) will be provided by the Office upon request and payment of the necessary fee.
FIG. 1. A schematic of an rAAV vector genome construct containing an expression cassette encoding the heavy and light chains of the Fab region of a therapeutic mAb controlled by expression elements, flanked by the AAV ITRs.

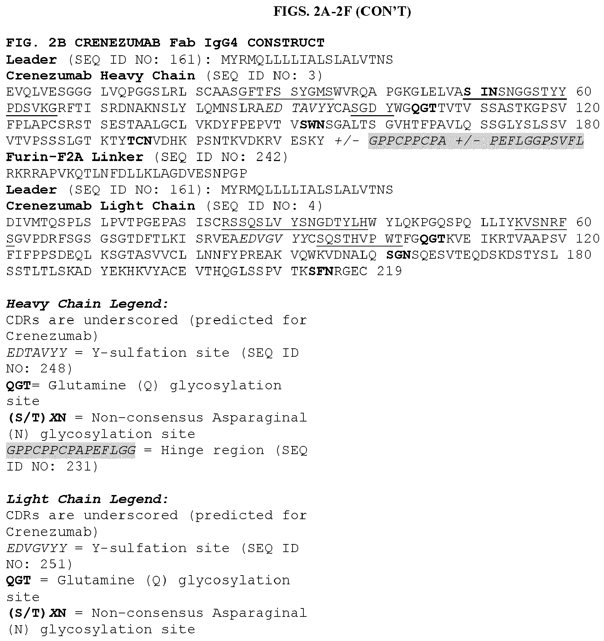

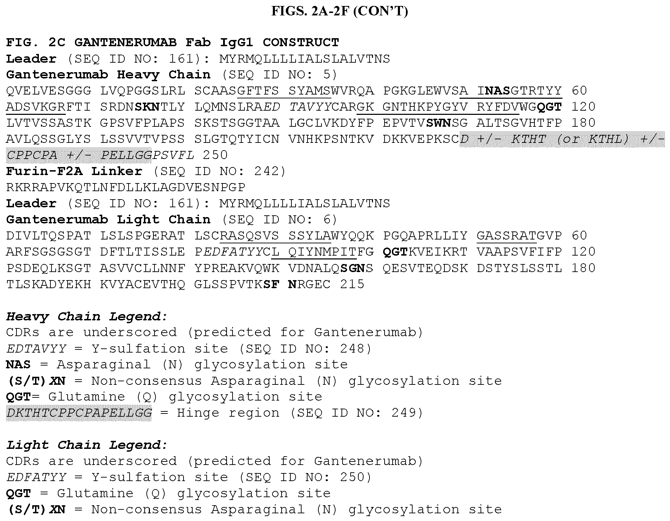

FIGS. 2A-F. The amino acid sequence of a transgene construct for the Fab region of therapeutic antibodies to CNS targets: anti-Aβ, aducanumab Fab (FIG. 2A); anti-Aβ, crenezumab Fab (FIG. 2B); anti-Aβ, gantenerumab Fab (FIG. 2C), ant-tau protein, aTAU Fab (FIG. 2D), and anti-CGRPR, erenumab Fab (FIG. 2E), and anti-Aβ BAN2401(FIG. 2F).. Glycosylation sites are boldface. Glutamine glycosylation sites are highlighted in green; asparaginal (N) glycosylation sites are highlighted in magenta; and non-consensus asparaginal (N) glycosylation sites are highlighted in blue; tyrosine-O-sulfation sites (italics) are highlighted in yellow. The heavy chain hinge regions are highlighted in grey.
FIGS. 3A-E. The amino acid sequence of a transgene construct for the Fab region of therapeutic antibodies to interleukins: anti-IL4R, dupilumab (FIG. 3A); anti-IL-17, ixekizumab (FIG. 3B); secukinumab (FIG. 3C); anti-IL-12/IL-23, ustekinumab (FIG. 3D); and anti-IL5, mepolizumab (FIG. 3E). Glycosylation sites are boldface. Glutamine glycosylation sites are highlighted in green and non-consensus asparaginal (N) glycosylation sites are highlighted in blue; tyrosine-O-sulfation sites (italics) are highlighted in yellow. The heavy chain hinge regions are highlighted in grey.
FIGS. 4A-4B. The amino acid sequence of a transgene construct for the Fab region of therapeutic antibodies to integrin: vedolizumab (FIG. 4A) and natalizumab (FIG. 4B). Glycosylation sites are boldface. Glutamine glycosylation sites are highlighted in green and non-consensus asparaginal (N) glycosylation sites are highlighted in blue; tyrosine-O-sulfation sites (italics) are highlighted in yellow. The heavy chain hinge regions are highlighted in grey.
FIGS. 5A-D. The amino acid sequence of a transgene construct for the Fab region of therapeutic antibodies to PCSK9: alirocumab (FIG. 5A); evolocumab (FIG. 5B); ANGPTL3: evinacumab (FIG. 5C), OxPL: E06-scFv. Glycosylation sites are boldface. Glutamine glycosylation sites are highlighted in green and non-consensus asparaginal (N) glycosylation sites are highlighted in blue; tyrosine-O-sulfation sites (italics) are highlighted in yellow. The hinge regions are highlighted in grey.
FIG. 6. The amino acid sequence of a transgene construct for the Fab region of denosumab, a therapeutic antibody to RANKL. Glycosylation sites are boldface. Glutamine glycosylation sites are highlighted in green and non-consensus asparaginal (N) glycosylation sites are highlighted in blue; tyrosine-O-sulfation sites (italics) are highlighted in yellow. The hinge region is highlighted in grey.
FIGS. 7A and B. The amino acid sequence of a transgene construct for the Fab region of therapeutic antibodies that are PD-1 blockers: nivolumab (FIG.7A); and pembrolizumab (FIG. 7B). Glycosylation sites are boldface. Glutamine glycosylation sites are highlighted in green and non-consensus asparaginal (N) glycosylation sites are highlighted in blue; tyrosine-O-sulfation sites (italics) are highlighted in yellow. The hinge regions are highlighted in grey.
FIGS. 8A-H. The amino acid sequence of a transgene construct for the Fab region of therapeutic antibodies directed at biological factors: anti-VEGF, ranibizumab (FIG. 8A), bevacizumab (FIG. 8B), and brolucizumab (FIG. 8D); anti-fD, lampalizumab (FIG. 8C); anti-BLyS, belimumab (FIG. 8E); anti-human C5 complement protein, eculizumab (FIG. 8F); anti-MMP 9, andecaliximab (FIG. 8G); and anti-kallikrein, lanadelumab (FIG. 8H). Glycosylation sites are boldface. Glutamine glycosylation sites are highlighted in green and non-consensus asparaginal (N) glycosylation sites are highlighted in blue; tyrosine-O-sulfation sites (italics) are highlighted in yellow. The hinge regions are highlighted in grey.
FIGS. 9A and B. The amino acid sequence of a transgene construct for the Fab region of therapeutic antibody directed at TNF-alpha: adalimumab (FIG. 9A) and infliximab (FIG. 9B). Glycosylation sites are boldface. Glutamine glycosylation sites are highlighted in green and non-consensus asparaginal (N) glycosylation sites are highlighted in blue; tyrosine-O-sulfation sites (italics) are highlighted in yellow. The hinge regions are highlighted in grey.
FIG. 10. Glycans that can be attached to HuGlyFab regions of full length mAbs or the antigen-binding domains. (Adapted from Bondt et al., 2014, Mol & Cell Proteomics 13.1: 3029-3039).
FIGS. 11A and B. Amino acid sequence alignment of the amino acid sequences of the heavy (FIG. 11A) (SEQ ID NOS 283-299, 59, 300-302, 313, 303, 39 and 304-310, respectively, in order of appearance) and light (FIG. 11B) (SEQ ID NOS 2, 4, 6, 8, 10, 12, 14, 16, 18, 20, 22, 24, 26, 28, 30, 32, 34, 60, 58, 36, 54, 311, 314, 312, 38, 234, 42, 44, 48, 247 and 235, respectively, in order of appearance) chain Fab portions of the therapeutic antibodies disclosed herein. Positions that may be substituted to produce hyperglycosylated variants of the Fab regions are highlighted in green. Four substitutions (one in the heavy chain and three in the light chain) that should result in hyperglycosylation of the Fab region by human cells are annotated above the amino acid residue positions. (For engineering mAbs or antigen-binding fragments to contain additional glycosylation sites on the Fab domain, see *e.g*., Courtois et al., 2016, mAbs 8: 99-112 for a description of derivatives of antibodies that are hyperglycosylated on the Fab domain of the full-length antibody).

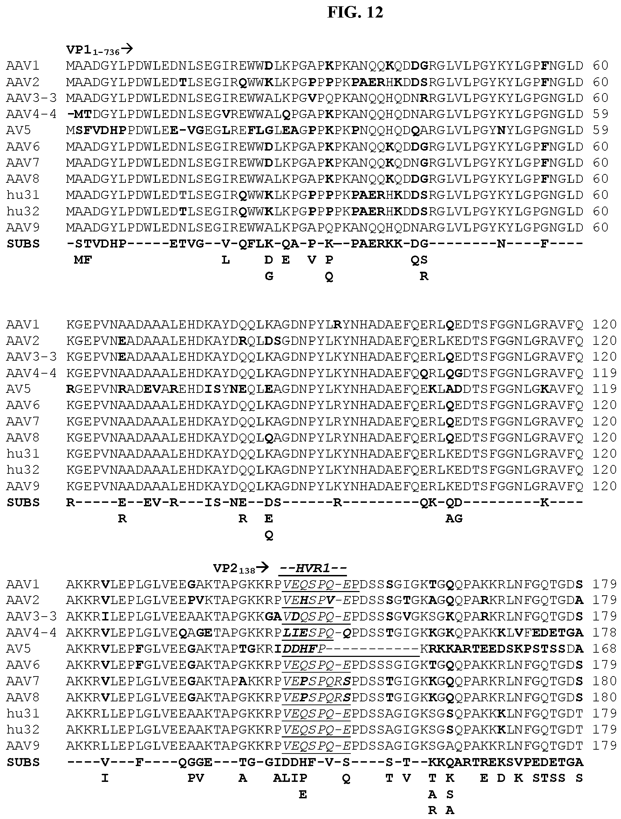

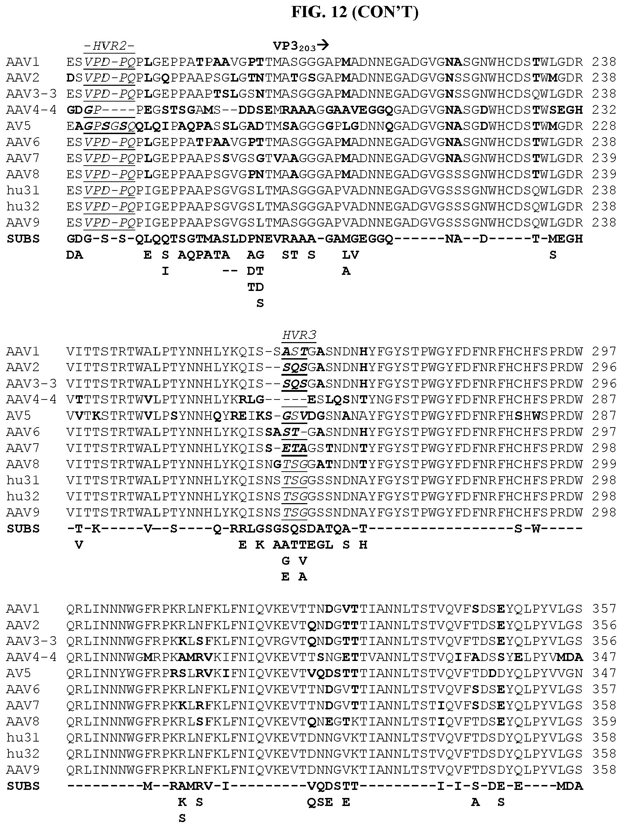

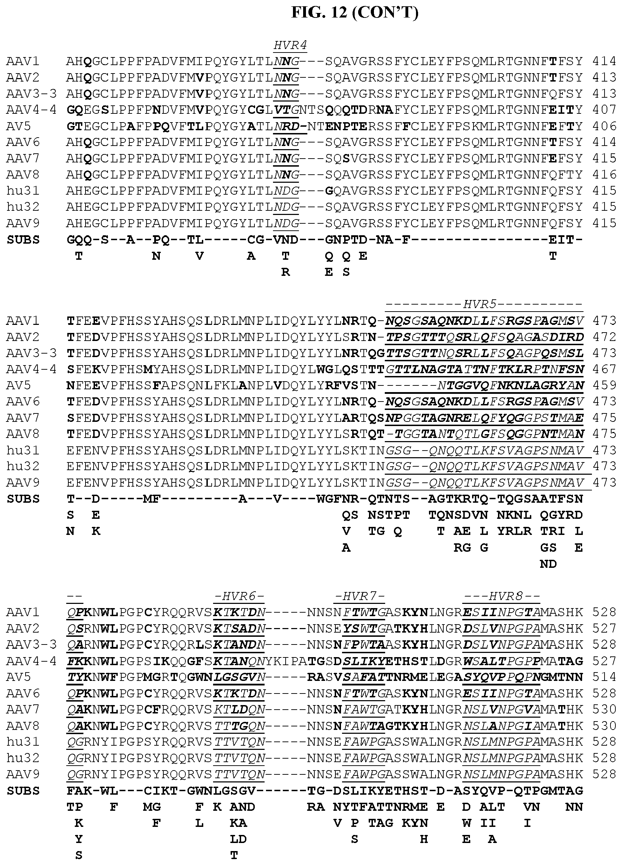

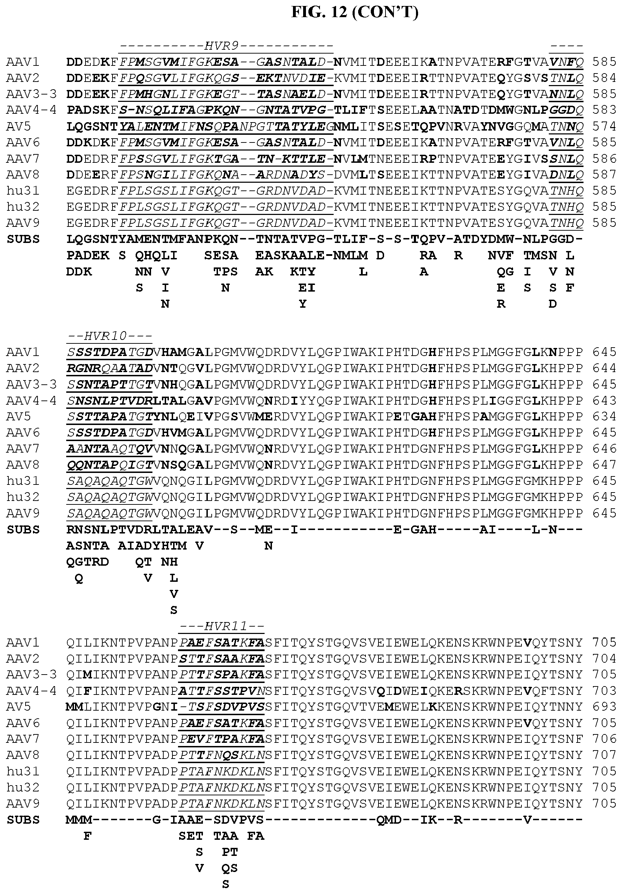

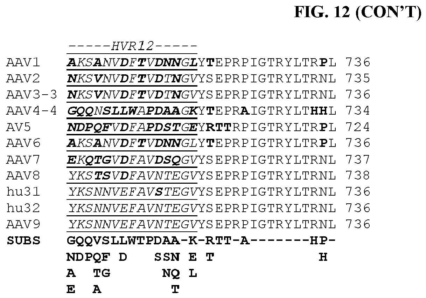

FIG. 12. Clustal Multiple Sequence Alignment of AAV capsids 1-9. Amino acid substitutions (shown in bold in the bottom rows) can be made to AAV9 and AAV8 capsids by "recruiting" amino acid residues from the corresponding position of other aligned AAV capsids. Sequence shown in Red = hypervariable regions. The amino acid sequences of the AAV capsids are assigned SEQ ID NOs as follows: AAV1 is SEQ ID NO: 71; AAV2 is SEQ ID NO: 72; AAV3-3 is SEQ ID NO: 73; AAV4-4 is SEQ ID NO: 74; AAV5 is SEQ ID NO: 75; AAV6 is SEQ ID NO: 76; AAV7 is SEQ ID NO: 77; AAV8 is SEQ ID NO: 78; AAV9 is SEQ ID NO: 79; hu31 is SEQ ID NO: 81; and hu32 is SEQ ID NO: 82.

### 5. DETAILED DESCRIPTION OF THE INVENTION

Compositions and methods are described for the delivery of a fully human post-translationally modified (HuPTM) therapeutic monoclonal antibody (mAb) or a HuPTM antigen-binding fragment of a therapeutic mAb (for example, a fully human-glycosylated Fab (HuGlyFab) of a therapeutic mAb) to a patient (human subject) diagnosed with a disease or condition indicated for treatment with the therapeutic mAb. Delivery may be advantageously accomplished via gene therapy-e.g., by administering a viral vector or other DNA expression construct encoding a therapeutic mAb or its antigen-binding fragment (or a hyperglycosylated derivative of either) to a patient (human subject) diagnosed with a condition indicated for treatment with the therapeutic mAb-to create a permanent depot in a tissue or organ of the patient that continuously supplies the HuPTM mAb or antigen-binding fragment of the therapeutic mAb, *e.g*., , a human-glycosylated transgene product, to a target tissue where the mAb or antigen-binding fragment there of exerts its therapeutic effect.

The HuPTM mAb or HuPTM antigen-binding fragment encoded by the transgene can include, but is not limited to, a full-length or an antigen-binding fragment of a therapeutic antibody that binds to:
- *Nervous system targets, including Amyloid beta (Aβ or A beta) peptides, Tau protein, and CGRP receptor,*
- *Interleukins or interleukin receptors, including IL4R, IL17A, IL-5, and IL12*/*IL23,*
- *Integrins, including integrin-alpha-4,*
- *PCSK9, ANGPTL3, or oxidized phospholipids, such as OxPL,*
- *RANKL,*
- *PD-1, or PD-L1 or PD-L2,*
- *BLyS (B-lymphocyte stimulator, also known as B-cell activating factor (BAFF)),*
- *Ocular Targets including VEGF, fD, and matrix metalloproteinase 9(MMP9),*
- *TNF-alpha,* and
- *Plasma Protein Targets, such as human complement proteins, including, C5 and C5a complement proteins, and plasma kallikrein,*
or such mAbs or antigen-binding fragments engineered to contain additional glycosylation sites on the Fab domain (*e.g.*, see Courtois et al., 2016, mAbs 8: 99-112 which is incorporated by reference herein in its entirety for it description of derivatives of antibodies that are hyperglycosylated on the Fab domain of the full-length antibody). The amino acid sequences of the heavy and light chains of antigen binding fragments of the foregoing are provided in Table 4, *infra,* and codon optimized nucleotide sequences encoding the heavy and light chains of these antigen binding fragments are provided in Table 5.

The recombinant vector used for delivering the transgene includes non-replicating recombinant adeno-associated virus vectors ("rAAV"). rAAVs are particularly attractive vectors for a number of reasons - they can transduce non-replicating cells, and therefore, can be used to deliver the transgene to tissues where cell division occurs at low levels, such as the CNS; they can be modified to preferentially target a specific organ of choice; and there are hundreds of capsid serotypes to choose from to obtain the desired tissue specificity, and/or to avoid neutralization by pre-existing patient antibodies to some AAVs. Such rAAVs include but are not limited to AAV based vectors comprising capsid components from one or more of AAV1, AAV2, AAV3, AAV4, AAV5, AAV6, AAV7, AAV8, AAV9, AAV10, AAV11, AAVrh10 or AAVrh20. In preferred embodiments, AAV based vectors provided herein comprise capsids from one or more of AAV8, AAV9, AAV10, AAV11, AAVrh10 or AAVrh20 serotypes.

However, other viral vectors may be used, including but not limited to lentiviral vectors; vaccinia viral vectors, or non-viral expression vectors referred to as "naked DNA" constructs. Expression of the transgene can be controlled by constitutive or tissue-specific expression control elements.

Gene therapy constructs are designed such that both the heavy and light chains are expressed. More specifically, the heavy and light chains should be expressed at about equal amounts, in other words, the heavy and light chains are expressed at approximately a 1:1 ratio of heavy chains to light chains. The coding sequences for the heavy and light chains can be engineered in a single construct in which the heavy and light chains are separated by a cleavable linker or IRES so that separate heavy and light chain polypeptides are expressed. In certain embodiments, the coding sequences encode for a Fab or F(ab')₂ or an scFv.

In certain embodiments, nucleic acids (e.g., polynucleotides) and nucleic acid sequences disclosed herein may be codon-optimized, for example, via any codon-optimization technique known to one of skill in the art (see, e.g., review by Quax et al., 2015, Mol Cell 59:149-161). Codon optimized nucleotide sequences of the heavy and light chain variable domains of the therapeutic antibodies are disclosed in Table 5. Each heavy and light chain requires a leader to ensure proper post-translation processing and secretion (unless expressed as an scFv, in which only the N-terminal chain requires a leader sequence). Useful leader sequences for the expression of the heavy and light chains of the therapeutic antibodies in human cells are disclosed herein. An exemplary recombinant expression construct is shown in FIG. 1.

The production of HuPTMmAb or HuPTM Fab (including an HuPTM scFv) should result in a "biobetter" molecule for the treatment of disease accomplished via gene therapy - *e.g.,* by administering a viral vector or other DNA expression construct encoding a full-length or HuPTM Fab or other antigen binding fragment, such as an scFv, of a therapeutic mAb to a patient (human subject) diagnosed with a disease indication for that mAb, to create a permanent depot in the subject that continuously supplies the human-glycosylated, sulfated transgene product produced by the subject's transduced cells. The cDNA construct for the HuPTMmAb or HuPTM Fab or HuPTM scFv should include a signal peptide that ensures proper co- and post-translational processing (glycosylation and protein sulfation) by the transduced human cells.

Pharmaceutical compositions suitable for administration to human subjects comprise a suspension of the recombinant vector in a formulation buffer comprising a physiologically compatible aqueous buffer, a surfactant and optional excipients. Such formulation buffer can comprise one or more of a polysaccharide, a surfactant, polymer, or oil.

As an alternative, or an additional treatment to gene therapy, the full-length or HuPTM Fab or other antigen binding fragment thereof can be produced in human cell lines by recombinant DNA technology, and the glycoprotein can be administered to patients. Human cell lines that can be used for such recombinant glycoprotein production include but are not limited to human embryonic kidney 293 cells (HEK293), fibrosarcoma HT-1080, HKB-11, CAP, HuH-7, and retinal cell lines, PER.C6, or RPE to name a few (*e.g.,* see Dumont et al., 2015, Crit. Rev. Biotechnol. 36(6):1110-1122, which is incorporated by reference in its entirety for a review of the human cell lines that could be used for the recombinant production of the HuPTM Fab or HuPTM scFv product, *e.g*., HuPTM Fab glycoprotein). To ensure complete glycosylation, especially sialylation, and tyrosine-sulfation, the cell line used for production can be enhanced by engineering the host cells to co-express α-2,6-sialyltransferase (or both α-2,3- and α-2,6-sialyltransferases) and/or TPST-1 and TPST-2 enzymes responsible for tyrosine-O-sulfation in human cells.

It is not essential that every molecule produced either in the gene therapy or protein therapy approach be fully glycosylated and sulfated. Rather, the population of glycoproteins produced should have sufficient glycosylation (including 2,6-sialylation) and sulfation to demonstrate efficacy. The goal of gene therapy treatment of the invention is to slow or arrest the progression of disease.

Combination therapies involving delivery of the full-length or HuPTM Fab or antigen binding fragment thereof to the patient accompanied by administration of other available treatments are encompassed by the methods of the invention. The additional treatments may be administered before, concurrently or subsequent to the gene therapy treatment. Such additional treatments can include but are not limited to co-therapy with the therapeutic mAb.

Also provided are methods of manufacturing the viral vectors, particularly the AAV based viral vectors. In specific embodiments, provided are methods of producing recombinant AAVs comprising culturing a host cell containing an artificial genome comprising a cis expression cassette flanked by AAV ITRs, wherein the cis expression cassette comprises a transgene encoding a therapeutic antibody operably linked to expression control elements that will control expression of the transgene in human cells; a trans expression cassette lacking AAV ITRs, wherein the trans expression cassette encodes an AAV rep and capsid protein operably linked to expression control elements that drive expression of the AAV rep and capsid proteins in the host cell in culture and supply the rep and cap proteins in trans; sufficient adenovirus helper functions to permit replication and packaging of the artificial genome by the AAV capsid proteins; and recovering recombinant AAV encapsidating the artificial genome from the cell culture.

### 5.1 CONSTRUCTS

Viral vectors or other DNA expression constructs encoding an HuPTMmAb or antigen-binding fragment thereof, particularly a HuGlyFab, or a hyperglycosylated derivative of a HuPTMmAb antigen-binding fragment are provided herein. The viral vectors and other DNA expression constructs provided herein include any suitable method for delivery of a transgene to a target cell. The means of delivery of a transgene include viral vectors, liposomes, other lipid-containing complexes, other macromolecular complexes, synthetic modified mRNA, unmodified mRNA, small molecules, non-biologically active molecules (*e.g.,* gold particles), polymerized molecules (*e.g.,* dendrimers), naked DNA, plasmids, phages, transposons, cosmids, or episomes. In some embodiments, the vector is a targeted vector, *e.g.,* a vector targeted to retinal pigment epithelial cells, CNS cells, muscle cells, or liver cells.

In some aspects, the disclosure provides for a nucleic acid for use, wherein the nucleic acid comprises a nucleotide sequence that encodes a HuPTMmAb or HuGlyFab or other antigen-binding fragment thereof, as a transgene described herein, operatively linked to a promoter selected for expression in tissue targeted for expression of the transgene, for example, but not limited to the CB7 promoter (see FIG. 1), cytomegalovirus (CMV) promoter, Rous sarcoma virus (RSV) promoter, GFAP promoter (glial fibrillary acidic protein), MBP promoter (myelin basic protein), MMT promoter, EF-1 alpha promoter, UB6 promoter, chicken beta-actin promoter, CAG promoter, RPE65 promoter and opsin promoter, liver-specific promoters, such as TBG (Thyroxine-binding Globulin) promoter, APOA2 promoter, SERPINA1 (hAAT) promoter, or mIR122 promoter, or muscle-specific promoter, such as a human desmin promoter or Pitx3 promoter, inducible promoters, such as a hypoxia-inducible promoter or a rapamycin-inducible promoter.

In certain embodiments, provided herein are recombinant vectors that comprise one or more nucleic acids (*e.g.* polynucleotides). The nucleic acids may comprise DNA, RNA, or a combination of DNA and RNA. In certain embodiments, the DNA comprises one or more of the sequences selected from the group consisting of promoter sequences, the sequence of the gene of interest (the transgene, *e.g.,* the nucleotide sequences encoding the heavy and light chains of the HuPTMmAb or HuGlyFab or other antigen-binding fragment), untranslated regions, and termination sequences. In certain embodiments, viral vectors provided herein comprise a promoter operably linked to the gene of interest.

In certain embodiments, nucleic acids (*e.g.,* polynucleotides) and nucleic acid sequences disclosed herein may be codon-optimized, for example, via any codon-optimization technique known to one of skill in the art *(see, e.g.,* review by Quax et al., 2015, Mol Cell 59:149-161). Codon optimized nucleotide sequences for expression in human cells are provided herein for the heavy and light chains of the HuGlyFabs in Table 5.

In a specific embodiment, the constructs described herein comprise the following components: (1) AAV2 inverted terminal repeats that flank the expression cassette; (2) one or more control elements, b) a chicken β-actin intron and c) a rabbit β-globin poly A signal; and (3) nucleic acid sequences coding for the heavy and light chains of anti-VEGF antigen-binding fragment, separated by a self-cleaving furin (F)/F2A linker, ensuring expression of equal amounts of the heavy and the light chain polypeptides. An exemplary construct is shown in FIG. 1.

### 5.1.1 mRNA Vectors

In certain embodiments, as an alternative to DNA vectors, the vectors provided herein are modified mRNA encoding for the gene of interest (e.g., the transgene, for example, HuPTMmAb or HuGlyFab or other antigen binding fragment thereof). The synthesis of modified and unmodified mRNA for delivery of a transgene to retinal pigment epithelial cells is taught, for example, in Hansson et al., J. Biol. Chem., 2015, 290(9):5661-5672, which is incorporated by reference herein in its entirety. In certain embodiments, provided herein is a modified mRNA encoding for a HuPTMmAb or HuPTM Fab, or HuPTM scFv.

### 5.1.2 Viral vectors

Viral vectors include adenovirus, adeno-associated virus (AAV, *e.g.,* AAV8, AAV9, AAVrh10), lentivirus, helper-dependent adenovirus, herpes simplex virus, poxvirus, hemagglutinin virus of Japan (HVJ), alphavirus, vaccinia virus, and retrovirus vectors. Retroviral vectors include murine leukemia virus (MLV)- and human immunodeficiency virus (HIV)-based vectors. Alphavirus vectors include semliki forest virus (SFV) and sindbis virus (SIN). In certain embodiments, the viral vectors provided herein are recombinant viral vectors. In certain embodiments, the viral vectors provided herein are altered such that they are replication-deficient in humans. In certain embodiments, the viral vectors are hybrid vectors, *e.g.,* an AAV vector placed into a "helpless" adenoviral vector. In certain embodiments, provided herein are viral vectors comprising a viral capsid from a first virus and viral envelope proteins from a second virus. In specific embodiments, the second virus is vesicular stomatitus virus (VSV). In more specific embodiments, the envelope protein is VSV-G protein.

In certain embodiments, the viral vectors provided herein are HIV based viral vectors. In certain embodiments, HIV-based vectors provided herein comprise at least two polynucleotides, wherein the gag and pol genes are from an HIV genome and the env gene is from another virus.

In certain embodiments, the viral vectors provided herein are herpes simplex virus-based viral vectors. In certain embodiments, herpes simplex virus-based vectors provided herein are modified such that they do not comprise one or more immediately early (IE) genes, rendering them non-cytotoxic.

In certain embodiments, the viral vectors provided herein are MLV based viral vectors. In certain embodiments, MLV-based vectors provided herein comprise up to 8 kb of heterologous DNA in place of the viral genes.

In certain embodiments, the viral vectors provided herein are lentivirus-based viral vectors. In certain embodiments, lentiviral vectors provided herein are derived from human lentiviruses. In certain embodiments, lentiviral vectors provided herein are derived from non-human lentiviruses. In certain embodiments, lentiviral vectors provided herein are packaged into a lentiviral capsid. In certain embodiments, lentiviral vectors provided herein comprise one or more of the following elements: long terminal repeats, a primer binding site, a polypurine tract, att sites, and an encapsidation site.

In certain embodiments, the viral vectors provided herein are alphavirus-based viral vectors. In certain embodiments, alphavirus vectors provided herein are recombinant, replication-defective alphaviruses. In certain embodiments, alphavirus replicons in the alphavirus vectors provided herein are targeted to specific cell types by displaying a functional heterologous ligand on their virion surface.

In certain embodiments, the viral vectors provided herein are AAV based viral vectors. In certain embodiments, the AAV-based vectors provided herein do not encode the AAV rep gene (required for replication) and/or the AAV cap gene (required for synthesis of the capsid proteins) (the rep and cap proteins may be provided by the packaging cells in *trans*)*.* Multiple AAV serotypes have been identified. In certain embodiments, AAV-based vectors provided herein comprise components from one or more serotypes of AAV In certain embodiments, AAV based vectors provided herein comprise capsid components from one or more of AAV1, AAV2, AAV3, AAV4, AAV5, AAV6, AAV7, AAV8, AAV9, AAV10, AAV11, or AAVrh10. In preferred embodiments, AAV based vectors provided herein comprise components from one or more of AAV8, AAV9, AAV10, AAV11, or AAVrh10 serotypes. Provided are viral vectors in which the capsid protein is a variant the AAV8 capsid protein (SEQ ID NO: 78), AAV9 capsid protein (SEQ ID NO: 79), or AAVrh10 capsid protein (SEQ ID NO: 80), more particularly, is at least 95%, 96%, 97%, 98%, 99% or 99.9% identical to the amino acid sequence of the AAV8 capsid protein (SEQ ID NO: 78), AAV9 capsid protein (SEQ ID NO: 79), or AAVrh10 capsid protein (SEQ ID NO: 80), while retaining the biological function of the native capsid. In certain embodiments, the encoded AAV capsid has the sequence of SEQ ID NO: 78, 79 or 80 with 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid substitutions and retaining the biological function of the AAV8 AAV9 or AAVrh10 capsid. FIG. 12 provides a comparative alignment of the amino acid sequences of the capsid proteins of different AAV serotypes with potential amino acids that may be substituted at certain positions in the aligned sequences based upon the comparison in the row labeled SUBS. Accordingly, in specific embodiments, the AAV vector comprises an AAV8, AAV9 or AAVrh10 capsid variant that has 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid substitutions that are not present at that position in the native AAV capsid sequence as identified in the SUBS row of FIG. 12. Sequence for AAVrh10 is provided in Table 4.

In certain embodiments, the AAV that is used in the compositions and methods described herein is Anc80 or Anc80L65, as described in Zinn et al., 2015, Cell Rep. 12(6): 1056-1068, which is incorporated by reference in its entirety. In certain embodiments, the AAV that is used in the methods described herein comprises one of the following amino acid insertions: LGETTRP (SEQ ID NO: 162) or LALGETTRP (SEQ ID NO: 163), as described in United States Patent Nos. 9,193,956; 9458517; and 9,587,282 and US patent application publication no. 2016/0376323, each of which is incorporated herein by reference in its entirety. In certain embodiments, the AAV that is used in the methods described herein is AAV.7m8 (including variants), as described in United States Patent Nos. 9,193,956; 9,458,517; and 9,587,282, US patent application publication no. 2016/0376323, and International Publication WO 2018/075798, each of which is incorporated herein by reference in its entirety. In certain embodiments, the AAV that is used in the methods described herein is any AAV disclosed in United States Patent No. 9,585,971, such as AAV-PHP.B. In certain embodiments, the AAV used in the compositions and methods described herein is an AAV2/Rec2 or AAV2/Rec3 vector, which have hybrid capsid sequences derived from AAV8 capsids and capsids of serotypes cy5, rh20 or rh39 as described in Charbel Issa et al., 2013, PLoS One 8(4): e60361, which is incorporated by reference herein for these vectors. In certain embodiments, the AAV that is used in the methods described herein is an AAV disclosed in any of the following patents and patent applications, each of which is incorporated herein by reference in its entirety: United States Patent Nos. 7,906,111; 8,524,446; 8,999,678; 8,628,966; 8,927,514; 8,734,809; US 9,284,357; 9,409,953; 9,169,299; 9,193,956; 9458517; and 9,587,282 US patent application publication nos. 2015/0374803; 2015/0126588; 2017/0067908; 2013/0224836; 2016/0215024; 2017/0051257; and International Patent Application Nos. PCT/US2015/034799; PCT/EP2015/053335.

AAV8-based, AAV9-based, and AAVrh10-based viral vectors are used in certain of the methods described herein. Nucleotide sequences of AAV based viral vectors and methods of making recombinant AAV and AAV capsids are taught, for example, in United States Patent No. 7,282,199 B2, United States Patent No. 7,790,449 B2, United States Patent No. 8,318,480 B2, United States Patent No. 8,962,332 B2 and International Patent Application No. PCT/EP2014/076466, each of which is incorporated herein by reference in its entirety. In one aspect, provided herein are AAV (e.g., AAV8, AAV9 or AAVrh10)-based viral vectors encoding a transgene (e.g., an HuPTM Fab). The amino acid sequences of AAV capsids, including AAV8, AAV9 and AAVrh10 are provided in Figure 12 and Table 4.

In certain embodiments, a single-stranded AAV (ssAAV) may be used supra. In certain embodiments, a self-complementary vector, *e.g.,* scAAV, may be used *(see, e.g.,* Wu, 2007, Human Gene Therapy, 18(2):171-82, McCarty et al, 2001, Gene Therapy, Vol 8, Number 16, Pages 1248-1254; and U.S. Patent Nos. 6,596,535; 7,125,717; and 7,456,683, each of which is incorporated herein by reference in its entirety).

In certain embodiments, the viral vectors used in the methods described herein are adenovirus based viral vectors. A recombinant adenovirus vector may be used to transfer in the transgene encoding the HuPTMmAb or HuGlyFab or antigen-binding fragment. The recombinant adenovirus can be a first generation vector, with an E1 deletion, with or without an E3 deletion, and with the expression cassette inserted into either deleted region. The recombinant adenovirus can be a second generation vector, which contains full or partial deletions of the E2 and E4 regions. A helper-dependent adenovirus retains only the adenovirus inverted terminal repeats and the packaging signal (phi). The transgene is inserted between the packaging signal and the 3'ITR, with or without stuffer sequences to keep the genome close to wild-type size of approximately 36 kb. An exemplary protocol for production of adenoviral vectors may be found in Alba et al., 2005, "Gutless adenovirus: last generation adenovirus for gene therapy," Gene Therapy 12:S18-S27, which is incorporated by reference herein in its entirety.

In certain embodiments, the viral vectors used in the methods described herein are lentivirus based viral vectors. A recombinant lentivirus vector may be used to transfer in the transgene encoding the HuPTM mAb antigen binding fragment. Four plasmids are used to make the construct: Gag/pol sequence containing plasmid, Rev sequence containing plasmids, Envelope protein containing plasmid (*i.e.* VSV-G), and Cis plasmid with the packaging elements and the anti-VEGF antigen-binding fragment gene.

For lentiviral vector production, the four plasmids are co-transfected into cells (*i.e.,* HEK293 based cells), whereby polyethylenimine or calcium phosphate can be used as transfection agents, among others. The lentivirus is then harvested in the supernatant (lentiviruses need to bud from the cells to be active, so no cell harvest needs/should be done). The supernatant is filtered (0.45 µm) and then magnesium chloride and benzonase added. Further downstream processes can vary widely, with using TFF and column chromatography being the most GMP compatible ones. Others use ultracentrifugation with/without column chromatography. Exemplary protocols for production of lentiviral vectors may be found in Lesch et al., 2011, "Production and purification of lentiviral vector generated in 293T suspension cells with baculoviral vectors," Gene Therapy 18:531-538, and Ausubel et al., 2012, "Production of CGMP-Grade Lentiviral Vectors," Bioprocess Int. 10(2):32-43, both of which are incorporated by reference herein in their entireties.

In a specific embodiment, a vector for use in the methods described herein is one that encodes an HuPTM mAb antigen binding fragment, such as an HuGlyFab, such that, upon introduction of the vector into a relevant cell, a glycosylated and/or tyrosine sulfated variant of the HuPTM mAb antigen binding fragment or HuGlyFab is expressed by the cell.

### 5.1.3 Promoters and Modifiers of Gene Expression

In certain embodiments, the vectors provided herein comprise components that modulate gene delivery or gene expression (*e.g.,* "expression control elements"). In certain embodiments, the vectors provided herein comprise components that modulate gene expression. In certain embodiments, the vectors provided herein comprise components that influence binding or targeting to cells. In certain embodiments, the vectors provided herein comprise components that influence the localization of the polynucleotide (*e.g.,* the transgene) within the cell after uptake. In certain embodiments, the vectors provided herein comprise components that can be used as detectable or selectable markers, *e.g.,* to detect or select for cells that have taken up the polynucleotide.

In certain embodiments, the viral vectors provided herein comprise one or more promoters that control expression of the transgene. In certain embodiments, the promoter is a constitutive promoter. In certain embodiments, the promoter is a CB7 promoter (see Dinculescu et al., 2005, Hum Gene Ther 16: 649-663, incorporated by reference herein in its entirety). In some embodiments, the CB7 promoter includes other expression control elements that enhance expression of the transgene driven by the vector. In certain embodiments, the other expression control elements include chicken β-actin intron and/or rabbit β-globin polA signal. In certain embodiments, the promoter comprises a TATA box. In certain embodiments, the promoter comprises one or more elements. In certain embodiments, the one or more promoter elements may be inverted or moved relative to one another. In certain embodiments, the elements of the promoter are positioned to function cooperatively. In certain embodiments, the elements of the promoter are positioned to function independently. In certain embodiments, the viral vectors provided herein comprise one or more promoters selected from the group consisting of the human CMV immediate early gene promoter, the SV40 early promoter, the Rous sarcoma virus (RS) long terminal repeat, and rat insulin promoter. In certain embodiments, the vectors provided herein comprise one or more long terminal repeat (LTR) promoters selected from the group consisting of AAV, MLV, MMTV, SV40, RSV, HIV-1, and HIV-2 LTRs. In certain embodiments, the vectors provided herein comprise one or more tissue specific promoters (e.g., a retinal pigment epithelial cell-specific promoter, a CNS-specific promoter, a liver-specific promoter or muscle specific). In certain embodiments, the viral vectors provided herein comprise a RPE65 promoter or an opsin promoter (a retinal cell/CNS specific promoter). In certain embodiments, the viral vectors provided herein comprises a liver cell specific promoter, such as, a TBG (Thyroxine-binding Globulin) promoter, an APOA2 promoter, a SERPINA1 (hAAT) promoter, or a MIR122 promoter. In certain embodiments, the viral vector provided herein comprises a muscle specific promoter, such as a human desmin promoter (Jonuschies et al., 2014, Curr. Gene Ther. 14:276-288) or a Pitx3 promoter (Coulon et al., 2007, JBC 282:33192). In other embodiments, the viral vector comprises a VMD2 promoter.

In certain embodiments, the promoter is an inducible promoter. In certain embodiments the promoter is a hypoxia-inducible promoter. In certain embodiments, the promoter comprises a hypoxia-inducible factor (HIF) binding site. In certain embodiments, the promoter comprises a HIF-1α binding site. In certain embodiments, the promoter comprises a HIF-2α binding site. In certain embodiments, the HIF binding site comprises an RCGTG motif. For details regarding the location and sequence of HIF binding sites, *see, e.g.,* Schödel, et al., Blood, 2011, 117(23):e207-e217, which is incorporated by reference herein in its entirety. In certain embodiments, the promoter comprises a binding site for a hypoxia induced transcription factor other than a HIF transcription factor. In certain embodiments, the viral vectors provided herein comprise one or more IRES sites that is preferentially translated in hypoxia. For teachings regarding hypoxia-inducible gene expression and the factors involved therein, *see, e.g.,* Kenneth and Rocha, Biochem J., 2008, 414:19-29, which is incorporated by reference herein in its entirety. In specific embodiments, the hypoxia-inducible promoter is the human N-WASP promoter, see, for example, Salvi, 2017, Biochemistry and Biophysics Reports 9:13-21 (incorporated by reference for the teaching of the N-WASP promoter) or is the hypoxia-induced promoter of human Epo, see, Tsuchiya et al., 1993, J. Biochem. 113:395-400 (incorporated by reference for the disclosure of the Epo hypoxia-inducible promoter). In other embodiments, the promoter is a drug inducible promoter, for example, a promoter that is induced by administration of rapamycin or analogs thereof. See, for example, the disclosure of rapamycin inducible promoters in PCT publications WO94/18317, WO 96/20951, WO 96/41865, WO 99/10508, WO 99/10510, WO 99/36553, and WO 99/41258, and US 7,067,526, which are hereby incorporated by reference in their entireties for the disclosure of drug inducible promoters.

In certain embodiments, the viral vectors provided herein comprise one or more regulatory elements other than a promoter. In certain embodiments, the viral vectors provided herein comprise an enhancer. In certain embodiments, the viral vectors provided herein comprise a repressor. In certain embodiments, the viral vectors provided herein comprise an intron or a chimeric intron. In certain embodiments, the viral vectors provided herein comprise a polyadenylation sequence.

### 5.1.4 Signal Peptides

In certain embodiments, the vectors provided herein comprise components that modulate protein delivery. In certain embodiments, the viral vectors provided herein comprise one or more signal peptides. Signal peptides may also be referred to herein as "leader sequences" or "leader peptides". In certain embodiments, the signal peptides allow for the transgene product to achieve the proper packaging (*e.g.* glycosylation) in the cell. In certain embodiments, the signal peptides allow for the transgene product to achieve the proper localization in the cell. In certain embodiments, the signal peptides allow for the transgene product to achieve secretion from the cell.

There are two general approaches to select a signal sequence for protein production in a gene therapy context or in cell culture. One approach is to use a signal peptide from proteins homologous to the protein being expressed. For example, a human antibody signal peptide may be used to express IgGs in CHO or other cells. Another approach is to identify signal peptides optimized for the particular host cells used for expression. Signal peptides may be interchanged between different proteins or even between proteins of different organisms, but usually the signal sequences of the most abundant secreted proteins of that cell type are used for protein expression. For example, the signal peptide of human albumin, the most abundant protein in plasma, was found to substantially increase protein production yield in CHO cells. However, certain signal peptides may retain function and exert activity after being cleaved from the expressed protein as "post-targeting functions". Thus, in specific embodiments, the signal peptide is selected from signal peptides of the most abundant proteins secreted by the cells used for expression to avoid the post-targeting functions. In a preferred embodiment, the signal sequence is fused to both the heavy and light chain sequences. A preferred sequence is MYRMQLLLLIALSLALVTNS (SEQ ID NO: 161) (see FIGS. 2-9). Alternatively, signal sequences that are appropriate for expression of the HuPTM mAb or Fab in eye (including CNS), muscle, or liver are provided in Tables 1, 2 and 3, respectively, below.

**Table 1. Signal peptides for expression in eye/CNS tissue**

| **Signal Peptide Origin** | **SEQ ID NO:** | **Sequence** |
|---|---|---|
| VEGF-A signal peptide | 164 | MNFLLSWVHWSLALLLYLHHAKWSQA |
| Fibulin-1 signal peptide | 165 | MERAAPSRRVPLPLLLLGGLALLAAGVDA |
| Vitronectin signal peptide | 166 | MAPLRPLLILALLAWVALA |
| Complement Factor H signal peptide | 167 | MRLLAKIICLMLWAICVA |
| Opticin signal peptide | 168 | MRLLAFLSLLALVLQETGT |
| Albumin signal peptide | 169 | MKWVTFISLLFLFSSAYS |
| Chymotrypsinogen signal peptide | 170 | MAFLWLLSCWALLGTTFG |
| Interleukin-2 signal peptide | 171 | MYRMQLLSCIALILALVTNS |
| Trypsinogen-2 signal peptide | 172 | MNLLLILTFVAAAVA |

**Table 2. Signal peptides for expression in muscle cells.**

| **Signal Peptide Origin** | **SEQ ID NO:** | **Sequence** |
|---|---|---|
| Human SPARC | 173 | MRAWIFFLLCLAGRALA |
| Human Collagen alpha-1(I) chain | 174 | MFSFVDLRLLLLLAATALLTHG |
| Human Lactotransferrin | 175 | MKLVFLVLLFLGALGLCLA |
| Human Complement C3 | 176 | MGPTS GPSLLLLLLTHI,PLALG |
| Human Lumican | 177 | MSLSAFTLFLALIGGTSG |
| Human Gelsolin isoform 1 | 178 | MAPHRPAPALLCALSLALCALSLPVRA |
| Human Pro-cathepsin H | 179 | MWATLPLLCAGAWLLGVPVCGA |
| Human SERPINF 1 | 180 | MQALVLLLCIGALLGHSSC |
| Human SERPINE1 | 181 | MQMSPALTCLVLGLALVFGEGSA |
| Human Cathepsin D | 182 | MQPSSLLPLALCLLAAP ASA |
| Human TIMP1 | 183 | MAPFEPLASGILLLLWLIAPSRA |
| Human Fibronectin | 184 | MLRGPGPGLLLLA VQCLGTA VPSTGASKSKR |
| Human Complement C1s subcomponent | 185 | MWCIVLFSLLAWVYA |
| Human Cathepsin L1 | 186 | MNPTLILAAFCLGIASA |
| Human Cathepsin B | 187 | MWQLWASLCCLLVLANA |
| Human Salivary acidic proline-rich phosphoprotein 1/2 | 188 | MLLILLSVALLAFSSA |
| Human Follistatin-related protein 1 | 189 | MWKRWLALALALVAVAWVRA |

**Table 3. Signal peptides for expression in liver cells.**

| **Signal Peptide** | **SEQ ID NO:** | | **Sequence** |
|---|---|---|---|
| Human Serum albumin | 169 | MKWVTFISLLFLFSSAYS | |
| Human α-1 Antitrypsin (SERPINA1) | 190 | MPSSVSWGILLLAGLCCLVPVSLA | |
| Human Apolipoprotein A-1 | 191 | MKAAVLTLAVLFLTGSQA | |
| Human Apolipoprotein A-2 | 192 | MKLLAATVLLLTICSLEG | |
| Human Apolipoprotein B-100 | 193 | MDPPRPALLALLALPALLLLLLAGARA | |
| Human Coagulation Factor IX | 194 | MQRVNMIMAESPGLITICLLGYLLSAEC | |
| Human Complement C2 | 195 | MGPLMVLFCLLFLYPGLADS | |
| Human Complement Factor H-related Protein 2 (CFHR2) | 196 | MWLLVSVILISRISSVGG | |
| Human Complement Factor H-related Protein 5 (CFHRS) | 197 | MLLLFSVILISWVSTVGG | |
| Human Fibrinogen α-chain (FGA) | 198 | MFSMRIVCLVLSWGTAWT | |
| Human Fibrinogen β-chain (FGB) | 199 | MKRMVSWSFHKLKTMKHLLLLLLCVFLVKS | |
| Human Fibrinogen γ-chain (FGG) | 200 | MSWSLHPRNLILYFYALLFLSSTCVA | |
| Human α-2-HS-Glycoprotein (AHSG) | 201 | MKSLVLLLCLAQLWGCHS | |
| Human Hemopexin (HPX) | 202 | MARVLGAPVALGLWSLCWSLAIA | |
| Human Kininogen-1 | 203 | MKLITILFLCSRLLLSLT | |
| Human Mannose-binding protein C (MBL2) | 204 | MSLFPSLPLLLLSMVAASYS | |
| Human Plasminogen (PLMN) | 205 | MEHKEWLLLLLFLKSGQG | |
| Human Prothrombin (Coagulation Factor II) | 206 | MAHVRGLQLPGCLALAALCSLVHS | |
| Human Secreted Phosphoprotein 24 | 207 | MISRMEKMTMMMKILIMF ALGMNYWSCSG | |
| Human Anti-thrombin-III (SERPINC1) | 208 | MYSNVIGTVTS GKRKVYLLSLLLIGFWDCVTC | |
| Human Serotransferrin (TF) | 209 | MRI,AVGALLVCAVLGLCLA | |

### 5.1.5 Polycistronic Messages - IRES and F2A linkers and scFv Constructs

*Internal ribosome entry sites.* A single construct can be engineered to encode both the heavy and light chains separated by a cleavable linker or IRES so that separate heavy and light chain polypeptides are expressed by the transduced cells. In certain embodiments, the viral vectors provided herein provide polycistronic (*e.g.,* bicistronic) messages. For example, the viral construct can encode the heavy and light chains separated by an internal ribosome entry site (IRES) elements (for examples of the use of IRES elements to create bicistronic vectors *see, e.g.,* Gurtu et al., 1996, Biochem. Biophys. Res. Comm. 229(1):295-8, which is herein incorporated by reference in its entirety). IRES elements bypass the ribosome scanning model and begin translation at internal sites. The use of IRES in AAV is described, for example, in Furling et al., 2001, Gene Ther 8(11): 854-73, which is herein incorporated by reference in its entirety. In certain embodiments, the bicistronic message is contained within a viral vector with a restraint on the size of the polynucleotide(s) therein. In certain embodiments, the bicistronic message is contained within an AAV virus-based vector (*e.g.,* an AAV8-based, AAV9-based or AAVrh10-based vector).

*Furin-F2A linkers.* In other embodiments, the viral vectors provided herein encode the heavy and light chains separated by a cleavable linker such as the self-cleaving furin/F2A (F/F2A) linkers (Fang et al., 2005, Nature Biotechnology 23: 584-590, and Fang, 2007, Mol Ther 15: 1153-9, each of which is incorporated by reference herein in its entirety). For example, a furin-F2A linker may be incorporated into an expression cassette to separate the heavy and light chain coding sequences, resulting in a construct with the structure:
Leader - Heavy chain - Furin site - F2A site - Leader - Light chain - Poly A.
The F2A site, with the amino acid sequence LLNFDLLKLAGDVESNPGP (SEQ ID NO: 210) is self-processing, resulting in "cleavage" between the final G and P amino acid residues. Additional linkers that could be used include but are not limited to:
T2A:(GSG) EGRGSLLTCGDVEENP**GP**(SEQ ID NO: 211);
P2A: (GSG)ATNFSLLKQAGDVEENP**GP**(SEQ ID NO: 212);
E2A: (GSG)QCTNYALLKLAGDVESNP**GP**(SEQID NO: 213);
F2A: (GSG)VKQTLNFDLLKLAGDVESNP**GP**(SEQ ID NO: 214).

A peptide bond is skipped when the ribosome encounters the F2A sequence in the open reading frame, resulting in the termination of translation, or continued translation of the downstream sequence (the light chain). This self-processing sequence results in a string of additional amino acids at the end of the C-terminus of the heavy chain. However, such additional amino acids are then cleaved by host cell Furin at the furin sites, located immediately prior to the F2A site and after the heavy chain sequence, and further cleaved by carboxypeptidases. The resultant heavy chain may have one, two, three, or more additional amino acids included at the C-terminus, or it may not have such additional amino acids, depending on the sequence of the Furin linker used and the carboxypeptidase that cleaves the linker in vivo (*See, e.g.,* Fang et al., 17 April 2005, Nature Biotechnol. Advance Online Publication; Fang et al., 2007, Molecular Therapy 15(6):1153-1159; Luke, 2012, Innovations in Biotechnology, Ch. 8, 161-186). Furin linkers that may be used comprise a series of four basic amino acids, for example, RKRR (SEQ ID NO: 215), RRRR (SEQ ID NO: 216), RRKR (SEQ ID NO: 217), or RKKR (SEQ ID NO: 218). Once this linker is cleaved by a carboxypeptidase, additional amino acids may remain, such that an additional zero, one, two, three or four amino acids may remain on the C-terminus of the heavy chain, for example, R, RR, RK, RKR, RRR, RRK, RKK, RKRR (SEQ ID NO: 215), RRRR (SEQ ID NO: 216), RRKR (SEQ ID NO: 217), or RKKR (SEQ ID NO: 218). In certain embodiments, one the linker is cleaved by a carboxypeptidase, no additional amino acids remain. In certain embodiments, 0.5% to 1%, 1% to 2%, 5%, 10%, 15%, or 20% of the antibody, *e.g.,* antigen-binding fragment, population produced by the constructs for use in the methods described herein has one, two, three, or four amino acids remaining on the C-terminus of the heavy chain after cleavage. In certain embodiments, the furin linker has the sequence R-X-K/R-R, such that the additional amino acids on the C-terminus of the heavy chain are R, RX, RXK, RXR, RXKR, or RXRR, where X is any amino acid, for example, alanine (A). In certain embodiments, no additional amino acids may remain on the C-terminus of the heavy chain.

*Flexible peptide linker.* In some embodiments, a single construct can be engineered to encode both the heavy and light chains (preferably the heavy and light chain variable domains) separated by a flexible peptide linker such as those encoding a scFv. A flexible peptide linker can be composed of flexible residues like glycine and serine so that the adjacent heavy chain and light chain domains are free to move relative to one another. The construct may be arranged such that the heavy chain variable domain is at the N-terminus of the scFv, followed by the linker and then the light chain variable domain. Alternatively, the construct may be arranged such that the light chain variable domain is at the N-terminus of the scFv, followed by the linker and then the heavy chain variable domain. That is, the components may be arranged as NH₂-V_{L}-linker-V_{H}-COOH or NH₂-V_{H}-linker-V_{L}-COOH.

In certain embodiments, an expression cassette described herein is contained within a viral vector with a restraint on the size of the polynucleotide(s) therein. In certain embodiments, the expression cassette is contained within an AAV virus-based vector. Due to the size restraints of certain vectors, the vector may or may not accommodate the coding sequences for the full heavy and light chains of the therapeutic antibody but may accommodate the coding sequences of the heavy and light chains of antigen binding fragments, such as the heavy and light chains of a Fab or F(ab')₂ fragment or an scFv. In particular, the AAV vectors described herein may accommodate a transgene of approximately 4.7 kilobases. For constructs such as that in FIG. 1 that contains the CB7 promoter, the chicken β-actin intron, rabbit β-globin polyA signal, and ITRs, the therapeutic antibody encoded may be approximately 752 amino acids. Substitution of smaller expression elements would permit the expression of larger protein products, such as full length therapeutic antibodies.

### 5.1.6 Untranslated regions

In certain embodiments, the viral vectors provided herein comprise one or more untranslated regions (UTRs), e*.g.*, 3' and/or 5' UTRs. In certain embodiments, the UTRs are optimized for the desired level of protein expression. In certain embodiments, the UTRs are optimized for the mRNA half-life of the transgene. In certain embodiments, the UTRs are optimized for the stability of the mRNA of the transgene. In certain embodiments, the UTRs are optimized for the secondary structure of the mRNA of the transgene.

### 5.1.7 Inverted terminal repeats

In certain embodiments, the viral vectors provided herein comprise one or more inverted terminal repeat (ITR) sequences. ITR sequences may be used for packaging the recombinant gene expression cassette into the virion of the viral vector. In certain embodiments, the ITR is from an AAV, *e.g.,* AAV8 or AAV2 *(see, e.g.,* Yan et al., 2005, J. Virol., 79(1):364-379; United States Patent No. 7,282,199 B2, United States Patent No. 7,790,449 B2, United States Patent No. 8,318,480 B2, United States Patent No. 8,962,332 B2 and International Patent Application No. PCT/EP2014/076466, each of which is incorporated herein by reference in its entirety).

In certain embodiments, the modified ITRs used to produce self-complementary vector, *e.g.,* scAAV, may be used *(see, e.g.,* Wu, 2007, Human Gene Therapy, 18(2):171-82, McCarty et al, 2001, Gene Therapy, Vol 8, Number 16, Pages 1248-1254; and U.S. Patent Nos. 6,596,535; 7,125,717; and 7,456,683, each of which is incorporated herein by reference in its entirety).

### 5.1.8 Transgenes

The transgenes encode a HuPTM mAb, either as a full length antibody or an antigen binding fragment thereof, preferably a Fab fragment (an HuGlyFab) or a F(ab')₂ or an scFv based upon a therapeutic antibody disclosed herein. In specific embodiments, the HuPTM mAb or antigen binding fragment, particularly the HuGlyFab, are engineered to contain additional glycosylation sites on the Fab domain (*e.g.,* see Courtois et al., 2016, mAbs 8: 99-112 which is incorporated by reference herein in its entirety for it description of sites of hyperglycosylation on a Fab domain). FIG. 11 provides alignments of the Fab heavy and light chains of the therapeutic antibodies disclosed herein and highlights in green residues that may be substituted with an asparagine or, in some instances, a serine, resulting in hyperglycosylation.

In certain embodiments, the transgenes encode either a full-length antibody or an antigen binding fragment thereof with the coding sequence of the heavy and light chains. When using a full-length antibody, a construct encoding a modified mAb may be used. For example, the C-terminal lysines (-K) conserved in the heavy chain genes of all human IgG subclases are generally absent from antibodies circulating in serum - the C-terminal lysines are cleaved off in circulation, resulting in a heterogenous population of circulating IgGs. (van den Bremer et al., 2015, mAbs 7:672-680). In the vectored constructs for full length mAbs, the DNA encoding the C-terminal lysine (-K) or glycine-lysine (-GK) of the Fc terminus can be deleted to produce a more homogeneous antibody product *in situ.* (See, Hu et al., 2017 Biotechnol. Prog. 33: 786-794 which is incorporated by reference herin in its entirety).

Alternatively, antigen binding fragments are advantageously used. FIGS. 2A-2F, 3A-3E, 4A-4B, 5A-5D, 6, 7A, 7B, 8A-8H and 9A-9B provide the amino acid sequences of the heavy and light chains of the Fab fragments and scFv of the therapeutic antibodies (see also Table 4, which provides the amino acid sequences of the heavy and light chains of the therapeutic antibodies). The transgene may comprise the nucleotide sequences encoding the heavy and light chain sequences using nucleotide sequences that encode the Fab portion of the heavy chain plus the constant domain portion of the heavy chain for the appropriate isotype as described further herein and the light chain. Nucleotide sequences that are codon optimized for expression in human cells encoding the Fab fragment portions of the heavy and light chains of the therapeutic antibodies disclosed herein are provided in Table 5. The transgene may encode an Fab fragment using nucleotide sequences encoding the sequences provided in FIGS. 2A-2F, 3A-3E, 4A-4B, 5A-5C, 6, 7A, 7B, 8A-8H and 9A-9B but not including the portion of the hinge region on the heavy chain that forms interchain di-sulfide bonds (i.e., the portion containing the sequence CPPCPA (SEQ ID NO: 219)). Heavy chain variable domain sequences that do not contain a CPPCP (SEQ ID NO: 220) sequence of the hinge region at the C-terminus will not form intrachain disulfide bonds and, thus, will form Fab fragments with the corresponding light chain variable domain sequences, whereas those heavy chain variable domain sequences with a portion of the hinge region at the C-terminus containing the sequence CPPCP (SEQ ID NO: 220) will form intrachain disulfide bonds and, thus, will form Fab₂ fragments. For example, in some embodiments, the transgene may encode a scFv comprising a light chain variable domain and a heavy chain variable domain connected by a flexible linker in between (where the heavy chain variable domain may be either at the N-terminal end or the C-terminal end of the scFv), for example, as depicted for brolucizumab in FIG. 8D and E06 in FIG. 5D. Alternatively, in other embodiments, the transgene may encode F(ab')₂ fragments comprising a nucleotide sequence that encodes the light chain and the heavy chain sequence that includes at least the sequence CPPCA (SEQ ID NO: 221) of the hinge region, as depicted in FIGS. 2A-2F, 3A-3E, 4A-4B, 5A-5C, 6, 7A, 7B, 8A-8C, 8E-8H and 9A-9B which depict various regions of the hinge region that may be included at the C-terminus of the heavy chain sequence. Pre-existing anti-hinge antibodies (AHA) may cause immunogenicity and reduce efficacy. Thus, in certain embodiments, for the IgG1 isotype, C-terminal ends with D221 or ends with a mutation T225L or with L242 can reduce binding to AHA. (See, e.g., Brezski, 2008, J Immunol 181: 3183-92 and Kim, 2016, 8: 1536-1547). For IgG2, the risk of AHA is lower since the hinge region of IgG2 is not as susceptible to enzymatic cleavage required to generate endogenous AHA. (*See, e.g.,* Brezski, 2011, MAbs 3: 558-567).

In certain embodiments, the viral vectors provided herein comprise the following elements in the following order: a) a constitutive or inducible (e.g., hypoxia-inducible or rifamycin-inducible) promoter sequence, and b) a sequence encoding the transgene (e.g., a HuGlyFab). In certain embodiments, the sequence encoding the transgene comprises multiple ORFs separated by IRES elements. In certain embodiments, the ORFs encode the heavy and light chain domains of the HuGlyFab. In certain embodiments, the sequence encoding the transgene comprises multiple subunits in one ORF separated by F/F2A sequences. In certain embodiments, the sequence comprising the transgene encodes the heavy and light chain domains of the HuGlyFab separated by an F/F2A sequence. In certain embodiments, the sequence comprising the transgene encodes the heavy and light chain variable domains of the HuGlyFab separated by a flexible peptide linker. In certain embodiments, the viral vectors provided herein comprise the following elements in the following order: a) a constitutive or a an inducible promoter sequence, and b) a sequence encoding the transgene *(e.g.,* a HuGlyFab), wherein the transgene comprises a nucleotide sequence encoding a signal peptide, a light chain and a heavy chain Fab portion separated by an IRES element. In certain embodiments, the viral vectors provided herein comprise the following elements in the following order: a) a constitutive or a hypoxia-inducible promoter sequence, and b) a sequence encoding the transgene comprising a signal peptide, a light chain and a heavy chain sequence separated by a cleavable F/F2A sequence or a flexible peptide linker.

In certain embodiments, the viral vectors provided herein comprise the following elements in the following order: a) a first ITR sequence, b) a first linker sequence, c) a constitutive or an inducible promoter sequence, d) a second linker sequence, e) an intron sequence, f) a third linker sequence, g) a first UTR sequence, h) a sequence encoding the transgene (*e.g.,* a HuGlyFab), i) a second UTR sequence, j) a fourth linker sequence, k) a poly A sequence, l) a fifth linker sequence, and m) a second ITR sequence.

In certain embodiments, the viral vectors provided herein comprise the following elements in the following order: a) a first ITR sequence, b) a first linker sequence, c) a constitutive or a an inducible promoter sequence, d) a second linker sequence, e) an intron sequence, f) a third linker sequence, g) a first UTR sequence, h) a sequence encoding the transgene (*e.g.,* HuGlyFab), i) a second UTR sequence, j) a fourth linker sequence, k) a poly A sequence, l) a fifth linker sequence, and m) a second ITR sequence, wherein the transgene comprises a signal, and wherein the transgene encodes a light chain and a heavy chain sequence separated by a cleavable F/F2A sequence.

### 5.1.9 Manufacture and testing of vectors

The viral vectors provided herein may be manufactured using host cells. The viral vectors provided herein may be manufactured using mammalian host cells, for example, A549, WEHI, 10T1/2, BHK, MDCK, COS1, COS7, BSC 1, BSC 40, BMT 10, VERO, W138, HeLa, 293, Saos, C2C12, L, HT1080, HepG2, primary fibroblast, hepatocyte, and myoblast cells. The viral vectors provided herein may be manufactured using host cells from human, monkey, mouse, rat, rabbit, or hamster.

The host cells are stably transformed with the sequences encoding the transgene and associated elements (*i.e.,* the vector genome), and the means of producing viruses in the host cells, for example, the replication and capsid genes *(e.g.,* the rep and cap genes of AAV). For a method of producing recombinant AAV vectors with AAV8 capsids, see Section IV of the Detailed Description of U.S. Patent No. 7,282,199 B2, which is incorporated herein by reference in its entirety. Genome copy titers of said vectors may be determined, for example, by TAQMAN^{®} analysis. Virions may be recovered, for example, by CsCl₂ sedimentation.

Alternatively, baculovirus expression systems in insect cells may be used to produce AAV vectors. For a review, see Aponte-Ubillus et al., 2018, Appl. Microbiol. Biotechnol. 102:1045-1054 which is incorporated by reference herein in its entirety for manufacturing techniques.

In vitro assays, *e.g.,* cell culture assays, can be used to measure transgene expression from a vector described herein, thus indicating, *e.g.,* potency of the vector. For example, the PER.C6^{®} Cell Line (Lonza), a cell line derived from human embryonic retinal cells, or retinal pigment epithelial cells, *e.g.,* the retinal pigment epithelial cell line hTERT RPE-1 (available from ATCC^{®}), can be used to assess transgene expression. Once expressed, characteristics of the expressed product can be determined, including determination of the glycosylation and tyrosine sulfation patterns associated with the HuGlyFab. Glycosylation patterns and methods of determining the same are discussed in Section 5.2.1, while tyrosine sulfation patterns and methods of determining the same are discussed in Section 5.2.2. In addition, benefits resulting from glycosylation/sulfation of the cell-expressed HuGlyFab can be determined using assays known in the art, *e.g.,* the methods described in Sections 5.2.1 and 5.2.2.

### 5.1.10 Compositions

Pharmaceutical compositions suitable for administration to human subjects comprise a suspension of the recombinant vector in a formulation buffer comprising a physiologically compatible aqueous buffer, a surfactant and optional excipients. Such formulation buffer can comprise one or more of a polysaccharide, a surfactant, polymer, or oil.

### 5.2 N-GLYCOSYLATION, TYROSINE SULFATION, AND O-GLYCOSYLATION

The amino acid sequence (primary sequence) of HuGlyFabs and HuPTM scFvs disclosed herein each comprises at least one site at which N-glycosylation or tyrosine sulfation takes place (see FIGS. 2A-2F, 3A-3E, 4A-4B, 5A-5D, 6, 7A-7B, 8A-8H and 9A-9B for glycosylation and/or sulfation positions within the amino acid sequences of the Fab fragments of the therapeutic antibodies).

### 5.2.1 N-Glycosylation

### Reverse Glycosylation Sites

The canonical N-glycosylation sequence is known in the art to be Asn-X-Ser(or Thr), wherein X can be any amino acid except Pro. However, it recently has been demonstrated that asparagine (Asn) residues of human antibodies can be glycosylated in the context of a reverse consensus motif, Ser(or Thr)-X-Asn, wherein X can be any amino acid except Pro. See Valliere-Douglass et al., 2009, J. Biol. Chem. 284:32493-32506; and Valliere-Douglass et al., 2010, J. Biol. Chem. 285:16012-16022. As disclosed herein, certain HuGlyFabs and HuPTM scFvs disclosed herein comprise such reverse consensus sequences.

### Non-Consensus Glycosylation Sites

In addition to reverse N-glycosylation sites, it recently has been demonstrated that glutamine (Gln) residues of human antibodies can be glycosylated in the context of a non-consensus motif, Gln-Gly-Thr. See Valliere-Douglass et al., 2010, J. Biol. Chem. 285:16012-16022. Surprisingly, certain of the HuGlyFab fragments disclosed herein comprise such non-consensus sequences. In addition, O-glycosylation comprises the addition of N-acetyl-galactosamine to serine or threonine residues by the enzyme. It has been demonstrated that amino acid residues present in the hinge region of antibodies can be O-glycosylated. The possibility of O-glycosylation confers another advantage to the therapeutic antibodies provided herein, as compared to, *e.g.,* antigen-binding fragments produced in *E. coli,* again because the *E. coli* naturally does not contain machinery equivalent to that used in human O-glycosylation. (Instead, O-glycosylation in *E. coli* has been demonstrated only when the bacteria is modified to contain specific O-glycosylation machinery. See, e.g., Farid-Moayer et al., 2007, J. Bacteriol. 189:8088-8098.)

### Engineered N-Glycosylation Sites

In certain embodiments, a nucleic acid encoding a HuGlyFab or HuTPM scFv is modified to include 1, 2, 3, 4, 5, 6, 7, 8, 9, 10, or more N-glycosylation sites (including the canonical N-glycosylation consensus sequence, reverse N-glycosylation site, and non-consensus N-glycosylation sites) than would normally be associated with the HuGlyFab or HuPTM scFv (*e.g.,* relative to the number of N-glycosylation sites associated with the HuGlyFab or HuPTM scFv in its unmodified state). In specific embodiments, introduction of glycosylation sites is accomplished by insertion of N-glycosylation sites (including the canonical N-glycosylation consensus sequence, reverse N-glycosylation site, and non-consensus N-glycosylation sites) anywhere in the primary structure of the antigen-binding fragment, so long as said introduction does not impact binding of the antigen-binding fragment to its antigen. Introduction of glycosylation sites can be accomplished by, *e.g.,* adding new amino acids to the primary structure of the antigen-binding fragment, or the antibody from which the antigen-binding fragment is derived (*i.e.,* the glycosylation sites are added, in full or in part), or by mutating existing amino acids in the antigen-binding fragment, or the antibody from which the antigen-binding fragment is derived, in order to generate the N-glycosylation sites (*i.e.,* amino acids are not added to the antigen-binding fragment/antibody, but selected amino acids of the antigen-binding fragment/antibody are mutated so as to form N-glycosylation sites). Those of skill in the art will recognize that the amino acid sequence of a protein can be readily modified using approaches known in the art, *e.g.,* recombinant approaches that include modification of the nucleic acid sequence encoding the protein.

In a specific embodiment, a HuGlyMab or antigen-binding fragment is modified such that, when expressed in mammalian cells, such as retina, CNS, liver or muscle cells, it can be hyperglycosylated. See Courtois et al., 2016, mAbs 8:99-112 which is incorporated by reference herein in its entirety.

### N-Glycosylation of HuPTM antigen-binding fragments

Unlike small molecule drugs, biologics usually comprise a mixture of many variants with different modifications or forms that could have a different potency, pharmacokinetics, and/or safety profile. It is not essential that every molecule produced either in the gene therapy or protein therapy approach be fully glycosylated and sulfated. Rather, the population of glycoproteins produced should have sufficient glycosylation (including 2,6-sialylation) and sulfation to demonstrate efficacy. The goal of gene therapy treatment provided herein can be, for example, to slow or arrest the progression of a disease or abnormal condition or to reduce the severity of one or more symptoms associated with the disease or abnormal condition.

When a HuGlyFab or HuPTM scFv is expressed in a human cell, the N-glycosylation sites of the antigen-binding fragment can be glycosylated with various different glycans. N-glycans of antigen-binding fragments have been characterized in the art. For example, Bondt et al., 2014, Mol. & Cell. Proteomics 13.11:3029-3039 (incorporated by reference herein in its entirety for its disclosure of Fab-associated N-glycans; see also, FIG. 10) characterizes glycans associated with Fabs, and demonstrates that Fab and Fc portions of antibodies comprise distinct glycosylation patterns, with Fab glycans being high in galactosylation, sialylation, and bisection (*e.g.,* with bisecting GlcNAc) but low in fucosylation with respect to Fc glycans. Like Bondt, Huang et al., 2006, Anal. Biochem. 349:197-207 (incorporated by reference herein in its entirety for it disclosure of Fab-associated N-glycans) found that most glycans of Fabs are sialylated. However, in the Fab of the antibody examined by Huang (which was produced in a murine cell background), the identified sialic residues were N-Glycolylneuraminic acid ("Neu5Gc" or "NeuGc") (which is not natural to humans) instead of N-acetylneuraminic acid ("Neu5Ac," the predominant human sialic acid). In addition, Song et al., 2014, Anal. Chem. 86:5661-5666 (incorporated by reference herein in its entirety for it disclosure of Fab-associated N-glycans) describes a library of N-glycans associated with commercially available antibodies.

Importantly, when the HuGlyFab or HuPTM scFv are expressed in human cells, the need for *in vitro* production in prokaryotic host cells (*e.g., E. coli*) or eukaryotic host cells *(e.g.,* CHO cells or NS0 cells) is circumvented. Instead, as a result of the methods described herein, N-glycosylation sites of the HuGlyFab or HuPTM scFv are advantageously decorated with glycans relevant to and beneficial to treatment of humans. Such an advantage is unattainable when CHO cells, NS0 cells, or *E. coli* are utilized in antibody/antigen-binding fragment production, because *e.g.,* CHO cells (1) do not express 2,6 sialyltransferase and thus cannot add 2,6 sialic acid during N-glycosylation; (2) can add Neu5Gc as sialic acid instead of Neu5Ac; and (3) can also produce an immunogenic glycan, the α-Gal antigen, which reacts with anti-α-Gal antibodies present in most individuals, which at high concentrations can trigger anaphylaxis; and because (4) *E. coli* does not naturally contain components needed for N-glycosylation.

Assays for determining the glycosylation pattern of antibodies, including antigen-binding fragments are known in the art. For example, hydrazinolysis can be used to analyze glycans. First, polysaccharides are released from their associated protein by incubation with hydrazine (the Ludger Liberate Hydrazinolysis Glycan Release Kit, Oxfordshire, UK can be used). The nucleophile hydrazine attacks the glycosidic bond between the polysaccharide and the carrier protein and allows release of the attached glycans. N-acetyl groups are lost during this treatment and have to be reconstituted by re-N-acetylation. Glycans may also be released using enzymes such as glycosidases or endoglycosidases, such as PNGase F and Endo H, which cleave cleanly and with fewer side reactions than hydrazines. The free glycans can be purified on carbon columns and subsequently labeled at the reducing end with the fluorophor 2-amino benzamide. The labeled polysaccharides can be separated on a GlycoSep-N column (GL Sciences) according to the HPLC protocol of Royle et al, Anal Biochem 2002, 304(1):70-90. The resulting fluorescence chromatogram indicates the polysaccharide length and number of repeating units. Structural information can be gathered by collecting individual peaks and subsequently performing MS/MS analysis. Thereby the monosaccharide composition and sequence of the repeating unit can be confirmed and additionally in homogeneity of the polysaccharide composition can be identified. Specific peaks of low or high molecular weight can be analyzed by MALDI-MS/MS and the result used to confirm the glycan sequence. Each peak in the chromatogram corresponds to a polymer, *e.g.,* glycan, consisting of a certain number of repeat units and fragments, *e.g.,* sugar residues, thereof. The chromatogram thus allows measurement of the polymer, *e.g.,* glycan, length distribution. The elution time is an indication for polymer length, while fluorescence intensity correlates with molar abundance for the respective polymer, *e.g.,* glycan. Other methods for assessing glycans associated with antigen-binding fragments include those described by Bondt et al., 2014, Mol. & Cell. Proteomics 13.11:3029-3039, Huang et al., 2006, Anal. Biochem. 349:197-207, and/or Song et al., 2014, Anal. Chem. 86:5661-5666.

Homogeneity or heterogeneity of the glycan patterns associated with antibodies (including antigen-binding fragments), as it relates to both glycan length or size and numbers glycans present across glycosylation sites, can be assessed using methods known in the art, *e.g.,* methods that measure glycan length or size and hydrodynamic radius. HPLC, such as size exclusion, normal phase, reversed phase, and anion exchange HPLC, as well as capillary electrophoresis, allows the measurement of the hydrodynamic radius. Higher numbers of glycosylation sites in a protein lead to higher variation in hydrodynamic radius compared to a carrier with less glycosylation sites. However, when single glycan chains are analyzed, they may be more homogenous due to the more controlled length. Glycan length can be measured by hydrazinolysis, SDS PAGE, and capillary gel electrophoresis. In addition, homogeneity can also mean that certain glycosylation site usage patterns change to a broader/narrower range. These factors can be measured by Glycopeptide LC-MS/MS.

In certain embodiments, the HuPTM mAbs, or antigen binding fragments thereof, also do not contain detectable NeuGc and/or α-Gal. By "detectable NeuGc" or "detectable α-Gal" or "does not contain or does not have NeuGc or α-Gal" means herein that the HuPTM mAb or antigen-binding fragment, does not contain NeuGc or α-Gal moieties detectable by standard assay methods known in the art. For example, NeuGc may be detected by HPLC according to Hara et al., 1989, "Highly Sensitive Determination of A-Acetyl-and N-Glycolylneuraminic Acids in Human Serum and Urine and Rat Serum by Reversed-Phase Liquid Chromatography with Fluorescence Detection." J. Chromatogr., B: Biomed. 377, 111-119, which is hereby incorporated by reference for the method of detecting NeuGc. Alternatively, NeuGc may be detected by mass spectrometry. The α-Gal may be detected using an ELISA, see, for example, Galili et al., 1998, "A sensitive assay for measuring α-Gal epitope expression on cells by a monoclonal anti-Gal antibody." Transplantation. 65(8): 1129-32, or by mass spectrometry, see, for example, Ayoub et al., 2013, "Correct primary structure assessment and extensive glyco-profiling of cetuximab by a combination of intact, middle-up, middle-down and bottom-up ESI and MALDI mass spectrometry techniques." Landes Bioscience. 5(5):699-710. See also the references cited in Platts-Mills et al., 2015, "Anaphylaxis to the Carbohydrate Side-Chain Alpha-gal" Immunol Allergy Clin North Am. 35(2): 247-260.

### Benefits of N-Glycosylation

N-glycosylation confers numerous benefits on the HuGlyFab or HuPTM scFv described herein. Such benefits are unattainable by production of antigen-binding fragments in *E. coli,* because *E. coli* does not naturally possess components needed for N-glycosylation. Further, some benefits are unattainable through antibody production in, *e.g.,* CHO cells (or murine cells such as NS0 cells), because CHO cells lack components needed for addition of certain glycans (*e.g.,* 2,6 sialic acid and bisecting GlcNAc) and because either CHO or murine cell lines add N-N-Glycolylneuraminic acid ("Neu5Gc" or "NeuGc") which is not natural to humans (and potentially immunogenic), instead of N-Acetylneuraminic acid ("Neu5Ac") the predominant human sialic acid. See, e.g., Dumont et al., 2015, Crit. Rev. Biotechnol. 36(6):1110-1122; Huang et al., 2006, Anal. Biochem. 349:197-207 (NeuGc is the predominant sialic acid in murine cell lines such as SP2/0 and NS0); and Song et al., 2014, Anal. Chem. 86:5661-5666, each of which is incorporated by reference herein in its entirety). Moreover, CHO cells can also produce an immunogenic glycan, the α-Gal antigen, which reacts with anti-α-Gal antibodies present in most individuals, which at high concentrations can trigger anaphylaxis. *See, e.g.,* Bosques, 2010, Nat. Biotech. 28:1153-1156. The human glycosylation pattern of the HuGlyFab of HuPTM scFv described herein should reduce immunogenicity of the transgene product and improve efficacy.

While non-canonical glycosylation sites usually result in low level glycosylation (*e.g.,* 1-5%) of the antibody population, the functional benefits may be significant (*See, e.g.,* van de Bovenkamp et al., 2016, J. Immunol. 196:1435-1441). For example, Fab glycosylation may affect the stability, half-life, and binding characteristics of an antibody. To determine the effects of Fab glycosylation on the affinity of the antibody for its target, any technique known to one of skill in the art may be used, for example, enzyme linked immunosorbent assay (ELISA), or surface plasmon resonance (SPR). To determine the effects of Fab glycosylation on the half-life of the antibody, any technique known to one of skill in the art may be used, for example, by measurement of the levels of radioactivity in the blood or organs in a subject to whom a radiolabelled antibody has been administered. To determine the effects of Fab glycosylation on the stability, for example, levels of aggregation or protein unfolding, of the antibody, any technique known to one of skill in the art may be used, for example, differential scanning calorimetry (DSC), high performance liquid chromatography (HPLC), *e.g.,* size exclusion high performance liquid chromatography (SEC-HPLC), capillary electrophoresis, mass spectrometry, or turbidity measurement.

The presence of sialic acid on HuGlyFab or HuPTM scFv used in the methods described herein can impact clearance rate of the HuGlyFab or HuPTM scFv. Accordingly, sialic acid patterns of a HuGlyFab or HuPTM scFv can be used to generate a therapeutic having an optimized clearance rate. Methods of assessing antigen-binding fragment clearance rate are known in the art. *See, e.g.,* Huang et al., 2006, Anal. Biochem. 349:197-207.

In another specific embodiment, a benefit conferred by N-glycosylation is reduced aggregation. Occupied N-glycosylation sites can mask aggregation prone amino acid residues, resulting in decreased aggregation. Such N-glycosylation sites can be native to an antigen-binding fragment used herein, or engineered into an antigen-binding fragment used herein, resulting in HuGlyFab or HuPTM scFv that is less prone to aggregation when expressed, *e.g.,* expressed in human cells. Methods of assessing aggregation of antibodies are known in the art. *See, e.g.,* Courtois et al., 2016, mAbs 8:99-112 which is incorporated by reference herein in its entirety.

In another specific embodiment, a benefit conferred by N-glycosylation is reduced immunogenicity. Such N-glycosylation sites can be native to an antigen-binding fragment used herein, or engineered into an antigen-binding fragment used herein, resulting in HuGlyFab or HuPTM scFv that is less prone to immunogenicity when expressed, *e.g.,* expressed in human retinal cells, human CNS cells, human liver cells or human muscle cells.

In another specific embodiment, a benefit conferred by N-glycosylation is protein stability. N-glycosylation of proteins is well-known to confer stability on them, and methods of assessing protein stability resulting from N-glycosylation are known in the art. *See, e.g.,* Sola and Griebenow, 2009, J Pharm Sci., 98(4): 1223-1245.

In another specific embodiment, a benefit conferred by N-glycosylation is altered binding affinity. It is known in the art that the presence of N-glycosylation sites in the variable domains of an antibody can increase the affinity of the antibody for its antigen. *See, e.g.,* Bovenkamp et al., 2016, J. Immunol. 196:1435-1441. Assays for measuring antibody binding affinity are known in the art. *See, e.g.,* Wright et al., 1991, EMBO J. 10:2717-2723; and Leibiger et al., 1999, Biochem. J. 338:529-538.

### 5.2.2 Tyrosine Sulfation

Tyrosine sulfation occurs at tyrosine (Y) residues with glutamate (E) or aspartate (D) within +5 to -5 position of Y, and where position -1 of Y is a neutral or acidic charged amino acid, but not a basic amino acid, *e.g.,* arginine (R), lysine (K), or histidine (H) that abolishes sulfation. Surprisingly, the HuGlyFabs and HuPTM scFvs described herein comprise tyrosine sulfation sites (see FIGS. 2A-2F, 3A-3E, 4A -4B, 5A-5D, 6, 7A-7B, 8A-8H, and 9A-9B).

Importantly, tyrosine-sulfated antigen-binding fragments cannot be produced in *E. coli,* which naturally does not possess the enzymes required for tyrosine-sulfation. Further, CHO cells are deficient for tyrosine sulfation-they are not secretory cells and have a limited capacity for post-translational tyrosine-sulfation. *See, e.g.,* Mikkelsen & Ezban, 1991, Biochemistry 30: 1533-1537. Advantageously, the methods provided herein call for expression of HuPTM Fab in human cells that are secretory and have capacity for tyrosine sulfation.

Tyrosine sulfation is advantageous for several reasons. For example, tyrosine-sulfation of the antigen-binding fragment of therapeutic antibodies against targets has been shown to dramatically increase avidity for antigen and activity. *See, e.g.,* Loos et al., 2015, PNAS 112: 12675-12680, and Choe et al., 2003, Cell 114: 161-170. Assays for detection tyrosine sulfation are known in the art. *See, e.g.,* Yang et al., 2015, Molecules 20:2138-2164.

### 5.2.3 O-Glycosylation

O-glycosylation comprises the addition of N-acetyl-galactosamine to serine or threonine residues by the enzyme. It has been demonstrated that amino acid residues present in the hinge region of antibodies can be O-glycosylated. In certain embodiments, the HuGlyFab comprise all or a portion of their hinge region, and thus are capable of being O-glycosylated when expressed in human cells. The possibility of O-glycosylation confers another advantage to the HuGlyFab provided herein, as compared to, *e.g.,* antigen-binding fragments produced in *E. coli,* again because the *E. coli* naturally does not contain machinery equivalent to that used in human O-glycosylation. (Instead, O-glycosylation in *E. coli* has been demonstrated only when the bacteria is modified to contain specific O-glycosylation machinery. *See, e.g.,* Farid-Moayer et al., 2007, J. Bacteriol. 189:8088-8098.) O-glycosylated HuGlyFab, by virtue of possessing glycans, shares advantageous characteristics with N-glycosylated HuGlyFab (as discussed above).

### 5.3 VECTORED THERAPEUTIC ANTIBODIES

### 5.3.1 Anti-ABeta HuPTM Constructs and Formulations for Alzheimer's Disease

Compositions and methods are described for the delivery of HuPTM mAbs and antigen-binding fragments thereof, such as HuPTM Fabs, that bind to amyloid beta (Aβ or Abeta) peptides derived from the amyloid precursor protein that may have benefit in treating Alzheimer's disease (AD) and the like. In particular embodiments, the HuPTM mAb is aducanumab, crenezumab, gantenerumab, or BAN2401, or an antigen binding fragment of one of the foregoing. The amino acid sequences of Fab fragments of these antibodies are provided in FIGS. 2A-2C and 2F. Delivery may be accomplished via gene therapy - e.g., by administering a viral vector or other DNA expression construct encoding an Aβ-binding HuPTM mAb (or an antigen binding fragment and/or a hyperglycosylated derivative or other derivative, thereof) to patients (human subjects) diagnosed with, or having one or more symptoms of, AD, to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to Aβ that can be administered to deliver the HuPTM mAb or antigen binding fragment in a patient. The transgene is a nucleic acid comprising the nucleotide sequences encoding an antigen binding fragment of an antibody that binds to Aβ, such as aducanumab, crenezumab, gantenerumab, or BAN2401, or variants there of as detailed herein. The transgene may also encode an anti-Aβ antigen binding fragment that contains additional glycosylation sites *(e.g.,* see Courtois et al., 2016, mAbs 8: 99-112 which is incorporated by reference herein in its entirety).

In certain embodiments, the anti-Aβ antigen-binding fragment transgene comprises the nucleotide sequences encoding the heavy and light chains of the Fab portion of aducanumab (having amino acid sequences of SEQ ID NOs. 1 and 2, respectively, see Table 4 and FIG 2A). The nucleotide sequences may be codon optimized for expression in human cells and may, for example, comprise the nucleotide sequences of SEQ ID NO: 101 (encoding the aducanumab heavy chain Fab portion) and SEQ ID NO: 102 (encoding the aducanumab light chain Fab portion) as set forth in Table 5. The heavy and light chain sequences both have a signal or leader sequence at the N-terminus appropriate for expression and secretion in human cells, in particular, human CNS cells. The signal sequence may have the amino acid sequence of MYRMQLLLLIALSLALVTNS (SEQ ID NO: 161) or the one of the sequences found in Table 1 *supra.*

In addition to the heavy and light chain variable domain sequences, the transgenes may comprise, at the C-terminus of the heavy chain variable domain sequence, all or a portion of the hinge region. In specific embodiments, the anti-Aβ-antigen binding domain has a heavy chain variable domain of SEQ ID NO: 1 with additional hinge region sequence starting after the C-terminal aspartate (D), contains all or a portion of the amino acid sequence KTHTCPPCPAPELLGG (SEQ ID NO: 222), and specifically, KTHL (SEQ ID NO: 223), KTHT (SEQ ID NO: 224), KTHTCPPCPA (SEQ ID NO: 225), KTHLCPPCPA (SEQ ID NO: 226), KTHTCPPCPAPELLGGPSVFL (SEQ ID NO: 227) or KTHLCPPCPAPELLGGPSVFL (SEQ ID NO: 228) as set forth in FIG 2A. These hinge regions may be encoded by nucleotide sequences at the 3' end of SEQ ID NO: 1 by the hinge region encoding sequences set forth in Table 4 (SEQ ID NO: 101).

In certain embodiments, the anti-Aβ antigen-binding fragment transgene encodes an Aβ antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 2. In certain embodiments, the anti-Aβ antigen-binding fragment transgene encodes an Aβ antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 1. In certain embodiments, the anti-Aβ antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 2 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 1. In specific embodiments, the Aβ antigen binding fragment comprises a heavy chain comprising an amino acid sequence of SEQ ID NO: 1 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2A) or are substitutions with an amino acid present at that position in the heavy chain of one or more of the other therapeutic antibodies, for example, as identified by the alignment in FIG. 11A. In specific embodiments, the Aβ antigen binding fragment comprises a light chain comprising an amino acid sequence of SEQ ID NO:2 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2A) or are substitutions with an amino acid present at that position in the light chain of one or more of the other therapeutic antibodies, for example, as identified by the alignment in FIG. 11B.

In certain embodiments, the anti-Aβ antigen-binding fragment transgene encodes a hyperglycosylated aducanumab Fab, comprising a heavy chain and a light chain of SEQ ID NOs: 1 and 2, respectively, with one or more of the following mutations: T119N (heavy chain), Q160N or Q160S (light chain), and/or E195N (light chain) (see FIGS. 11A (heavy chain) and B (light chain)).

In certain embodiments, the anti-Aβ antigen-binding fragment transgene encodes an antigen-binding fragment and comprises the nucleotide sequences encoding the six aducanumab CDRs which are underlined in the heavy and light chain variable domain sequences of FIG.2A which are spaced between framework regions, generally human framework regions, and associated with constant domains depending upon the form of the antigen-binding molecule, as is known in the art to form the heavy and/or light chain variable domain of an anti-Aβ antibody or antigen-binding fragment thereof.

In certain embodiments, the anti-Aβ antigen-binding fragment transgene comprises the nucleotide sequences encoding the heavy and light chains of the Fab portion of crenezumab (having amino acid sequences of SEQ ID NOs. 3 and 4, respectively, see Table 4 and FIG. 2B). The nucleotide sequences may be codon optimized for expression in human cells and may, for example, comprise the nucleotide sequences of SEQ ID NO: 103 (encoding the crenezumab heavy chain Fab portion) and SEQ ID NO: 104 (encoding the crenezumab light chain Fab portion) as set forth in Table 5. The heavy and light chain sequences both have a signal or leader sequence at the N-terminus appropriate for expression and secretion in human cells, in particular, human CNS cells. The signal sequence may have the amino acid sequence of MYRMQLLLLIALSLALVTNS (SEQ ID NO: 161) or a signal sequence found in Table 1.

In addition to the heavy and light chain variable domain sequences, the transgenes may comprise, at the C-terminus of the heavy chain variable domain sequence, all or a portion of the hinge region. In specific embodiments, the anti-Aβ-antigen binding domain has a heavy chain variable domain of SEQ ID NO: 3 with additional hinge region sequence starting after the C-terminal tyrosine (Y), contains all or a portion of the amino acid sequence GPPCPPCPA (SEQ ID NO: 229) or GPPCPPCPAPEFLGGPSVFL (SEQ ID NO: 230) as set forth in FIG 2B. These hinge regions may be encoded by nucleotide sequences at the 3' end of SEQ ID NO: 3 by the hinge region encoding sequences set forth in Table 5 (SEQ ID NO: 103).

In certain embodiments, the anti-Aβ antigen-binding fragment transgene encodes an Aβ antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 4. In certain embodiments, the anti-Aβ antigen-binding fragment transgene encodes an Aβ antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 3. In certain embodiments, the anti-Aβ antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 4 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 3. In specific embodiments, the Aβ antigen binding fragment comprises a heavy chain comprising an amino acid sequence of SEQ ID NO: 3 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2B) or are substitutions with an amino acid present at that position in the heavy chain of one or more of the other therapeutic antibodies, for example, as identified by the alignment in FIG. 11A. In specific embodiments, the Aβ antigen binding fragment comprises a light chain comprising an amino acid sequence of SEQ ID NO: 4 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2B) or are substitutions with an amino acid present at that position in the light chain of one or more of the other therapeutic antibodies, for example, as identified by the alignment in FIG. 11B.

In certain embodiments, the anti-Aβ antigen-binding fragment transgene encodes a hyperglycosylated crenezumab Fab, comprising a heavy chain and a light chain of SEQ ID NOs: 3 and 4, respectively, with one or more of the following mutations: T107N (heavy chain), Q165N or Q165S (light chain), and/or E200N (light chain) (see FIGS. 11A (heavy chain) and B (light chain)).

In certain embodiments, the anti-Aβ antigen-binding fragment transgene encodes an antigen-binding fragment and comprises the nucleotide sequences encoding the six crenezumab CDRs which are underlined in the heavy and light chain variable domain sequences of FIG. 2B which are spaced between framework regions, generally human framework regions, and associated with constant domains depending upon the form of the antigen-binding molecule, as is known in the art to form the heavy and/or light chain variable domain of an anti-Aβ antibody or antigen-binding fragment thereof.

In certain embodiments, the anti-Aβ antigen-binding fragment transgene comprises the nucleotide sequences encoding the heavy and light chains of the Fab portion of gantenerumab (having amino acid sequences of SEQ ID NOs. 5 and 6, respectively, see Table 4 and FIG. 2C). The nucleotide sequences may be codon optimized for expression in human cells and may, for example, comprise the nucleotide sequences of SEQ ID NO: 105 (encoding the gantenerumab heavy chain Fab portion) and SEQ ID NO: 106 (encoding the gantenerumab light chain Fab portion) as set forth in Table 5. The heavy and light chain sequences both have a signal or leader sequence at the N-terminus appropriate for expression and secretion in human cells, in particular, human CNS cells. The signal sequence may have the amino acid sequence of MYRMQLLLLIALSLALVTNS (SEQ ID NO: 161) or a signal sequence found in Table 1.

In addition to the heavy and light chain variable domain sequences, the transgenes may comprise, at the C-terminus of the heavy chain variable domain sequence, all or a portion of the hinge region. In specific embodiments, the anti-Aβ-antigen binding domain has a heavy chain variable domain of SEQ ID NO: 5 with additional hinge region sequence starting at the C-terminal aspartate (D), contains all or a portion of the amino acid sequence KTHTCPPCPAPELLGG (SEQ ID NO: 222), and specifically, KTHL (SEQ ID NO: 223), KTHT (SEQ ID NO: 224), KTHTCPPCPA (SEQ ID NO: 225), KTHLCPPCPA (SEQ ID NO: 226), KTHTCPPCPAPELLGGPSVFL (SEQ ID NO: 227) or KTHLCPPCPAPELLGGPSVFL (SEQ ID NO: 228) as set forth in FIG 2C. These hinge regions may be encoded by nucleotide sequences at the 3' end of SEQ ID NO: 5 by the hinge region encoding sequences set forth in Table 5 (SEQ ID NO: 105).

In certain embodiments, the anti-Aβ antigen-binding fragment transgene encodes an Aβ antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 6. In certain embodiments, the anti-Aβ antigen-binding fragment transgene encodes an Aβ antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 5. In certain embodiments, the anti-Aβ antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 6 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 5. In specific embodiments, the Aβ antigen binding fragment comprises a heavy chain comprising an amino acid sequence of SEQ ID NO:5 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2C) or are substitutions with an amino acid present at that position in the heavy chain of one or more of the other therapeutic antibodies, for example, as identified by the alignment in FIG. 11A. In specific embodiments, the Aβ antigen binding fragment comprises a light chain comprising an amino acid sequence of SEQ ID NO: 6 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2C) or are substitutions with an amino acid present at that position in the light chain of one or more of the other therapeutic antibodies, for example, as identified by the alignment in FIG. 11B.

In certain embodiments, the anti-Aβ antigen-binding fragment transgene encodes a hyperglycosylated gantenerumab Fab, comprising a heavy chain and a light chain of SEQ ID NOs: 5 and 6, respectively, with one or more of the following mutations: L121N (heavy chain), Q161N or Q161S (light chain), and/or E196N (light chain) (see FIGS. 11A (heavy chain) and B (light chain)).

In certain embodiments, the anti-Aβ antigen-binding fragment transgene encodes an antigen-binding fragment and comprises the nucleotide sequences encoding the six gantenerumab CDRs which are underlined in the heavy and light chain variable domain sequences of FIG. 2C which are spaced between framework regions, generally human framework regions, and associated with constant domains depending upon the form of the antigen-binding molecule, as is known in the art to form the heavy and/or light chain variable domain of an anti-Aβ antibody or antigen-binding fragment thereof.

In certain embodiments, the anti-Aβ antigen-binding fragment transgene comprises the nucleotide sequences encoding the heavy and light chains of the Fab portion of BAN2401 (having amino acid sequences of SEQ ID NOs. 57 and 58, respectively, see Table 4 and FIG 2F). The nucleotide sequences may be codon optimized for expression in human cells and may, for example, comprise the nucleotide sequences of SEQ ID NO: 157 (encoding the BAN2401 heavy chain Fab portion) and SEQ ID NO: 158 (encoding the BAN2401 light chain Fab portion) as set forth in Table 5. The heavy and light chain sequences both have a signal or leader sequence at the N-terminus appropriate for expression and secretion in human cells, in particular, human CNS cells. The signal sequence may have the amino acid sequence of MYRMQLLLLIALSLALVTNS (SEQ ID NO: 161) or the one of the sequences found in Table 1 *supra.*

In addition to the heavy and light chain variable domain sequences, the transgenes may comprise, at the C-terminus of the heavy chain variable domain sequence, all or a portion of the hinge region. In specific embodiments, the anti-Aβ-antigen binding domain has a heavy chain variable domain of SEQ ID NO: 57 with additional hinge region sequence starting after the C-terminal aspartate (D), contains all or a portion of the amino acid sequence KTHTCPPCPAPELLGG (SEQ ID NO: 222) or KTHLCPPCPAPELLGG (SEQ ID NO: 239), and specifically, KTHL (SEQ ID NO: 223), KTHT (SEQ ID NO: 224), KTHTCPPCPA (SEQ ID NO: 225), or KTHLCPPCPA (SEQ ID NO: 226), as set forth in FIG 2F. These hinge regions may be encoded by nucleotide sequences at the 3' end of SEQ ID NO: 57 by the hinge region encoding sequences set forth in Table 4 (SEQ ID NO: 157).

In certain embodiments, the anti-Aβ antigen-binding fragment transgene encodes an Aβ antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 58. In certain embodiments, the anti-Aβ antigen-binding fragment transgene encodes an Aβ antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 57. In certain embodiments, the anti-Aβ antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 58 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 57. In specific embodiments, the Aβ antigen binding fragment comprises a heavy chain comprising an amino acid sequence of SEQ ID NO: 57 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2F) or are substitutions with an amino acid present at that position in the heavy chain of one or more of the other therapeutic antibodies, for example, as identified by the alignment in FIG. 11A. In specific embodiments, the Aβ antigen binding fragment comprises a light chain comprising an amino acid sequence of SEQ ID NO:58 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2F) or are substitutions with an amino acid present at that position in the light chain of one or more of the other therapeutic antibodies, for example, as identified by the alignment in FIG. 11B.

In certain embodiments, the anti-Aβ antigen-binding fragment transgene encodes a hyperglycosylated BAN2401Fab, comprising a heavy chain and a light chain of SEQ ID NOs: 57 and 58, respectively, with one or more of the following mutations: T119N (heavy chain), Q165N or Q165S (light chain), and/or E200N (light chain) (see FIGS. 11A (heavy chain) and B (light chain)).

In certain embodiments, the anti-Aβ antigen-binding fragment transgene encodes an antigen-binding fragment and comprises the nucleotide sequences encoding the six BAN2401 CDRs which are underlined in the heavy and light chain variable domain sequences of FIG.2F which are spaced between framework regions, generally human framework regions, and associated with constant domains depending upon the form of the antigen-binding molecule, as is known in the art to form the heavy and/or light chain variable domain of an anti-Aβ antibody or antigen-binding fragment thereof.

### Gene Therapy Methods

Provided are methods of treating human subjects for AD by administration of a viral vector containing a transgene encoding an anti-Aβ antibody, or antigen binding fragment thereof. The antibody may be aducanumab, crenezumab, gantenerumab, or BAN2401 and is preferably a Fab fragment thereof, or other antigen-binding fragment thereof. In certain embodiments, the patient has been diagnosed with and/or has symptoms associated with prodromal AD, *i.e.,* a mild cognitive impairment associated with early AD or even pre-AD. Recombinant vectors used for delivering the transgene are described in Section 5.4.1 and shown at FIGS 2A-C. Such vectors should have a tropism for human CNS cells and can include non-replicating rAAV, particularly those bearing an AAV9, AAVrh10, AAVrh20, AAVrh39, or AAVcy5 capsid. The recombinant vectors can be administered in any manner such that the recombinant vector enters the CNS, preferably by introducing the recombinant vector into the cerebral spinal fluid (CSF). See Section 5.5.1 for details regarding the methods of treatment.

Subjects to whom such gene therapy is administered can be those responsive to anti-Aβ therapy. In particular embodiments, the methods encompass treating patients who have been diagnosed with AD, or have one or more symptoms associated therewith, and identified as responsive to treatment with an anti-Aβ antibody or considered a good candidate for therapy with an anti-Aβ antibody. In specific embodiments, the patients have previously been treated with aducanumab, crenezumab, gantenerumab, or BAN2401, and have been found to be responsive to one or more of aducanumab, crenezumab, gantenerumab, or BAN2401. To determine responsiveness, the anti-Aβ antibody or antigen-binding fragment transgene product (e.g., produced in human cell culture, bioreactors, etc.) may be administered directly to the subject.

### Human Post Translationally Modified Antibodies

The production of the anti-Aβ HuPTM mAb or HuPTM Fab, should result in a "biobetter" molecule for the treatment of AD accomplished via gene therapy - e.g., by administering a viral vector or other DNA expression construct encoding the antiAβ HuPTM Fab, intrathecally, particularly intracisternal or lumbar administration, or intravenous administration to human subjects (patients) diagnosed with or having one or more symptoms of AD, to create a permanent depot in the CNS that continuously supplies the fully-human post-translationally modified, e.g., human-glycosylated, sulfated transgene product produced by transduced CNS cells.

The cDNA construct for the anti-Aβ HuPTMmAb or anti-Aβ HuPTM Fab should include a signal peptide that ensures proper co- and post-translational processing (glycosylation and protein sulfation) by the transduced CNS cells. For example, the signal sequence may be MYRMQLLLLIALSLALVTNS (SEQ ID NO: 161).

As an alternative, or an additional treatment to gene therapy, the anti-Aβ HuPTM mAb or HuPTM Fab can be produced in human cell lines by recombinant DNA technology, and administered to patients diagnosed with AD, or for whom therapy for AD is considered appropriate.

In specific embodiments, the anti-Aβ HuPTM mAb or antigen-binding fragment thereof has heavy and light chains with the amino acid sequences of the heavy and light chain Fab portions of aducanumab as set forth in FIG. 2A (with non-consensus asparagine (N) glycosylation sites highlighted in green, glutamine (Q) glycosylation sites highlighted in blue, and Y-sulfation sites highlighted in yellow) has glycosylation, particularly a 2,6-sialylation, at one or more of the amino acid positions N166 of the heavy chain (SEQ ID NO:1) or N158 and/or N210 of the light chain (SEQ ID NO: 2). Alternatively or in addition to, the HuPTM mAb or antigen binding-fragment thereof with the heavy and light chain variable domain sequences of aducanumab has a sulfation group at Y 94 and/or Y95 of the heavy chain (SEQ ID NO: 1) and/or Y86 and/or Y87 of the light chain (SEQ ID NO: 2). In other embodiments, the anti-Aβ HuPTM mAb or antigen-binding fragment thereof does not contain any detectable (e.g., as detected by assays known in the art, for example, those described in section 5.2, *infra*) NeuGc moieties and/or does not contain any detectable *(e.g.,* as detected by assays known in the art, for example, those described in section 5.2, *infra*) alpha-Gal moieties.

In specific embodiments, the anti-Aβ HuPTM mAb or antigen-binding fragment thereof has heavy and light chains with the amino acid sequences of the heavy and light chain Fab portions of crenezumab as set forth in FIG. 2B (with non-consensus asparagine (N) glycosylation sites highlighted in green, glutamine (Q) glycosylation sites highlighted in blue, and Y-sulfation sites highlighted in yellow) has glycosylation, particularly a 2,6-sialylation, at one or more of the amino acid positions N52, Q104, N154, and/or N196 of the heavy chain (SEQ ID NO: 3) or Q105, N163 and/or N215 of the light chain (SEQ ID NO: 4). Alternatively or in addition to, the HuPTM mAb or antigen binding-fragment thereof with the heavy and light chain variable domain sequences of crenezumab has a sulfation group at Y94 and/or Y95 of the heavy chain (SEQ ID NO: 3) and/or Y91 and/or Y92 of the light chain (SEQ ID NO: 4). In other embodiments, the anti-Aβ HuPTM mAb or antigen-binding fragment thereof does not contain any detectable NeuGc moieties and/or does not contain any detectable alpha-Gal moieties.

In specific embodiments, the anti-Aβ HuPTM mAb or antigen-binding fragment thereof has heavy and light chains with the amino acid sequences of the heavy and light chain Fab portions of gantenerumab as set forth in FIG. 2C (with asparagine (N) glycosylation sites highlighted in magenta, non-consensus asparagine (N) glycosylation sites highlighted in green, glutamine (Q) glycosylation sites highlighted in blue, and Y-sulfation sites highlighted in yellow) has a glycosylation, particularly a 2,6-sialylation, at one or more of the amino acid positions N52, N77, Q118 and/or N168 of the heavy chain (SEQ ID NO: 5) or Q101, N159 and/or N211 of the light chain (SEQ ID NO: 6). Alternatively or in addition to, the HuPTM mAb or antigen binding-fragment thereof with the heavy and light chain variable domain sequences of gantenerumab has a sulfation group at Y94 and/or Y95 of the heavy chain (SEQ ID NO: 5) and/or Y87 and/or Y88 of the light chain (SEQ ID NO: 6). In other embodiments, the anti-Aβ HuPTM mAb or antigen-binding fragment thereof does not contain any detectable NeuGc moieties and/or does not contain any detectable alpha-Gal moieties.

In specific embodiments, the anti-Aβ HuPTM mAb or antigen-binding fragment thereof has heavy and light chains with the amino acid sequences of the heavy and light chain Fab portions of BAN2401 as set forth in FIG. 2F (with non-consensus asparagine (N) glycosylation sites highlighted in green, glutamine (Q) glycosylation sites highlighted in blue, and Y-sulfation sites highlighted in yellow) has a glycosylation, particularly a 2,6-sialylation, at one or more of the amino acid positions Q116 and/or N166 of the heavy chain (SEQ ID NO: 57) or N163 and/or N215 of the light chain (SEQ ID NO: 58). Alternatively or in addition to, the HuPTM mAb or antigen binding-fragment thereof with the heavy and light chain variable domain sequences of BAN2401 has a sulfation group at Y94 and/or Y95 of the heavy chain (SEQ ID NO: 57) and/or Y91 of the light chain (SEQ ID NO: 58). In other embodiments, the anti-Aβ HuPTM mAb or antigen-binding fragment thereof does not contain any detectable NeuGc moieties and/or does not contain any detectable alpha-Gal moieties.

In certain embodiments, the HuPTM mAb or Fab is therapeutically effective and is at least 0.5%, 1% or 2% 2,6 sialylated and/or sulfated and may be at least 5%, 10% or even 50% or 100% glycosylated 2,6 sialylation and/or sulfated. The goal of gene therapy treatment provided herein is to slow or arrest the progression of AD, particular cognitive impairment. Efficacy may be monitored by measuring a reduction in plaque formation and/or an improvement in cognitive function or a reduction in the decline in cognitive function.

Combinations of delivery of the anti-Aβ HuPTM mAb or antigen-binding fragment thereof, to the CNS accompanied by delivery of other available treatments are encompassed by the methods provided herein. The additional treatments may be administered before, concurrently or subsequent to the gene therapy treatment. Available treatments for AD that could be combined with the gene therapy provided herein include but are not limited to ARICEPT^{®} (donepezil), RAZADYNE^{®} (galantamine), NAMENDA^{®} (rivastigmine), and NAMZARIC^{®} (donepezil and memantine), to name a few, and administration with anti-Aβ agents, including but not limited to aducanumab, crenezumab, gantenerumab, or BAN2401, or anti-Tau agents, such as aTAU.

### 5.3.2. Anti-Tau HuPTM Constructs and Formulations for Tauopathies like Alzheimer's Disease, Chronic Traumatic Encephalopathy, Progressive Supranuclear Palsy, or Frontotemporal Dementia

Compositions and methods are described for the delivery of HuPTM mAbs and antigen-binding fragments thereof, such as HuPTM Fabs, that bind to Tau protein (Tau), such as monomeric Tau, oligomeric Tau, non-phosphorylated Tau, and phosphorylated Tau, that may have benefit in treating Alzheimer's Disease (AD), Chronic Traumatic Encephalopathy (CTE), Pick's Complex, primary age-related tauopathy, progressive supranuclear palsy (PSP), frontotemporal dementia (FD), and other tauopathies. In particular embodiments, the HuPTM mAb is an antibody having the Fab fragments provided in FIGS. 2D (referred to herein as "aTAU") or an antigen binding fragment thereof. Delivery may be accomplished via gene therapy - e.g., by administering a viral vector or other DNA expression construct encoding a Tau-binding HuPTM mAb (or an antigen binding fragment and/or a hyperglycosylated derivative or other derivative, thereof) to patients (human subjects) diagnosed with, or having one or more symptoms of, AD, CTE, PSP, FD, or other tauopathies, to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to Tau that can be administered to deliver the HuPTM mAb or antigen binding fragment in a patient. The transgene is a nucleic acid comprising the nucleotide sequences encoding an antigen binding fragment of an antibody that binds to Tau, such as aTAU or variants there of as detailed herein. The transgene may also encode anti-Tau antigen binding fragment that contains additional glycosylation sites *(e.g.,* see Courtois et al., 2016, mAbs 8: 99-112 which is incorporated by reference herein in its entirety).

In certain embodiments, the anti-Tau antigen-binding fragment transgene comprises the nucleotide sequences encoding the heavy and light chains of the Fab portion of aTAU (having amino acid sequences of SEQ ID NOs. 53 and 54, respectively, see Table 4 and FIG 2D). The nucleotide sequences may be codon optimized for expression in human cells and may, for example, comprise the nucleotide sequences of SEQ ID NO: 153 (encoding the aTAU heavy chain Fab portion) and SEQ ID NO: 154 (encoding the aTAU light chain Fab portion) as set forth in Table 5. The heavy and light chain sequences both have a signal or leader sequence at the N-terminus appropriate for expression and secretion in human cells, in particular, human CNS cells. The signal sequence may have the amino acid sequence of MYRMQLLLLIALSLALVTNS (SEQ ID NO: 161) or the one of the sequences found in Table 1 *supra.*

In addition to the heavy and light chain variable domain sequences, the transgenes may comprise, at the C-terminus of the heavy chain variable domain sequence, all or a portion of the hinge region. In specific embodiments, the anti-Tau-antigen binding domain has a heavy chain variable domain of SEQ ID NO: 53 with additional hinge region sequence starting after the C-terminal aspartate (D), contains all or a portion of the amino acid sequence GPPCPPCPAPEFLGG (SEQ ID NO: 231), and specifically, GPPCPPCPA (SEQ ID NO: 229) or GPPCPPCPAPEFLGGPSVFL (SEQ ID NO: 230) as set forth in FIG 2D. These hinge regions may be encoded by nucleotide sequences at the 3' end of SEQ ID NO: 53 by the hinge region encoding sequences set forth in Table 4 (SEQ ID NO: 153).

In certain embodiments, the anti-Tau antigen-binding fragment transgene encodes a Tau antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 54. In certain embodiments, the anti-Tau antigen-binding fragment transgene encodes a Tau antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 53. In certain embodiments, the anti-Tau antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 54 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 53. In specific embodiments, the Tau antigen-binding fragment comprises a heavy chain comprising an amino acid sequence of SEQ ID NO: 53 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2D) or are substitutions with an amino acid present at that position in the heavy chain of one or more of the other therapeutic antibodies, for example, as identified by the alignment in FIG. 11A. In specific embodiments, the Tau antigen binding fragment comprises a light chain comprising an amino acid sequence of SEQ ID NO:54 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2D) or are substitutions with an amino acid present at that position in the light chain of one or more of the other therapeutic antibodies, for example, as identified by the alignment in FIG. 11B.

In certain embodiments, the anti-Tau antigen-binding fragment transgene encodes a hyperglycosylated aTAU Fab, comprising a heavy chain and a light chain of SEQ ID NOs: 53 and 54, respectively, with one or more of the following mutations: T110N (heavy chain), Q164N or Q164S (light chain), and/or E199N (light chain) (see FIGS. 11A (heavy chain) and B (light chain)).

In certain embodiments, the anti-Tau antigen-binding fragment transgene encodes an antigen-binding fragment and comprises the nucleotide sequences encoding the six aTAU CDRs which are underlined in the heavy and light chain variable domain sequences of FIG. 2D which are spaced between framework regions, generally human framework regions, and associated with constant domains depending upon the form of the antigen-binding molecule, as is known in the art to form the heavy and/or light chain variable domain of an anti-Tau antibody or antigen-binding fragment thereof.

### Gene Therapy Methods

Provided are methods of treating human subjects for AD, CTE, PSP, FD, or other tauopathies by administration of a viral vector containing a transgene encoding an anti-Tau antibody, or antigen binding fragment thereof. The antibody may be aTAU, and is preferably a Fab fragment thereof, or other antigen-binding fragment thereof. In certain embodiments, the patient has been diagnosed with and/or has symptoms associated with prodromal AD, *i.e.,* a mild cognitive impairment associated with early AD or even pre-AD. A recombinant vector used for delivering the transgene is described in Section 5.4.1 and shown in FIG 2D. Such vectors should have a tropism for human CNS cells and can include non-replicating rAAV, particularly those bearing an AAV9, AAVrh10, AAVrh20, AAVrh39, or AAVcy5 capsid. The recombinant vectors can be administered in any manner such that the recombinant vector enters the CNS, preferably by introducing the recombinant vector into the cerebral spinal fluid (CSF). See Section 5.5.1 for details regarding the methods of treatment.

Subjects to whom such gene therapy is administered can be those responsive to anti-Tau therapy. In particular embodiments, the methods encompass treating patients who have been diagnosed with AD, PSP, or FD, or have one or more symptoms associated therewith, and identified as responsive to treatment with an anti-Tau antibody or considered a good candidate for therapy with an anti-Tau antibody. In specific embodiments, the patients have previously been treated with aTAU, and have been found to be responsive to one or more of aTAU. To determine responsiveness, the anti-Tau antibody or antigen-binding fragment transgene product (e.g., produced in human cell culture, bioreactors, etc.) may be administered directly to the subject.

### Human Post Translationally Modified Antibodies

The production of the anti-Tau HuPTM mAb or HuPTM Fab, should result in a "biobetter" molecule for the treatment of AD, PSP, or FD accomplished via gene therapy - e.g., by administering a viral vector or other DNA expression construct encoding the anti-Tau HuPTM Fab, intrathecally, particularly intracisternal or lumbar administration, or intravenous administration to human subjects (patients) diagnosed with or having one or more symptoms of AD, PSP, or FD, to create a permanent depot in the CNS that continuously supplies the fully-human post-translationally modified, e.g., human-glycosylated, sulfated transgene product produced by transduced CNS cells.

The cDNA construct for the anti-Tau HuPTMmAb or anti-Tau HuPTM Fab should include a signal peptide that ensures proper co- and post-translational processing (glycosylation and protein sulfation) by the transduced CNS cells. For example, the signal sequence may be MYRMQLLLLIALSLALVTNS (SEQ ID NO: 161).

As an alternative, or an additional treatment to gene therapy, the anti-Tau HuPTM mAb or HuPTM Fab can be produced in human cell lines by recombinant DNA technology, and administered to patients diagnosed with AD, PSP, or FD, or for whom therapy for AD, PSP, or FD is considered appropriate.

In specific embodiments, the anti-Tau HuPTM mAb or antigen-binding fragment thereof has heavy and light chains with the amino acid sequences of the heavy and light chain Fab portions of aTAU as set forth in FIG. 2D (with non-consensus asparagine (N) glycosylation sites highlighted in green, glutamine (Q) glycosylation sites highlighted in blue, and Y-sulfation sites highlighted in yellow) has glycosylation, particularly a 2,6-sialylation, at one or more of the amino acid positions N57 and/or Q107 and/or N157 and/or N199 of the heavy chain (SEQ ID NO:53) or N78 and/or Q104 and/or N162 and/or N214 of the light chain (SEQ ID NO: 54). Alternatively or in addition to, the HuPTM mAb or antigen binding-fragment thereof with the heavy and light chain variable domain sequences of aTAU has a sulfation group at Y96 and/or Y97 and/or Y104 of the heavy chain (SEQ ID NO: 53) and/or Y90 and/or Y91 of the light chain (SEQ ID NO: 54). In other embodiments, the anti-Tau HuPTM mAb or antigen-binding fragment thereof does not contain any detectable (*e.g.,* as detected by assays known in the art, for example, those described in section 5.2, *infra*) NeuGc moieties and/or does not contain any detectable (e.g., as detected by assays known in the art, for example, those described in section 5.2, *infra*) alpha-Gal moieties.

In certain embodiments, the HuPTM mAb or Fab is therapeutically effective and is at least 0.5%, 1% or 2% 2,6 sialylated and/or sulfated and may be at least 5%, 10% or even 50% or 100% glycosylated 2,6 sialylation and/or sulfated. The goal of gene therapy treatment provided herein is to slow or arrest the progression of AD, PSP, or FD, particularly cognitive impairment, gross or fine motor skill impairment, or vision impairment. Efficacy may be monitored by measuring a reduction in plaque formation and/or an improvement in cognitive function, with motor skills, or with vision or a reduction in the decline in cognitive function, motor skills, or vision.

Combinations of delivery of the anti-Tau HuPTM mAb or antigen-binding fragment thereof, to the CNS accompanied by delivery of other available treatments are encompassed by the methods provided herein. The additional treatments may be administered before, concurrently or subsequent to the gene therapy treatment. Available treatments for AD, PSP, or FD that could be combined with the gene therapy provided herein include but are not limited to ARICEPT^{®} (donepezil), RAZADYNE^{®} (galantamine), NAMENDA^{®} (rivastigmine), and NAMZARIC^{®} (donepezil and memantine), to name a few, and administration with anti-Tau agents, including but not limited to aTAU and anti-Aβ agents, such as, but not limited to aducanumab, crenezumab, and gantenerumab.

### 5.3.3. Anti-CGRPR HuPTM Constructs and Formulations for Migraines and Cluster Headaches.

Compositions and methods are described for the delivery of HuPTM mAbs and antigen-binding fragments thereof, such as HuPTM Fabs, that bind to calcitonin gene-related peptide receptor (CGRPR) that may have benefit in treating migraines and cluster headaches (referred to collectively as headache disorders). In particular embodiments, the HuPTM mAb is erenumab, eptinezumab, fremanezumab, galcanezumab or an antigen binding fragment of one of the foregoing. An amino acid sequence for Fab fragments of erenumab is provided in FIG. 2E. Delivery may be accomplished via gene therapy - e.g., by administering a viral vector or other DNA expression construct encoding an CGRPR-binding HuPTM mAb (or an antigen binding fragment and/or a hyperglycosylated derivative or other derivative, thereof) to patients (human subjects) diagnosed with, or having one or more symptoms of, migraines and cluster headaches, to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to CGRPR that can be administered to deliver the HuPTM mAb or antigen binding fragment in a patient. The transgene is a nucleic acid comprising the nucleotide sequences encoding an antigen binding fragment of an antibody that binds to CGRPR, such as erenumab, eptinezumab, fremanezumab, galcanezumab or variants thereof as detailed herein or in accordance with the details herein. The transgene may also encode anti-CGRPR antigen binding fragment that contains additional glycosylation sites *(e.g.,* see Courtois et al., 2016, mAbs 8: 99-112 which is incorporated by reference herein in its entirety).

In certain embodiments, the anti-CGRPR antigen-binding fragment transgene comprises the nucleotide sequences encoding the heavy and light chains of the Fab portion of erenumab (having amino acid sequences of SEQ ID NOs. 55 and 56, respectively, see Table 4 and FIG 2E). The nucleotide sequences may be codon optimized for expression in human cells and may, for example, comprise the nucleotide sequences of SEQ ID NO: 155 (encoding the erenumab heavy chain Fab portion) and SEQ ID NO: 156 (encoding the erenumab light chain Fab portion) as set forth in Table 5. The heavy and light chain sequences both have a signal or leader sequence at the N-terminus appropriate for expression and secretion in human cells, in particular, human CNS cells. The signal sequence may have the amino acid sequence of MYRMQLLLLIALSLALVTNS (SEQ ID NO: 161) or the one of the sequences found in Table 1 *supra.*

In addition to the heavy and light chain variable domain sequences, the transgenes may comprise, at the C-terminus of the heavy chain variable domain sequence, all or a portion of the hinge region. In specific embodiments, the anti-CGRPR-antigen binding domain has a heavy chain variable domain of SEQ ID NO: 55 with additional hinge region sequence starting after the C-terminal aspartate (D), contains all or a portion of the amino acid sequence CPPCPAPPVAGG (SEQ ID NO: 232), and specifically, CPPCPA (SEQ ID NO: 219) or CPPCPAPPVAG (SEQ ID NO: 233) as set forth in FIG 2E. These hinge regions may be encoded by nucleotide sequences at the 3' end of SEQ ID NO: 55 by the hinge region encoding sequences set forth in Table 4 (SEQ ID NO: 155).

In certain embodiments, the anti-CGRPR antigen-binding fragment transgene encodes a CGRPR antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 56. In certain embodiments, the anti-CGRPR antigen-binding fragment transgene encodes a CGRPR antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 55. In certain embodiments, the anti-CGRPR antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 56 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 55. In specific embodiments, the CGRPR antigen-binding fragment comprises a heavy chain comprising an amino acid sequence of SEQ ID NO: 55 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2E) or are substitutions with an amino acid present at that position in the heavy chain of one or more of the other therapeutic antibodies, for example, as identified by the alignment in FIG. 11A. In specific embodiments, the Tau antigen binding fragment comprises a light chain comprising an amino acid sequence of SEQ ID NO:56 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 2E) or are substitutions with an amino acid present at that position in the light chain of one or more of the other therapeutic antibodies, for example, as identified by the alignment in FIG. 11B.

In certain embodiments, the anti-CGRPR antigen-binding fragment transgene encodes a hyperglycosylated erenumab Fab, comprising a heavy chain and a light chain of SEQ ID NOs: 55 and 56, respectively, with one or more of the following mutations: T125N (heavy chain) and/or Q198N (light chain) (see FIGS. 11A (heavy chain) and B (light chain)).

In certain embodiments, the anti-CGRPR antigen-binding fragment transgene encodes an antigen-binding fragment and comprises the nucleotide sequences encoding the six erenumab CDRs which are underlined in the heavy and light chain variable domain sequences of FIG. 2E which are spaced between framework regions, generally human framework regions, and associated with constant domains depending upon the form of the antigen-binding molecule, as is known in the art to form the heavy and/or light chain variable domain of an anti-Tau antibody or antigen-binding fragment thereof.

### Gene Therapy Methods

Provided are methods of treating human subjects for migraines and cluster headaches by administration of a viral vector containing a transgene encoding an anti-CGRPR antibody, or antigen binding fragment thereof. The antibody may be erenumab, eptinezumab, fremanezumab, or galcanezumab and is preferably a Fab fragment thereof, or other antigen-binding fragment thereof. In certain embodiments, the patient has been diagnosed with and/or has symptoms associated with episodic migraines or chronic migraines. In certain embodiments, the patient has been diagnosed with and/or has symptoms associated with episodic cluster headaches or chronic cluster headaches. A recombinant vector used for delivering the transgene is described in Section 5.4.1 and shown in FIG 2E. Such vectors should have a tropism for human CNS cells and can include non-replicating rAAV, particularly those bearing an AAV9, AAVrh10, AAVrh20, AAVrh39, or AAVcy5 capsid. The recombinant vectors can be administered in any manner such that the recombinant vector enters the CNS, preferably by introducing the recombinant vector into the cerebral spinal fluid (CSF). See Section 5.5.1 for details regarding the methods of treatment.

Subjects to whom such gene therapy is administered can be those responsive to anti-CGRPR therapy. In particular embodiments, the methods encompass treating patients who have been diagnosed with migraines or cluster headaches or have one or more symptoms associated therewith, and identified as responsive to treatment with an anti-CGRPR antibody or considered a good candidate for therapy with an anti-CGRPR antibody. In specific embodiments, the patients have previously been treated with erenumab, eptinezumab, fremanezumab, or galcanezumab, and have been found to be responsive to one or more of erenumab, eptinezumab, fremanezumab, and galcanezumab. To determine responsiveness, the anti-CGRPR antibody or antigen-binding fragment transgene product (e.g., produced in human cell culture, bioreactors, etc.) may be administered directly to the subject.

### Human Post Translationally Modified Antibodies

The production of the anti-CGRPR HuPTM mAb or HuPTM Fab, should result in a "biobetter" molecule for the treatment of migraines or cluster headaches accomplished via gene therapy - e.g., by administering a viral vector or other DNA expression construct encoding the anti-CGRPR HuPTM Fab, intrathecally, particularly intracisternal or lumbar administration, or intravenous administration to human subjects (patients) diagnosed with or having one or more symptoms of migraines or cluster headaches, to create a permanent depot in the CNS that continuously supplies the fully-human post-translationally modified, e.g., human-glycosylated, sulfated transgene product produced by transduced CNS cells.

The cDNA construct for the anti-CGRPR HuPTM mAb or anti-CGRPR HuPTM Fab should include a signal peptide that ensures proper co- and post-translational processing (glycosylation and protein sulfation) by the transduced CNS cells. For example, the signal sequence may be MYRMQLLLLIALSLALVTNS (SEQ ID NO: 161).

As an alternative, or an additional treatment to gene therapy, the anti-CGRPR HuPTM mAb or HuPTM Fab can be produced in human cell lines by recombinant DNA technology, and administered to patients diagnosed with migraines or cluster headaches, or for whom therapy for migraines or cluster headaches is considered appropriate.

In specific embodiments, the anti-CGRPR HuPTM mAb or antigen-binding fragment thereof has heavy and light chains with the amino acid sequences of the heavy and light chain Fab portions of erenumab as set forth in FIG. 2E (with non-consensus asparagine (N) glycosylation sites highlighted in green, glutamine (Q) glycosylation sites highlighted in blue, and Y-sulfation sites highlighted in yellow) has glycosylation, particularly a 2,6-sialylation, at one or more of the amino acid positions N77 and/or Q122 and/or N172 and/or N205 and/or N214 of the heavy chain (SEQ ID NO:55) or N28 and/or N174 of the light chain (SEQ ID NO: 56). Alternatively or in addition to, the HuPTM mAb or antigen binding-fragment thereof with the heavy and light chain variable domain sequences of erenumab has a sulfation group at Y94 and/or Y95 of the heavy chain (SEQ ID NO: 55) and/or Y87 and/or Y88 of the light chain (SEQ ID NO: 56). In other embodiments, the anti-CGRPR HuPTM mAb or antigen-binding fragment thereof does not contain any detectable (*e.g.,* as detected by assays known in the art, for example, those described in section 5.2, *infra*) NeuGc moieties and/or does not contain any detectable (*e.g.,* as detected by assays known in the art, for example, those described in section 5.2, *infra*) alpha-Gal moieties.

In certain embodiments, the HuPTM mAb or Fab is therapeutically effective and is at least 0.5%, 1% or 2% 2,6 sialylated and/or sulfated and may be at least 5%, 10% or even 50% or 100% glycosylated 2,6 sialylation and/or sulfated. The goal of gene therapy treatment provided herein is to prevent or reduce the intensity or frequency of migraines, cluster headaches, or one or more of the symptoms associated therewith, including nausea, light sensitivity, sound sensitivity, red eye, eyelid edema, forehead and facial sweating, tearing (lacrimation), abnormal small size of the pupil (miosis), nasal congestion, runny nose (rhinorrhea), and drooping eyelid (ptosis). Efficacy may be monitored by measuring a reduction in the intensity or frequency of migraines or cluster headaches, or a reduction in the amount of acute migraine-specific medication used over a defined period of time.

Combinations of delivery of the anti-CGRPR HuPTM mAb or antigen-binding fragment thereof, to the CNS accompanied by delivery of other available treatments are encompassed by the methods provided herein. The additional treatments may be administered before, concurrently or subsequent to the gene therapy treatment. Available treatments for cluster headaches or migraines that could be combined with the gene therapy provided herein include but are not limited to triptans, ergotamine derivatives and NSAIDs, to name a few, and administration with anti-CGRPR agents, including but not limited to erenumab, eptinezumab, fremanezumab, and galcanezumab.

### 5.3.4 Anti-Interleukin and Anti-Interleukin Receptor HuPTM Constructs and Formulations for Autoimmune Disorders

Compositions and methods are described for the delivery of HuPTM mAbs and antigen-binding fragments thereof, such as HuPTM Fabs, that bind to interleukins (IL) or interleukin receptors (ILR) (e.g., *IL4R, IL17A, IL12*/*IL23,* or *IL-5)* derived from anti-ILs or anti-ILRs indicated for treating one or more autoimmune-related disorders, such as atopic dermatitis, psoriasis (e.g., plaque psoriasis, pustular psoriasis, and erythrodermic psoriasis), arthritis (e.g., psoriatic arthritis, and alkylating spondylitis), Crohn's disease, or asthma (collectively referred to hereinafter as "subject AI-Ds"). In particular embodiments, the HuPTM mAb has the amino acid sequence of dupilumab, ixekizumab, secukinumab, ustekinumab, or mepolizumab or an antigen binding fragment of one of the foregoing. The amino acid sequences of Fab fragments of these antibodies are provided in FIGS. 3A to 3E, respectively. Delivery may be accomplished via gene therapy - e.g., by administering a viral vector or other DNA expression construct encoding an IL/ILR-binding HuPTM mAb (or an antigen binding fragment and/or a hyperglycosylated derivative or other derivative, thereof) to patients (human subjects) diagnosed with, or having one or more symptoms of atopic dermatitis, psoriasis (e.g., plaque psoriasis), arthritis (e.g., psoriatic arthritis, and alkylating spondylitis), Crohn's disease, or asthma to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to IL/ILR that can be administered to deliver the HuPTM mAb or antigen binding fragment in a patient. The transgene is a nucleic acid comprising the nucleotide sequences encoding an antigen binding fragment of an antibody that binds to IL/ILR, such as dupilumab, ixekizumab, secukinumab, ustekinumab, mepolizumab, or variants thereof as detailed herein. The transgene may also encode an anti-IL/ILR antigen binding fragment that contains additional glycosylation sites *(e.g.,* see Courtois et al.).

In certain embodiments, the anti-IL4R antigen-binding fragment transgene comprises the nucleotide sequences encoding the heavy and light chains of the Fab portion of dupilumab (having amino acid sequences of SEQ ID NOs. 7 and 8, respectively, see Table 4 and FIG. 3A). The nucleotide sequences may be codon optimized for expression in human cells and may, for example, comprise the nucleotide sequences of SEQ ID NO: 107 (encoding the dupilumab heavy chain Fab portion) and SEQ ID NO: 108 (encoding the dupilumab light chain Fab portion) as set forth in Table 5. The heavy and light chain sequences both have a signal or leader sequence at the N-terminus appropriate for expression and secretion in human cells, in particular, human liver cells (e.g., hepatocytes) or human muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-integrin-antigen binding domain has a heavy chain variable domain of SEQ ID NO: 7 with additional hinge region sequence starting after the C-terminal tyrosine (Y), contains all or a portion of the amino acid sequence GPPCPPCPAPEFLGG (SEQ ID NO: 231), and specifically, GPPCPPCPA (SEQ ID NO: 229) or GPPCPPCPAPEFLGGPSVFL (SEQ ID NO: 230) as set forth in FIG 3A. These hinge regions may be encoded by nucleotide sequences at the 3' end of SEQ ID NO: 7 by the hinge region encoding sequences set forth in Table 5.

In certain embodiments, the anti-IL4R antigen-binding fragment transgene encodes an IL4R antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 8. In certain embodiments, the anti-IL4R antigen-binding fragment transgene encodes an IL4R antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 7. In certain embodiments, the anti-IL4R antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 8 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 7. In specific embodiments, the IL4R antigen binding fragment comprises a heavy chain comprising an amino acid sequence of SEQ ID NO: 7 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 3A) or are substitutions with an amino acid present at that position in the heavy chain of one or more of the other therapeutic antibodies, for example, as identified by the alignment in FIG. 11A. In specific embodiments, the IL4R antigen binding fragment comprises a light chain comprising an amino acid sequence of SEQ ID NO: 8 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 3A) or are substitutions with an amino acid present at that position in the light chain of one or more of the other therapeutic antibodies, for example, as identified by the alignment in FIG. 11B.

In certain embodiments, the anti-IL4R antigen-binding fragment transgene encodes a hyperglycosylated dupilumab Fab, comprising a heavy chain and a light chain of SEQ ID NOs: 7 and 8, respectively, with one or more of the following mutations: T120N (heavy chain), Q165N or Q165S (light chain), and/or E200N (light chain) (see FIGS. 11A (heavy chain) and B (light chain)).

In certain embodiments, the anti-IL4R antigen-binding fragment transgene encodes an antigen-binding fragment and comprises the nucleotide sequences encoding the six dupilumab CDRs which are underlined in the heavy and light chain variable domain sequences of FIG. 3A which are spaced between framework regions, generally human framework regions, and associated with constant domains depending upon the form of the antigen-binding molecule, as is known in the art to form the heavy and/or light chain variable domain of an anti-IL4R antibody or antigen-binding fragment thereof.

In certain embodiments, the anti-IL17A antigen-binding fragment transgene comprises the nucleotide sequences encoding the heavy and light chains of the Fab portion of ixekizumab (having amino acid sequences of SEQ ID NOs. 9 and 10, respectively, see Table 4 and FIG. 3B). The nucleotide sequences may be codon optimized for expression in human cells and may, for example, comprise the nucleotide sequences of SEQ ID NO: 109 (encoding the ixekizumab heavy chain Fab portion) and SEQ ID NO: 110 (encoding the ixekizumab light chain Fab portion) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region.. In specific embodiments, the anti-integrin-antigen binding domain has a heavy chain variable domain of SEQ ID NO: 9 with additional hinge region sequence starting after the C-terminal tyrosine (Y), contains all or a portion of the amino acid sequence GPPCPPCPAPEFLGG (SEQ ID NO: 231), and specifically, GPPCPPCPA (SEQ ID NO: 229) or GPPCPPCPAPEFLGGPSVFL (SEQ ID NO: 230) as set forth in FIG 3B. These hinge regions may be encoded by nucleotide sequences at the 3' end of SEQ ID NO: 9 by the hinge region encoding sequences set forth in Table 5.

In certain embodiments, the anti-IL17A antigen-binding fragment transgene encodes an IL17A antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 10. In certain embodiments, the anti- IL17A antigen-binding fragment transgene encodes an IL17A antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 9. In certain embodiments, the anti-IL17A antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 10 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 9. In specific embodiments, the IL17A antigen binding fragment comprises a heavy chain comprising an amino acid sequence of SEQ ID NO: 9 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (*i.e.,* those regions outside of the CDRs, which CDRs are underlined in FIG. 3B) or are substitutions with an amino acid present at that position in the heavy chain of one or more of the other therapeutic antibodies, for example, as identified by the alignment in FIG. 11A. In specific embodiments, the IL17A antigen binding fragment comprises a light chain comprising an amino acid sequence of SEQ ID NO: 10 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 3B) or are substitutions with an amino acid present at that position in the light chain of one or more of the other therapeutic antibodies, for example, as identified by the alignment in FIG. 11B.

In certain embodiments, the anti-IL17A antigen-binding fragment transgene encodes a hyperglycosylated ixekizumab Fab, comprising a heavy chain and a light chain of SEQ ID NOs: 9 and 10, respectively, with one or more of the following mutations: L114N (heavy chain), Q165N or Q165S (light chain), and/or E200N (light chain) (see FIGS. 11A (heavy chain) and B (light chain)).

In certain embodiments, the anti-IL17A antigen-binding fragment transgene encodes an antigen-binding fragment and comprises the nucleotide sequences encoding the six ixekizumab CDRs which are underlined in the heavy and light chain variable domain sequences of FIG. 3B which are spaced between framework regions, generally human framework regions, and associated with constant domains depending upon the form of the antigen-binding molecule, as is known in the art to form the heavy and/or light chain variable domain of an anti-IL17A antibody or antigen-binding fragment thereof.

In certain embodiments, the anti-IL17A antigen-binding fragment transgene comprises the nucleotide sequences encoding the heavy and light chains of the Fab portion of secukinumab (having amino acid sequences of SEQ ID NOs. 11 and 12, respectively, see Table 4 and FIG. 3C). The nucleotide sequences may be codon optimized for expression in human cells and may, for example, comprise the nucleotide sequences of SEQ ID NO: 111 (encoding the secukinumab heavy chain Fab portion) and SEQ ID NO: 112 (encoding the secukinumab light chain Fab portion) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to myocyte or hepatocyte secreted proteins, respectively.

In addition to the heavy and light chain variable domain sequences, the transgenes may comprise, at the C-terminus of the heavy chain variable domain sequence, all or a portion of the hinge region. In specific embodiments, the anti-integrin-antigen binding domain has a heavy chain variable domain of SEQ ID NO: 11 with additional hinge region sequence starting after the C-terminal aspartic acid (D), contains all or a portion of the amino acid sequence KTHT CPPCPAPELLGGPSVFL (SEQ ID NO: 227), and specifically, KTHT (SEQ ID NO: 224), KTHL (SEQ ID NO: 223), KTHTCPPCPA (SEQ ID NO: 225), KTHI,CPPCPA (SEQ ID NO: 226), KTHTCPPCPAPELLGGPSVFL (SEQ ID NO: 227), or KTHLCPPCPAPELLGGPSVFL (SEQ ID NO: 228) as set forth in FIG 3C. These hinge regions may be encoded by nucleotide sequences at the 3' end of SEQ ID NO: 11 by the hinge region encoding sequences set forth in Table 5.

In certain embodiments, the anti-IL17A antigen-binding fragment transgene encodes an IL17A antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 12. In certain embodiments, the anti-IL17A antigen-binding fragment transgene encodes an IL17A antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 11. In certain embodiments, the anti-IL17A antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 12 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 11. In specific embodiments, the IL17A antigen binding fragment comprises a heavy chain comprising an amino acid sequence of SEQ ID NO: 11 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 3C) or are substitutions with an amino acid present at that position in the heavy chain of one or more of the other therapeutic antibodies, for example, as identified by the alignment in FIG. 11A. In specific embodiments, the IL17A antigen binding fragment comprises a light chain comprising an amino acid sequence of SEQ ID NO: 12 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 3C) or are substitutions with an amino acid present at that position in the light chain of one or more of the other therapeutic antibodies, for example, as identified by the alignment in FIG. 11B.

In certain embodiments, the anti-IL17A antigen-binding fragment transgene encodes a hyperglycosylated secukinumab Fab, comprising a heavy chain and a light chain of SEQ ID NOs: 11 and 12, respectively, with one or more of the following mutations: L122N (heavy chain), Q161N or Q161S (light chain), and/or E196N (light chain) (see FIGS. 11A (heavy chain) and B (light chain)).

In certain embodiments, the anti-IL17A antigen-binding fragment transgene encodes an antigen-binding fragment and comprises the nucleotide sequences encoding the six secukinumab CDRs which are underlined in the heavy and light chain variable domain sequences of FIG. 3C which are spaced between framework regions, generally human framework regions, and associated with constant domains depending upon the form of the antigen-binding molecule, as is known in the art to form the heavy and/or light chain variable domain of an anti-IL/ILR antibody or antigen-binding fragment thereof.

In certain embodiments, the anti-IL12/IL23 antigen-binding fragment transgene comprises the nucleotide sequences encoding the heavy and light chains of the Fab portion of ustekinumab (having amino acid sequences of SEQ ID NOs. 13 and 14, respectively, see Table 4 and FIG. 3D). The nucleotide sequences may be codon optimized for expression in human cells and may, for example, comprise the nucleotide sequences of SEQ ID NO: 113 (encoding the ustekinumab heavy chain Fab portion) and SEQ ID NO: 114 (encoding the ustekinumab light chain Fab portion) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-integrin-antigen binding domain has a heavy chain variable domain of SEQ ID NO: 13 with additional hinge region sequence starting after the C-terminal aspartic acid (D), contains all or a portion of the amino acid sequence KTHT CPPCPAPELLGGPSVFL (SEQ ID NO: 227), and specifically, KTHT (SEQ ID NO: 224), KTHL (SEQ ID NO: 223), KTHTCPPCPA (SEQ ID NO: 225), KTHI,CPPCPA (SEQ ID NO: 226), KTHTCPPCPAPELLGGPSVFL (SEQ ID NO: 227), or KTHLCPPCPAPELLGGPSVFL (SEQ ID NO: 228) as set forth in FIG 3D. These hinge regions may be encoded by nucleotide sequences at the 3' end of SEQ ID NO: 13 by the hinge region encoding sequences set forth in Table 5.

In certain embodiments, the anti-IL12/IL23 antigen-binding fragment transgene encodes an IL/ILR antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 14. In certain embodiments, the anti-IL12/IL23 antigen-binding fragment transgene encodes an IL12/IL23 antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 13. In certain embodiments, the anti-IL12/IL23 antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 14 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 13. In specific embodiments, the IL12/IL23 antigen binding fragment comprises a heavy chain comprising an amino acid sequence of SEQ ID NO: 13 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 3D) or are substitutions with an amino acid present at that position in the heavy chain of one or more of the other therapeutic antibodies, for example, as identified by the alignment in FIG. 11A. In specific embodiments, the IL12/IL23 antigen binding fragment comprises a light chain comprising an amino acid sequence of SEQ ID NO: 14 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 3D) or are substitutions with an amino acid present at that position in the light chain of one or more of the other therapeutic antibodies, for example, as identified by the alignment in FIG. 11B.

In certain embodiments, the anti-IL12/IL23 antigen-binding fragment transgene encodes a hyperglycosylated ustekinumab Fab, comprising a heavy chain and a light chain of SEQ ID NOs: 13 and 14, respectively, with one or more of the following mutations: L114N (heavy chain), Q160N or Q160S (light chain), and/or E195N (light chain) (see FIGS. 11A (heavy chain) and B (light chain)).

In certain embodiments, the anti-IL12/IL23 antigen-binding fragment transgene encodes an antigen-binding fragment and comprises the nucleotide sequences encoding the six ustekinumab CDRs which are underlined in the heavy and light chain variable domain sequences of FIG. 3D which are spaced between framework regions, generally human framework regions, and associated with constant domains depending upon the form of the antigen-binding molecule, as is known in the art to form the heavy and/or light chain variable domain of an anti-IL12/IL23 antibody or antigen-binding fragment thereof.

In certain embodiments, the anti-IL-5 antigen-binding fragment transgene comprises the nucleotide sequences encoding the heavy and light chains of the Fab portion of mepolizumab (having amino acid sequences of SEQ ID NOs. 15 and 16, respectively, see Table 4 and FIG. 3E). The nucleotide sequences may be codon optimized for expression in human cells and may, for example, comprise the nucleotide sequences of SEQ ID NO: 115 (encoding the mepolizumab heavy chain Fab portion) and SEQ ID NO: 116 (encoding the mepolizumab light chain Fab portion) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-integrin-antigen binding domain has a heavy chain variable domain of SEQ ID NO: 15 with additional hinge region sequence starting after the C-terminal aspartic acid (D), contains all or a portion of the amino acid sequence KTHT CPPCPAPELLGGPSVFL (SEQ ID NO: 227), and specifically, KTHT (SEQ ID NO: 224), KTHL (SEQ ID NO: 223), KTHTCPPCPA (SEQ ID NO: 225), KTHI,CPPCPA (SEQ ID NO: 226), KTHTCPPCPAPELLGGPSVFL (SEQ ID NO: 227), or KTHLCPPCPAPELLGGPSVFL (SEQ ID NO: 228) as set forth in FIG 3E. These hinge regions may be encoded by nucleotide sequences at the 3' end of SEQ ID NO: 15 by the hinge region encoding sequences set forth in Table 5.

In certain embodiments, the anti-IL-5 antigen-binding fragment transgene encodes an IL-5 antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 16. In certain embodiments, the anti-IL-5 antigen-binding fragment transgene encodes an IL-5 antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 15. In certain embodiments, the anti-IL-5 antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 16 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 15. In specific embodiments, the IL-5 antigen binding fragment comprises a heavy chain comprising an amino acid sequence of SEQ ID NO: 15 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 3E) or are substitutions with an amino acid present at that position in the heavy chain of one or more of the other therapeutic antibodies, for example, as identified by the alignment in FIG. 11A. In specific embodiments, the IL-5 antigen binding fragment comprises a light chain comprising an amino acid sequence of SEQ ID NO: 16 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 3E) or are substitutions with an amino acid present at that position in the light chain of one or more of the other therapeutic antibodies, for example, as identified by the alignment in FIG. 11B.

In certain embodiments, the anti-IL-5 antigen-binding fragment transgene encodes a hyperglycosylated mepolizumab Fab, comprising a heavy chain and a light chain of SEQ ID NOs: 15 and 16, respectively, with one or more of the following mutations: T114N (heavy chain), Q166N or Q166S (light chain), and/or E201N (light chain) (see FIGS. 11A (heavy chain) and B (light chain)).

In certain embodiments, the anti-IL-5 antigen-binding fragment transgene encodes an antigen-binding fragment and comprises the nucleotide sequences encoding the six mepolizumab CDRs which are underlined in the heavy and light chain variable domain sequences of FIG. 3E which are spaced between framework regions, generally human framework regions, and associated with constant domains depending upon the form of the antigen-binding molecule, as is known in the art to form the heavy and/or light chain variable domain of an anti-IL-5 antibody or antigen-binding fragment thereof.

### Gene Therapy Methods

Provided are methods of treating human subjects for one or more of the subject AI-Ds by administration of a viral vector containing a transgene encoding an anti-IL/ILR antibody, or antigen binding fragment thereof. The antibody may be dupilumab, ixekizumab, secukinumab, ustekinumab, or mepolizumab, and is preferably a Fab fragment thereof, or other antigen-binding fragment thereof. In embodiments, the patient has been diagnosed with and/or has symptoms associated with one or more of the subject AI-Ds. Recombinant vectors used for delivering the transgene are described in Section 5.4.2. Such vectors should have a tropism for human liver or muscle cells and can include non-replicating rAAV, particularly those bearing an AAV8 or AAV9 capsid. The recombinant vectors, such as those shown in FIGS. 3A-3E, can be administered in any manner such that the recombinant vector enters the liver or muscle tissue, preferably by introducing the recombinant vector into the bloodstream (or in an alternative embodiment into the hepatic bloodstream, such as through the hepatic artery). See Section 5.5.2 for details regarding the methods of treatment.

Subjects to whom such gene therapy is administered can be those responsive to anti-IL/ILR therapy. In particular embodiments, the methods encompass treating patients who have been diagnosed with one or more of the subject AI-Ds, or have one or more symptoms associated therewith, and identified as responsive to treatment with an anti-IL/ILR antibody or considered a good candidate for therapy with an anti-IL/ILR antibody. In specific embodiments, the patients have previously been treated with dupilumab, ixekizumab, secukinumab, ustekinumab, or mepolizumab, and have been found to be responsive to dupilumab, ixekizumab, secukinumab, ustekinumab, or mepolizumab. To determine responsiveness, the anti-IL/ILR antibody or antigen-binding fragment transgene product (e.g., produced in human cell culture, bioreactors, etc.) may be administered directly to the subject.

### Human Post Translationally Modified Antibodies

The production of the anti-IL/ILR HuPTM mAb or HuPTM Fab, should result in a "biobetter" molecule for the treatment of one or more of the subject AI-Ds accomplished via gene therapy - e.g., by administering a viral vector or other DNA expression construct encoding the anti-IL/ILR HuPTM Fab, subcutaneously, intramuscularly, or intravenously to human subjects (patients) diagnosed with or having one or more symptoms of one or more of the subject AI-Ds, to create a permanent depot in the liver or muscle tissue that continuously supplies the fully-human post-translationally modified, e.g., human-glycosylated, sulfated transgene product produced by transduced liver or muscle cells.

The cDNA construct for the anti-IL/ILR HuPTMmAb or anti-IL/ILR HuPTM Fab should include a signal peptide that ensures proper co- and post-translational processing (glycosylation and protein sulfation) by the transduced liver or muscle cells. For example, the signal sequence may be MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

As an alternative, or an additional treatment to gene therapy, the anti-IL/ILR HuPTM mAb or HuPTM Fab can be produced in human cell lines by recombinant DNA technology, and administered to patients diagnosed with one or more of the subject AI-Ds, or for whom therapy for one or more of the subject AI-Ds is considered appropriate..

In specific embodiments, the anti-IL4R HuPTM mAb or antigen-binding fragment thereof has heavy and light chains with the amino acid sequences of the heavy and light chain Fab portions of dupilumab as set forth in FIG. 3A (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N77, N167, and/or Q117 of the heavy chain (SEQ ID NO:7) or Q105, N163, and/or N215 of the light chain (SEQ ID NO:8). Alternatively or in addition to, the HuPTM mAb or antigen binding-fragment thereof with the heavy and light chain variable domain sequences of dupilumab has a sulfation group at Y94 and/or Y95 of the heavy chain (SEQ ID NO: 7) and/or Y91 and/or Y92 of the light chain (SEQ ID NO: 8). In other embodiments, the anti-IL4R HuPTM mAb or antigen-binding fragment thereof does not contain any detectable NeuGc moieties and/or does not contain any detectable alpha-Gal moieties.

In specific embodiments, the anti-IL17A HuPTM mAb or antigen-binding fragment thereof has heavy and light chains with the amino acid sequences of the heavy and light chain Fab portions of ixekizumab as set forth in FIG. 3B (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions Q111, N161, and/or N203 of the heavy chain (SEQ ID NO: 9) or Q105, N163 and/or N215 of the light chain (SEQ ID NO: 10). Alternatively or in addition to, the HuPTM mAb or antigen binding-fragment thereof with the heavy and light chain variable domain sequences of ixekizumab has a sulfation group at Y94 and/or Y95 of the heavy chain (SEQ ID NO: 9) and/or Y91 and/or Y92 of the light chain (SEQ ID NO: 10). In other embodiments, the anti-IL17A HuPTM mAb or antigen-binding fragment thereof does not contain any detectable NeuGc moieties and/or does not contain any detectable α-Gal moieties.

In specific embodiments, the anti-IL17A HuPTM mAb or antigen-binding fragment thereof has heavy and light chains with the amino acid sequences of the heavy and light chain Fab portions of secukinumab as set forth in FIG. 3C (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N169 of the heavy chain (SEQ ID NO:11) or Q101, N159, and/or N211 of the light chain (SEQ ID NO: 12). Alternatively or in addition to, the HuPTM mAb or antigen binding-fragment thereof with the heavy and light chain variable domain sequences of secukinumab has a sulfation group at Y94 and/or Y95 and/or Y107 and/or Y108 of the heavy chain (SEQ ID NO: 11) and/or Y 87 and/or Y88 of the light chain (SEQ ID NO: 12. In other embodiments, the anti-II,17A HuPTM mAb or antigen-binding fragment thereof does not contain detectable NeuGc moieties and/or does not contain detectable α-Gal moieties.

In specific embodiments, the anti-IL12/IL23 HuPTM mAb or antigen-binding fragment thereof has heavy and light chains with the amino acid sequences of the heavy and light chain Fab portions of ustekinumab as set forth in FIG. 3D (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions Q111 and/or N161 of the heavy chain (SEQ ID NO: 13) or Q100, N158, and/or N210 of the light chain (SEQ ID NO: 14). Alternatively or in addition to, the HuPTM mAb or antigen binding-fragment thereof with the heavy and light chain variable domain sequences of ustekinumab has a sulfation group at Y86 and/or Y87 of the light chain (SEQ ID NO: 14). In other embodiments, the anti-IL12/IL23 HuPTM mAb or antigen-binding fragment thereof does not contain detectable NeuGc moieties and/or does not contain detectable α-Gal moieties.

In specific embodiments, the anti-IL-5 HuPTM mAb or antigen-binding fragment thereof has heavy and light chains with the amino acid sequences of the heavy and light chain Fab portions of mepolizumab as set forth in FIG. 3E (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N76 and/or N161 of the heavy chain (SEQ ID NO:15) or N22, N34, N164, and/or N216 of the light chain (SEQ ID NO: 16). Alternatively or in addition to, the HuPTM mAb or antigen binding-fragment thereof with the heavy and light chain variable domain sequences of mepolizumab has a sulfation group at Y93 and/or Y94 of the heavy chain (SEQ ID NO: 15) and/or Y92 and/or Y93 of the light chain (SEQ ID NO:16). In other embodiments, the anti-IL-5 HuPTM mAb or antigen-binding fragment thereof does not contain detectable NeuGc moieties and/or does not contain detectable alpha-Gal moieties.

In certain embodiments, the HuPTM mAb or Fab is therapeutically effective and is at least 0.5%, 1% or 2% 2,6-sialylation and/or sulfated and may be at least 5%, 10% or even 50% or 100% glycosylated and/or sulfated. The goal of gene therapy treatment provided herein is to slow or arrest the progression of subject AI-Ds.

Efficacy may be monitored by scoring the symptoms or degree of inflammation in the affected tissue or area of the body, e.g., such as the skin, colon, or joints. For example, with regard to CD, efficacy can be monitored by assessing Crohn's Disease Activity Index [CDAI] over the course of treatment (e.g., see Best WR et al. (1976) Gastroenterology 70(3):439-44, "Development of a Crohn's disease activity index. National Cooperative Crohn's Disease Study."). With regard to psoriasis and atopic dermatitis, efficacy can be monitored by assessing changes in the affected skin or in the quality of the patient's life over the course of treatment. One or more standardized assessments can be used to assess the change. (see e.g., Feldman & Krueger, (2005) Ann. Rheum. Dis. 64(Suppl II):ii65-ii68: "Psoriasis assessment tools in clinical trials" describing standardized assessments including the Psoriasis Area and Severity Index (PASI), Physician Global Assessment (PGA), lattice system, NPF Psoriasis Score (NPF-PS), Medical Outcome Survey Short Form 36 (SF-36), the Euro QoL, Dermatology Life Quality Index (DLQI), and the Skindex; Schram et al. (2012) Allergy; 67: 99-106: "EASI, (objective) SCORAD and POEM for atopic eczema: responsiveness and minimal clinically important difference" describing standardized assessments including Eczema Area and Severity Index (EASI) and the Severity Scoring of Atopic Dermatitis Index (SCORAD)). With regard to arthritis, efficacy can be monitored by assessing one or more of the activity of the disease, the patient's level of function, or the degree of structural damage to patient's joints (e.g., see Zockling & Braun (2005) Clin. Exp. Rheumatol 23 (Suppl. 39) S133-S141: "Assessment of ankylosing spondylitis" describing standardized assessment for ankylosing spondylitis; see also Coates et al. (2011) J. Rheumatol. 38(7):1496-1501: "Development of a disease severity and responder index for psoriatic arthritis (PsA)-report of the OMERACT 10 PsA special interest group" describing standardized assessments for psoriatic arthritis.).

Combinations of delivery of the anti-IL/ILR HuPTM mAb or antigen-binding fragment thereof, to the liver or muscle accompanied by delivery of other available treatments are encompassed by the methods provided herein. The additional treatments may be administered before, concurrently, or subsequent to the gene therapy treatment. Available treatments for subject AI-Ds that could be combined with the gene therapy provided herein include but are not limited to phototherapy for psoriasis, aminosalicylates, immunomodulatory agents (e.g., azathioprine (AZA), 6-mercaptopurine (6-MP), methotrexate (MTX)), oral or topical corticosteroids (e.g., prednisone or budesonide), topical calcineurin inhibitors, inhaled corticosteroids for asthma, and/or antibiotics for Crohn's Disease and administration with anti-IL/ILR agents, including but not limited to dupilumab, ixekizumab, secukinumab, ustekinumab, or mepolizumab.

### 5.3.5 Anti-Integrin HuPTM Constructs and Formulations for IBD or Multiple Sclerosis

Compositions and methods are described for the delivery of HuPTM mAbs and antigen-binding fragments thereof, such as HuPTM Fabs, that bind to integrin (e.g., α4 or α4β7 integrin) derived from anti-α4 integrin or anti-α4β7 integrin and indicated for treating inflammatory bowel disease (IBD), such as ulcerative colitis (UC) or Crohn's disease (CD), and multiple sclerosis (MS). In particular embodiments, the HuPTM mAb has the amino acid sequence of vedolizumab, natalizumab, or an antigen binding fragment of one of the foregoing. The amino acid sequences of Fab fragments of vedolizumab and natalizumab are provided in FIGS. 4A and 4B, respectively. Delivery may be accomplished via gene therapy - e.g., by administering a viral vector or other DNA expression construct encoding an integrin-binding HuPTM mAb (or an antigen binding fragment and/or a hyperglycosylated derivative or other derivative, thereof) to patients (human subjects) diagnosed with, or having one or more symptoms of IBD or MS to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to integrin that can be administered to deliver the HuPTM mAb or antigen binding fragment in a patient. The transgene is a nucleic acid comprising the nucleotide sequences encoding an antigen binding fragment of an antibody that binds to integrin, such as vedolizumab, natalizumab, or variants thereof as detailed herein. The transgene may also encode an anti-integrin antigen binding fragment that contains additional glycosylation sites *(e.g.,* see Courtois et al.).

In certain embodiments, the anti-integrin antigen-binding fragment transgene comprises the nucleotide sequences encoding the heavy and light chains of the Fab portion of vedolizumab (having amino acid sequences of SEQ ID NOs. 17 and 18, respectively, see Table 4 and FIG. 4A). The nucleotide sequences may be codon optimized for expression in human cells and may, for example, comprise the nucleotide sequences of SEQ ID NO: 117 (encoding the vedolizumab heavy chain Fab portion) and SEQ ID NO: 118 (encoding the vedolizumab light chain Fab portion) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-integrin-antigen binding domain has a heavy chain variable domain of SEQ ID NO: 17 with additional hinge region sequence starting after the C-terminal aspartate (D), contains all or a portion of the amino acid sequence KTHTCPPCPAPELAGA (SEQ ID NO: 236), and specifically, KTHL (SEQ ID NO: 223), KTHT (SEQ ID NO: 224), KTHTCPPCPA (SEQ ID NO: 225), KTHLCPPCPA (SEQ ID NO: 226), KTHTCPPCPAPELAGAPSVFL (SEQ ID NO: 237) or KTHLCPPCPAPELAGAPSVFL (SEQ ID NO: 238) as set forth in FIG. 4A. These hinge regions may be encoded by nucleotide sequences at the 3' end of SEQ ID NO: 117 by the hinge region encoding sequences set forth in Table 5.

In certain embodiments, the anti-integrin antigen-binding fragment transgene encodes an integrin antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 18. In certain embodiments, the anti-integrin antigen-binding fragment transgene encodes an integrin antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 17. In certain embodiments, the anti-integrin antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 18 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 17. In specific embodiments, the integrin antigen binding fragment comprises a heavy chain comprising an amino acid sequence of SEQ ID NO: 17 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 4A) or are substitutions with an amino acid present at that position in the heavy chain of one or more of the other therapeutic antibodies, for example, as identified by the alignment in FIG. 11A. In specific embodiments, the integrin antigen binding fragment comprises a light chain comprising an amino acid sequence of SEQ ID NO: 18 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 4A) or are substitutions with an amino acid present at that position in the light chain of one or more of the other therapeutic antibodies, for example, as identified by the alignment in FIG. 11B.

In certain embodiments, the anti-integrin antigen-binding fragment transgene encodes a hyperglycosylated vedolizumab Fab, comprising a heavy chain and a light chain of SEQ ID NOs: 17 and 18, respectively, with one or more of the following mutations: L116N (heavy chain), Q165N or Q165S (light chain), and/or E200N (light chain) (see FIGS. 11A (heavy chain) and B (light chain)).

In certain embodiments, the anti-integrin antigen-binding fragment transgene encodes an antigen-binding fragment and comprises the nucleotide sequences encoding the six vedolizumab CDRs which are underlined in the heavy and light chain variable domain sequences of FIG. 4A which are spaced between framework regions, generally human framework regions, and associated with constant domains depending upon the form of the antigen-binding molecule, as is known in the art to form the heavy and/or light chain variable domain of an anti-integrin antibody or antigen-binding fragment thereof.

In certain embodiments, the anti-integrin antigen-binding fragment transgene comprises the nucleotide sequences encoding the heavy and light chains of the Fab portion of natalizumab (having amino acid sequences of SEQ ID NOs. 19 and 20, respectively, see Table 4 and FIG. 4B). The nucleotide sequences may be codon optimized for expression in human cells and may, for example, comprise the nucleotide sequences of SEQ ID NO: 119 (encoding the natalizumab heavy chain Fab portion) and SEQ ID NO: 120 (encoding the natalizumab light chain Fab portion) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively. Alternatively, particularly for the treatment of MS, the heavy and light chains have a signal or leader sequence at the N-terminus appropriate for expression and secretion in human CNS cells, for example, any one of the signal sequences set forth in Table 1.

In addition to the heavy and light chain variable domain sequences, the transgenes may comprise, at the C-terminus of the heavy chain variable domain sequence, all or a portion of the hinge region. In specific embodiments, the anti-integrin-antigen binding domain has a heavy chain variable domain of SEQ ID NO: 19 with additional hinge region sequence starting after the C-terminal aspartate (D), contains all or a portion of the amino acid sequence GPPCPPCPAPEFLGG (SEQ ID NO: 231), and specifically, GPPCPPCPA (SEQ ID NO: 229) or GPPCPPCPAPEFLGGPSVFL (SEQ ID NO: 230) as set forth in FIG. 4B. These hinge regions may be encoded by nucleotide sequences at the 3' end of SEQ ID NO: 119 by the hinge region encoding sequences set forth in Table 5.

In certain embodiments, the anti-integrin antigen-binding fragment transgene encodes an integrin antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 20. In certain embodiments, the anti-integrin antigen-binding fragment transgene encodes an integrin antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 19. In certain embodiments, the anti-integrin antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 20 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 19. In specific embodiments, the integrin antigen binding fragment comprises a heavy chain comprising an amino acid sequence of SEQ ID NO: 19 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 4B) or are substitutions with an amino acid present at that position in the heavy chain of one or more of the other therapeutic antibodies, for example, as identified by the alignment in FIG. 11A. In specific embodiments, the integrin antigen binding fragment comprises a light chain comprising an amino acid sequence of SEQ ID NO: 20 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 4B) or are substitutions with an amino acid present at that position in the light chain of one or more of the other therapeutic antibodies, for example, as identified by the alignment in FIG. 11B.

In certain embodiments, the anti-integrin antigen-binding fragment transgene encodes a hyperglycosylated natalizumab Fab, comprising a heavy chain and a light chain of SEQ ID NOs: 19 and 20, respectively, with one or more of the following mutations: L118N (heavy chain), Q159N or Q159S (light chain), and/or E194N (light chain) (see FIGS. 11A (heavy chain) and B (light chain)).

In certain embodiments, the anti-integrin antigen-binding fragment transgene encodes an antigen-binding fragment and comprises the nucleotide sequences encoding the six natalizumab CDRs which are underlined in the heavy and light chain variable domain sequences of FIG. 4B which are spaced between framework regions, generally human framework regions, and associated with constant domains depending upon the form of the antigen-binding molecule, as is known in the art to form the heavy and/or light chain variable domain of an anti-integrin antibody or antigen-binding fragment thereof.

In specific embodiments, provided are AAV vectors comprising a viral capsid that is at least 95% identical to the amino acid sequence of an AAV8 capsid (SEQ ID NO: 78), AAV9 capsid (SEQ ID NO: 79) or AANrh10 capsid (SEQ ID NO:80); and an artificial genome comprising an expression cassette flanked by AAV inverted terminal repeats (ITRs), wherein the expression cassette comprises a transgene encoding an anti-integrin mAb, or an antigen-binding fragment thereof, operably linked to one or more regulatory sequences that control expression of the transgene in human liver or muscle cells.

### Gene Therapy Methods

Provided are methods of treating human subjects for IBD or MS by administration of a viral vector containing a transgene encoding an anti-integrin antibody, or antigen binding fragment thereof. The antibody may be vedolizumab or natalizumab, and is preferably a Fab fragment thereof, or other antigen-binding fragment thereof. In embodiments, the patient has been diagnosed with and/or has symptoms associated with IBD, such as UC or CD, or MS. In particular embodiments, IBD can be moderately to severely active. Recombinant vector used for delivering the transgene are described in Section 5.4.1 and 5.4.2. In some embodiments, such vectors should have a tropism for human liver cells and can include non-replicating rAAV, particularly those bearing an AAV8 or AAV9 capsid. The recombinant vectors, such as those shown in FIGS. 4A and 4B, can be administered in any manner such that the recombinant vector enters the liver or muscle tissue, preferably by introducing the recombinant vector into the bloodstream. See 5.5.2 for details regarding the methods of treatment. In other embodiments, such vectors should have a tropism for human CNS cells and can include non-replicating rAAV, particularly those bearing an AAV9, AAVrh10, AAVrh20, AAVrh39, or AAVcy5 capsid. The recombinant vector, such as shown in FIG. 4B, can be administered in any manner such that the recombinant vector enters the CNS, preferably by introducing the recombinant vector into the cerebral spinal fluid (CSF). See Section 5.5.1 for details regarding the methods of treatment.

Subjects to whom such gene therapy is administered can be those responsive to anti-integrin therapy. In particular embodiments, the methods encompass treating patients who have been diagnosed with IBD, MS, or have one or more symptoms associated therewith, and identified as responsive to treatment with an anti-integrin antibody or considered a good candidate for therapy with an anti-integrin antibody. In specific embodiments, the patients have previously been treated with vedolizumab and/or natalizumab, and have been found to be responsive to vedolizumab or natalizumab. To determine responsiveness, the anti-integrin antibody or antigen-binding fragment transgene product (e.g., produced in cell culture, bioreactors, etc.) may be administered directly to the subject.

### Human Post Translationally Modified Antibodies

The production of the anti-integrin HuPTM mAb or HuPTM Fab, should result in a "biobetter" molecule for the treatment of IBD or MS accomplished via gene therapy - e.g., by administering a viral vector or other DNA expression construct encoding the anti-integrin HuPTM Fab, subcutaneously, intramuscularly, or intravenously to human subjects (patients) diagnosed with or having one or more symptoms of IBD or MS, to create a permanent depot in the liver, muscle or CNS tissue that continuously supplies the fully-human post-translationally modified, such as human-glycosylated, sulfated transgene product produced by transduced liver, muscle or CNS cells.

The cDNA construct for the anti-integrin HuPTMmAb or anti-integrin HuPTM Fab should include a signal peptide that ensures proper co- and post-translational processing (glycosylation and protein sulfation) by the transduced liver or muscle cells. For example, the signal sequence may be MYRMQLLLLIALSLALVTNS (SEQ ID NO: 161). Alternatively, in some embodiments, the signal sequence may have an amino acid sequence selected from any one of the signal sequences set forth in Tables 1, 2 or 3 that correspond to the proteins secreted by CNS cells, myocytes or hepatocytes, respectively.

As an alternative, or an additional treatment to gene therapy, the anti-integrin HuPTM mAb or HuPTM Fab can be produced in human cell lines by recombinant DNA technology, and administered to patients diagnosed with IBD or MS, or for whom therapy for IBD or MS is considered appropriate.

In specific embodiments, the anti-integrin HuPTM mAb or antigen-binding fragment thereof has heavy and light chains with the amino acid sequences of the heavy and light chain Fab portions of vedolizumab as set forth in FIG. 4A (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions Q113 and/or N163 of the heavy chain (SEQ ID NO:17) or Q105 and/or N163 and/or N215 of the light chain (SEQ ID NO: 18). Alternatively or in addition to, the HuPTM mAb or antigen binding-fragment thereof with the heavy and light chain variable domain sequences of vedolizumab has a sulfation group at Y94, Y95 and/or Y106 of the heavy chain (SEQ ID NO: 17) and/or Y91 and/or Y92 of the light chain (SEQ ID NO: 18). In other embodiments, the anti-integrin HuPTM mAb or antigen-binding fragment thereof does not contain detectable NeuGc moieties and/or does not contain detectable alpha-Gal moieties.

In specific embodiments, the anti-integrin HuPTM mAb or antigen-binding fragment thereof has heavy and light chains with the amino acid sequences of the heavy and light chain Fab portions of natalizumab as set forth in FIG. 4B (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions Q115, N165, and/or N207 of the heavy chain (SEQ ID NO: 19) or Q99, N157 and/or N209 of the light chain (SEQ ID NO:20). Alternatively or in addition to, the HuPTM mAb or antigen binding-fragment thereof with the heavy and light chain variable domain sequences of natalizumab has a sulfation group at Y94 and/or Y95 of the heavy chain (SEQ ID NO: 19) and/or Y86 and/or Y87 of the light chain (SEQ ID NO:20). In other embodiments, the anti-integrin HuPTM mAb or antigen-binding fragment thereof does not contain detectable NeuGc moieties and/or does not contain detectable alpha-Gal moieties.

In certain embodiments, the HuPTM mAb or Fab is therapeutically effective and is at least 0.5%, 1% or 2% glycosylated and/or sulfated and may be at least 5%, 10% or even 50% or 100% glycosylated and/or sulfated. The goal of gene therapy treatment provided herein is to slow or arrest the progression of IBD or MS, particularly a reduction in pain and discomfort for the patient and/or in the case of MS, improvements in mobility. Efficacy may be monitored by scoring the symptoms or degree of inflammation in the affected tissue. For example, with regard to UC, efficacy can be monitored by assessing a Mayo score and an endoscopy subscore over the course of treatment (e.g., see Lobaton et al. (2015) J. Crohns Colitis. 2015 Oct;9(10):846-52, "The Modified Mayo Endoscopic Score (MMES): A New Index for the Assessment of Extension and Severity of Endoscopic Activity in Ulcerative Colitis Patients."). With regard to CD, efficacy can be monitored by assessing Crohn's Disease Activity Index [CDAI] over the course of treatment (e.g., see Best WR et al. (1976) Gastroenterology, Mar;70(3):439-44, "Development of a Crohn's disease activity index. National Cooperative Crohn's Disease Study."). For example, with regard to MS, efficacy can be monitored by assessing frequency of relapses (e.g., Annualized Relapse Rate), physical disability status (e.g., scoring Kurtzke Expanded Disability Status Scale (EDSS)), and biological markers, including brain scans using MRI (e.g., evaluation of T1-weighted gadolinium (Gd)-enhancing lesions and T2-hyperintense lesions through magnetic resonance imaging).

Combinations of delivery of the anti-integrin HuPTM mAb or antigen-binding fragment thereof, to the CNS, liver, or muscles accompanied by delivery of other available treatments are encompassed by the methods provided herein. The additional treatments may be administered before, concurrently, or subsequent to the gene therapy treatment. Available treatments for IBD that could be combined with the gene therapy provided herein include but are not limited to aminosalicylates, corticosteroids, and immunomodulators (e.g, azathioprine, 6-mercaptopurine, and/or methotrexate) and administration with anti-integrin agents, including but not limited to vedolizumab or natalizumab. Available treatments for MS that could be combined with the gene therapy provided herein include but are not limited to interferon beta, interferon beta 1a, glatiramer acetate, cyclophosphamide, corticosteroids, immunomodulators (e.g, azathioprine, 6-mercaptopurine, and/or methotrexate), and mitoxantrone and administration with anti-integrin agents, including but not limited to natalizumab.

### 5.3.6 Anti-PCSK9 and anti-ANGPTL3 HuPTM mAbs

Compositions and methods are described for the delivery of a HuPTM mAbs and antigen-binding fragments thereof, such as HuPTM Fabs, that bind to proprotein convertase subtilisin/kexin type 9 (PCSK9) derived from anti-PCSK9 or angiopoetin-like 3 (ANGPTL3) indicated for treating heterozygous familial hypercholesterolemia (HeFH), homozygous familial hypercholesterolemia (HoFH), or atherosclerotic cardiovascular disease (ACD), lowering low density lipoprotein cholesterol (LDL-C), triglyceride (TG), and/or total cholesterol (TC) levels, and/or reducing or slowing atherosclerotic plaque formation. In particular embodiments, the HuPTM mAb has the amino acid sequence of alirocumab, evolocumab, evinacumab, or an antigen binding fragment of one of the foregoing. The amino acid sequences of Fab fragments of alirocumab, evolocumab, and evinacumab are provided in FIGS. 5A to 5C, respectively. Delivery may be accomplished via gene therapy - e.g., by administering a viral vector or other DNA expression construct encoding an PCSK9-binding or anti-ANGPTL3-binding HuPTM mAb (or an antigen binding fragment and/or a hyperglycosylated derivative or other derivative, thereof) to patients (human subjects) diagnosed with, or having one or more symptoms of HeFH, HoFH, or ACD; abnormally high levels of LDL-C, TG, and/or TC; or abnormal atherosclerotic plaque to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to PCSK9 or ANGPTL3 that can be administered to deliver the HuPTM mAb or antigen binding fragment in a patient. The transgene is a nucleic acid comprising the nucleotide sequences encoding an antigen binding fragment of an antibody that binds to PCSK9 or ANGPTL3, such as alirocumab, evolocumab, evinacumab, or variants thereof as detailed herein. The transgene may also encode an anti-PCSK9 or anti-ANGPTL3 antigen binding fragment that contains additional glycosylation sites *(e.g.,* see Courtois et al.).

In certain embodiments, the anti-PCSK9 antigen-binding fragment transgene comprises the nucleotide sequences encoding the heavy and light chains of the Fab portion of alirocumab (having amino acid sequences of SEQ ID NOs. 21 and 22, respectively, see Table 4 and FIG. 5A). The nucleotide sequences may be codon optimized for expression in human cells and may, for example, comprise the nucleotide sequences of SEQ ID NO: 121 (encoding the alirocumab heavy chain Fab portion) and SEQ ID NO: 122 (encoding the alirocumab light chain Fab portion) as set forth in Table 5. The heavy and light chain sequences both have a signal or leader sequence at the N-terminus appropriate for expression and secretion in human cells, in particular, human liver cells (e.g., hepatocytes) or human muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-PCSK9-antigen binding domain has a heavy chain variable domain of SEQ ID NO: 21 with additional hinge region sequence starting after the C-terminal aspartic acid (D), contains all or a portion of the amino acid sequence KTHTCPPCPAPELLGG (SEQ ID NO: 222), and specifically, KTHT (SEQ ID NO: 224), KTHL (SEQ ID NO: 223), KTHTCPPCPA (SEQ ID NO: 225), KTHI,CPPCPA (SEQ ID NO: 226), KTHTCPPCPAPELLGGPSVFL (SEQ ID NO: 227), or KTHLCPPCPAPELLGGPSVFL (SEQ ID NO: 228) as set forth in FIG 5A. These hinge regions may be encoded by nucleotide sequences at the 3' end of SEQ ID NO: 21 by the hinge region encoding sequences set forth in Table 5.

In certain embodiments, the anti-PCSK9 antigen-binding fragment transgene encodes an PCSK9 antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 22. In certain embodiments, the anti-PCSK9 antigen-binding fragment transgene encodes an PCSK9 antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 21. In certain embodiments, the anti-PCSK9 antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 22 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 21. In specific embodiments, the PCSK9 antigen binding fragment comprises a heavy chain comprising an amino acid sequence of SEQ ID NO: 21 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 5A) or are substitutions with an amino acid present at that position in the heavy chain of one or more of the other therapeutic antibodies, for example, as identified by the alignment in FIG. 11A. In specific embodiments, the PCSK9 antigen binding fragment comprises a light chain comprising an amino acid sequence of SEQ ID NO: 22 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 5A) or are substitutions with an amino acid present at that position in the light chain of one or more of the other therapeutic antibodies, for example, as identified by the alignment in FIG. 11B.

In certain embodiments, the anti-PCSK9 antigen-binding fragment transgene encodes a hyperglycosylated alirocumab Fab, comprising a heavy chain and a light chain of SEQ ID NOs: 21 and 22, respectively, with one or more of the following mutations: L113N (heavy chain), Q166N or Q166S (light chain), and/or E201N (light chain) (see FIGS. 11A (heavy chain) and B (light chain)).

In certain embodiments, the anti-PCSK9 antigen-binding fragment transgene encodes an antigen-binding fragment and comprises the nucleotide sequences encoding the six alirocumab CDRs which are underlined in the heavy and light chain variable domain sequences of FIG. 5A which are spaced between framework regions, generally human framework regions, and associated with constant domains depending upon the form of the antigen-binding molecule, as is known in the art to form the heavy and/or light chain variable domain of an anti-PCSK9 antibody or antigen-binding fragment thereof.

In certain embodiments, the anti-PCSK9 antigen-binding fragment transgene comprises the nucleotide sequences encoding the heavy and light chains of the Fab portion of evolocumab (having amino acid sequences of SEQ ID NOs. 23 and 24, respectively, see Table 4 and FIG. 5B). The nucleotide sequences may be codon optimized for expression in human cells and may, for example, comprise the nucleotide sequences of SEQ ID NO: 123 (encoding the evolocumab heavy chain Fab portion) and SEQ ID NO: 124 (encoding the evolocumab light chain Fab portion) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-PCSK9 antigen binding domain has a heavy chain variable domain of SEQ ID NO: 23 with additional hinge region sequence starting after the C-terminal glutamic acid (E), contains all or a portion of the amino acid sequence KTHTCPPCPAPELLGG (SEQ ID NO: 222), and specifically, KTHT (SEQ ID NO: 224), KTHL (SEQ ID NO: 223), KTHTCPPCPA (SEQ ID NO: 225), KTHI,CPPCPA (SEQ ID NO: 226), KTHTCPPCPAPELLGGPSVFL (SEQ ID NO: 227), or KTHLCPPCPAPELLGGPSVFL (SEQ ID NO: 228) as set forth in FIG 5B. These hinge regions may be encoded by nucleotide sequences at the 3' end of SEQ ID NO: 23 by the hinge region encoding sequences set forth in Table 5.

In certain embodiments, the anti-PCSK9 antigen-binding fragment transgene encodes an PCSK9 antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 24. In certain embodiments, the anti-PCSK9 antigen-binding fragment transgene encodes an PCSK9 antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 23. In certain embodiments, the anti-PCSK9 antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 24 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 23. In specific embodiments, the PCSK9 antigen binding fragment comprises a heavy chain comprising an amino acid sequence of SEQ ID NO: 23 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 5B) or are substitutions with an amino acid present at that position in the heavy chain of one or more of the other therapeutic antibodies, for example, as identified by the alignment in FIG. 11A. In specific embodiments, the PCSK9 antigen binding fragment comprises a light chain comprising an amino acid sequence of SEQ ID NO: 24 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 5B) or are substitutions with an amino acid present at that position in the light chain of one or more of the other therapeutic antibodies, for example, as identified by the alignment in FIG. 11B.

In certain embodiments, the anti-PCSK9 antigen-binding fragment transgene encodes a hyperglycosylated evolocumab Fab, comprising a heavy chain and a light chain of SEQ ID NOs: 23 and 24, respectively, with one or more of the following mutations: T110N (heavy chain) and/or Q197N (light chain) (see FIGS. 11A (heavy chain) and B (light chain)).

In certain embodiments, the anti-PCSK9 antigen-binding fragment transgene encodes an antigen-binding fragment and comprises the nucleotide sequences encoding the six evolocumab CDRs which are underlined in the heavy and light chain variable domain sequences of FIG. 5B which are spaced between framework regions, generally human framework regions, and associated with constant domains depending upon the form of the antigen-binding molecule, as is known in the art to form the heavy and/or light chain variable domain of an anti-PCSK9 antibody or antigen-binding fragment thereof.

In certain embodiments, the anti-ANGPTL3 antigen-binding fragment transgene comprises the nucleotide sequences encoding the heavy and light chains variable domains of evinacumab (having amino acid sequences of SEQ ID NOs. 25 and 26, respectively, see Table 4 and FIG. 5C). These may be fused to heavy chain C1 constant domain and/or the light chain constant domain to form a Fab fragment. The nucleotide sequences may be codon optimized for expression in human cells and may, for example, comprise the nucleotide sequences of SEQ ID NO: 25 (encoding the evinacumab heavy chain variable domain) and SEQ ID NO: 26 (encoding the evinacumab light chain variable domain) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain sequence, all or a portion of the hinge region. In specific embodiments, the anti-ANGPTL3 antigen binding domain has a heavy chain variable domain of SEQ ID NO: 25 with additional hinge region sequence starting after the C-terminal aspartic acid (D), contains all or a portion of the amino acid sequence KTHT CPPCPAPELLGGPSVFL (SEQ ID NO: 227), and specifically, KTHT (SEQ ID NO: 224), KTHL (SEQ ID NO: 223), KTHTCPPCPA (SEQ ID NO: 225), KTHI,CPPCPA (SEQ ID NO: 226), KTHTCPPCPAPELLGGPSVFL (SEQ ID NO: 227), or KTHLCPPCPAPELLGGPSVFL (SEQ ID NO: 228) as set forth in FIG 5C. These hinge regions may be encoded by nucleotide sequences at the 3' end of SEQ ID NO: 25 by the hinge region encoding sequences set forth in Table 5.

In certain embodiments, the anti-ANGPTL3 antigen-binding fragment transgene encodes an ANGPTL3 antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 26. In certain embodiments, the anti-ANGPTL3 antigen-binding fragment transgene encodes an ANGPTL3 antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 25. In certain embodiments, the anti-ANGPTL3 antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 26 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 25. In specific embodiments, the ANGPTL3 antigen binding fragment comprises a heavy chain comprising an amino acid sequence of SEQ ID NO: 25 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 5C) or are substitutions with an amino acid present at that position in the heavy chain of one or more of the other therapeutic antibodies, for example, as identified by the alignment in FIG. 11A. In specific embodiments, the ANGPTL3 antigen binding fragment comprises a light chain comprising an amino acid sequence of SEQ ID NO: 26 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 5C) or are substitutions with an amino acid present at that position in the light chain of one or more of the other therapeutic antibodies, for example, as identified by the alignment in FIG. 11B.

In certain embodiments, the anti-ANGPTL3 antigen-binding fragment transgene encodes a hyperglycosylated evinacumab Fab, comprising a heavy chain and a light chain of SEQ ID NOs: 25 and 26, respectively, with one or more of the following mutations: M121N (heavy chain), Q160N or Q160S (light chain), and/or E195N (light chain) (see FIGS. 11A (heavy chain) and B (light chain)).

In certain embodiments, the anti-ANGPTL3 antigen-binding fragment transgene encodes an antigen-binding fragment and comprises the nucleotide sequences encoding the six evinacumab CDRs which are underlined in the heavy and light chain variable domain sequences of FIG. 5C which are spaced between framework regions, generally human framework regions, and associated with constant domains depending upon the form of the antigen-binding molecule, as is known in the art to form the heavy and/or light chain variable domain of an anti-ANGPTL3 antibody or antigen-binding fragment thereof.

### Gene Therapy Methods

Provided are methods of treating human subjects for HeFH, HoFH, or ADC by administration of a viral vector containing a transgene encoding an anti-PCSK9 or anti-ANGPTL3 mAb, or antigen binding fragment thereof. The antibody may be alirocumab, evolocumab, or evinacumab, and is preferably a Fab fragment thereof, or other antigen-binding fragment thereof. In embodiments, the patient has been diagnosed with and/or has symptoms associated with HeFH, HoFH, or ADC. Recombinant vectors used for delivering the transgene are described in Section 5.4.2. Such vectors should have a tropism for human liver or muscle cells and can include non-replicating rAAV, particularly those bearing an AAV8 or AAV9 capsid. The recombinant vectors, such as those shown in FIGS. 5A-5C, can be administered in any manner such that the recombinant vector enters the liver or muscle tissue, preferably by introducing the recombinant vector into the bloodstream. See Section 5.5.2 for details regarding the methods of treatment.

Subjects to whom such gene therapy is administered can be those responsive to anti-PCSK9 or anti-ANGPTL3 therapy. In particular embodiments, the methods encompass treating patients who have been diagnosed with HeFH, HoFH, or ADC, or have one or more symptoms associated therewith, and identified as responsive to treatment with an anti-PCSK9 or anti-ANGPTL3 antibody or considered a good candidate for therapy with an anti-PCSK9 or anti-ANGPTL3 antibody. In specific embodiments, the patients have previously been treated with alirocumab, evolocumab, or evinacumab, and have been found to be responsive to alirocumab, evolocumab, or evinacumab. To determine responsiveness, the anti-PCSK9 or anti-ANGPTL3 antibody or antigen-binding fragment transgene product (e.g., produced in cell culture, bioreactors, etc.) may be administered directly to the subject.

### Human Post Translationally Modified Antibodies

The production of the anti-PCSK9 or anti-ANGPTL3 HuPTM mAb or HuPTM Fab, should result in a "biobetter" molecule for the treatment of HeFH, HoFH, or ADC accomplished via gene therapy - e.g., by administering a viral vector or other DNA expression construct encoding the anti-PCSK9 or anti-ANGPTL3 HuPTM Fab, subcutaneously, intramuscularly, or intravenously to human subjects (patients) diagnosed with or having one or more symptoms of HeFH, HoFH, or ADC, to create a permanent depot in the liver or muscle tissue that continuously supplies the fully-human post-translationally modified, e.g., human-glycosylated, sulfated transgene product produced by transduced liver or muscle cells.

In specific embodiments, the anti-PCSK9 HuPTM mAb or antigen-binding fragment thereof has heavy and light chains with the amino acid sequences of the heavy and light chain Fab portions of alirocumab as set forth in FIG. 5A (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N30, N59, and/or N160 of the heavy chain (SEQ ID NO:21) or N22, N35, Q106, N164, and/or N216 of the light chain (SEQ ID NO: 22). Alternatively or in addition to, the HuPTM mAb or antigen binding-fragment thereof with the heavy and light chain variable domain sequences of alirocumab has a sulfation group at Y94 and/or Y95 of the heavy chain (SEQ ID NO: 21) and/or Y92 and/or Y93 of the light chain (SEQ ID NO: 22). In other embodiments, the anti-PCSK9 HuPTM mAb or antigen-binding fragment thereof does not contain detectable NeuGc moieties and/or does not contain detectable alpha-Gal moieties.

In specific embodiments, the anti-PCSK9 HuPTM mAb or antigen-binding fragment thereof has heavy and light chains with the amino acid sequences of the heavy and light chain Fab portions of evolocumab as set forth in FIG. 5B (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions Q107 and/or N157 and/or N190 and/or N199 of the heavy chain (SEQ ID NO: 23) or N71 and/or N173 of the light chain (SEQ ID NO: 24). Alternatively or in addition to, the HuPTM mAb or antigen binding-fragment thereof with the heavy and light chain variable domain sequences of evolocumab has a sulfation group at Y94 and/or Y95 of the heavy chain (SEQ ID NO: 23) and/or Y88 and/or Y89 of the light chain (SEQ ID NO: 24). In other embodiments, the anti-PCSK9 HuPTM mAb or antigen-binding fragment thereof does not contain detectable NeuGc moieties and/or does not contain detectable alpha-Gal moieties.

In specific embodiments, the anti-ANGPTL3 HuPTM mAb or antigen-binding fragment thereof has heavy and light chains with the amino acid sequences of the heavy and light chain Fab portions of evinacumab as set forth in FIG. 5C (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N77, Q118, and/or N168 of the heavy chain (SEQ ID NO: 25) or Q100, N158 and/or N210 of the light chain (SEQ ID NO: 26). Alternatively or in addition to, the HuPTM mAb or antigen binding-fragment thereof with the heavy and light chain variable domain sequences of evinacumab has a sulfation group at Y95 of the heavy chain (SEQ ID NO: 25) and/or Y86 and/or Y87 of the light chain (SEQ ID NO: 26). In other embodiments, the anti-PCSK9 HuPTM mAb or antigen-binding fragment thereof does not contain detectable NeuGc moieties and/or does not contain detectable alpha-Gal moieties.

In certain embodiments, the HuPTM mAb or Fab is therapeutically effective and is at least 0.5%, 1% or 2% glycosylated and/or sulfated and may be at least 5%, 10% or even 50% or 100% glycosylated and/or sulfated. The goal of gene therapy treatment provided herein is to slow or arrest the progression of HeFH, HoFH, or ADC and/or to lower the low density lipoprotein cholesterol (LDL-C) levels. Efficacy may be monitored by monitoring LDL-C levels. For example, efficacy can be monitored by assessing mean percent change in LDL-C from baseline.

Combinations of delivery of the anti-PCSK9 or anti-ANGPTL3 HuPTM mAb or antigen-binding fragment thereof, to the liver or muscle accompanied by delivery of other available treatments are encompassed by the methods provided herein. The additional treatments may be administered before, concurrently, or subsequent to the gene therapy treatment. Available treatments for HeFH, HoFH, or ACD that could be combined with the gene therapy provided herein include but are not limited to diet, statins, ezetimibe, and LDL apheresis and administration with anti-PCSK9 or anti-ANGPTL3 agents, including but not limited to alirocumab, evolocumab, or evinacumab.

### 5.3.7 Anti-OxPL HuPTM mAbs

Compositions and methods are described for the delivery of a HuPTM mAbs and antigen-binding fragments thereof, such as HuPTM Fabs, that bind to oxidized phospholipids (OxPL) indicated for treating and/or reducing and/or slowing cardiovascular disease including atherosclerotic cardiovascular disease (ACD), atherosclerotic plaque formation, abnormally high levels of non-HDL cholesterol and LDL, aortic stenosis, hepatic stenosis, or hypercholesterolemia. In particular embodiments, the HuPTM mAb has the amino acid sequence of E06-scFv, or an antigen binding fragment thereof. The amino acid sequences of E06-scFv is provided in FIG. 5D. Delivery may be accomplished via gene therapy - e.g., by administering a viral vector or other DNA expression construct encoding an OxPL-binding HuPTM mAb (or an antigen binding fragment and/or a hyperglycosylated derivative or other derivative, thereof) to patients (human subjects) diagnosed with, or having one or more symptoms of cardiovascular disease, ACD, hypercholesterolemia, abnormally high levels of non-HDL cholesterol and/or LDL, aortic stenosis, hepatic stenosis, and/or abnormal atherosclerotic plaque to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to OxPL that can be administered to deliver the HuPTM mAb or antigen binding fragment in a patient. The transgene is a nucleic acid comprising the nucleotide sequences encoding an antigen binding fragment of an antibody that binds to OxPL, such as E06-scFv or variants thereof as detailed herein. The transgene may also encode an anti-OxPL antigen binding fragment that contains additional glycosylation sites *(e.g.,* see Courtois et al.).

In certain embodiments, the anti-OxPL antigen-binding fragment transgene comprises the nucleotide sequences encoding the heavy and light chain variable domains of E06-scFv (having amino acid sequences of SEQ ID NOs. 59 and 60, respectively, see Table 4 and FIG. 5D). E06-scFv is a scFv molecule and, thus, contains the heavy and light chain variable domains of an anti-OxPL mAb connected by a flexible linker. The nucleotide sequences may be codon optimized for expression in human cells and may, for example, comprise the nucleotide sequences of SEQ ID NO: 159 (encoding the E06-scFv heavy chain variable domain) and SEQ ID NO: 160 (encoding the E06-scFv light chain variable) as set forth in Table 5. E06 is an scFv and, as such, the scFv is expressed as one protein chain, with a linker between the light and heavy chains. The scFv has a leader sequence at the N-terminus for appropriate expression and secretion in human cells, particularly, human liver cells (such as hepatocytes) or human muscle cells. In other embodiments where the heavy and light chains are expressed as separate proteins, both have a signal or leader sequence at the N-terminus appropriate for expression and secretion in human cells, in particular, human liver cells (e.g., hepatocytes) or human muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the light chain variable domain sequence, a flexible peptide linker. The flexible peptide linker sequence can comprise flexible residues such as glycine (G) or serine (S). In some embodiments, the flexible peptide linker can comprise 10-30 residues or G, S, or both G and S. Charged residues such as E and K can be used and interspersed to enhance solubility. The flexible peptide linker sequence can have the amino acid sequence of (GGGGS)ₙ, wherein n can be 1, 2, 3, 4, 5, or 6 (SEQ ID NO: 243). In this case, the signal sequence is fused to the N-terminus of the scFv, either the heavy or light chain variable domain sequence, as the case may be.

In certain embodiments, the anti-OxPL antigen-binding fragment transgene encodes an OxPL antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 60. In certain embodiments, the anti-OxPL antigen-binding fragment transgene encodes an OxPL antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 59. In certain embodiments, the anti-OxPL antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 60 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 59. In specific embodiments, the OxPL antigen binding fragment comprises a heavy chain comprising an amino acid sequence of SEQ ID NO: 59 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 5D) or are substitutions with an amino acid present at that position in the heavy chain of one or more of the other therapeutic antibodies, for example, as identified by the alignment in FIG. 11A. In specific embodiments, the OxPL antigen binding fragment comprises a light chain comprising an amino acid sequence of SEQ ID NO: 60 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 5D) or are substitutions with an amino acid present at that position in the light chain of one or more of the other therapeutic antibodies, for example, as identified by the alignment in FIG. 11B.

In certain embodiments, the anti-OxPL antigen-binding fragment transgene encodes a hyperglycosylated E06-scFv Fab, comprising a heavy chain and a light chain of SEQ ID NOs: 59 and 60, respectively, with optionally the mutation T118N (heavy chain) (see FIGS. 11A (heavy chain) and B (light chain)).

In certain embodiments, the anti-OxPL antigen-binding fragment transgene encodes an antigen-binding fragment and comprises the nucleotide sequences encoding the six E06-scFv CDRs which are underlined in the heavy and light chain variable domain sequences of FIG. 5D which are spaced between framework regions, generally human framework regions, and associated with constant domains depending upon the form of the antigen-binding molecule, as is known in the art to form the heavy and/or light chain variable domain of an anti-OxPL antibody or antigen-binding fragment thereof.

### Gene Therapy Methods

Provided are methods of treating human subjects for cardiovascular disease, ACD, hypercholesterolemia, abnormally high levels of non-HDL cholesterol and/or LDL, aortic stenosis, hepatic stenosis, and/or abnormal atherosclerotic plaque by administration of a viral vector containing a transgene encoding an anti-OxPL mAb, or antigen binding fragment thereof. The antibody may be E06-scFv, and is preferably a Fab fragment thereof, or other antigen-binding fragment thereof. In embodiments, the patient has been diagnosed with and/or has symptoms associated with cardiovascular disease, ACD, hypercholesterolemia, abnormally high levels of non-HDL cholesterol and/or LDL, aortic stenosis, hepatic stenosis, and/or abnormal atherosclerotic plaque. Recombinant vectors used for delivering the transgene are described in Section 5.4.2. Such vectors should have a tropism for human liver or muscle cells and can include non-replicating rAAV, particularly those bearing an AAV8 or AAV9 capsid. The recombinant vectors, such as shown in FIG. 5D, can be administered in any manner such that the recombinant vector enters the liver or muscle tissue, preferably by introducing the recombinant vector into the bloodstream. See Section 5.5.2 for details regarding the methods of treatment.

Subjects to whom such gene therapy is administered can be those responsive to anti-OxPL therapy. In particular embodiments, the methods encompass treating patients who have been diagnosed with cardiovascular disease, ACD, hypercholesterolemia, abnormally high levels of non-HDL cholesterol and/or LDL, aortic stenosis, hepatic stenosis, and/or abnormal atherosclerotic plaque, or have one or more symptoms associated therewith, and identified as responsive to treatment with an anti-OxPL antibody or considered a good candidate for therapy with an anti-OxPL antibody. In specific embodiments, the patients have previously been treated with E06-scFv or E06, and have been found to be responsive to E06-scFv or E06. To determine responsiveness, the anti-OxPL antibody or antigen-binding fragment transgene product (e.g., produced in cell culture, bioreactors, etc.) may be administered directly to the subject.

### Human Post Translationally Modified Antibodies

The production of the anti-OxPL HuPTM mAb or HuPTM Fab, should result in a "biobetter" molecule for the treatment of cardiovascular disease, ACD, hypercholesterolemia, abnormally high levels of non-HDL cholesterol and/or LDL, aortic stenosis, hepatic stenosis, and/or abnormal atherosclerotic plaque accomplished via gene therapy - e.g., by administering a viral vector or other DNA expression construct encoding the anti-OxPL HuPTM Fab, subcutaneously, intramuscularly, or intravenously to human subjects (patients) diagnosed with or having one or more symptoms of cardiovascular disease, ACD, hypercholesterolemia, abnormally high levels of non-HDL cholesterol and/or LDL, aortic stenosis, hepatic stenosis, and/or abnormal atherosclerotic plaque, to create a permanent depot in the liver or muscle tissue that continuously supplies the fully-human post-translationally modified, e.g., human-glycosylated, sulfated transgene product produced by transduced liver or muscle cells.

In specific embodiments, the anti-OxPL HuPTM mAb or antigen-binding fragment thereof has heavy and light chains with the amino acid sequences of the heavy and light chain Fab portions of E06-scFv as set forth in FIG. 5D (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N53 of the heavy chain (SEQ ID NO:59). Alternatively or in addition to, the HuPTM scFv or other antigen binding-fragment thereof with the heavy and light chain variable domain sequences of E06-scFv has a sulfation group at Y58 and/or Y62 and/or Y96 and/or Y97 of the heavy chain (SEQ ID NO: 59) and/or Y42 of the light chain (SEQ ID NO: 60). In other embodiments, the anti-OxPL HuPTM mAb or antigen-binding fragment thereof does not contain detectable NeuGc moieties and/or does not contain detectable alpha-Gal moieties.

In certain embodiments, the HuPTM mAb or Fab of scFv is therapeutically effective and is at least 0.5%, 1% or 2% glycosylated and/or sulfated and may be at least 5%, 10% or even 50% or 100% glycosylated and/or sulfated. The goal of gene therapy treatment provided herein is to treat, slow and/or arrest the progression of cardiovascular disease, ACD, hypercholesterolemia, abnormally high levels of non-HDL cholesterol and/or LDL, aortic stenosis, hepatic stenosis, and/or abnormal atherosclerotic plaque. Efficacy may be monitored by monitoring LDL levels, inflammation markers, or for changes in the degree of aortic stenosis, such as by monitoring for changes in the aortic valve area, peak and mean transvalvular gradients, and/or maximum aortic velocity. For example, efficacy can be monitored by assessing mean percent change in LDL from baseline.

Combinations of delivery of the anti-OxPL HuPTM mAb or antigen-binding fragment thereof, to the liver or muscle accompanied by delivery of other available treatments are encompassed by the methods provided herein. The additional treatments may be administered before, concurrently, or subsequent to the gene therapy treatment. Available treatments for cardiovascular disease, ACD, hypercholesterolemia, abnormally high levels of non-HDL cholesterol and/or LDL, aortic stenosis, hepatic stenosis, and/or abnormal atherosclerotic plaque that could be combined with the gene therapy provided herein include but are not limited to diet, statins, ezetimibe, and LDL apheresis and administration with anti-OxPL agents, including but not limited to E06-scFv.

### 5.3.8. Anti-RANKL HuPTM Constructs and Formulations for Osteoporosis

Compositions and methods are described for the delivery of HuPTM mAbs and antigen-binding fragments thereof, such as HuPTM Fabs, that bind to receptor activator of nuclear factor kappa-B ligand (RANKL) derived from anti-RANKL antibody, such as denosumab (FIG. 6), and indicated for treating osteoporosis or abnormal bone loss or weakness (e.g., treating giant cell tumor of bone, treating treatment-induced bone loss, slowing the loss of (or increasing) bone mass in breast and prostate cancer patients, preventing skeletal-related events due to bone metastasis or for decreasing bone resorption and turnover. In particular embodiments, the HuPTM mAb has the amino acid sequence of denosumab or an antigen binding fragment thereof. The amino acid sequence of Fab fragment of this antibody is provided in FIG. 6. Delivery may be accomplished via gene therapy - e.g., by administering a viral vector or other DNA expression construct encoding an RANKL-binding HuPTM mAb (or an antigen binding fragment and/or a hyperglycosylated derivative or other derivative, thereof) to patients (human subjects) diagnosed with osteoporosis or suffering bone loss to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to RANKL that can be administered to deliver the HuPTM mAb or antigen binding fragment in a patient. The transgene is a nucleic acid comprising the nucleotide sequences encoding an antigen binding fragment of an antibody that binds to RANKL, such as denosumab or variants thereof as detailed herein. The transgene may also encode an anti-RANKL antigen binding fragment that contains additional glycosylation sites *(e.g.,* see Courtois et al.).

In certain embodiments, the anti-RANKL antigen-binding fragment transgene comprises the nucleotide sequences encoding the heavy and light chains of the Fab portion of denosumab (having amino acid sequences of SEQ ID NOs. 27 and 28, respectively, see Table 4 and FIG 6). The nucleotide sequences may be codon optimized for expression in human cells and may, for example, comprise the nucleotide sequences of SEQ ID NO: 127 (encoding the denosumab heavy chain Fab portion) and SEQ ID NO: 128 (encoding the denosumab light chain Fab portion) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-RANKL-antigen binding domain has a heavy chain variable domain of SEQ ID NO: 27 with additional hinge region sequence starting after the C-terminal aspartate (D), contains all or a portion of the amino acid sequence KTHTCPPCPAPELLGG (SEQ ID NO: 222), and specifically, KTHL (SEQ ID NO: 223), KTHT (SEQ ID NO: 224), KTHTCPPCPA (SEQ ID NO: 225), KTHLCPPCPA (SEQ ID NO: 226), KTHTCPPCPAPELLGGPSVFL (SEQ ID NO: 227) or KTHLCPPCPAPELLGGPSVFL (SEQ ID NO: 228) as set forth in FIG 6. These hinge regions may be encoded by nucleotide sequences at the 3' end of SEQ ID NO: 27 by the hinge region encoding sequences set forth in Table 5.

In certain embodiments, the anti-RANKL antigen-binding fragment transgene encodes an RANKL antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 28. In certain embodiments, the anti-RANKL antigen-binding fragment transgene encodes an RANKL antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 27. In certain embodiments, the anti-RANKL antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 28 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 27. In specific embodiments, the RANKL antigen binding fragment comprises a heavy chain comprising an amino acid sequence of SEQ ID NO: 27 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 6) or are substitutions with an amino acid present at that position in the heavy chain of one or more of the other therapeutic antibodies, for example, as identified by the alignment in FIG. 11A. In specific embodiments, the RANKL antigen binding fragment comprises a light chain comprising an amino acid sequence of SEQ ID NO: 28 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 6) or are substitutions with an amino acid present at that position in the light chain of one or more of the other therapeutic antibodies, for example, as identified by the alignment in FIG. 11B.

In certain embodiments, the anti-RANKL antigen-binding fragment transgene encodes a hyperglycosylated denosumab Fab, comprising a heavy chain and a light chain of SEQ ID NOs: 27 and 28, respectively, with one or more of the following mutations: L117N (heavy chain), Q161N or Q161S (light chain), and/or E196N (light chain) (see FIGS. 11A (heavy chain) and B (light chain)).

In certain embodiments, the anti-RANKL antigen-binding fragment transgene encodes an antigen-binding fragment and comprises the nucleotide sequences encoding the six denosumab CDRs which are underlined in the heavy and light chain variable domain sequences of FIG. 6 which are spaced between framework regions, generally human framework regions, and associated with constant domains depending upon the form of the antigen-binding molecule, as is known in the art to form the heavy and/or light chain variable domain of an anti-RANKL antibody or antigen-binding fragment thereof.

### Gene Therapy Methods

Provided are methods of treating human subjects for osteoporosis or abnormal bone loss (for example, in breast or prostate cancer patients or due to bone metastases) by administration of a viral vector containing a transgene encoding an anti-RANKL antibody, or antigen binding fragment thereof. The antibody may be denosumab, and is preferably a Fab fragment thereof, or other antigen-binding fragment thereof. In embodiments, the patient has been diagnosed with and/or has symptoms associated with osteoporosis or abnormal bone loss. Recombinant vectors used for delivering the transgene are described in Section 5.4.2. Such vectors should have a tropism for human liver or muscle cells and can include non-replicating rAAV, particularly those bearing an AAV8 or AAV9 capsid. The recombinant vector, such as shown in FIG. 6, can be administered in any manner such that the recombinant vector enters the liver or muscle tissue, preferably by introducing the recombinant vector into the bloodstream. See Section 5.5.2 for details regarding the methods of treatment.

Subjects to whom such gene therapy is administered can be those responsive to anti-RANKL therapy. In particular embodiments, the methods encompass treating patients who have been diagnosed with osteoporosis or abnormal bone loss, or have one or more symptoms associated therewith, and identified as responsive to treatment with an anti-RANKL antibody or considered a good candidate for therapy with an anti-RANKL antibody. In specific embodiments, the patients have previously been treated with denosumab, and have been found to be responsive to denosumab. To determine responsiveness, the anti-RANKL antibody or antigen-binding fragment transgene product (e.g., produced in cell culture, bioreactors, etc.) may be administered directly to the subject.

### Human Post Translationally Modified Antibodies

The production of the anti-RANKL HuPTM mAb or HuPTM Fab, should result in a "biobetter" molecule for the treatment of osteoporosis or bone loss accomplished via gene therapy - e.g., by administering a viral vector or other DNA expression construct encoding the anti-RANKL HuPTM Fab, intravenously to human subjects (patients) diagnosed with or having one or more symptoms of osteoporosis or bone loss, to create a permanent depot in the liver or muscle tissue that continuously supplies the fully-human post-translationally modified, e.g., human-glycosylated, sulfated transgene product produced by transduced liver or muscle cells.

The cDNA construct for the anti-RANKL HuPTMmAb or anti-RANKL HuPTM Fab should include a signal peptide that ensures proper co- and post-translational processing (glycosylation and protein sulfation) by the transduced liver or muscle cells. For example, the signal sequence may be MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

As an alternative, or an additional treatment to gene therapy, the anti-RANKL HuPTM mAb or HuPTM Fab can be produced in human cell lines by recombinant DNA technology, and administered to patients diagnosed with osteoporosis or bone loss, or for whom therapy for osteoporosis or bone loss is considered appropriate.

In specific embodiments, the anti-RANKL HuPTM mAb or antigen-binding fragment thereof has heavy and light chains with the amino acid sequences of the heavy and light chain Fab portions of denosumab as set forth in FIG. 6 (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N77 and/or N164 and/or Q114 of the heavy chain (SEQ ID NO:27) or N159 and/or N211 and/or Q101 of the light chain (SEQ ID NO:28). Alternatively or in addition to, the HuPTM mAb or antigen binding-fragment thereof with the heavy and light chain variable domain sequences of denosumab has a sulfation group at Y94 and/or Y95 of the heavy chain (SEQ ID NO:27) and/or Y88 of the light chain (SEQ ID NO:28). In other embodiments, the anti-RANKL HuPTM mAb or antigen-binding fragment thereof does not contain detectable NeuGc moieties and/or does not contain detectable alpha-Gal moieties.

In certain embodiments, the HuPTM mAb or Fab is therapeutically effective and is at least 0.5%, 1% or 2% glycosylated and/or sulfated and may be at least 5%, 10% or even 50% or 100% glycosylated and/or sulfated. The goal of gene therapy treatment provided herein is to slow or arrest the progression of osteoporosis or bone loss. Efficacy may be monitored by evaluating bone tissue or skeletal events or the lack of skeletal events. For example, with regard to osteoporosis, efficacy can be monitored by a bone mineral content assessment, assessment of radiographs for vertebral fractures, or diagnostic imaging for clinical fractures confirmation.

Combinations of delivery of the anti-RANKL HuPTM mAb or antigen-binding fragment thereof, to the liver or muscles accompanied by delivery of other available treatments are encompassed by the methods provided herein. The additional treatments may be administered before, concurrently, or subsequent to the gene therapy treatment. Available treatments for osteoporosis or bone loss that could be combined with the gene therapy provided herein include but are not limited to bisphosphonates (e.g., zoledronic acid), parathyroid hormone (e.g., teriparatide [PTH 1-34] and/or full-length PTH 1-84), calcium, vitamin D, and chemotherapy, cryotherapy, or radiotherapy in patients diagnosed with cancer, and administration with anti-RANKL agents, including but not limited to denosumab.

### 5.3.9 PD Blocker HuPTM Constructs and Formulations for Cancer and Lymphoma

Compositions and methods are described for the delivery of HuPTM mAbs and antigen-binding fragments thereof, such as HuPTM Fabs, that bind to programmed cell death protein 1 (PD-1), programmed death-ligand 1 (PD-L1), or programmed death-ligand 2 (PD-L2) derived from PD-1 blockers (e.g., anti-PD-1, anti-PD-L1, or anti-PD-L2), indicated for treating unresectable/metastatic melanoma, lymphomas (e.g., Hodgkin lymphoma), and carcinomas (e.g., renal cell carcinoma, squamous cell carcinoma, and non-small cell lung carcinomas). In particular embodiments, the HuPTM mAb has the amino acid sequence of nivolumab, pembrolizumab, or an antigen binding fragment of one of the foregoing. The amino acid sequences of Fab fragments of nivolumab and pembrolizumab are provided in FIGS. 7A and 7B, respectively. Delivery may be accomplished via gene therapy - e.g., by administering a viral vector or other DNA expression construct encoding an PD-1/PD-L1/PD-L2 binding HuPTM mAb (or an antigen binding fragment and/or a hyperglycosylated derivative or other derivative, thereof) to patients (human subjects) diagnosed with, or having one or more symptoms of melanoma, carcinomas, or lymphomas to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to PD-1/PD-L1/PD-L2 that can be administered to deliver the HuPTM mAb or antigen binding fragment in a patient. The transgene is a nucleic acid comprising the nucleotide sequences encoding an antigen binding fragment of an antibody that binds to PD-1/PD-L1/PD-L2, such as nivolumab, pembrolizumab, or variants thereof as detailed herein. The transgene may also encode an anti-PD-1, anti-PD-L1, or an anti-PD-L2 antigen binding fragment that contains additional glycosylation sites *(e.g.,* see Courtois et al.).

In certain embodiments, the anti-PD-1 antigen-binding fragment transgene comprises the nucleotide sequences encoding the heavy and light chains of the Fab portion of nivolumab (having amino acid sequences of SEQ ID NOs. 29 and 30, respectively, see Table 4 and FIG. 7A). The nucleotide sequences may be codon optimized for expression in human cells and may, for example, comprise the nucleotide sequences of SEQ ID NO: 129 (encoding the nivolumab heavy chain Fab portion) and SEQ ID NO: 130 (encoding the nivolumab light chain Fab portion) as set forth in Table 5. The heavy and light chain sequences both have a signal or leader sequence at the N-terminus appropriate for expression and secretion in human cells, in particular, human liver cells (e.g., hepatocytes) or human muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-integrin-antigen binding domain has a heavy chain variable domain of SEQ ID NO: 29 with additional hinge region sequence starting after the C-terminal tyrosine (Y), contains all or a portion of the amino acid sequence GPPCPPCPAPEFLG (SEQ ID NO: 240), and specifically, GPPCPPCPA (SEQ ID NO: 229) or GPPCPPCPAPEFLGPSVFL (SEQ ID NO: 241) as set forth in FIG 7A. These hinge regions may be encoded by nucleotide sequences at the 3' end of SEQ ID NO: 29 by the hinge region encoding sequences set forth in Table 5.

In certain embodiments, the anti-PD-1 antigen-binding fragment transgene encodes an PD-1 antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 30. In certain embodiments, the anti-PD-1 antigen-binding fragment transgene encodes an PD-1 antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 29. In certain embodiments, the anti-PD-1, antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 30 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 29. In specific embodiments, the PD-1 antigen binding fragment comprises a heavy chain comprising an amino acid sequence of SEQ ID NO: 29 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 7A) or are substitutions with an amino acid present at that position in the heavy chain of one or more of the other therapeutic antibodies, for example, as identified by the alignment in FIG. 11A. In specific embodiments, the PD-1 antigen binding fragment comprises a light chain comprising an amino acid sequence of SEQ ID NO: 30 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 7A) or are substitutions with an amino acid present at that position in the light chain of one or more of the other therapeutic antibodies, for example, as identified by the alignment in FIG. 11B.

In certain embodiments, the anti-PD-1 antigen-binding fragment transgene encodes a hyperglycosylated nivolumab Fab, comprising a heavy chain and a light chain of SEQ ID NOs: 29 and 30, respectively, with one or more of the following mutations: L108N (heavy chain), Q160N or Q160S (light chain), and/or E195N (light chain) (see FIGS. 11A (heavy chain) and B (light chain)).

In certain embodiments, the anti-PD-1 antigen-binding fragment transgene encodes an antigen-binding fragment and comprises the nucleotide sequences encoding the six nivolumab CDRs which are underlined in the heavy and light chain variable domain sequences of FIG. 7A which are spaced between framework regions, generally human framework regions, and associated with constant domains depending upon the form of the antigen-binding molecule, as is known in the art to form the heavy and/or light chain variable domain of an anti-PD-1 antibody or antigen-binding fragment thereof.

In certain embodiments, the anti-PD-1 antigen-binding fragment transgene comprises the nucleotide sequences encoding the heavy and light chains of the Fab portion of pembrolizumab (having amino acid sequences of SEQ ID NOs. 31 and 32, respectively, see Table 4 and FIG. 7B). The nucleotide sequences may be codon optimized for expression in human cells and may, for example, comprise the nucleotide sequences of SEQ ID NO: 131 (encoding the pembrolizumab heavy chain Fab portion) and SEQ ID NO: 132 (encoding the pembrolizumab light chain Fab portion) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-integrin-antigen binding domain has a heavy chain variable domain of SEQ ID NO: 31 with additional hinge region sequence starting after the C-terminal tyrosine (Y), contains all or a portion of the amino acid sequence GPPCPPCPAPEFLG (SEQ ID NO: 240), and specifically, GPPCPPCPA (SEQ ID NO: 229) or GPPCPPCPAPEFLGPSVFL (SEQ ID NO: 241) as set forth in FIG 7B. These hinge regions may be encoded by nucleotide sequences at the 3' end of SEQ ID NO: 31 by the hinge region encoding sequences set forth in Table 5.

In certain embodiments, the anti-PD-1 antigen-binding fragment transgene encodes an PD-1 antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 32. In certain embodiments, the anti-PD-1 antigen-binding fragment transgene encodes an PD-1 antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 31. In certain embodiments, the anti-PD-1 antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 32 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 31. In specific embodiments, the PD-1 antigen binding fragment comprises a heavy chain comprising an amino acid sequence of SEQ ID NO: 31 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 7B) or are substitutions with an amino acid present at that position in the heavy chain of one or more of the other therapeutic antibodies, for example, as identified by the alignment in FIG. 11A. In specific embodiments, the PD-1 antigen binding fragment comprises a light chain comprising an amino acid sequence of SEQ ID NO: 32 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 7B) or are substitutions with an amino acid present at that position in the light chain of one or more of the other therapeutic antibodies, for example, as identified by the alignment in FIG. 11B.

In certain embodiments, the anti-PD-1 antigen-binding fragment transgene encodes a hyperglycosylated pembrolizumab Fab, comprising a heavy chain and a light chain of SEQ ID NOs: 31 and 32, respectively, with one or more of the following mutations: T115N (heavy chain) Q164N or Q164S (light chain), and/or E199N (light chain) (see FIGS. 11A (heavy chain) and B (light chain)).

In certain embodiments, the anti-PD-1 antigen-binding fragment transgene encodes an antigen-binding fragment and comprises the nucleotide sequences encoding the six pembrolizumab CDRs which are underlined in the heavy and light chain variable domain sequences of FIG. 7B which are spaced between framework regions, generally human framework regions, and associated with constant domains depending upon the form of the antigen-binding molecule, as is known in the art to form the heavy and/or light chain variable domain of an anti-PD-1 antibody or antigen-binding fragment thereof.

### Gene Therapy Methods

Provided are methods of treating human subjects for melanoma, carcinoma, or lymphoma by administration of a viral vector containing a transgene encoding one or more of the anti-PD-1, anti-PD-L1, and anti-PD-L2 antibody, or antigen binding fragment thereof. In particular, methods are provided for treatment of metastatic melanoma, lymphoma, non-small cell lung carcinoma, head and neck squamous cell cancer, urothelial carcinoma, microsatellite instability-high cancer, gastric cancer, renal cell carcinoma, mismatch repair deficit metastatic colon cancer, or hepatocellular carcinoma by administration of a viral vector containing a transgene encoding one or more of the anti-PD-1, anti-PD-L1, and anti-PD-L2 antibody, or antigen binding fragment thereof. The antibody may be nivolumab and pembrolizumab, and are preferably a Fab fragment thereof, or other antigen-binding fragment thereof. In embodiments, the patient has been diagnosed with and/or has symptoms associated with melanoma, carcinoma, or lymphoma. Recombinant vectors used for delivering the transgene are described in Section 5.4.2. Such vectors should have a tropism for human liver or muscle cells and can include non-replicating rAAV, particularly those bearing an AAV8 or AAV9 capsid. The recombinant vectors, such as those shown in FIGS. 7A and 7B, can be administered in any manner such that the recombinant vector enters the liver or muscle tissue, preferably by introducing the recombinant vector into the bloodstream. See Section 5.5.2 for details regarding the methods of treatment.

Subjects to whom such gene therapy is administered can be those responsive to anti-PD-1, anti-PD-L1, or anti-PD-L2 therapy. In particular embodiments, the methods encompass treating patients who have been diagnosed with melanoma, carcinoma, or lymphoma, or have one or more symptoms associated therewith, and identified as responsive to treatment with an anti-PD-1, anti-PD-L1, or anti-PD-L2 antibody or considered a good candidate for therapy with an anti-PD-1, anti-PD-L1, or anti-PD-L2 antibody. In specific embodiments, the patients have previously been treated with nivolumab or pembrolizumab, and have been found to be responsive to nivolumab or pembrolizumab. To determine responsiveness, the anti-PD-1, anti-PD-L1, or anti-PD-L2 antibody or antigen-binding fragment transgene product (e.g., produced in cell culture, bioreactors, etc.) may be administered directly to the subject.

### Human Post Translationally Modified Antibodies

The production of the anti-PD-1, anti-PD-L1, and/or anti-PD-L2 HuPTM mAb or HuPTM Fab, should result in a "biobetter" molecule for the treatment of melanoma, carcinomas, or lymphomas accomplished via gene therapy - e.g., by administering a viral vector or other DNA expression construct encoding the anti-PD-1, anti-PD-L1, and/or anti-PD-L2 HuPTM Fab, subcutaneously, intramuscularly, or intravenously to human subjects (patients) diagnosed with or having one or more symptoms of melanoma, carcinomas, lymphoma, or other cancers to create a permanent depot in the liver or muscle tissue that continuously supplies the fully-human post-translationally modified, e.g., human-glycosylated, sulfated transgene product produced by transduced liver or muscle cells.

The cDNA constructs for the HuPTMmAb or HuPTM Fab should include a signal peptide that ensures proper co- and post-translational processing (glycosylation and protein sulfation) by the transduced liver or muscle cells. For example, the signal sequence may be MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

As an alternative, or an additional treatment to gene therapy, the anti-PD-1, anti-PD-L1, or anti-PD-L2 HuPTM mAb or HuPTM Fab can be produced in human cell lines by recombinant DNA technology, and administered to patients diagnosed with metastatic melanoma, lymphoma, non-small cell lung carcinoma, head and neck squamous cell cancer, urothelial carcinoma, microsatellite instability-high cancer, gastric cancer, renal cell carcinoma, mismatch repair deficit metastatic colon cancer, or hepatocellular carcinoma, or for whom therapy for metastatic melanoma, lymphoma, non-small cell lung carcinoma, head and neck squamous cell cancer, urothelial carcinoma, microsatellite instability-high cancer, gastric cancer, renal cell carcinoma, mismatch repair deficit metastatic colon cancer, or hepatocellular carcinoma is considered appropriate.

In specific embodiments, the anti-PD-1 HuPTM mAb or antigen-binding fragment thereof has heavy and light chains with the amino acid sequences of the heavy and light chain Fab portions of nivolumab as set forth in FIG. 7A (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N77, Q105, and/or N155 of the heavy chain (SEQ ID NO:29) or N93, Q100, N158, and/or N210 of the light chain (SEQ ID NO:30). Alternatively or in addition to, the HuPTM mAb or antigen binding-fragment thereof with the heavy and light chain variable domain sequences of nivolumab has a sulfation group at Y94 and/or Y95 of the heavy chain (SEQ ID NO: 29) and/or Y86 and/or Y87 of the light chain (SEQ ID NO: 30). In other embodiments, the anti-PD-1 HuPTM mAb or antigen-binding fragment thereof does not contain detectable NeuGc moieties and/or does not contain detectable alpha-Gal moieties.

In specific embodiments, the anti-PD-1 HuPTM mAb or antigen-binding fragment thereof has heavy and light chains with the amino acid sequences of the heavy and light chain Fab portions of pembrolizumab as set forth in FIG. 7B (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions Q112, N162, and/or N204 of the heavy chain (SEQ ID NO: 31) or N162 and/or N214 of the light chain (SEQ ID NO: 32). Alternatively or in addition to, the HuPTM mAb or antigen binding-fragment thereof with the heavy and light chain variable domain sequences of pembrolizumab has a sulfation group at Y94 and/or Y95 of the heavy chain (SEQ ID NO: 31) and/or Y90 and/or Y91 of the light chain (SEQ ID NO: 32). In other embodiments, the anti-PD-1 HuPTM mAb or antigen-binding fragment thereof does not contain detectable NeuGc moieties and/or does not contain detectable alpha-Gal moieties.

In certain embodiments, the HuPTM mAb or Fab is therapeutically effective and is at least 0.5%, 1% or 2% glycosylated and/or sulfated and may be at least 5%, 10% or even 50% or 100% glycosylated and/or sulfated. The goal of gene therapy treatment provided herein is to slow or arrest the progression of metastatic melanoma, lymphoma, non-small cell lung carcinoma, head and neck squamous cell cancer, urothelial carcinoma, microsatellite instability-high cancer, gastric cancer, renal cell carcinoma, mismatch repair deficit metastatic colon cancer, or hepatocellular carcinoma. Efficacy may be monitored by one or more oncology endpoints including overall survival, progression-free survival, time to progression, time to treatment failure, event-free survival, time to next treatment, objective response rate, or duration of response (see, e.g., U.S. Department of Health and Human Services Food and Drug Administration Center for Drug Evaluation and Research, Center for Biologics Evaluation and Research. Guidance for industry: clinical trial endpoints for the approval of cancer drugs and biologics. https://www.fda.gov/downloads/Drugs/Guidances/ucm071590.pdf. Published May 2007. Accessed October 13, 2017; Oncology Endpoints in a Changing Landscape. Manag. Care. 2016; 1(suppl):1-12).

Combinations of delivery of the one or more anti-PD-1, anti-PD-L1, and anti-PD-L2 HuPTM mAbs or antigen-binding fragments thereof, to the liver or muscle accompanied by delivery of other available treatments are encompassed by the methods provided herein. The additional treatments may be administered before, concurrently, or subsequent to the gene therapy treatment. Available treatments for metastatic melanoma, lymphoma, non-small cell lung carcinoma, head and neck squamous cell cancer, urothelial carcinoma, microsatellite instability-high cancer, gastric cancer, renal cell carcinoma, mismatch repair deficit metastatic colon cancer, or hepatocellular carcinoma that could be combined with the gene therapy provided herein include but are not limited to chemotherapy (e.g., cisplatin, gemcitabine, pemetrexed, carboplatin, and/or paclitaxel), radiotherapy, cryotherapy, targeted small molecule therapies, other antibodies, and vaccine therapy and administration with one or more of the anti-PD-1, anti-PD-L1, and anti-PD-L2 agents, including but not limited to nivolumab and pembrolizumab.

### 5.3.10 Anti-VEGF or anti-fD HuPTM Constructs and Formulations for Ocular Disorders

Compositions and methods are described for the delivery of HuPTM mAb and antigen-binding fragments thereof, such as HuPTM Fabs, that bind to vascular endothelial growth factor (VEGF) or complement (e.g., factor D (fD)) derived from anti-VEGF or anti-complement (e.g., anti-fD), respectively, indicated for treating one or more retinal disorders including diabetic retinopathy, myopic choroidal neovascularization (mCNV), macular degeneration (e.g., neovascular (wet) agerelated macular degeneration (AMD)), macular edema (e.g., macular edema following a retinal vein occlusion (RVO) or diabetic macular edema (DME)); for suppressing angiogenesis; or, in the of case those derived from anti-VEGF, for treating one or more types of cancer including epithelial ovarian cancer, fallopian tube cancer, peritoneal cancer cervical cancer, metastatic colorectal cancer, metastatic HER2 negative breast cancer, metastatic renal cell carcinoma, glioblastoma, non-small cell lung cancer (NSCLC). In particular embodiments, the HuPTM mAb has the amino acid sequence of ranibizumab, bevacizumab, lampalizumab, brolucizumab, or an antigen binding fragment of one of the foregoing. The amino acid sequences of Fab fragments of ranibizumab, bevacizumab, and lampalizumab, and the scFv of brolucizumab are provided in FIGS. 8A to 8D, respectively. Delivery may be accomplished via gene therapy - e.g., by administering a viral vector or other DNA expression construct encoding a VEGF-binding or Factor D-binding HuPTM mAb (or an antigen binding fragment and/or a hyperglycosylated derivative or other derivative, thereof, including an scFv) to patients (human subjects) diagnosed with, or having one or more symptoms of a retinal disorder (e.g. diabetic retinopathy, mCNV, macular degeneration, or macular edema) or cancer (e.g., epithelial ovarian cancer, fallopian tube cancer, peritoneal cancer cervical cancer, metastatic colorectal cancer, metastatic HER2 negative breast cancer, metastatic renal cell carcinoma, glioblastoma, or NSCLC) to create a permanent depot that continuously supplies the human PTM, e.g., human-glycosylated, transgene product.

Provided are recombinant vectors containing a transgene encoding a HuPTM mAb or HuPTM Fab (or other antigen binding fragment of the HuPTM mAb) that binds to VEGF or fD that can be administered to deliver the HuPTM mAb or antigen binding fragment in a patient. The transgene is a nucleic acid comprising the nucleotide sequences encoding an antigen binding fragment of an antibody that binds to VEGF or fD, such as ranibizumab, bevacizumab, lampalizumab, brolucizumab, or variants thereof as detailed herein. The transgene may also encode an anti-VEGF or anti-fD antigen binding fragment that contains additional glycosylation sites *(e.g.,* see Courtois et al.).

In certain embodiments, the anti-VEGF antigen-binding fragment transgene comprises the nucleotide sequences encoding the heavy and light chains of the Fab portion of ranibizumab (having amino acid sequences of SEQ ID NOs. 33 and 34, respectively, see Table 4 and FIG. 8A). The nucleotide sequences may be codon optimized for expression in human cells and may, for example, comprise the nucleotide sequences of SEQ ID NO: 133 (encoding the ranibizumab heavy chain Fab portion) and SEQ ID NO: 134 (encoding the ranibizumab light chain Fab portion) as set forth in Table 5. The heavy and light chain sequences both have a signal or leader sequence at the N-terminus appropriate for expression and secretion in human cells, in particular, one or more cells forming the retina. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 1 that correspond to the proteins secreted by one or more cells forming the retina. Alternatively, the signal sequence may be appropriate for expression in muscle or liver cells, such as those listed in Tables 2 and 3 *infra.*

In addition to the heavy and light chain variable domain sequences, the transgenes may comprise, at the C-terminus of the heavy chain variable domain sequence, all or a portion of the hinge region. In specific embodiments, the anti-VEGF antigen binding domain has a heavy chain variable domain of SEQ ID NO: 33 with additional hinge region sequence starting after the C-terminal aspartic acid (D), contains all or a portion of the amino acid sequence KTHTCPPCPAPELLGG (SEQ ID NO: 222), and specifically, KTHT (SEQ ID NO: 224), KTHL (SEQ ID NO: 223), KTHTCPPCPA (SEQ ID NO: 225), KTHI,CPPCPA (SEQ ID NO: 226), KTHTCPPCPAPELLGGPSVFL (SEQ ID NO: 227), or KTHLCPPCPAPELLGGPSVFL (SEQ ID NO: 228) as set forth in FIG 8A. These hinge regions may be encoded by nucleotide sequences at the 3' end of SEQ ID NO: 33 by the hinge region encoding sequences set forth in Table 5.

In certain embodiments, the anti-VEGF antigen-binding fragment transgene encodes an VEGF antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 34. In certain embodiments, the anti-VEGF antigen-binding fragment transgene encodes an VEGF antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 33. In certain embodiments, the anti-VEGF antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 34 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 33. In specific embodiments, the VEGF antigen binding fragment comprises a heavy chain comprising an amino acid sequence of SEQ ID NO: 33 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8A) or are substitutions with an amino acid present at that position in the heavy chain of one or more of the other therapeutic antibodies, for example, as identified by the alignment in FIG. 11A. In specific embodiments, the VEGF antigen binding fragment comprises a light chain comprising an amino acid sequence of SEQ ID NO: 34 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8A) or are substitutions with an amino acid present at that position in the light chain of one or more of the other therapeutic antibodies, for example, as identified by the alignment in FIG. 11B.

In certain embodiments, the anti-VEGF antigen-binding fragment transgene encodes a hyperglycosylated ranibizumab Fab, comprising a heavy chain and a light chain of SEQ ID NOs: 33 and 34, respectively, with one or more of the following mutations: L118N (heavy chain), Q160N or Q160S (light chain), and/or E195N (light chain) (see FIGS. 11A (heavy chain) and B (light chain)).

In certain embodiments, the anti-VEGF antigen-binding fragment transgene encodes an antigen-binding fragment and comprises the nucleotide sequences encoding the six ranibizumab CDRs which are underlined in the heavy and light chain variable domain sequences of FIG. 8A which are spaced between framework regions, generally human framework regions, and associated with constant domains depending upon the form of the antigen-binding molecule, as is known in the art to form the heavy and/or light chain variable domain of an anti-VEGF antibody or antigen-binding fragment thereof.

In certain embodiments, the anti-VEGF antigen-binding fragment transgene comprises the nucleotide sequences encoding the heavy and light chains of the Fab portion of bevacizumab (having amino acid sequences of SEQ ID NOs. 35 and 36, respectively, see Table 4 and FIG. 8B). The nucleotide sequences may be codon optimized for expression in human cells and may, for example, comprise the nucleotide sequences of SEQ ID NO: 135 (encoding the bevacizumab heavy chain Fab portion) and SEQ ID NO: 136 (encoding the bevacizumab light chain Fab portion) as set forth in Table 5. The heavy and light chain sequences both have a signal or leader sequence at the N-terminus appropriate for expression and secretion in human cells, in particular, one or more retina cell or liver cell types. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 1 or 3 that correspond to the proteins secreted by retina cell or liver cell types, respectively.

In addition to the heavy and light chain variable domain sequences, the transgenes may comprise, at the C-terminus of the heavy chain variable domain sequence, all or a portion of the hinge region. In specific embodiments, the anti-integrin-antigen binding domain has a heavy chain variable domain of SEQ ID NO: 35 with additional hinge region sequence starting after the C-terminal aspartic acid (D), contains all or a portion of the amino acid sequence KTHTCPPCPAPELLGG (SEQ ID NO: 222), and specifically, KTHT (SEQ ID NO: 224), KTHL (SEQ ID NO: 223), KTHTCPPCPA (SEQ ID NO: 225), KTHI,CPPCPA (SEQ ID NO: 226), KTHTCPPCPAPELLGGPSVFL (SEQ ID NO: 227), or KTHLCPPCPAPELLGGPSVFL (SEQ ID NO: 228) as set forth in FIG 8B. These hinge regions may be encoded by nucleotide sequences at the 3' end of SEQ ID NO: 35 by the hinge region encoding sequences set forth in Table 5.

In certain embodiments, the anti-VEGF antigen-binding fragment transgene encodes an VEGF antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 36. In certain embodiments, the anti-VEGF antigen-binding fragment transgene encodes an VEGF antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 35. In certain embodiments, the anti-VEGF antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 36 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 35. In specific embodiments, the VEGF antigen binding fragment comprises a heavy chain comprising an amino acid sequence of SEQ ID NO: 35 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8B) or are substitutions with an amino acid present at that position in the heavy chain of one or more of the other therapeutic antibodies, for example, as identified by the alignment in FIG. 11A. In specific embodiments, the VEGF antigen binding fragment comprises a light chain comprising an amino acid sequence of SEQ ID NO: 36 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8B) or are substitutions with an amino acid present at that position in the light chain of one or more of the other therapeutic antibodies, for example, as identified by the alignment in FIG. 11B.

In certain embodiments, the anti-VEGF antigen-binding fragment transgene encodes a hyperglycosylated bevacizumab Fab, comprising a heavy chain and a light chain of SEQ ID NOs: 35 and 36, respectively, with one or more of the following mutations: L118N (heavy chain) and/or Q160N or Q160S (light chain), and/or E195N (light chain) (see FIGS. 11A (heavy chain) and B (light chain)).

In certain embodiments, the anti-VEGF antigen-binding fragment transgene encodes an antigen-binding fragment and comprises the nucleotide sequences encoding the six bevacizumab CDRs which are underlined in the heavy and light chain variable domain sequences of FIG. 8B which are spaced between framework regions, generally human framework regions, and associated with constant domains depending upon the form of the antigen-binding molecule, as is known in the art to form the heavy and/or light chain variable domain of an anti-VEGF antibody or antigen-binding fragment thereof.

In certain embodiments, the anti-fD antigen-binding fragment transgene comprises the nucleotide sequences encoding the heavy and light chains of the Fab portion of lampalizumab (having amino acid sequences of SEQ ID NOs. 37 and 38, respectively, see Table 4 and FIG. 8C). The nucleotide sequences may be codon optimized for expression in human cells and may, for example, comprise the nucleotide sequences of SEQ ID NO: 137 (encoding the lampalizumab heavy chain Fab portion) and SEQ ID NO: 138 (encoding the lampalizumab light chain Fab portion) as set forth in Table 5. The heavy and light chain sequences both have a signal or leader sequence at the N-terminus appropriate for expression and secretion in human cells, in particular, human one or more cells forming the retina. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 1 that correspond to the proteins secreted by cells forming the retina.

In addition to the heavy and light chain variable domain sequences, the transgenes may comprise, at the C-terminus of the heavy chain variable domain sequence, all or a portion of the hinge region. In specific embodiments, the anti-integrin-antigen binding domain has a heavy chain variable domain of SEQ ID NO: 37 with additional hinge region sequence starting after the C-terminal aspartic acid (D), contains all or a portion of the amino acid sequence KTHT CPPCPAPELLGGPSVFL (SEQ ID NO: 227), and specifically, KTHT (SEQ ID NO: 224), KTHL (SEQ ID NO: 223), KTHTCPPCPA (SEQ ID NO: 225), KTHI,CPPCPA (SEQ ID NO: 226), KTHTCPPCPAPELLGGPSVFL (SEQ ID NO: 227), or KTHLCPPCPAPELLGGPSVFL (SEQ ID NO: 228) as set forth in FIG 8C. These hinge regions may be encoded by nucleotide sequences at the 3' end of SEQ ID NO: 37 by the hinge region encoding sequences set forth in Table 5.

In certain embodiments, the anti-fD antigen-binding fragment transgene encodes an fD antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 38. In certain embodiments, the anti-fD antigen-binding fragment transgene encodes an fD antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 37. In certain embodiments, the anti-fD antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 38 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 37. In specific embodiments, the fD antigen binding fragment comprises a heavy chain comprising an amino acid sequence of SEQ ID NO: 37 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8C) or are substitutions with an amino acid present at that position in the heavy chain of one or more of the other therapeutic antibodies, for example, as identified by the alignment in FIG. 11A. In specific embodiments, the fD antigen binding fragment comprises a light chain comprising an amino acid sequence of SEQ ID NO: 38 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8C) or are substitutions with an amino acid present at that position in the light chain of one or more of the other therapeutic antibodies, for example, as identified by the alignment in FIG. 11B.

In certain embodiments, the anti-fD antigen-binding fragment transgene encodes a hyperglycosylated lampalizumab Fab, comprising a heavy chain and a light chain of SEQ ID NOs: 37 and 38, respectively, with one or more of the following mutations: L110N (heavy chain), Q160N or Q160S (light chain), and/or E195N (light chain) (see FIGS. 11A (heavy chain) and B (light chain)).

In certain embodiments, the anti-fD antigen-binding fragment transgene encodes an antigen-binding fragment and comprises the nucleotide sequences encoding the six lampalizumab CDRs which are underlined in the heavy and light chain variable domain sequences of FIG. 8C which are spaced between framework regions, generally human framework regions, and associated with constant domains depending upon the form of the antigen-binding molecule, as is known in the art to form the heavy and/or light chain variable domain of an anti-fD antibody or antigen-binding fragment thereof.

In certain embodiments, the anti-VEGF antigen-binding fragment transgene comprises the nucleotide sequences encoding the heavy and light chain variable domains of brolucizumab (having amino acid sequences of SEQ ID NOs. 39 and 40, respectively, see Table 4 and FIG. 8D). Brolucizumab is a scFv molecule and, thus, contains the heavy and light chain variable domains of an anti-VEGF mAb connected by a flexible linker. The nucleotide sequences may be codon optimized for expression in human cells and may, for example, comprise the nucleotide sequences of SEQ ID NO: 139 (encoding the brolucizumab heavy chain variable domain portion) and SEQ ID NO: 142 (encoding the brolucizumab light chain variable domain portion) as set forth in Table 5. In the even the heavy and light chain variable domains are expressed as separate proteins, the heavy and light chain sequences each have a signal or leader sequence at the N-terminus appropriate for expression and secretion in human cells, in particular, one or more cells forming the retina. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 1 that correspond to the proteins secreted by one or more cells forming the retina.

In addition to the heavy and light chain variable domain sequences, the transgenes may comprise, at the C-terminus of the light chain variable domain sequence, a flexible peptide linker. The flexible peptide linker sequence can comprise flexible residues such as glycine (G) or serine (S). In some embodiments, the flexible peptide linker can comprise 10-30 residues or G, S, or both G and S. Charged residues such as E and K can be used and interspersed to enhance solubility. The flexible peptide linker sequence can have the amino acid sequence of (GGGGS)ₙ, wherein n can be 1, 2, 3, 4, 5, or 6 (SEQ ID NO: 243). In this case, the signal sequence is fused to the N-terminus of the scFv, either the heavy or light chain variable domain sequence, as the case may be.

In certain embodiments, the anti-VEGF antigen-binding fragment transgene encodes an VEGF antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 40. In certain embodiments, the anti-VEGF antigen-binding fragment transgene encodes an VEGF antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 39. In certain embodiments, the anti-VEGF antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 40 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 39. In specific embodiments, the VEGF antigen binding fragment comprises a heavy chain comprising an amino acid sequence of SEQ ID NO: 39 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8D) or are substitutions with an amino acid present at that position in the heavy chain of one or more of the other therapeutic antibodies, for example, as identified by the alignment in FIG. 11A. In specific embodiments, the VEGF antigen binding fragment comprises a light chain comprising an amino acid sequence of SEQ ID NO: 40 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8D) or are substitutions with an amino acid present at that position in the light chain of one or more of the other therapeutic antibodies, for example, as identified by the alignment in FIG. 11B.

In certain embodiments, the anti-VEGF antigen-binding fragment transgene encodes a hyperglycosylated brolucizumab scFv, comprising a single chain of SEQ ID NOs: 39 and 40, respectively, with the following mutation: L115N (heavy chain) (see FIGS. 11A (heavy chain).

In certain embodiments, the anti-VEGF antigen-binding fragment transgene encodes an antigen-binding fragment and comprises the nucleotide sequences encoding the six brolucizumab CDRs which are underlined in the single chain variable domain sequences of FIG. 8D which are spaced between framework regions, generally human framework regions, and associated with constant domains depending upon the form of the antigen-binding molecule, as is known in the art to form the heavy and/or light chain variable domain of an anti-VEGF antibody or antigen-binding fragment thereof.

### Gene Therapy Methods

Provided are methods of treating human subjects for one or more retinal disorders (such as diabetic retinopathy, mCNV, macular degeneration, or macular edema) or cancer (such as epithelial ovarian cancer, fallopian tube cancer, peritoneal cancer cervical cancer, metastatic colorectal cancer, metastatic HER2 negative breast cancer, metastatic renal cell carcinoma, glioblastoma, or NSCLC) by administration of a viral vector containing a transgene encoding an anti-VEGF antibody or antigen binding fragment thereof. The antibody or Fab fragment thereof may be ranibizumab bevacizumab, or brolucizumab. In embodiments, the patient has been diagnosed with and/or has symptoms associated with one or more of the various retinal disorders or cancers listed above.

Also, provided are methods of treating human subjects for one or more retinal disorders (such as diabetic retinopathy, mCNV, macular degeneration, or macular edema) by administration of a viral vector containing a transgene encoding an anti-fD antibody or antigen binding fragment thereof. The antibody may be lampalizumab, and is preferably a Fab fragment thereof, or other antigen-binding fragment thereof. In embodiments, the patient has been diagnosed with and/or has symptoms associated with one or more of the various retinal disorders listed above.

Recombinant vector used for delivering the transgene are described in Section 5.4.3. Such vectors should have a tropism for human retina-type cells and can include non-replicating rAAV, particularly those bearing an AAV8 capsid. Alternatively, vectors bearing an AAV.7m8 capsid can be used for ocular indications. The recombinant vectors, such as those shown in FIGS. 8A-8D, can be administered in any manner such that the recombinant vector enters the retina, preferably by introducing the recombinant vector into the eye. See Section 5.5.3 for details regarding the methods of treatment. For delivery to the liver, for example, for the treatment of cancer, recombinant vector used for delivering the transgene are described in Section 5.4.2. Such vectors should have a tropism for human liver cells and can include non-replicating rAAV, particularly those bearing an AAV8 or AAV9 capsid. The recombinant vectors, such as those shown in FIGS. 8A-8C, can be administered in any manner such that the recombinant vector enters the liver, preferably by introducing the recombinant vector into the bloodstream. See Section 5.5.2 for details regarding the methods of treatment.

Subjects to whom such gene therapy is administered can be those responsive to anti-VEGF or anti-fD therapy. In particular embodiments, the methods encompass treating patients who have been diagnosed with one or more retinal disorders or types of cancer, or have one or more symptoms associated therewith, and identified as responsive to treatment with an anti-VEGF antibody or anti-fD antibody, or considered a good candidate for therapy with an anti-VEGF antibody or anti-fD antibody. In specific embodiments, the patients have previously been treated with ranibizumab, bevacizumab, lampalizumab, or brolucizumab, and have been found to be responsive to ranibizumab, bevacizumab, lampalizumab, or brolucizumab. To determine responsiveness, the anti-VEGF or anti-fD antibody or antigen-binding fragment transgene product (e.g., produced in cell culture, bioreactors, etc.) may be administered directly to the subject.

### Human Post Translationally Modified Antibodies

The production of the anti-VEGF or anti-fD HuPTM mAb or HuPTM Fab, should result in a "biobetter" molecule for the treatment of one or more retinal disorders or cancers accomplished via gene therapy - e.g., by administering a viral vector or other DNA expression construct encoding the anti-VEGF or anti-fD HuPTM Fab, subretinally, intravitreally, or suprachoroidally to human subjects (patients) diagnosed with or having one or more symptoms of one or more retinal disorders, or by administering a viral vector or other DNA expression construct encoding the anti-VEGF HuPTM Fab, subcutaneously, intramuscularly, or intravenously to human subjects (patients) diagnosed with a cancer, to create a permanent depot in the retina or liver that continuously supplies the fully-human post-translationally modified, e.g., human-glycosylated, sulfated transgene product produced by transduced cells of the retina or liver.

As an alternative, or an additional treatment to gene therapy, the anti-VEGF or anti-fD HuPTM mAb or HuPTM Fab can be produced in human cell lines by recombinant DNA technology, and administered to patients diagnosed with a retinal disorder or cancer for whom therapy for a retinal disorder or cancer is considered appropriate.

In specific embodiments, the anti-VEGF HuPTM mAb or antigen-binding fragment thereof has heavy and light chains with the amino acid sequences of the heavy and light chain Fab portions of ranibizumab as set forth in FIG. 8A (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions Q115 and/or N165 of the heavy chain (SEQ ID NO:33) or Q100, N158, and/or N210 of the light chain (SEQ ID NO:34). Alternatively or in addition to, the HuPTM mAb or antigen binding-fragment thereof with the heavy and light chain variable domain sequences of ranibizumab has a sulfation group at Y94 and/or Y95 of the heavy chain (SEQ ID NO: 33) and/or Y86 and/or Y87 of the light chain (SEQ ID NO: 34). In other embodiments, the anti-VEGF HuPTM mAb or antigen-binding fragment thereof does not contain detectable NeuGc moieties and/or does not contain detectable alpha-Gal moieties.

In specific embodiments, the anti-VEGF HuPTM mAb or antigen-binding fragment thereof has heavy and light chains with the amino acid sequences of the heavy and light chain Fab portions of bevacizumab as set forth in FIG. 8B (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions Q115, and/or N165 of the heavy chain (SEQ ID NO: 35) or Q100, N158, and/or N210 of the light chain (SEQ ID NO: 36). Alternatively or in addition to, the HuPTM mAb or antigen binding-fragment thereof with the heavy and light chain variable domain sequences of bevacizumab has a sulfation group at Y94 and/or Y95 of the heavy chain (SEQ ID NO: 35) and/or Y86 and/or Y87 of the light chain (SEQ ID NO: 36). In other embodiments, the anti-VEGF HuPTM mAb or antigen-binding fragment thereof does not contain detectable NeuGc moieties and/or does not contain detectable alpha-Gal moieties.

In specific embodiments, the anti-fD HuPTM mAb or antigen-binding fragment thereof has heavy and light chains with the amino acid sequences of the heavy and light chain Fab portions of lampalizumab as set forth in FIG. 8C (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions Q107 and/or N157 of the heavy chain (SEQ ID NO: 37) or Q100 and/or N158 and/or N210 of the light chain (SEQ ID NO: 38). Alternatively or in addition to, the HuPTM mAb or antigen binding-fragment thereof with the heavy and light chain variable domain sequences of lampalizumab has a sulfation group at Y60 and/or Y94 and/or Y95 of the heavy chain (SEQ ID NO: 37) and/or Y86 and/or Y87 of the light chain (SEQ ID NO: 38). In other embodiments, the anti-fD HuPTM mAb or antigen-binding fragment thereof does not contain detectable NeuGc moieties and/or does not contain detectable alpha-Gal moieties.

In specific embodiments, the anti-VEGF HuPTM mAb or antigen-binding fragment thereof has heavy and light chains with the amino acid sequences of the heavy and light chain variable domains of brolucizumab as set forth in FIG. 8D (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N77 and/or Q112 of the heavy chain (SEQ ID NO: 39) or N97 and/or Q103 of the light chain (SEQ ID NO: 40). Alternatively or in addition to, the HuPTM mAb or antigen binding-fragment thereof with the heavy and light chain variable domain sequences of brolucizumab has a sulfation group at Y32 and/or Y33 and/or Y34 and/or Y59 and/or Y60 and/or Y94 and/or Y95 of the heavy chain (SEQ ID NO: 39) and/or Y86 and/or Y87 of the light chain (SEQ ID NO: 40). In other embodiments, the anti-VEGF HuPTM mAb or antigen-binding fragment thereof does not contain detectable NeuGc moieties and/or does not contain detectable alpha-Gal moieties.

In certain embodiments, the HuPTM mAb or Fab is therapeutically effective and is at least 0.5%, 1% or 2% glycosylated and/or sulfated and may be at least 5%, 10% or even 50% or 100% glycosylated and/or sulfated. The goal of gene therapy treatment provided herein is to slow or arrest the progression of a retinal disorder or type of cancer, and/or to suppress angiogenesis. In the case of retinal disorders, efficacy may be monitored by monitoring vision acuity. For example, efficacy can be monitored by assessing change in vision acuity from baseline. In the case of a cancer, efficacy can be monitored by assessing one or more oncology endpoints including overall survival, progressionfree survival, time to progression, time to treatment failure, event-free survival, time to next treatment, objective response rate, or duration of response. (see, e.g., U.S. Department of Health and Human Services Food and Drug Administration Center for Drug Evaluation and Research, Center for Biologics Evaluation and Research. Guidance for industry: clinical trial endpoints for the approval of cancer drugs and biologics. https://www.fda.gov/downloads/Drugs/Guidances/ucm071590.pdf. Published May 2007. Accessed October 13, 2017; Oncology Endpoints in a Changing Landscape. Manag. Care. 2016; 1(suppl):1-12).

Combinations of delivery of the anti-VEGF or anti-fD HuPTM mAb or antigen-binding fragment thereof to the retina or liver accompanied by delivery of other available treatments are encompassed by the methods provided herein. The additional treatments may be administered before, concurrently, or subsequent to the gene therapy treatment. Available treatments for diabetic retinopathy, mCNV, macular degeneration, or macular edema that could be combined with the gene therapy provided herein include but are not limited to laser photocoagulation, photodynamic therapy with verteporfin, aflibercept, and/or intravitreal steroids and administration with anti-VEGF or anti-fD agents, including but not limited to ranibizumab, bevacizumab, lampalizumab, or brolucizumab. Available treatments for epithelial ovarian cancer, fallopian tube cancer, peritoneal cancer cervical cancer, metastatic colorectal cancer, metastatic HER2 negative breast cancer, metastatic renal cell carcinoma, glioblastoma, or NSCLC that could be combined with the gene therapy provided herein include but are not limited to chemotherapy (e.g., cisplatin, gemcitabine, pemetrexed, 5-fluorouracil, carboplatin, irinotecan, interferon alfa, oxaliplatin, paclitaxel pegylated liposomal doxorubicin, and/or topotecan), chemotherapy protective drugs (e.g., leucovorin), radiotherapy, cryotherapy, targeted small molecule therapies, other antibodies, afilbercept, and/or vaccine therapy and administration with anti-VEGF, including but not limited to ranibizumab or bevacizumab.

### 5.3.11. Anti-BLyS HuPTM Constructs and Formulations for Systemic Lupus Erythematosus

Compositions and methods are described for the delivery of HuPTM mAbs and antigen-binding fragments thereof, such as HuPTM Fabs, that bind to B-lymphocyte stimulator (BLyS) derived from an anti-BLyS antibody, such as belimumab (FIG. 8E), and indicated for treating systemic lupus erythematosus (SLE) and reducing levels of autoreactive B cells and immunoglobulin producing plasma cells. In particular embodiments, the HuPTM mAb has the amino acid sequence of belimumab or an antigen binding fragment thereof. The amino acid sequence of Fab fragment of this antibody is provided in FIG. 8E. Delivery may be accomplished via gene therapy - e.g., by administering a viral vector or other DNA expression construct encoding an BLyS-binding HuPTM mAb (or an antigen binding fragment and/or a hyperglycosylated derivative or other derivative, thereof) to patients (human subjects) diagnosed with SLE to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to BLyS that can be administered to deliver the HuPTM mAb or antigen binding fragment in a patient. The transgene is a nucleic acid comprising the nucleotide sequences encoding an antigen binding fragment of an antibody that binds to BLyS, such as belimumab or variants thereof as detailed herein. The transgene may also encode an anti-BLyS antigen binding fragment that contains additional glycosylation sites (*e.g.,* see Courtois et al.).

In certain embodiments, the anti-BLyS antigen-binding fragment transgene comprises the nucleotide sequences encoding the heavy and light chains of the Fab portion of belimumab (having amino acid sequences of SEQ ID NOs. 41 and 42, respectively, see Table 4 and FIG. 8E). The nucleotide sequences may be codon optimized for expression in human cells and may, for example, comprise the nucleotide sequences of SEQ ID NO: 141 (encoding the belimumab heavy chain Fab portion) and SEQ ID NO: 142 (encoding the belimumab light chain Fab portion) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-BLyS-antigen binding domain has a heavy chain variable domain of SEQ ID NO: 41 with additional hinge region sequence starting after the C-terminal aspartate (D), contains all or a portion of the amino acid sequence KTHTCPPCPAPELLGG (SEQ ID NO: 222), and specifically, KTHL (SEQ ID NO: 223), KTHT (SEQ ID NO: 224), KTHTCPPCPA (SEQ ID NO: 225), KTHLCPPCPA (SEQ ID NO: 226), KTHTCPPCPAPELLGGPSVFL (SEQ ID NO: 227) or KTHLCPPCPAPELLGGPSVFL (SEQ ID NO: 228) as set forth in FIG 8E. These hinge regions may be encoded by nucleotide sequences at the 3' end of SEQ ID NO: 41 by the hinge region encoding sequences set forth in Table 5.

In certain embodiments, the anti-BLyS antigen-binding fragment transgene encodes an BLyS antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 42. In certain embodiments, the anti-BLyS antigen-binding fragment transgene encodes an BLyS antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 41. In certain embodiments, the anti-BLyS antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 42 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 41. In specific embodiments, the BLyS antigen binding fragment comprises a heavy chain comprising an amino acid sequence of SEQ ID NO: 41 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8E) or are substitutions with an amino acid present at that position in the heavy chain of one or more of the other therapeutic antibodies, for example, as identified by the alignment in FIG. 11A. In specific embodiments, the BLyS antigen binding fragment comprises a light chain comprising an amino acid sequence of SEQ ID NO: 42 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8E) or are substitutions with an amino acid present at that position in the light chain of one or more of the other therapeutic antibodies, for example, as identified by the alignment in FIG. 11B.

In certain embodiments, the anti-BLyS antigen-binding fragment transgene encodes a hyperglycosylated belimumab Fab, comprising a heavy chain and a light chain of SEQ ID NOs: 41 and 42, respectively, with one or more of the following mutations: M118N (heavy chain) and/or Q196N (light chain) (see FIGS. 11A (heavy chain) and B (light chain)).

In certain embodiments, the anti-BLyS antigen-binding fragment transgene encodes an antigen-binding fragment and comprises the nucleotide sequences encoding the six belimumab CDRs which are underlined in the heavy and light chain variable domain sequences of FIG. 8E which are spaced between framework regions, generally human framework regions, and associated with constant domains depending upon the form of the antigen-binding molecule, as is known in the art to form the heavy and/or light chain variable domain of an anti-BLyS antibody or antigen-binding fragment thereof.

### Gene Therapy Methods

Provided are methods of treating human subjects for SLE by administration of a viral vector containing a transgene encoding an anti-BLyS antibody, or antigen binding fragment thereof. The antibody may be belimumab, and is preferably a Fab fragment thereof, or other antigen-binding fragment thereof. In embodiments, the patient has been diagnosed with and/or has symptoms associated with SLE. Recombinant vectors used for delivering the transgene are described in Section 5.4.2. Such vectors should have a tropism for human liver or muscle cells and can include non-replicating rAAV, particularly those bearing an AAV8 or AAV9 capsid. The recombinant vectors, such as those shown in FIG. 8E, can be administered in any manner such that the recombinant vector enters the liver or muscle tissue, preferably by introducing the recombinant vector into the bloodstream. See Section 5.5.2 for details regarding the methods of treatment.

Subjects to whom such gene therapy is administered can be those responsive to anti-BLyS therapy. In particular embodiments, the methods encompass treating patients who have been diagnosed with SLE, or have one or more symptoms associated therewith, and identified as responsive to treatment with an anti-BLyS antibody or considered a good candidate for therapy with an anti-BLyS antibody. In specific embodiments, the patients have previously been treated with belimumab, and have been found to be responsive to belimumab. To determine responsiveness, the anti-BLyS antibody or antigen-binding fragment transgene product (e.g., produced in cell culture, bioreactors, etc.) may be administered directly to the subject.

### Human Post Translationally Modified Antibodies

The production of the anti-BLyS HuPTM mAb or HuPTM Fab, should result in a "biobetter" molecule for the treatment of SLE accomplished via gene therapy - e.g., by administering a viral vector or other DNA expression construct encoding the anti-BLyS HuPTM Fab, intravenously to human subjects (patients) diagnosed with or having one or more symptoms of SLE, to create a permanent depot in the liver or muscle tissue that continuously supplies the fully-human post-translationally modified, e.g., human-glycosylated, sulfated transgene product produced by transduced liver or muscle cells.

The cDNA construct for the anti-BLyS HuPTMmAb or anti-BLyS HuPTM Fab should include a signal peptide that ensures proper co- and post-translational processing (glycosylation and protein sulfation) by the transduced liver or muscle cells. For example, the signal sequence may be MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

As an alternative, or an additional treatment to gene therapy, the anti-BLyS HuPTM mAb or HuPTM Fab can be produced in human cell lines by recombinant DNA technology, and administered to patients diagnosed with SLE, or for whom therapy for SLE is considered appropriate.

In specific embodiments, the anti-BLyS HuPTM mAb or antigen-binding fragment thereof has heavy and light chains with the amino acid sequences of the heavy and light chain Fab portions of belimumab as set forth in FIG. 8E (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N30 and/or N63 and/or N165 of the heavy chain (SEQ ID NO:41) or N68 and/or N95 of the light chain (SEQ ID NO:42). Alternatively or in addition to, the HuPTM mAb or antigen binding-fragment thereof with the heavy and light chain variable domain sequences of belimumab has a sulfation group at Y94 and/or Y95 of the heavy chain (SEQ ID NO:41) and/or Y85 and/or Y86 of the light chain (SEQ ID NO:42). In other embodiments, the anti-BLyS HuPTM mAb or antigen-binding fragment thereof does not contain detectable NeuGc moieties and/or does not contain detectable alpha-Gal moieties.

In certain embodiments, the HuPTM mAb or Fab is therapeutically effective and is at least 0.5%, 1% or 2% glycosylated and/or sulfated and may be at least 5%, 10% or even 50% or 100% glycosylated and/or sulfated. The goal of gene therapy treatment provided herein is to slow or arrest the progression of SLE, reduce the levels of pain or discomfort for the patient, or reduce levels of autoreactive B cells and immunoglobulin producing plasma cells. Efficacy may be monitored by scoring the function, symptoms, or degree of inflammation in the affected tissue or area of the body, e.g., such as the skin, joints, kidneys, lungs, blood cells, heart, and brain. For example, efficacy can be monitored by monitoring the presence, extent, or rate of one or more symptoms including seizure, psychosis, organic brain syndrome, visual disturbance, other neurological problems, alopecia, skin rash, muscle weakness, arthritis, blood vessel inflammation, mucosal ulcers, chest pain worse with deep breathing and manifestations of pleurisy and/or pericarditis and fever. Standardized disease indexes can be used, such as Safety of Estrogens in Lupus Erythematosus National Assessment Systemic Lupus Erythematosus Disease Activity Index (SELENA-SLEDAI); British Isles Lupus Assessment Group (BILAG) A, BILAG B, Systemic Lupus Activity Measure (SLAM), or PGA score. (See e.g., Liang MH et al. (1988) "Measurement of systemic lupus erythematosus activity in clinical research," Arthritis Rheum. 31:817-25; Diaz et al. (2011) "Measures of adult systemic lupus erythematosus: updated version of British Isles Lupus Assessment Group (BILAG 2004), European Consensus Lupus Activity Measurements (ECLAM), Systemic Lupus Activity Measure, Revised (SLAM-R), Systemic Lupus Activity Questionnaire for Population Studies (SLAQ), Systemic Lupus Erythematosus Disease Activity Index 2000 (SLEDAI-2 K), and Systemic Lupus," International Collaborating Clinics/American College of Rheumatology Damage Index (SDI) Arthritis Care Res. 63:S37-46).

Combinations of delivery of the anti-BLyS HuPTM mAb or antigen-binding fragment thereof, to the liver or muscles accompanied by delivery of other available treatments are encompassed by the methods provided herein. The additional treatments may be administered before, concurrently, or subsequent to the gene therapy treatment. Available treatments for SLE that could be combined with the gene therapy provided herein include but are not limited to corticosteroids, antimalarials, NSAIDs, and immunosuppressives, and administration with anti-BLyS agents, including but not limited to belimumab.

### 5.3.12. Anti-CP-C5 HuPTM Constructs and Formulations for Paroxysmal Nocturnal Hemoglobinuria and Atypical Hemolytic Uremic Syndrome

Compositions and methods are described for the delivery of HuPTM mAbs and antigen-binding fragments thereof, such as HuPTM Fabs, that bind to complement protein C5 (or C5a) (CP-C5) derived from an anti-CP-C5 antibody, such as eculizumab (FIG. 8F), and indicated for treating paroxysmal nocturnal hemoglobinuria (PNH), treating atypical hemolytic uremic syndrome (aHUS), reducing the destruction of blood cells, and/or reducing the need for blood transfusions. In particular embodiments, the HuPTM mAb has the amino acid sequence of eculizumab or an antigen binding fragment thereof. The amino acid sequence of the Fab fragment of this antibody is provided in FIG. 8F. Delivery may be accomplished via gene therapy - e.g., by administering a viral vector or other DNA expression construct encoding an CP-C5-binding HuPTM mAb (or an antigen binding fragment and/or a hyperglycosylated derivative or other derivative, thereof) to patients (human subjects) diagnosed with PNH or aHUS to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to CP-C5 that can be administered to deliver the HuPTM mAb or antigen binding fragment in a patient. The transgene is a nucleic acid comprising the nucleotide sequences encoding an antigen binding fragment of an antibody that binds to CP-C5, such as eculizumab or variants thereof as detailed herein. The transgene may also encode an anti-CP-C5 antigen binding fragment that contains additional glycosylation sites (*e.g.,* see Courtois et al.).

In certain embodiments, the anti-CP-C5 antigen-binding fragment transgene comprises the nucleotide sequences encoding the heavy and light chains of the Fab portion of eculizumab (having amino acid sequences of SEQ ID NOs. 43 and 44, respectively, see Table 4 and FIG. 8F). The nucleotide sequences may be codon optimized for expression in human cells and may, for example, comprise the nucleotide sequences of SEQ ID NO: 143 (encoding the eculizumab heavy chain Fab portion) and SEQ ID NO: 144 (encoding the eculizumab light chain Fab portion) as set forth in Table 5. The heavy and light chain sequences both have a signal or leader sequence at the N-terminus appropriate for expression and secretion in human cells, in particular, human liver cells (e.g., hepatocytes). The signal sequence may have the amino acid sequence of MYRMQLLLLIALSLALVTNS (SEQ ID NO: 161). Alternatively, the signal sequence may have an amino acid sequence selected from any one of the signal sequences set forth in Table 3 that correspond to the proteins secreted by hepatocytes.

In addition to the heavy and light chain variable domain sequences, the transgenes may comprise, at the C-terminus of the heavy chain variable domain sequence, all or a portion of the hinge region. In specific embodiments, the anti-CP-C5-antigen binding domain has a heavy chain variable domain of SEQ ID NO: 43 with additional hinge region sequence starting after the C-terminal glutamic acid (E), contains all or a portion of the amino acid sequence CPPCPAPPVAGG (SEQ ID NO: 232), and specifically, CPPCPA (SEQ ID NO: 219) or CPPCPAPPVAG (SEQ ID NO: 233) as set forth in FIG 8F. These hinge regions may be encoded by nucleotide sequences at the 3' end of SEQ ID NO: 43 by the hinge region encoding sequences set forth in Table 5.

In certain embodiments, the anti-CP-C5 antigen-binding fragment transgene encodes an CP-C5 antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 44. In certain embodiments, the anti-CP-C5 antigen-binding fragment transgene encodes an CP-C5 antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 43. In certain embodiments, the anti-CP-C5 antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 44 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 43. In specific embodiments, the CP-C5 antigen binding fragment comprises a heavy chain comprising an amino acid sequence of SEQ ID NO: 43 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8F) or are substitutions with an amino acid present at that position in the heavy chain of one or more of the other therapeutic antibodies, for example, as identified by the alignment in FIG. 11A. In specific embodiments, the CP-C5 antigen binding fragment comprises a light chain comprising an amino acid sequence of SEQ ID NO: 44 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8F) or are substitutions with an amino acid present at that position in the light chain of one or more of the other therapeutic antibodies, for example, as identified by the alignment in FIG. 11B.

In certain embodiments, the anti-CP-C5 antigen-binding fragment transgene encodes a hyperglycosylated eculizumab Fab, comprising a heavy chain and a light chain of SEQ ID NOs: 43 and 44, respectively, with one or more of the following mutations: L117N (heavy chain), Q160N or Q160S (light chain), and/or E195N (light chain) (see FIGS. 11A (heavy chain) and B (light chain)).

In certain embodiments, the anti-CP-C5 antigen-binding fragment transgene encodes an antigen-binding fragment and comprises the nucleotide sequences encoding the six eculizumab CDRs which are underlined in the heavy and light chain variable domain sequences of FIG. 8F which are spaced between framework regions, generally human framework regions, and associated with constant domains depending upon the form of the antigen-binding molecule, as is known in the art to form the heavy and/or light chain variable domain of an anti-CP-C5 antibody or antigen-binding fragment thereof.

### Gene Therapy Methods

Provided are methods of treating human subjects for PNH or aHUS by administration of a viral vector containing a transgene encoding an anti-CP-C5 antibody, or antigen binding fragment thereof. The antibody may be eculizumab, and is preferably a Fab fragment thereof, or other antigen-binding fragment thereof. In embodiments, the patient has been diagnosed with and/or has symptoms associated with PNH or aHUS. Recombinant vectors used for delivering the transgene are described in Section 5.4.2. Such vectors should have a tropism for human liver cells and can include non-replicating rAAV, particularly those bearing an AAV8 or AAV9 capsid. The recombinant vectors, such as those shown in FIG. 8F, can be administered in any manner such that the recombinant vector enters the liver, preferably by introducing the recombinant vector into the bloodstream. See Section 5.5.2 for details regarding the methods of treatment.

Subjects to whom such gene therapy is administered can be those responsive to anti-CP-C5 therapy. In particular embodiments, the methods encompass treating patients who have been diagnosed with PNH or aHUS, or have one or more symptoms associated therewith, and identified as responsive to treatment with an anti-CP-C5 antibody or considered a good candidate for therapy with an anti-CP-C5 antibody. In specific embodiments, the patients have previously been treated with eculizumab, and have been found to be responsive to eculizumab. To determine responsiveness, the anti-CP-C5 antibody or antigen-binding fragment transgene product (e.g., produced in cell culture, bioreactors, etc.) may be administered directly to the subject.

### Human Post Translationally Modified Antibodies

The production of the anti-CP-C5 HuPTM mAb or HuPTM Fab, should result in a "biobetter" molecule for the treatment of PNH or aHUS accomplished via gene therapy - e.g., by administering a viral vector or other DNA expression construct encoding the anti-CP-C5 HuPTM Fab, intravenously to human subjects (patients) diagnosed with or having one or more symptoms of PNH or aHUS, to create a permanent depot in the liver tissue that continuously supplies the fully-human post-translationally modified, e.g., human-glycosylated, sulfated transgene product produced by transduced liver cells.

The cDNA construct for the anti-CP-C5 HuPTMmAb or anti-CP-C5 HuPTM Fab should include a signal peptide that ensures proper co- and post-translational processing (glycosylation and protein sulfation) by the transduced liver cells. For example, the signal sequence may be MYRMQLLLLIALSLALVTNS (SEQ ID NO: 161). Alternatively, the signal sequence may have an amino acid sequence selected from any one of the signal sequences set forth in Table 3 that correspond to the proteins secreted by hepatocytes.

As an alternative, or an additional treatment to gene therapy, the anti-CP-C5 HuPTM mAb or HuPTM Fab can be produced in human cell lines by recombinant DNA technology, and administered to patients diagnosed with PNH or aHUS, or for whom therapy for PNH or aHUS is considered appropriate.

In specific embodiments, the anti-CP-C5 HuPTM mAb or antigen-binding fragment thereof has heavy and light chains with the amino acid sequences of the heavy and light chain Fab portions of eculizumab as set forth in FIG. 8F (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N63 and/or Q114 and/or N164 and/or N197 and/or N206 of the heavy chain (SEQ ID NO:43) or N28 and/or Q100 and/or N158 and/or N210 of the light chain (SEQ ID NO:44). Alternatively or in addition to, the HuPTM mAb or antigen binding-fragment thereof with the heavy and light chain variable domain sequences of eculizumab has a sulfation group at Y94 and/or Y95 of the heavy chain (SEQ ID NO:43) and/or Y86 and/or Y87 of the light chain (SEQ ID NO:44). In other embodiments, the anti-CP-C5 HuPTM mAb or antigen-binding fragment thereof does not contain any detectable NeuGc moieties and/or does not contain any detectable alpha-Gal moieties.

In certain embodiments, the HuPTM mAb or Fab is therapeutically effective and is at least 0.5%, 1% or 2% glycosylated and/or sulfated and may be at least 5%, 10% or even 50% or 100% glycosylated and/or sulfated. The goal of gene therapy treatment provided herein is to slow or arrest the progression of PNH or aHUS, reduce the need for a blood transfusion, or reduce the destruction of red blood cells. Efficacy may be monitored by measuring hemoglobin stabilization and/or the number of RBC units transfused or scoring fatigue levels and/or health-related quality of life over the course of treatment.

Combinations of delivery of the anti-CP-C5 HuPTM mAb or antigen-binding fragment thereof, to the liver accompanied by delivery of other available treatments are encompassed by the methods provided herein. The additional treatments may be administered before, concurrently, or subsequent to the gene therapy treatment. Available treatments for PNH or aHUS that could be combined with the gene therapy provided herein include but are not limited to anticoagulants and steroids/immunosuppressant treatments, and administration with anti-CP-C5 agents, including but not limited to eculizumab.

### 5.3.13 anti-MMP9 HuPTM Constructs and Formulations for Ocular Disorders, Cystic Fibrosis, Rheumatoid Arthritis, Inflammatory Bowel Disease, and Cancer

Compositions and methods are described for the delivery of HuPTM mAbs and antigen-binding fragments thereof, such as HuPTM Fabs, that bind to matrix metalloproteinase 9 (MMP9) derived from anti-MMP9 indicated for treating one or more retinal disorders including macular degeneration (e.g., dry age-related macular degeneration (AMD)), cystic fibrosis (CF), rheumatoid arthritis (RA), IBD (e.g., UC and CD), and one or more types of cancer (e.g., solid tumors, pancreatic adenocarcinoma, lung adenocarcinoma, lung squamous cell carcinoma, esophagogastric adenocarcinoma, gastric cancer, colorectal cancer, or breast cancer), or for suppressing extracellular matrix degradation. In particular embodiments, the HuPTM mAb has the amino acid sequence of andecaliximab or an antigen binding fragment thereof. The amino acid sequence of Fab fragments of andecaliximab is provided in FIG. 8G. Delivery may be accomplished via gene therapy - e.g., by administering a viral vector or other DNA expression construct encoding an MMP9-binding HuPTM mAb (or an antigen binding fragment and/or a hyperglycosylated derivative or other derivative, thereof) to patients (human subjects) diagnosed with, or having one or more symptoms of a retinal disorder (e.g. macular degeneration), RA, CF, IBD (e.g., UC or CD), or one or more cancers (such as those listed above) to create a permanent depot that continuously supplies the human PTM, e.g., human-glycosylated, transgene product.

Provided are recombinant vectors containing a transgene encoding a HuPTM mAb or HuPTM Fab (or other antigen binding fragment of the HuPTM mAb) that binds to MMP9 that can be administered to deliver the HuPTM mAb or antigen binding fragment in a patient. The transgene is a nucleic acid comprising the nucleotide sequences encoding an antigen binding fragment of an antibody that binds to MMP9, such as andecaliximab, or variants thereof as detailed herein. The transgene may also encode an anti-MMP9 antigen binding fragment that contains additional glycosylation sites (*e.g.,* see Courtois et al.).

In certain embodiments, the anti-MMP9 antigen-binding fragment transgene comprises the nucleotide sequences encoding the heavy and light chains of the Fab portion of andecaliximab (having amino acid sequences of SEQ ID NOs. 45 and 46, respectively, see Table 4 and FIG. 8G). The nucleotide sequences may be codon optimized for expression in human cells and may, for example, comprise the nucleotide sequences of SEQ ID NO: 145 (encoding the andecaliximab heavy chain Fab portion) and SEQ ID NO: 146 (encoding the andecaliximab light chain Fab portion) as set forth in Table 5. In the case of treating ocular diseases, the heavy and light chain sequences both have a signal or leader sequence at the N-terminus appropriate for expression and secretion in human cells, in particular, one or more cells forming the retina. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 1 that correspond to the proteins secreted by one or more cells forming the retina. In the case of treating non-ocular diseases, the heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-MMP9 antigen binding domain has a heavy chain variable domain of SEQ ID NO: 45 with additional hinge region sequence starting after the C-terminal aspartic acid (D), contains all or a portion of the amino acid sequence GPPCPPCPAPEFLGG (SEQ ID NO: 231), and specifically, GPPCPPCPA (SEQ ID NO: 229) or GPPCPPCPAPEFLGGPSVFL (SEQ ID NO: 230) as set forth in FIG 8G. These hinge regions may be encoded by nucleotide sequences at the 3' end of SEQ ID NO: 45 by the hinge region encoding sequences set forth in Table 5.

In certain embodiments, the anti-MMP9 antigen-binding fragment transgene encodes an MMP9 antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 46. In certain embodiments, the anti-MMP9 antigen-binding fragment transgene encodes an MMP9 antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 45. In certain embodiments, the anti-MMP9 antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 46 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 45. In specific embodiments, the MMP9 antigen binding fragment comprises a heavy chain comprising an amino acid sequence of SEQ ID NO: 45 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8G) or are substitutions with an amino acid present at that position in the heavy chain of one or more of the other therapeutic antibodies, for example, as identified by the alignment in FIG. 11A. In specific embodiments, the MMP9 antigen binding fragment comprises a light chain comprising an amino acid sequence of SEQ ID NO: 46 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8G) or are substitutions with an amino acid present at that position in the light chain of one or more of the other therapeutic antibodies, for example, as identified by the alignment in FIG. 11B.

In certain embodiments, the anti-MMP9 antigen-binding fragment transgene encodes a hyperglycosylated andecaliximab Fab, comprising a heavy chain and a light chain of SEQ ID NOs: 45 and 46, respectively, with one or more of the following mutations: L110N (heavy chain), Q160N or Q160S (light chain), and/or E195N (light chain) (see FIGS. 11A (heavy chain) and B (light chain)).

In certain embodiments, the anti-MMP9 antigen-binding fragment transgene encodes an antigen-binding fragment and comprises the nucleotide sequences encoding the six andecaliximab CDRs which are underlined in the heavy and light chain variable domain sequences of FIG. 8G which are spaced between framework regions, generally human framework regions, and associated with constant domains depending upon the form of the antigen-binding molecule, as is known in the art to form the heavy and/or light chain variable domain of an anti-MMP9 antibody or antigen-binding fragment thereof.

### Gene Therapy Methods

Provided are methods of treating human subjects for one or more retinal disorders (such as macular degeneration), IBD, CF, RA, or cancers by administration of a viral vector containing a transgene encoding an anti-MMP9 antibody or antigen binding fragment thereof. The antibody may be andecaliximab, and is preferably a Fab fragment thereof, or other antigen-binding fragment thereof. In embodiments, the patient has been diagnosed with and/or has symptoms associated with one or more of retinal disorders, IBD, CF, RA, or cancers.

Recombinant vector used for delivering the transgene are described in Section 5.4.3. Such vectors should have a tropism for human retina-type cells and can include non-replicating rAAV, particularly those bearing an AAV8 capsid. The recombinant vectors, such as those shown in FIG. 8G, can be administered in any manner such that the recombinant vector enters the retina, preferably by introducing the recombinant vector into the eye. See Section 5.5.3 for details regarding the methods of treatment. For delivery to the liver, for example, for the treatment of cancer, recombinant vector used for delivering the transgene are described in Section 5.4.2. Such vectors should have a tropism for human liver cells and can include non-replicating rAAV, particularly those bearing an AAV8 or AAV9 capsid. The recombinant vectors, such as those shown in FIG. 8G, can be administered in any manner such that the recombinant vector enters the liver, preferably by introducing the recombinant vector into the bloodstream. See Section 5.5.2 for details regarding the methods of treatment.

Subjects to whom such gene therapy is administered can be those responsive to anti-MMP9 therapy. In particular embodiments, the methods encompass treating patients who have been diagnosed with one or more retinal disorders or types of cancer, or have one or more symptoms associated therewith, and identified as responsive to treatment with an anti-MMP9 antibody, or considered a good candidate for therapy with an anti-MMP9 antibody. In specific embodiments, the patients have previously been treated with andecaliximab, and have been found to be responsive to andecaliximab. To determine responsiveness, the anti-MMP9 or antigen-binding fragment transgene product (e.g., produced in cell culture, bioreactors, etc.) may be administered directly to the subject.

### Human Post Translationally Modified Antibodies

The production of the anti-MMP9 HuPTM mAb or HuPTM Fab, should result in a "biobetter" molecule for the treatment of one or more retinal disorders, CF, RA, IBD, or cancers accomplished via gene therapy - e.g., by administering a viral vector or other DNA expression construct encoding the anti-MMP9 HuPTM Fab, subretinally, intravitreally or suprachoroidally to human subjects (patients) diagnosed with or having one or more symptoms of one or more retinal disorders, or by administering a viral vector or other DNA expression construct encoding the anti-MMP9 HuPTM Fab, subcutaneously, intramuscularly, or intravenously to human subjects (patients) diagnosed with RA, CF, IBD, or cancer, to create a permanent depot in the retina or liver that continuously supplies the fully-human post-translationally modified, e.g., human-glycosylated, sulfated transgene product produced by transduced cells of the retina or liver.

The cDNA construct for the anti-MMP9 HuPTMmAb or anti-MMP9 HuPTM Fab should include a signal peptide that ensures proper co- and post-translational processing (glycosylation and protein sulfation) by the transduced cells of the retina or liver. For example, the signal sequence may be MYRMQLLLLIALSLALVTNS (SEQ ID NO: 161). Alternatively, the signal sequence may have an amino acid sequence selected from any one of the signal sequences set forth in Table 1 or 3 that correspond to the proteins secreted by cells of the retina or liver, respectively.

As an alternative, or an additional treatment to gene therapy, the anti-MMP9 HuPTM mAb or HuPTM Fab can be produced in human cell lines by recombinant DNA technology, and administered to patients diagnosed with a retinal disorder or cancer for whom therapy for a retinal disorder, IBD, CF, RA, or cancer is considered appropriate.

In specific embodiments, the anti-MMP9 HuPTM mAb or antigen-binding fragment thereof has heavy and light chains with the amino acid sequences of the heavy and light chain Fab portions of andecaliximab as set forth in FIG. 8G (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N58, N76, Q107, N157, and/or N199 of the heavy chain (SEQ ID NO:45) or N158 and/or N210 of the light chain (SEQ ID NO:46). Alternatively or in addition to, the HuPTM mAb or antigen binding-fragment thereof with the heavy and light chain variable domain sequences of andecaliximab has a sulfation group at Y93 and/or Y94 of the heavy chain (SEQ ID NO: 45) and/or Y86 and/or Y87 of the light chain (SEQ ID NO: 46). In other embodiments, the anti-MMP9 HuPTM mAb or antigen-binding fragment thereof does not contain detectable NeuGc moieties and/or does not contain detectable alpha-Gal moieties.

. In certain embodiments, the HuPTM mAb or Fab is therapeutically effective and is at least 0.5%, 1% or 2% glycosylated and/or sulfated and may be at least 5%, 10% or even 50% or 100% glycosylated and/or sulfated. The goal of gene therapy treatment provided herein is to slow or arrest the progression of the disease being treated or alleviate one or more symptoms thereof. In the case of retinal disorders, efficacy may be monitored by monitoring vision acuity. For example, efficacy can be monitored by assessing change in vision acuity from baseline. In the case of a cancer, efficacy can be monitored by assessing one or more oncology endpoints including overall survival, progressionfree survival, time to progression, time to treatment failure, event-free survival, time to next treatment, objective response rate, or duration of response. (see, e.g., U.S. Department of Health and Human Services Food and Drug Administration Center for Drug Evaluation and Research, Center for Biologics Evaluation and Research. Guidance for industry: clinical trial endpoints for the approval of cancer drugs and biologics. https://www.fda.gov/downloads/Drugs/Guidances/ucm071590.pdf. Published May 2007. Accessed October 13, 2017; Oncology Endpoints in a Changing Landscape. Manag. Care. 2016; 1(suppl): 1-12). In the case of RA, efficacy can be monitored by assessing one or more of (1) swollen joint count, (2) tender joint count, (3) global physician's assessment of disease activity, (4) patient self-report of functional status, (5) patient self-report of pain, (6) global patient assessment of disease activity, (7) a laboratory measures of C-reactive protein and erythrocyte sedimentation rate, and (8) radiographic progression. (see, e.g., Smolen JS, Aletaha D. "Assessment of rheumatoid arthritis activity in clinical trials and clinical practice" UptoDate.com Wolters Kluwer Health. Accessed at: www.uptodate.com Dec. 2017) For example, with regard to CD, efficacy can be monitored by assessing Crohn's Disease Activity Index [CDAI] over the course of treatment (e.g., see Best WR et al. (1976) Gastroenterology, Mar;70(3):439-44, "Development of a Crohn's disease activity index. National Cooperative Crohn's Disease Study."). With regard to UC, efficacy can be monitored by assessing a Mayo score and an endoscopy subscore over the course of treatment (e.g., see Lobaton et al., "The Modified Mayo Endoscopic Score (MMES): A New Index for the Assessment of Extension and Severity of Endoscopic Activity in Ulcerative Colitis Patients," J. Crohns Colitis. 2015 Oct:9(10):846-52). In the case of CF, efficacy can be monitored by assessing Forced Expiratory Volume in 1s (FEV1), decreased frequency of pulmonary exacerbations, quality of life (QoL) improvement, and, for younger patients, growth improvement, (e.g., see VanDevanter and Konstan, "Outcome measurement for clinical trials assessing treatment of cystic fibrosis lung disease," Clin. Investig. 2(2): 163-175 (2012)).

Combinations of delivery of the anti-MMP9 HuPTM mAb or antigen-binding fragment thereof to the retina or liver accompanied by delivery of other available treatments are encompassed by the methods provided herein. The additional treatments may be administered before, concurrently, or subsequent to the gene therapy treatment. Available treatments for macular degeneration that could be combined with the gene therapy provided herein include but are not limited to laser photocoagulation, photodynamic therapy with verteporfin, aflibercept, and/or intravitreal steroids and administration with anti-MMP9 agents, including but not limited to andecaliximab. Available treatments for the one or more above listed cancers that could be combined with the gene therapy provided herein include but are not limited to chemotherapy (e.g., cisplatin, gemcitabine, pemetrexed, 5-fluorouracil, carboplatin, irinotecan, interferon alfa, oxaliplatin, paclitaxel pegylated liposomal doxorubicin, and/or topotecan), chemotherapy protective drugs (e.g., leucovorin), radiotherapy, cryotherapy, targeted small molecule therapies, other antibodies, afilbercept, and/or vaccine therapy and administration with anti-MMP9, including but not limited to andecaliximab. Available treatments for RA that could be combined with the gene therapy provided herein include but are not limited to bisphosphonates, nonsteroidal anti-inflammatory drugs (e.g., celecoxib, naproxen, aspirin, indomethacin, sulfasalazine, and ketoprofen), steroids (e.g., prednisone), disease modifying antirheumatic drugs and other immunosupressants (e.g., leflunomide, methotrexate, tofactinib, azathioprine, mycophenolate, cyclosphophamide, cyclosporine), hydroxychloroquine, abatacept, anakinra, apremilast, TNF inhibitors, other antibodies (e.g., tocilizumab, secukinimab, rituximab) and administration with anti-MMP9, including but not limited to andecaliximab. Available treatments for IBD that could be combined with the gene therapy provided herein include but are not limited to nonsteroidal anti-inflammatory drugs (e.g., mesalamine, sulfasalazine), steroids (e.g., hydrocortisone, prednisone, budesonide), immunosuppressants (e.g., methotrexate, mercaptopurine, azathioprine), vitamins (e.g., iron, cholecalciferol), antibiotics (e.g., amino salicylic acid, metronidazole), other antibodies (e.g., infliximab, adalimumab) and administration with anti-MMP9, including but not limited to andecaliximab. Available treatments for CF that could be combined with the gene therapy provided herein include but are not limited to antibiotics, vaccines, and cough medicines (e.g., acetylcysteine and dornasa alfa) and administration with anti-MMP9, including but not limited to andecaliximab.

### 5.3.14. Anti-pKal HuPTM Constructs and Formulations for Angioedema

Compositions and methods are described for the delivery of HuPTM mAbs and antigen-binding fragments thereof, such as HuPTM Fabs, that bind to kallikrein (pKal) derived from an anti-pKal antibody and indicated for treating angioedema, such as hereditary angioedema. In particular embodiments, the HuPTM mAb has the amino acid sequence of lanadelumab or an antigen binding fragment thereof. The amino acid sequence of Fab fragment of this antibody is provided in FIG. 8H. Delivery may be accomplished via gene therapy - e.g., by administering a viral vector or other DNA expression construct encoding an pKal-binding HuPTM mAb (or an antigen binding fragment and/or a hyperglycosylated derivative or other derivative, thereof) to patients (human subjects) diagnosed with angioedema to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to pKal that can be administered to deliver the HuPTM mAb or antigen binding fragment in a patient. The transgene is a nucleic acid comprising the nucleotide sequences encoding an antigen binding fragment of an antibody that binds to pKal, such as lanadelumab or variants thereof as detailed herein. The transgene may also encode an anti-pKal antigen binding fragment that contains additional glycosylation sites (*e.g.,* see Courtois et al.).

In certain embodiments, the anti-pKal antigen-binding fragment transgene comprises the nucleotide sequences encoding the heavy and light chains of the Fab portion of lanadelumab (having amino acid sequences of SEQ ID NOs. 47 and 48, respectively, see Table 4 and FIG. 8H). The nucleotide sequences may be codon optimized for expression in human cells and may, for example, comprise the nucleotide sequences of SEQ ID NO: 147 (encoding the lanadelumab heavy chain Fab portion) and SEQ ID NO: 148 (encoding the lanadelumab light chain Fab portion) as set forth in Table 5. The heavy and light chain sequences both have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-pKal-antigen binding domain has a heavy chain variable domain of SEQ ID NO: 47 with additional hinge region sequence starting after the C-terminal aspartate (D), contains all or a portion of the amino acid sequence KTHTCPPCPAPELLGG (SEQ ID NO: 222), and specifically, KTHL (SEQ ID NO: 223), KTHT (SEQ ID NO: 224), KTHTCPPCPA (SEQ ID NO: 225), KTHLCPPCPA (SEQ ID NO: 226), KTHTCPPCPAPELLGGPSVFL (SEQ ID NO: 227) or KTHLCPPCPAPELLGGPSVFL (SEQ ID NO: 228) as set forth in FIG 8H. These hinge regions may be encoded by nucleotide sequences at the 3' end of SEQ ID NO: 47 by the hinge region encoding sequences set forth in Table 5.

In certain embodiments, the anti-pKal antigen-binding fragment transgene encodes an pKal antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 48. In certain embodiments, the anti-pKal antigen-binding fragment transgene encodes an pKal antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 47. In certain embodiments, the anti-pKal antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 48 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 47. In specific embodiments, the pKal antigen binding fragment comprises a heavy chain comprising an amino acid sequence of SEQ ID NO: 47 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8H) or are substitutions with an amino acid present at that position in the heavy chain of one or more of the other therapeutic antibodies, for example, as identified by the alignment in FIG. 11A. In specific embodiments, the pKal antigen binding fragment comprises a light chain comprising an amino acid sequence of SEQ ID NO: 48 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 8H) or are substitutions with an amino acid present at that position in the light chain of one or more of the other therapeutic antibodies, for example, as identified by the alignment in FIG. 11B.

In certain embodiments, the anti-pKal antigen-binding fragment transgene encodes a hyperglycosylated lanadelumab Fab, comprising a heavy chain and a light chain of SEQ ID NOs: 47 and 48, respectively, with one or more of the following mutations: M117N (heavy chain) and/or Q159N, Q159S, and/or E194N (light chain) (see FIGS. 11A (heavy chain) and B (light chain)).

In certain embodiments, the anti-pKal antigen-binding fragment transgene encodes an antigen-binding fragment and comprises the nucleotide sequences encoding the six lanadelumab CDRs which are underlined in the heavy and light chain variable domain sequences of FIG. 8H which are spaced between framework regions, generally human framework regions, and associated with constant domains depending upon the form of the antigen-binding molecule, as is known in the art to form the heavy and/or light chain variable domain of an anti-pKal antibody or antigen-binding fragment thereof.

### Gene Therapy Methods

Provided are methods of treating human subjects for angioedema by administration of a viral vector containing a transgene encoding an anti-pKal antibody, or antigen binding fragment thereof. The antibody may be lanadelumab, and is preferably a Fab fragment thereof, or other antigen-binding fragment thereof. In embodiments, the patient has been diagnosed with and/or has symptoms associated with angioedema. Recombinant vectors used for delivering the transgene are described in Section 5.4.2. Such vectors should have a tropism for human liver or muscle cells and can include non-replicating rAAV, particularly those bearing an AAV8 or AAV9 capsid. The recombinant vectors, such as shown in FIG. 8H, can be administered in any manner such that the recombinant vector enters the liver or muscle tissue, preferably by introducing the recombinant vector into the bloodstream. See Section 5.5.2 for details regarding the methods of treatment

Subjects to whom such gene therapy is administered can be those responsive to anti-pKal therapy. In particular embodiments, the methods encompass treating patients who have been diagnosed with angioedema, or have one or more symptoms associated therewith, and identified as responsive to treatment with an anti-pKal antibody or considered a good candidate for therapy with an anti-pKal antibody. In specific embodiments, the patients have previously been treated with lanadelumab, and have been found to be responsive to lanadelumab. To determine responsiveness, the anti-pKal antibody or antigen-binding fragment transgene product (e.g., produced in cell culture, bioreactors, etc.) may be administered directly to the subject.

### Human Post Translationally Modified Antibodies

The production of the anti-pKal HuPTM mAb or HuPTM Fab, should result in a "biobetter" molecule for the treatment of angioedema accomplished via gene therapy - e.g., by administering a viral vector or other DNA expression construct encoding the anti-pKal HuPTM Fab, intravenously to human subjects (patients) diagnosed with or having one or more symptoms of angioedema, to create a permanent depot in the liver or muscle tissue that continuously supplies the fully-human post-translationally modified, e.g., human-glycosylated, sulfated transgene product produced by transduced liver or muscle cells.

In specific embodiments, the anti-pKal HuPTM mAb or antigen-binding fragment thereof has heavy and light chains with the amino acid sequences of the heavy and light chain Fab portions of lanadelumab as set forth in FIG. 8H (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N77, Q114 and/or N164 of the heavy chain (SEQ ID NO:47) or Q99, N157, and/or N209 of the light chain (SEQ ID NO:48). Alternatively or in addition to, the HuPTM mAb or antigen binding-fragment thereof with the heavy and light chain variable domain sequences of lanadelumab has a sulfation group at Y94 and/or Y95 of the heavy chain (SEQ ID NO:47) and/or Y86 and/or Y87 of the light chain (SEQ ID NO:48). In other embodiments, the anti-pKal HuPTM mAb or antigen-binding fragment thereof does not contain detectable NeuGc moieties and/or does not contain detectable alpha-Gal moieties.

In certain embodiments, the HuPTM mAb or Fab (or a hyperglycosylated derivative of either) is therapeutically effective and is at least 0. 5%, 1% or 2% glycosylated and/or sulfated and may be at least 5%, 10% or even 50% or 100% glycosylated and/or sulfated. The goal of gene therapy treatment provided herein is to slow or arrest the progression of angioedema, reduce the levels of pain or discomfort for the patient, or reduce levels of autoreactive B cells and immunoglobulin producing plasma cells. Efficacy may be monitored by scoring the function, symptoms, or degree of inflammation in the affected tissue or area of the body, e.g., such as the skin, joints, kidneys, lungs, blood cells, heart, and brain. For example, efficacy can be monitored by assessing changes in attack severity or frequency.

Combinations of delivery of the anti-pKal HuPTM mAb or antigen-binding fragment thereof, to the liver or muscle accompanied by delivery of other available treatments are encompassed by the methods provided herein. The additional treatments may be administered before, concurrently, or subsequent to the gene therapy treatment. Available treatments for angioedema that could be combined with the gene therapy provided herein include but are not limited to danazol, bradykinin receptor antagonist (e.g., icatibant), plasma kallikrein inhibitor (e.g., ecallantide), C1 esterase inhibitor, conestat alfa, anti-fibrinolytic agents (e.g., tranexamic acid), omalizumab, and fresh frozen plasma transfusions, antihistamines, and corticosteroids and administration with anti-pKal agents, including but not limited to lanadelumab.

### 5.3.15. Anti-TNFα HuPTM Constructs and Formulations for various auto-immune disorders - Adalimumab and Infliximab

Compositions and methods are described for the delivery of HuPTM mAbs and antigen-binding fragments thereof, such as HuPTM Fabs, that bind to tumor necrosis factor-alpha (TNFα) derived from an anti-TNFα antibody, such as adalimumab (FIG. 9A) or infliximab (FIG. 9B), and indicated for treating one or more autoimmune-related disorders, such as hidradenitis suppurativa (HS), atopic dermatitis, psoriasis (e.g., plaque psoriasis, pustular psoriasis, and erythrodermic psoriasis), arthritis (e.g., juvenile idiopathic arthritis, rheumatoid arthritis, psoriatic arthritis, and alkylating spondylitis), and/or IBD (e.g., Crohn's disease and ulcerative colitis) (collectively referred to hereinafter as "subject AI-Ds(2)"). In particular embodiments, the HuPTM mAb has the amino acid sequence of adalimumab or an antigen binding fragment thereof. In other embodiments, the HuPTM mAb has the amino acid sequence of infliximab or an antigen binding fragment thereof. Amino acid sequences of Fab fragments of the antibody are provided in FIGS. 9A and 9B. Delivery may be accomplished via gene therapy - e.g., by administering a viral vector or other DNA expression construct encoding an TNFα-binding HuPTM mAb (or an antigen binding fragment and/or a hyperglycosylated derivative or other derivative, thereof) to patients (human subjects) diagnosed with one or more subject AI-Ds(2) to create a permanent depot that continuously supplies the human PTM, e.g., human-glycosylated, transgene product.

### Transgenes

Provided are recombinant vectors containing a transgene encoding a HuPTM mAb or HuPTM Fab (or other antigen binding fragment of the HuPTM mAb) that binds to TNFα that can be administered to deliver the HuPTM mAb or antigen binding fragment in a patient. The transgene is a nucleic acid comprising the nucleotide sequences encoding an antigen binding fragment of an antibody that binds to TNFα, such as adalimumab or infliximab or variants thereof as detailed herein. The transgene may also encode an anti-TNFα antigen binding fragment that contains additional glycosylation sites (*e.g.,* see Courtois et al.).

In certain embodiments, the anti-TNFα antigen-binding fragment transgene comprises the nucleotide sequences encoding the heavy and light chains of the Fab portion of adalimumab (having amino acid sequences of SEQ ID NOs. 49 and 50, respectively, see Table 4 and FIG. 9A). The nucleotide sequences may be codon optimized for expression in human cells and may, for example, comprise the nucleotide sequences of SEQ ID NO: 149 (encoding the adalimumab heavy chain Fab portion) and SEQ ID NO: 150 (encoding the adalimumab light chain Fab portion) as set forth in Table 5. The heavy and light chain sequences each have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-TNFα-antigen binding domain has a heavy chain variable domain of SEQ ID NO: 49 with additional hinge region sequence starting after the C-terminal aspartate (D), contains all or a portion of the amino acid sequence KTHTCPPCPAPELLGG (SEQ ID NO: 222), and specifically, KTHL (SEQ ID NO: 223), KTHT (SEQ ID NO: 224), KTHTCPPCPA (SEQ ID NO: 225), KTHLCPPCPA (SEQ ID NO: 226), KTHTCPPCPAPELLGGPSVFL (SEQ ID NO: 227) or KTHLCPPCPAPELLGGPSVFL (SEQ ID NO: 228) as set forth in FIG 9A. These hinge regions may be encoded by nucleotide sequences at the 3' end of SEQ ID NO: 49 by the hinge region encoding sequences set forth in Table 5.

In certain embodiments, the anti-TNFα antigen-binding fragment transgene encodes an TNFα antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 50. In certain embodiments, the anti-TNFα antigen-binding fragment transgene encodes an TNFα antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 49. In certain embodiments, the anti-TNFα antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 50 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 49. In specific embodiments, the TNFα antigen binding fragment comprises a heavy chain comprising an amino acid sequence of SEQ ID NO: 49 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 9A) or are substitutions with an amino acid present at that position in the heavy chain of one or more of the other therapeutic antibodies, for example, as identified by the alignment in FIG. 11A. In specific embodiments, the TNFα antigen binding fragment comprises a light chain comprising an amino acid sequence of SEQ ID NO: 50 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 9A) or are substitutions with an amino acid present at that position in the light chain of one or more of the other therapeutic antibodies, for example, as identified by the alignment in FIG. 11B.

In certain embodiments, the anti-TNFα antigen-binding fragment transgene encodes a hyperglycosylated adalimumab Fab, comprising a heavy chain and a light chain of SEQ ID NOs: 49 and 50, respectively, with one or more of the following mutations: L116N (heavy chain), Q160N or Q160S (light chain), and/or E195N (light chain) (see FIGS. 11A (heavy chain) and B (light chain)).

In certain embodiments, the anti-TNFα antigen-binding fragment transgene encodes an antigen-binding fragment and comprises the nucleotide sequences encoding the six adalimumab CDRs which are underlined in the heavy and light chain variable domain sequences of FIG. 9A which are spaced between framework regions, generally human framework regions, and associated with constant domains depending upon the form of the antigen-binding molecule, as is known in the art to form the heavy and/or light chain variable domain of an anti-TNFα antibody or antigen-binding fragment thereof.

In certain embodiments, the anti-TNFα antigen-binding fragment transgene comprises the nucleotide sequences encoding the heavy and light chains of the Fab portion of infliximab (having amino acid sequences of SEQ ID NOs. 51 and 52, respectively, see Table 4 and FIG. 9B). The nucleotide sequences may be codon optimized for expression in human cells and may, for example, comprise the nucleotide sequences of SEQ ID NO: 151 (encoding the infliximab heavy chain Fab portion) and SEQ ID NO: 152 (encoding the infliximab light chain Fab portion) as set forth in Table 5. The heavy and light chain sequences each have a signal or leader sequence at the N-terminus appropriate for expression and secretion in human cells, in particular, human liver cells (e.g., hepatocytes) or muscle cells. The signal sequence may have the amino acid sequence of MYRMQLLLLIALSLALVTNS (SEQ ID NO: 161). Alternatively, the signal sequence may have an amino acid sequence selected from any one of the signal sequences set forth in Table 2 or 3 that correspond to the proteins secreted by myocytes or hepatocytes, respectively.

In addition to the heavy and light chain variable domain sequences, the transgenes may comprise, at the C-terminus of the heavy chain variable domain sequence, all or a portion of the hinge region. In specific embodiments, the anti-TNFα-antigen binding domain has a heavy chain variable domain of SEQ ID NO: 51 with additional hinge region sequence starting after the C-terminal aspartate (D), contains all or a portion of the amino acid sequence KTHTCPPCPAPELLGG (SEQ ID NO: 222), and specifically, KTHL (SEQ ID NO: 223), KTHT (SEQ ID NO: 224), KTHTCPPCPA (SEQ ID NO: 225), KTHLCPPCPA (SEQ ID NO: 226), KTHTCPPCPAPELLGGPSVFL (SEQ ID NO: 227) or KTHLCPPCPAPELLGGPSVFL (SEQ ID NO: 228) as set forth in FIG 9B. These hinge regions may be encoded by nucleotide sequences at the 3' end of SEQ ID NO: 51 by the hinge region encoding sequences set forth in Table 5.

In certain embodiments, the anti-TNFα antigen-binding fragment transgene encodes an TNFα antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 52. In certain embodiments, the anti-TNFα antigen-binding fragment transgene encodes an TNFα antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 51. In certain embodiments, the anti-TNFα antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 52 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 51. In specific embodiments, the TNFα antigen binding fragment comprises a heavy chain comprising an amino acid sequence of SEQ ID NO: 51 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 9B) or are substitutions with an amino acid present at that position in the heavy chain of one or more of the other therapeutic antibodies, for example, as identified by the alignment in FIG. 11A. In specific embodiments, the TNFα antigen binding fragment comprises a light chain comprising an amino acid sequence of SEQ ID NO: 52 with 1, 2, 3, 4, 5, 6, 7, 8, 9, 10, 11, 12, 13, 14, 15 or more amino acid substitutions, insertions or deletions, and the substitutions, insertions or deletions preferably are made in the framework regions (i.e., those regions outside of the CDRs, which CDRs are underlined in FIG. 9B) or are substitutions with an amino acid present at that position in the light chain of one or more of the other therapeutic antibodies, for example, as identified by the alignment in FIG. 11B.

In certain embodiments, the anti-TNFα antigen-binding fragment transgene encodes a hyperglycosylated infliximab Fab, comprising a heavy chain and a light chain of SEQ ID NOs: 51 and 52, respectively, with one or more of the following mutations: T115N (heavy chain), Q160N or Q160S (light chain), and/or E195N (light chain) (see FIGS. 11A (heavy chain) and B (light chain)).

In certain embodiments, the anti-TNFα antigen-binding fragment transgene encodes an antigen-binding fragment and comprises the nucleotide sequences encoding the six infliximab CDRs which are underlined in the heavy and light chain variable domain sequences of FIG. 9B which are spaced between framework regions, generally human framework regions, and associated with constant domains depending upon the form of the antigen-binding molecule, as is known in the art to form the heavy and/or light chain variable domain of an anti-TNFα antibody or antigen-binding fragment thereof.

### Gene Therapy Methods

Provided are methods of treating human subjects for one or more of the subject AI-Ds(2) by administration of a viral vector containing a transgene encoding an anti-TNFα antibody, or antigen binding fragment thereof. The antibody may be adalimumab or infliximab, and is preferably a Fab fragment thereof, or other antigen-binding fragment thereof. In embodiments, the patient has been diagnosed with and/or has symptom(s) associated with one or more of the subject AI-Ds(2). Recombinant vectors used for delivering the transgene are described in Section 5.4.2. Such vectors should have a tropism for human liver or muscle cells and can include non-replicating rAAV, particularly those bearing an AAV8 or AAV9 capsid. The recombinant vectors, such as those shown in FIGS. 9A and 9B, can be administered in any manner such that the recombinant vector enters the liver or muscle tissue, preferably by introducing the recombinant vector into the bloodstream. See Section 5.5.2 for details regarding the methods of treatment

Subjects to whom such gene therapy is administered can be those responsive to anti-TNFα therapy. In particular embodiments, the methods encompass treating patients who have been diagnosed with one or more of the subject AI-Ds(2), or have one or more symptoms associated therewith, and identified as responsive to treatment with an anti-TNFα antibody or considered a good candidate for therapy with an anti-TNFα antibody. In specific embodiments, the patients have previously been treated with adalimumab or infliximab, and have been found to be responsive to adalimumab or infliximab. In other embodiments, the patients have been previously treated with an anti-TNF-alpha antibody or fusion protein such as etanercept, golimumab, or certolizumab, or other anti-TNF-alpha agent. To determine responsiveness, the anti-TNFα antibody or antigen-binding fragment transgene product (e.g., produced in cell culture, bioreactors, etc.) may be administered directly to the subject.

### Human Post Translationally Modified Antibodies

The production of the anti-TNFα HuPTM mAb or HuPTM Fab, should result in a "biobetter" molecule for the treatment of one or more subject AI-Ds(2) accomplished via gene therapy - e.g., by administering a viral vector or other DNA expression construct encoding the anti-TNFα HuPTM Fab, intravenously to human subjects (patients) diagnosed with or having one or more symptoms of one or more subject AI-Ds(2), to create a permanent depot in the liver or muscle tissue that continuously supplies the fully-human post-translationally modified, e.g., human-glycosylated, sulfated transgene product produced by transduced liver or muscle cells.

In specific embodiments, the anti-TNFα HuPTM mAb or antigen-binding fragment thereof has heavy and light chains with the amino acid sequences of the heavy and light chain Fab portions of adalimumab as set forth in FIG. 9A (with non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N54 and/or N163 and/or Q113 of the heavy chain (SEQ ID NO:49) or Q100 and/or N158 and/or N210 of the light chain (SEQ ID NO:50). Alternatively or in addition to, the HuPTM mAb or antigen binding-fragment thereof with the heavy and light chain variable domain sequences of adalimumab has a sulfation group at Y94 and/or Y95 and/or Y32 of the heavy chain (SEQ ID NO:49) and/or Y86 and/or Y87 of the light chain (SEQ ID NO:50). In other embodiments, the anti-TNFα HuPTM mAb or antigen-binding fragment thereof does not contain any detectable NeuGc moieties and/or does not contain any detectable alpha-Gal moieties.

In specific embodiments, the anti-TNFα HuPTM mAb or antigen-binding fragment thereof has heavy and light chains with the amino acid sequences of the heavy and light chain Fab portions of infliximab as set forth in FIG. 9B (with consensus and non-consensus asparagine (N) glycosylation sites highlighted in aqua, glutamine (Q) glycosylation sites highlighted in green, and Y-sulfation sites highlighted in yellow) has a glycosylation, particularly a 2,6-sialylation, at one or more of the amino acid positions N57 and/or N101 and/or Q112 and/or N162 of the heavy chain (SEQ ID NO:51) or N41 and/or N76 and/or N158 and/or N210 of the light chain (SEQ ID NO:52). Alternatively or in addition to, the HuPTM mAb or antigen binding-fragment thereof with the heavy and light chain variable domain sequences of adalimumab has a sulfation group at Y96 and/or Y97 of the heavy chain (SEQ ID NO:51) and/or Y86 and/or Y87 of the light chain (SEQ ID NO:52). In other embodiments, the anti-TNFα HuPTM mAb or antigen-binding fragment thereof does not contain any detectable NeuGc moieties and/or does not contain any detectable alpha-Gal moieties.

In certain embodiments, the HuPTM mAb or Fab is therapeutically effective and is at least 0.5%, 1% or 2% glycosylated and/or sulfated and may be at least 5%, 10% or even 50% or 100% glycosylated and/or sulfated. The goal of gene therapy treatment provided herein is to slow or arrest the progression of or relieve one or more symptoms of the one or more of the subject AI-Ds(2), such as reduce the levels of pain or discomfort for the patient.

Efficacy may be monitored by scoring the symptoms or degree of inflammation in the affected tissue or area of the body, e.g., such as the skin, colon, or joints. For example, with regard to CD, efficacy can be monitored by assessing Crohn's Disease Activity Index [CDAI] over the course of treatment (e.g., see Best WR et al. (1976) Gastroenterology, Mar;70(3):439-44, "Development of a Crohn's disease activity index. National Cooperative Crohn's Disease Study."). With regard to UC, efficacy can be monitored by assessing a Mayo score and an endoscopy subscore over the course of treatment (e.g., see Lobaton et al. (2015) J. Crohns Colitis. 2015 Oct;9(10):846-52, "The Modified Mayo Endoscopic Score (MMES): A New Index for the Assessment of Extension and Severity of Endoscopic Activity in Ulcerative Colitis Patients."). With regard to psoriasis, HS, and atopic dermatitis, efficacy can be monitored by assessing changes in the affected skin or in the quality of the patient's life over the course of treatment. One or more standardized assessments can be used to assess the change. (see e.g., Feldman & Krueger, (2005) Ann. Rheum. Dis. 64(Suppl II):ii65-ii68: "Psoriasis assessment tools in clinical trials" describing standardized assessments including the Psoriasis Area and Severity Index (PASI), Physician Global Assessment (PGA), lattice system, NPF Psoriasis Score (NPF-PS), Medical Outcome Survey Short Form 36 (SF-36), the Euro QoL, Dermatology Life Quality Index (DLQI), and the Skindex; Schram et al. (2012) Allergy; 67: 99-106: "EASI, (objective) SCORAD and POEM for atopic eczema: responsiveness and minimal clinically important difference" describing standardized assessments including Eczema Area and Severity Index (EASI) and the Severity Scoring of Atopic Dermatitis Index (SCORAD); Sisic et al. (2017) J Cutan Med Surg. 21(2): 152-155 "Development of a Quality-of-Life Measure for Hidradenitis Suppurativa."). With regard to arthritis, efficacy can be monitored by assessing one or more of the activity of the disease, the patient's level of function, or the degree of structural damage to patient's joints (e.g., see Zockling & Braun (2005) Clin. Exp. Rheumatol 23 (Suppl. 39) S133-S141: "Assessment of ankylosing spondylitis" describing standardized assessment for ankylosing spondylitis; see also Coates et al. (2011) J. Rheumatol. 38(7):1496-1501: "Development of a disease severity and responder index for psoriatic arthritis (PsA)-report of the OMERACT 10 PsA special interest group" describing standardized assessments for psoriatic arthritis.).

Combinations of delivery of the anti-TNFα HuPTM mAb or antigen-binding fragment thereof, to the liver or muscles accompanied by delivery of other available treatments are encompassed by the methods provided herein. The additional treatments may be administered before, concurrently, or subsequent to the gene therapy treatment. Available treatments for subject AI-Ds(2) that could be combined with the gene therapy provided herein include but are not limited to phototherapy for psoriasis, aminosalicylates, immunomodulatory agents (e.g., azathioprine (AZA), 6-mercaptopurine (6-MP), methotrexate (MTX)), oral or topical corticosteroids (e.g., prednisone or budesonide), topical calcineurin inhibitors, antibiotics for IBD, and administration with anti-TNFα agents, including but not limited to adalimumab or infliximab.

### 5.4 Delivery of Gene Therapy Constructs

### 5.4.1 Constructs for delivery to CNS

Sections 5.3.1, 5.3.2, 5.3.3, and 5.3.4 describe recombinant vectors that contain a transgene encoding a HuPTM mAb or HuPTM Fab (or other antigen binding fragment of the HuPTM mAb) that binds to Aβ, Tau protein, CGRPR, and integrin, respectively. Such recombinant vector used for delivering the transgene should have a tropism for human CNS cells, such as glial and neuronal cells. Such vectors can include non-replicating recombinant adeno-associated virus vectors ("rAAV"), particularly those bearing an AAV9, AAVrh10, AAVrh20, AAVrh39, or AAVcy5 capsid. However, other viral vectors may be used, including but not limited to lentiviral vectors, vaccinia viral vectors, or non-viral expression vectors referred to as "naked DNA" constructs.

In specific embodiments, provided are constructs for gene therapy administration to a human subject, comprising an AAV vector, which comprises a viral capsid that is at least 95% identical to the amino acid sequence of an AAV9 capsid (SEQ ID NO: 79); and a viral or artificial genome comprising an expression cassette flanked by AAV inverted terminal repeats (ITRs) wherein the expression cassette comprises a transgene encoding the heavy and light chains of the therapeutic antibody, operably linked to one or more regulatory sequences that control expression of the transgene in human cells that express and deliver the therapeutic antibody in a therapeutically appropriate manner as disclosed herein, particularly expressed from CNS cells. In certain embodiments, the encoded AAV9 capsid has the sequence of SEQ ID NO: 79 with 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid substitutions, particularly substitutions with amino acid residues found in the corresponding position in other AAV capsids, for example, in the SUBS row of FIG. 12 which provides a comparison of the amino acid sequences of the capsid sequences of various AAVs, highlighting amino acids appropriate for substitution at different positions within the capsid sequence.

In other specific embodiments, provided are constructs for gene therapy administration to a human subject, comprising an AAV vector, which comprises a viral capsid that is at least 95% identical to the amino acid sequence of an AAVrh10 capsid (SEQ ID NO: 80); and a viral or artificial genome comprising an expression cassette flanked by AAV inverted terminal repeats (ITRs) wherein the expression cassette comprises a transgene encoding the heavy and light chains of the therapeutic antibody, operably linked to one or more regulatory sequences that control expression of the transgene in human cells that express and deliver the therapeutic antibody in a therapeutically appropriate manner as disclosed herein, particularly from CNS cells. In certain embodiments, the encoded AAVrh10 capsid has the sequence of SEQ ID NO: 80 with 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid substitutions, particularly substitutions with amino acid residues found in the corresponding position in other AAV capsids, for example, in the SUBS row of FIG. 12 which provides a comparison of the amino acid sequences of the capsid sequences of various AAVs, highlighting amino acids appropriate for substitution at different positions within the capsid sequence.

Preferably, the HuPTM mAb or antigen binding fragment thereof, including the HuPTM Fab transgene should be controlled by appropriate expression control elements for expression of the HuPTM Fab in human CNS cells, for example, the CB7 promoter (a chicken β-actin promoter and CMV enhancer), RSV promoter, GFAP prmoter (glial fibrillary acidic protein), MBP promoter (myelin basic protein), MMT promoter, EF-1α, U86 promoter, RPE65 promoter or opsin promoter, an inducible promoter, for example, a hypoxia-inducible promoter or a drug inducible promoter, such as a promoters induced by rapamycin and related agents, and other expression control elements that enhance expression of the transgene driven by the vector (e.g., introns such as the chicken β-actin intron, minute virus of mice (MVM) intron, human factor IX intron (e.g., FIX truncated intron 1), β-globin splice donor/immunoglobulin heavy chain spice acceptor intron, adenovirus splice donor /immunoglobulin splice acceptor intron, SV40 late splice donor /splice acceptor (19S/16S) intron, and hybrid adenovirus splice donor/IgG splice acceptor intron and polyA signals such as the rabbit β-globin polyA signal, human growth hormone (hGH) polyA signal, SV40 late polyA signal, synthetic polyA (SPA) signal, and bovine growth hormone (bGH) polyA signal). See, e.g., Powell and Rivera-Soto, 2015, Discov. Med., 19(102):49-57.

Gene therapy constructs are designed such that both the heavy and light chains are expressed. More specifically, the heavy and light chains should be expressed at about equal amounts, in other words, the heavy and light chains are expressed at approximately a 1:1 ratio of heavy chains to light chains. The coding sequences for the heavy and light chains can be engineered in a single construct in which the heavy and light chains are separated by a cleavable linker or IRES so that separate heavy and light chain polypeptides are expressed. The leader sequence for each of the heavy and light chains is preferably MYRMQLLLLIALSLALVTNS (SEQ ID NO: 161). Section 5.1.5, *supra*, provides specific IRES, 2A, and other linker sequences that can be used with the methods and compositions provided herein. In specific embodiments, the linker is a Furin-F2A linker RKRRAPVKQTLNFDLLKLAGDVESNPGP (SEQ ID NO: 242). In specific embodiments, the transgene is a nucleotide sequence that encodes the following: Signal sequence-heavy chain Fab portion-Furin-F2A linker-signal sequence-light chain Fab portion. See, e.g., FIGS. 2A-2C and 2F for sequences for aducanumab, crenezumab, gantenerumab, or BAN2401 Fab expression, respectively; FIG. 2D for the sequence for aTAU Fab expression; FIG. 2E for the sequence for erenumab Fab expression; and FIG. 4B for the sequence for natalizumab Fab expression.

In a specific embodiment, the constructs described herein comprise the following components: (1) AAV2 inverted terminal repeats that flank the expression cassette; (2) Control elements, which include a) the CB7 promoter, comprising the CMV enhancer/chicken β-actin promoter, b) a chicken β-actin intron and c) a rabbit β-globin poly A signal; and (3) nucleic acid sequences coding for the heavy and light chains of the Aβ-binding , Tau-binding, CGRPR-binding, integrin-binding Fab, separated by a self-cleaving furin (F)/F2A linker, ensuring expression of equal amounts of the heavy and the light chain polypeptides. An exemplary construct is provided in FIG. 1.

In specific embodiments, provided are AAV vectors comprising a viral capsid that is at least 95% identical to the amino acid sequence of an AAV9 capsid (SEQ ID NO: 79) or AAVrh10 (SEQ ID NO: 80); and an artificial genome comprising an expression cassette flanked by AAV inverted terminal repeats (ITRs), wherein the expression cassette comprises a transgene encoding an anti- Aβ, anti-Tau, anti-CGRPR, or anti-integrin mAb, or an antigen-binding fragment thereof, operably linked to one or more regulatory sequences that control expression of the transgene in human CNS cells.

### 5.4.2 Constructs for delivery to Liver or Muscle Cells

Sections 5.3.4, 5.3.5, 5.3.6, 5.3.7, 5.3.8, 5.3.9, 5.3.10, 5.3.11, 5.3.12, 5.3.13, 5.3.14 , 5.3.15 describe recombinant vectors that contain a transgene encoding a HuPTM mAb or HuPTM Fab (or other antigen binding fragment of the HuPTM mAb) that binds to interleukins (IL) or interleukin receptors (ILR), integrin, PCSK9, ANGPTL3, OxPL RANKL, PD-1/PD-L1/PD-L2, VEGF, factor D (fD), BLyS, CP-C5, MMP9, pKal, or TNFα. Such recombinant vector used for delivering the transgene can have a tropism for human liver or muscle cells. Such vectors can include non-replicating recombinant adeno-associated virus vectors ("rAAV"), particularly those bearing an AAV8 or AAV9 capsid are preferred. However, other viral vectors may be used, including but not limited to lentiviral vectors, vaccinia viral vectors, or non-viral expression vectors referred to as "naked DNA" constructs.

In specific embodiments, provided are constructs for gene therapy administration to a human subject, comprising an AAV vector, which comprises a viral capsid that is at least 95% identical to the amino acid sequence of an AAV8 capsid (SEQ ID NO: 78); and a viral or artificial genome comprising an expression cassette flanked by AAV inverted terminal repeats (ITRs) wherein the expression cassette comprises a transgene encoding the heavy and light chains of the therapeutic antibody, operably linked to one or more regulatory sequences that control expression of the transgene in human cells (e.g., human muscle or liver cells) that express and deliver the therapeutic antibody in a therapeutically appropriate manner as disclosed herein. In certain embodiments, the encoded AAV8 capsid has the sequence of SEQ ID NO: 78 with 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid substitutions, particularly substitutions with amino acid residues found in the corresponding position in other AAV capsids, for example, in the SUBS row of FIG. 12 which provides a comparison of the amino acid sequences of the capsid sequences of various AAVs, highlighting amino acids appropriate for substitution at different positions within the capsid sequence.

In specific embodiments, provided are constructs for gene therapy administration to a human subject, comprising an AAV vector, which comprises a viral capsid that is at least 95% identical to the amino acid sequence of an AAV9 capsid (SEQ ID NO: 79); and a viral or artificial genome comprising an expression cassette flanked by AAV inverted terminal repeats (ITRs) wherein the expression cassette comprises a transgene encoding the heavy and light chains of the therapeutic antibody, operably linked to one or more regulatory sequences that control expression of the transgene in human cells (e.g., human muscle or liver cells) that express and deliver the therapeutic antibody in a therapeutically appropriate manner as disclosed herein. In certain embodiments, the encoded AAV9 capsid has the sequence of SEQ ID NO: 79 with 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid substitutions, particularly substitutions with amino acid residues found in the corresponding position in other AAV capsids, for example, in the SUBS row of FIG. 12 which provides a comparison of the amino acid sequences of the capsid sequences of various AAVs, highlighting amino acids appropriate for substitution at different positions within the capsid sequence.

Preferably, the HuPTM mAb or antigen binding fragment thereof, including the HuPTM Fab transgene should be controlled by appropriate expression control elements for expression of the HuPTM Fab in human liver or muscle cells, for example, the CB7 promoter (a chicken β-actin promoter and CMV enhancer), liver specific promoters such as the TBG (Thyroxine-binding Globulin) promoter, the APOA2 promoter, the SERPINA1 (hAAT) promoter or the MIR122 promoter, or muscle specific promoters, such as the human desmin promoter or the human Pitx3 promoter, or inducible promoters, such as hypoxia-inducible promoters or rapamycin-inducible promoter, and can include other expression control elements that enhance expression of the transgene driven by the vector (e.g., introns such as the chicken β-actin intron, minute virus of mice (MVM) intron, human factor IX intron (e.g., FIX truncated intron 1), β-globin splice donor/immunoglobulin heavy chain spice acceptor intron, adenovirus splice donor /immunoglobulin splice acceptor intron, SV40 late splice donor /splice acceptor (19S/16S) intron, and hybrid adenovirus splice donor/IgG splice acceptor intron and polyA signals such as the rabbit β-globin polyA signal, human growth hormone (hGH) polyA signal, SV40 late polyA signal, synthetic polyA (SPA) signal, and bovine growth hormone (bGH) polyA signal). See, e.g., Powell and Rivera-Soto, 2015, Discov. Med., 19(102):49-57.

Gene therapy constructs are designed such that both the heavy and light chains are expressed. More specifically, the heavy and light chains should be expressed at about equal amounts, in other words, the heavy and light chains are expressed at approximately a 1:1 ratio of heavy chains to light chains. The coding sequences for the heavy and light chains can be engineered in a single construct in which the heavy and light chains are separated by a cleavable linker or IRES so that separate heavy and light chain polypeptides are expressed. The leader sequence for each of the heavy and light chains is preferably MYRMQLLLLIALSLALVTNS (SEQ ID NO: 161). Section 5.1.5, *supra*, provides specific IRES, 2A, and other linker sequences that can be used with the methods and compositions provided herein. In specific embodiments, the linker is a Furin-F2A linker RKRRAPVKQTLNFDLLKLAGDVESNPGP (SEQ ID NO: 242). In specific embodiments, the transgene is a nucleotide sequence that encodes the following: Signal sequence-heavy chain Fab portion-Furin-F2A linker-signal sequence-light chain Fab portion. See, e.g., FIGS. 3A to 3E for sequences for dupilumab, ixekizumab, secukinumab, ustekinumab, and mepolizumab Fab expression, respectively; FIGS. 4A and 4B for a sequence for vedolizumab and natalizumab Fab expression, respectively; FIGS. 5A to 5D for sequences for alirocumab, evolocumab, evinacumab, and E06-scFv Fab expression, respectively; FIG. 6 for a sequence for denosumab Fab expression; FIGS. 7A and 7B for sequences for nivolumab and pembrolizumab Fab expression, respectively; FIGS. 8A to 8C for sequences for ranibizumab, bevacizumab, and lampalizumab Fab expression, respectively; FIG. 8E for a sequence for belimumab Fab expression; FIG. 8F for a sequence for eculizumab Fab expression; FIG. 8G for a sequence for andecaliximab Fab expression; FIG. 8H for a sequence for lanadelumab Fab expression; and FIGS. 9A and 9B for a sequence for adalimumab Fab expression and infliximab Fab expression, respectively.

In a specific embodiment, the constructs described herein comprise the following components: (1) AAV2 inverted terminal repeats that flank the expression cassette; (2) Control elements, which include a) an inducible promoter, preferably a hypoxia-inducible promoter, b) a chicken β-actin intron and c) a rabbit β-globin poly A signal; and (3) nucleic acid sequences coding for the heavy and light chains of the IL/ILR-binding, integrin-binding, PCSK9-binding,, ANGPTL3-binding, RANKL-binding, OxPL-binding, PD-1/PD-L1/PD-L2 binding, VEGF-binding Fab, fD-binding, BLyS-binding, pKal-binding, or TNFα-binding Fab, separated by a self-cleaving furin (F)/F2A linker, ensuring expression of equal amounts of the heavy and the light chain polypeptides. An exemplary construct is provided in FIG. 1.

In a specific embodiment, the constructs described herein comprise the following components: (1) AAV2 inverted terminal repeats that flank the expression cassette; (2) Control elements, which include a) an inducible promoter, preferably a hypoxia-inducible promoter, b) a chicken β-actin intron and c) a rabbit β-globin poly A signal; and (3) nucleic acid sequences coding for the heavy and light chains of the IL/ILR-binding, integrin-binding, PCSK9-binding,, ANGPTL3-binding, OxPL-binding, RANKL-binding, PD-1/PD-L1/PD-L2 binding, VEGF-binding Fab, fD-binding, BLyS-binding, CP-C5-binding, MMP9-binding, pKal-binding, TNFα-binding Fab, separated by a self-cleaving furin (F)/F2A linker, ensuring expression of equal amounts of the heavy and the light chain polypeptides. An exemplary construct is provided in FIG. 1.

In specific embodiments, provided are AAV vectors comprising a viral capsid that is at least 95% identical to the amino acid sequence of an AAV8 capsid (SEQ ID NO: 78); and an artificial genome comprising an expression cassette flanked by AAV inverted terminal repeats (ITRs), wherein the expression cassette comprises a transgene encoding an anti-IL/ILR, anti-integrin, anti-PCSK9, anti-ANGPTL3, anti-OxPL, anti-RANKL, anti-PD-1, anti-PD-L1, anti-PD-L2, anti-VEGF, anti-fd, anti-BLyS, anti-CP-C5, anti-MMP9, anti-pKal, or anti-TNFα mAb, or an antigen-binding fragment thereof, operably linked to one or more regulatory sequences that control expression of the transgene in human liver or muscle cells.

In specific embodiments, provided are AAV vectors comprising a viral capsid that is at least 95% identical to the amino acid sequence of an AAV9 (SEQ ID NO: 79); and an artificial genome comprising an expression cassette flanked by AAV inverted terminal repeats (ITRs), wherein the expression cassette comprises a transgene encoding an anti-IL/ILR, anti-integrin, anti-PCSK9, anti-ANGPTL3, anti-RANKL, anti-OxPL, anti-PD-1, anti-PD-L1, anti-PD-L2, anti-VEGF, anti-fD, anti-BLyS, anti-pKal, or anti-TNFα mAb, or an antigen-binding fragment thereof, operably linked to one or more regulatory sequences that control expression of the transgene in human muscle cells.

### 5.4.3 Constructs for delivery to Retinal Cell Types

Sections 5.3.9 and 5.3.12 describe recombinant vectors that contain a transgene encoding a HuPTM mAb or HuPTM Fab (or other antigen binding fragment of the HuPTM mAb) that binds to VEGF, factor D (fD), or MMP9. Such recombinant vectors used for delivering the transgene can have a tropism for one or more human retina cell types. Such vectors can include non-replicating recombinant adeno-associated virus vectors ("rAAV"), particularly those bearing an AAV8 capsid are preferred. Alternatively, an AAV vector bearing an AAV.7m8 capsid can be used. However, other viral vectors may be used, including but not limited to lentiviral vectors, vaccinia viral vectors, or non-viral expression vectors referred to as "naked DNA" constructs.

In specific embodiments, provided are constructs for gene therapy administration to a human subject, comprising an AAV vector, which comprises a viral capsid that is at least 95% identical to the amino acid sequence of an AAV8 capsid (SEQ ID NO: 78); and a viral or artificial genome comprising an expression cassette flanked by AAV inverted terminal repeats (ITRs) wherein the expression cassette comprises a transgene encoding the heavy and light chains of the therapeutic antibody, operably linked to one or more regulatory sequences that control expression of the transgene in human cells (e.g., retina cell or liver cell types) that express and deliver the therapeutic antibody in a therapeutically appropriate manner as disclosed herein. In certain embodiments, the encoded AAV8 capsid has the sequence of SEQ ID NO: 78 with 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid substitutions, particularly substitutions with amino acid residues found in the corresponding position in other AAV capsids, for example, in the SUBS row of FIG. 12 which provides a comparison of the amino acid sequences of the capsid sequences of various AAVs, highlighting amino acids appropriate for substitution at different positions within the capsid sequence.

Preferably, the HuPTM mAb or antigen binding fragment thereof, including the HuPTM Fab transgene should be controlled by appropriate expression control elements for expression of the HuPTM Fab in human retina or liver cell types, for example, CB7 promoter (a chicken β-actin promoter and CMV enhancer), or tissue-specific promoters such as RPE-specific promoters e.g., the RPE65 promoter, or cone-specific promoters, e.g., the opsin promoter, or liver specific promoters, such as, the TBG (Thyroxine-binding Globulin) promoter, the APOA2 promoter, the SERPINA1 (hAAT) promoter or the MIR122 promoter, inducible promoters, for example, hypoxia-induced promoters and drug inducible promoters, such as promoters induced by rapamycin and related agents, and can include other expression control elements that enhance expression of the transgene driven by the vector (e.g., introns such as the chicken β-actin intron, minute virus of mice (MVM) intron, human factor IX intron (e.g., FIX truncated intron 1), β-globin splice donor/immunoglobulin heavy chain spice acceptor intron, adenovirus splice donor /immunoglobulin splice acceptor intron, SV40 late splice donor /splice acceptor (19S/16S) intron, and hybrid adenovirus splice donor/IgG splice acceptor intron and polyA signals such as the rabbit β-globin polyA signal, human growth hormone (hGH) polyA signal, SV40 late polyA signal, synthetic polyA (SPA) signal, and bovine growth hormone (bGH) polyA signal). See, e.g., Powell and Rivera-Soto, 2015, Discov. Med., 19(102):49-57.

Gene therapy constructs are designed such that both the heavy and light chains are expressed. More specifically, the heavy and light chains should be expressed at about equal amounts, in other words, the heavy and light chains are expressed at approximately a 1:1 ratio of heavy chains to light chains. The coding sequences for the heavy and light chains can be engineered in a single construct in which the heavy and light chains are separated by a cleavable linker or IRES so that separate heavy and light chain polypeptides are expressed. The leader sequence for each of the heavy and light chains is preferably MYRMQLLLLIALSLALVTNS (SEQ ID NO: 161). Section 5.1.5, *supra,* provides specific IRES, 2A, and other linker sequences that can be used with the methods and compositions provided herein. In specific embodiments, the linker is a Furin-F2A linker RKRRAPVKQTLNFDLLKLAGDVESNPGP (SEQ ID NO: 242). In specific embodiments, the transgene is a nucleotide sequence that encodes the following: Signal sequence-heavy chain Fab portion-Furin-F2A linker-signal sequence-light chain Fab portion. See FIGS. 8A to 8C for sequences for ranibizumab, bevacizumab, and lampalizumab Fab expression, respectively, and FIG. 8G for a sequence for andecaliximab Fab expression.

In a specific embodiment, the constructs described herein comprise the following components: (1) AAV2 inverted terminal repeats that flank the expression cassette; (2) Control elements, which include a) the CB7 promoter, comprising the CMV enhancer/chicken β-actin promoter, b) a chicken β-actin intron and c) a rabbit β-globin poly A signal; and (3) nucleic acid sequences coding for the heavy and light chains of the VEGF-binding, fD-binding, or MMP9-binding Fab, separated by a self-cleaving furin (F)/F2A linker, ensuring expression of equal amounts of the heavy and the light chain polypeptides. An exemplary construct is provided in FIG. 1.

In another embodiment, the constructs described herein comprise the following components: (1) AAV2 inverted terminal repeats that flank the expression cassette; (2) Control elements, which include a) the CB7 promoter, comprising the CMV enhancer/chicken β-actin promoter, b) a chicken β-actin intron and c) a rabbit β-globin poly A signal; and (3) nucleic acid sequences coding for the heavy and light chains of the VEGF-binding, fD-binding, or MMP9-binding Fab, separated by flexible peptide linker, ensuring proper folding and solubility.

In specific embodiments, provided are AAV vectors comprising a viral capsid that is at least 95% identical to the amino acid sequence of an AAV8 capsid (SEQ ID NO: 78); and an artificial genome comprising an expression cassette flanked by AAV inverted terminal repeats (ITRs), wherein the expression cassette comprises a transgene encoding an anti-VEGF mAb, anti-fD, or anti-MMP9 mAb, or an antigen-binding fragment thereof, operably linked to one or more regulatory sequences that control expression of the transgene in one or more retina cell types (such as human photoreceptor cells (cone cells, rod cells); horizontal cells; bipolar cells; amarcrine cells; retina ganglion cells (midget cell, parasol cell, bistratified cell, giant retina ganglion cell, photosensitive ganglion cell, and muller glia); and retinal pigment epithelial cells).

### 5.5 Dose Administration

### 5.5.1 Administration for delivering to CNS.

Sections 5.3.1, 5.3.2, 5.3.3, and 5.3.4 describe recombinant vectors that contain a transgene encoding a HuPTM mAb or HuPTM Fab (or other antigen binding fragment of the HuPTM mAb) that binds to Aβ, Tau protein, CGRPR, and integrin, respectively. Therapeutically effective doses of any such recombinant vector should be administered in any manner such that the recombinant vector enters the CNS, preferably by introducing the recombinant vector into the cerebral spinal fluid (CSF). In specific, embodiments, the vector is administered intrathecally, specifically intracisternally (such as to the cisterna magna) or, alternatively, lumbar delivery. Alternatively, the recombinant vector may be administered intravenously. In particular, recombinant AAV9 vectors have been shown to cross the blood-brain barrier and, as such, may be useful to deliver the anti- Aβ, anti-Tau, anti-CGRPR, or anti-integrin antibody transgene product to the CNS. Specifically, an scAAV9 may be particularly useful for intravenous administration. Intrathecal, including intracisternal or lumbar administration, or intravenous administration should result expression of the soluble transgene product in cells of the CNS. The expression of the transgene product (e.g., the encoded anti- Aβ, anti-Tau, anti-CGRPR, or anti-integrin antibody) results in delivery and maintenance of the transgene product in the CNS. Because the transgene product is continuously produced, maintenance of lower concentrations can be effective. The concentration of the transgene product can be measured in patient samples of the CSF.

Pharmaceutical compositions suitable for intrathecal, intracisternal, lumbar or intravenous administration comprise a suspension of the recombinant vector comprising the transgene encoding the anti- Aβ, anti-Tau, anti-CGRPR, or anti-integrin antibody, or antigen-binding fragment thereof, in a formulation buffer comprising a physiologically compatible aqueous buffer. The formulation buffer can comprise one or more of a polysaccharide, a surfactant, polymer, or oil.

### 5.5.2 Administration for delivering to liver or muscle tissue

Sections 5.3.4, 5.3.5, 5.3.6, 5.3.7, 5.3.8, 5.3.9, 5.3.10, 5.3.11, 5.3.12, 5.3.13, and 5.3.14 describe recombinant vectors that contain a transgene encoding a HuPTM mAb or HuPTM Fab (or other antigen binding fragment of the HuPTM mAb) that binds to interleukins (IL) or interleukin receptors (ILR), integrin, PCSK9, ANGPTL3, RANKL, PD-1/PD-L1/PD-L2, VEGF, factor D (fD), BLyS, CP-C5, MMP9, pKal, or TNFα. Therapeutically effective doses of any such recombinant vector should be administered in any manner such that the recombinant vector enters the liver or muscle (e.g., skeletal muscle), preferably by introducing the recombinant vector into the bloodstream. Alternatively, the vector may be administered directly to the liver through hepatic blood flow, e.g., via the suprahepatic veins or via the hepatic artery. In specific, embodiments, the vector is administered subcutaneously, intramuscularly or intravenously. Intramuscular, subcutaneous, intravenous or hepatic administration should result in expression of the soluble transgene product in cells of the liver or muscle. Alternatively, the vector may be administered directly to the liver through hepatic blood flow, e.g., via the suprahepatic veins or via the hepatic artery. The expression of the transgene product (e.g., the encoded an anti-IL/ILR, anti-integrin, anti-PCSK9, anti-ANGPTL3, anti-RANKL, anti-PD-1, anti-PD-L1, anti-PD-L2, anti-VEGF, anti-fD, anti-BLyS, anti-CP-C5, anti-MMP9, anti-pKal, or anti-TNFα antibody) results in delivery and maintenance of the transgene product in the liver or muscle.

The concentration of the transgene product can be measured in patient blood serum samples. In specific embodiments, doses that maintain a concentration of the anti-IL/ILR antibody transgene product at a Cₘᵢₙ of at least 2 µg/mL are desired, such as Cₘᵢₙ of 5 to 30 µg/ml, 5 to 50 µg/ml, or 5 to 80 µg/ml, or 5 to 100 µg/ml, or 5 to 200 µg/ml depending upon the mAb used. For example, to achieve a Cₘᵢₙ of approximately 60 to 90 µg/ml of dupilumab (comparable to biweekly dosing) or 170 to 200 µg/ml dupilumab (comparable to weekly dosing), or approximately 2 µg/ml to 12 µg/ml of ixekizumab, or approximately 13 µg/ml to 50 µg/ml secukinumab.

In specific embodiments, doses that maintain a concentration of the anti-TNFα antibody transgene product at a Cₘᵢₙ of at least .5 µg/mL or at least 1 µg/mL (e.g., Cₘᵢₙ of 1 to 10 µg/ml, 3 to 30 µg/ml or 5 to 15 µg/mL or 5 to 30 µg/mL) are desired.

In specific embodiments, doses that maintain a concentration of the anti-integrin antibody transgene product at a Cₘᵢₙ of at least 10 µg/ml (e.g., Cₘᵢₙ of 10 to 60 µg/ml) are desired.

In specific embodiments, doses that maintain a concentration of the anti-PCSK9 or anti-ANGPTL3 antibody transgene product at a Cₘᵢₙ of at least 10 µg/mL are desired, such as Cₘᵢₙ of 10 to 80 µg/ml.

In specific embodiments, doses that maintain a concentration of the anti-RANKL antibody transgene product at a Cₘᵢₙ of at least 10 µg/mL(e.g., Cₘᵢₙ of 10 to 50 µg/ml or 15 to 30 µg/mL) are desired.

In specific embodiments, doses that maintain a concentration of the anti-PD-1, anti-PD-L1, or anti-PD-L2 antibody transgene product at a Cₘᵢₙ of at least 10 µg/mL are desired, such as Cₘᵢₙ of 10 to 100 µg/ml or 100 to 300 µg/ml or 300 to 600 µg/ml are desired.

In specific embodiments, doses that maintain a concentration of the anti-BLyS antibody transgene product at a Cₘᵢₙ of at least 70 µg/mL (e.g., Cₘᵢₙ of 70 to 150 µg/ml or 100 to 200 µg/mL or 200 to 350 µg/mL) are desired.

In specific embodiments, doses that maintain a concentration of the anti-pKal antibody transgene product at a Cₘᵢₙ of at least 70 µg/mL (e.g., Cₘᵢₙ of 70 to 150 µg/ml or 100 to 200 µg/mL or 200 to 350 µg/mL) are desired.

The expression of the transgene product (e.g., the encoded anti-VEGF antibody) results in delivery and maintenance of the transgene product in the liver. In some embodiments, doses that maintain a concentration of the VEGF transgene product at a Cₘᵢₙ of at least 90 µg/mL are desired, such as Cₘᵢₙ of 90 µg/mL to 200 µg/mL. In specific embodiments, doses that maintain a concentration of the anti-CP-C5 antibody transgene product at a Cₘᵢₙ of at least 30 mcg/mL, e.g., Cₘᵢₙ of 30 to 300 mcg/ml or 100 to 200 mcg/mL, are desired.

However, in all cases because the transgene product is continuously produced, maintenance of lower concentrations can be effective. Notwithstanding, because the transgene product is continuously produced, maintenance of lower concentrations can be effective. The concentration of the transgene product can be measured in patient blood serum samples.

Pharmaceutical compositions suitable for intravenous, intramuscular, subcutaneous or hepatic administration comprise a suspension of the recombinant vector comprising the transgene encoding the anti-IL/ILR, anti-integrin, anti-PCSK9, anti-ANGPTL3, anti-RANKL, anti-PD-1, anti-PD-L1, anti-PD-L2, anti-VEGF, anti-fD, anti-BLyS, anti-CP-C5, anti-MMP9, anti-pKal, or anti-TNFα antibody, or antigen-binding fragment thereof, in a formulation buffer comprising a physiologically compatible aqueous buffer. The formulation buffer can comprise one or more of a polysaccharide, a surfactant, polymer, or oil.

### 5.5.3 Administration for delivering to retinal type cells

Therapeutically effective doses of the recombinant vector should be administered in any manner such that the recombinant vector enters the retina, preferably by introducing the recombinant vector directly into the eye. In specific, embodiments, the vector is administered subretinally (a surgical procedure performed by trained retinal surgeons that involves a partial vitrectomy with the subject under local anesthesia, and injection of the gene therapy into the retina; see, e.g., Campochiaro et al., 2016, Hum Gen Ther Sep 26 epub:doi: 10.1089/hum.2016.117, which is incorporated by reference herein in its entirety), or intravitreally, or suprachoroidally such as by microinjection or microcannulation. (See, e.g., Patel et al., 2012, Invest Ophth & Vis Sci 53:4433-4441; Patel et al., 2011, Pharm Res 28:166-176; Olsen, 2006, Am J Ophth 142:777-787 each of which is incorporated by reference in its entirety). Subretinal, intravitreal or suprachoroidal administration should result in expression of the soluble transgene product in one or more of the following retinal cell types: human photoreceptor cells (cone cells, rod cells); horizontal cells; bipolar cells; amarcrine cells; retina ganglion cells (midget cell, parasol cell, bistratified cell, giant retina ganglion cell, photosensitive ganglion cell, and muller glia); and retinal pigment epithelial cells. The expression of the transgene product (e.g., the encoded anti-VEGF, anti-fD, anti-MMP9 antibody) results in delivery and maintenance of the transgene product in the retina.

The concentration of the transgene product can be measured in patient samples of the vitreous humour and/or anterior chamber of the treated eye. In specific embodiments, doses that maintain a concentration of the anti-VEGF, anti-fD transgene product at a Cₘᵢₙ of at least 0.33 µg/mL in the vitreous humour, or 0.11 µg/mL in the aqueous humour (the anterior chamber of the eye) for three months are desired; thereafter, vitreous Cₘᵢₙ concentrations of the transgene product ranging from 1.70 to 6.60 µg/mL, and/or aqueous Cₘᵢₙ concentrations ranging from 0.567 to 2.20 µg/mL should be maintained. However, because the transgene product is continuously produced, maintenance of lower concentrations can be effective. Alternatively, vitreous humour concentrations can be estimated and/or monitored by measuring the patient's serum concentrations of the transgene product - the ratio of systemic to vitreal exposure to the transgene product is about 1:90,000. (*E.g.,* see, vitreous humor and serum concentrations of ranibizumab reported in Xu L, et al., 2013, Invest. Opthal. Vis. Sci. 54: 1616-1624, at p. 1621 and Table 5 at p. 1623, which is incorporated by reference herein in its entirety). However, because the transgene product is continuously produced, maintenance of lower concentrations can be effective.

Pharmaceutical compositions suitable for administration comprise a suspension of the recombinant vector comprising the transgene encoding the anti-VEGF, anti-fD, or anti-MMP9 antibody, or antigen-binding fragment thereof, in a formulation buffer comprising a physiologically compatible aqueous buffer. The formulation buffer can comprise one or more of a polysaccharide, a surfactant, polymer, or oil.

The invention is further described in the following non-limiting embodiments.
1. A pharmaceutical composition for treating Alzheimer's disease, migraines, cluster headaches, or tauopathies including chronic traumatic encephalopathy, progressive supranuclear palsy, and frontotemporal dementia in a human subject in need thereof, comprising an adeno-associated virus (AAV) vector having:
   (a) a viral capsid that is at least 95% identical to the amino acid sequence of an AAV9 capsid (SEQ ID NO: 78) or AAVrh10 (SEQ ID NO: 80); and
   (b) an artificial genome comprising an expression cassette flanked by AAV inverted terminal repeats (ITRs), wherein the expression cassette comprises a transgene encoding an anti-amyloid beta, anti-Tau, or anti-CGRPR mAb, or an antigen-binding fragment thereof, operably linked to one or more regulatory sequences that control expression of the transgene in human CNS cells;
   wherein said AAV vector is formulated for intrathecal administration to the CNS of said subject.
2. The pharmaceutical composition of embodiment 1, wherein the anti-amyloid β mAb is aducanumab, crenezumab, BAN2401, or gantenerumab and the anti-Tau mAb is aTAU and the anti-CGRPR is erenumab, eptinezumab, fremanezumab, or galcanezumab.
3. A pharmaceutical composition for treating psoriasis, psoriatic arthritis, ankylosing spondylitis, or Crohn's disease in a human subject in need thereof, comprising an AAV vector comprising:
   (a) a viral capsid that is at least 95% identical to the amino acid sequence of an AAV8 capsid (SEQ ID NO: 78) or AAV9 capsid (SEQ ID NO: 79); and
   (b) an artificial genome comprising an expression cassette flanked by AAV ITRs wherein the expression cassette comprises a transgene encoding an anti-IL17A or anti-IL12/IL23 mAb, or an antigen-binding fragment thereof, operably linked to one or more regulatory sequences that control expression of the transgene in human liver cells or in human muscle cells;
   wherein said AAV vector is formulated for intravenous administration to the liver or muscle of said subject.
4. The pharmaceutical composition of embodiment 3 wherein the anti-IL17A or anti IL12/IL23 mAb is ixekizumab, secukinumab, or ustekinumab.
5. A pharmaceutical composition for treating multiple sclerosis, ulcerative colitis or Crohn's disease in a human subject in need thereof, comprising an AAV vector comprising:
   (a) a viral capsid that is at least 95% identical to the amino acid sequence of an AAV8 capsid (SEQ ID NO: 78), AAV9 capsid (SEQ ID NO: 79), or AAVrh10 (SEQ ID NO: 80); and
   (b) an artificial genome comprising an expression cassette flanked by AAV ITRs wherein the expression cassette comprises a transgene encoding an anti-integrin mAb, or an antigen-binding fragment thereof, operably linked to one or more regulatory sequences that control expression of the transgene in human liver cells, human muscle cells or human CNS cells;
   wherein said AAV vector is formulated for intravenous administration to the liver or muscle of said subject or intrathecal administration to the CNS of said subject.
6. The pharmaceutical composition of embodiment 5, wherein the anti-integrin mAb is vedolizumab or natalizumab.
7. A pharmaceutical composition for treating atopic dermatitis in a human subject in need thereof, comprising AAV vector comprising:
   (a) a viral capsid that is at least 95% identical to the amino acid sequence of an AAV8 capsid (SEQ ID NO: 78) or an AAV9 capsid (SEQ ID NO: 79); and
   (b) an artificial genome comprising an expression cassette flanked by AAV ITRs wherein the expression cassette comprises a transgene encoding an anti-IL4R mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
8. The pharmaceutical composition of embodiment 7, wherein the anti-IL-4R mAb is dupilumab.
9. A pharmaceutical composition for treating asthma in a human subject in need thereof, comprising an AAV vector comprising:
   (a) a viral capsid that is at least 95% identical to the amino acid sequence of an AAV8 capsid (SEQ ID NO: 78) or an AAV9 capsid (SEQ ID NO: 79); and
   (b) an artificial genome comprising an expression cassette flanked by AAV ITRs wherein the expression cassette comprises a transgene encoding an anti-IL-5 mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
10. The pharmaceutical composition of embodiment 9, wherein the anti-IL-5 mAb is mepolizumab.
11. A pharmaceutical composition for treating HeFH, HoFH, dyslipidemia, cardiovascular disease including atherosclerotic cardiovascular disease (ACD), atherosclerotic plaque formation, abnormally high levels of non-HDL cholesterol and LDL, aortic stenosis, hepatic stenosis, or hypercholesterolemia in a human subject in need thereof, comprising an AAV vector comprising:
   (a) a viral capsid that is at least 95% identical to the amino acid sequence of an AAV8 capsid (SEQ ID NO: 78) or an AAV9 capsid (SEQ ID NO: 79); and
   (b) an artificial genome comprising an expression cassette flanked by AAV inverted terminal repeats (ITRs) wherein the expression cassette comprises a transgene encoding an anti-PCSK9, anti-ANGPTL3, anti-OxPL mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
12. The pharmaceutical composition of embodiment 11, wherein the anti-PCSK9 or anti-ANGPTL3 mAb is alirocumab, evolocumab or evinacumab or wherein the anti-OxPL is E06.
13. A pharmaceutical composition for treating osteoporosis in a human subject in need thereof, comprising an AAV vector comprising:
   (a) a viral capsid that is at least 95% identical to the amino acid sequence of an AAV8 capsid (SEQ ID NO: 78) or AAV9 capsid (SEQ ID NO: 79); and
   (b) an artificial genome comprising an expression cassette flanked by AAV ITRs wherein the expression cassette comprises a transgene encoding an anti-RANKL mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
14. The pharmaceutical composition of embodiment 13, wherein the anti-RANLK mAb is densomab.
15. A pharmaceutical composition for treating metastatic melanoma, lymphoma or non-small cell lung carcinoma in a human subject in need thereof, comprising an AAV vector comprising:
   (a) a viral capsid that is at least 95% identical to the amino acid sequence of an AAV8 capsid (SEQ ID NO: 78) or AAV9 capsid (SEQ ID NO: 79); and
   (b) an artificial genome comprising an expression cassette flanked by AAV ITRs wherein the expression cassette comprises a transgene encoding a PD-1 blocker mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
16. The pharmaceutical composition of embodiment 15, wherein the PD-1 blocker mAb is nivolumab or pembrolizumab.
17. A pharmaceutical composition for treating systemic lupus erythromatosis (SLE) in a human subject in need thereof, comprising an AAV vector comprising:
   (a) a viral capsid that is at least 95% identical to the amino acid sequence of an AAV8 capsid (SEQ ID NO: 78) or an AAV9 capsid (SEQ ID NO: 79); and
   (b) an artificial genome comprising an expression cassette flanked by AAV ITRs wherein the expression cassette comprises a transgene encoding an anti-BLyS mAb, or an antigen-binding fragment thereof, operably linked to one or more regulatory sequences that control expression of the transgene in human liver cells or human muscle cells;
   wherein said AAV vector is formulated for intravenous administration to the liver or muscle of said subject.
18. The pharmaceutical composition of embodiment 17, wherein the anti-BLyS mAb is belimumab.
19. A pharmaceutical composition for treating ocular disorders, including age-related macular degeneration, in a human subject in need thereof, comprising an AAV vector comprising:
   (a) a viral capsid that is at least 95% identical to the amino acid sequence of an AAV8 capsid (SEQ ID NO: 78) or an AAV9 capsid (SEQ ID NO: 79); and
   (b) an artificial genome comprising an expression cassette flanked by AAV ITRs wherein the expression cassette comprises a transgene encoding an anti-VEGF, anti-MMP9, or anti-fD mAb, or an antigen-binding fragment thereof, operably linked to one or more regulatory sequences that control expression of the transgene in human retinal cells;
   wherein said AAV vector is formulated for subretinal, intravitreal or suprachoroidal administration to the eye of said subject.
20. The pharmaceutical composition of embodiment 19, wherein the anti-MMP9 is andecaliximab, the anti-VEGF is ranibizumab, bevacizumab, brolucizumab, and anti-fD mAb is lampalizumab.
21. A pharmaceutical composition for treating cystic fibrosis (CF), rheumatoid arthritis (RA), UC ,CD, solid tumors, pancreatic adenocarcinoma, lung adenocarcinoma, lung squamous cell carcinoma, esophagogastric adenocarcinoma, gastric cancer, colorectal cancer, or breast cancer in a human subject in need thereof, comprising an AAV vector comprising:
   (a) a viral capsid that is at least 95% identical to the amino acid sequence of an AAV8 capsid (SEQ ID NO: 78) or an AAV9 capsid (SEQ ID NO: 79); and
   (b) an artificial genome comprising an expression cassette flanked by AAV ITRs wherein the expression cassette comprises a transgene encoding an anti-MMP9 or an antigen-binding fragment thereof, operably linked to one or more regulatory sequences that control expression of the transgene in human liver cells or human muscle cells;
   wherein said AAV vector is formulated for intravenous administration to the liver or muscle of said subject.
22. The pharmaceutical composition of embodiment 21, wherein the anti-MMP9 mAb is andecaliximab.
23. A pharmaceutical composition for treating hereditary angioedema in a human subject in need thereof, comprising an AAV vector comprising:
   (a) a viral capsid that is at least 95% identical to the amino acid sequence of an AAV8 capsid (SEQ ID NO: 78) or an AAV9 capsid (SEQ ID NO: 79); and
   (b) an artificial genome comprising an expression cassette flanked by AAV ITRs wherein the expression cassette comprises a transgene encoding an anti-kallikrein or an antigen-binding fragment thereof, operably linked to one or more regulatory sequences that control expression of the transgene in human muscle cells or human liver cells;
   wherein said AAV vector is formulated for intravenous administration to the liver or muscle of said subject.
24. The composition of embodiment 23, wherein the anti-kallikrein mAb is lanadelumab.
25. A pharmaceutical composition for treating rheumatoid arthritis, psoriatic arthritis, ankylosing spondylitis, Crohn's disease, plaque psoriasis, or ulcerative colitis, in a human subject in need thereof, comprising an AAV vector comprising:
   (a) a viral capsid that is at least 95% identical to the amino acid sequence of an AAV8 capsid (SEQ ID NO: 78) or AAV9 capsid (SEQ ID NO: 79); and
   (b) an artificial genome comprising an expression cassette flanked by AAV ITRs wherein the expression cassette comprises a transgene encoding an anti-TNF-alpha mAb, or an antigen-binding fragment thereof, operably linked to one or more regulatory sequences that control expression of the transgene in human muscle or liver cells;
   wherein said AAV vector is formulated for intravenous administration to the liver or muscle of said subject.
26. The pharmaceutical composition of embodiment 25, wherein the anti-TNF-alpha mAb is adalimumab or infliximab.

The invention is further illustrated in the following non-limiting examples.

### 6. EXAMPLES

### 6.1 EXAMPLE 1: Aducanumab Fab cDNA-Based Vector

An aducanumab Fab cDNA-based vector is constructed comprising a transgene comprising nucleotide sequences encoding the Fab portion of the heavy and light chain sequences of aducanumab (amino acid sequences being SEQ ID NOs. 1 and 2, respectively). The nucleotide sequence coding for the Fab portion of the heavy and light chain is codon optimized for expression in human CNS cells and may be the nucleotide sequence of SEQ ID NOS: 101 and 102, respectively. The transgene also comprises nucleotide sequences that encodes a signal peptide, particularly, MYRMQLLLLIALSLALVTNS (SEQ ID NO: 161). The nucleotide sequences encoding the light chain and heavy chain are separated by IRES elements or 2A cleavage sites to create a bicistronic vector. See FIG. 2A for amino acid sequence of a transgene product. The vector additionally includes a constitutive promoter, such as CB7 or an inducible promoter, such as a hypoxia-inducible promoter.

### 6.2 EXAMPLE 2: Crenezumab Fab cDNA-Based Vector

A crenezumab Fab cDNA-based vector is constructed comprising a transgene comprising nucleotide sequences encoding the Fab portion of the heavy and light chain sequences of crenezumab (amino acid sequences being SEQ ID NOs. 3 and 4, respectively). The nucleotide sequence coding for the Fab portion of the heavy and light chain is codon optimized for expression in human CNS cells and may be the nucleotide sequence of SEQ ID NOS: 103 and 104, respectively. The transgene also comprises nucleotide sequences that encodes a signal peptide, particularly, MYRMQLLLLIALSLALVTNS (SEQ ID NO: 161). The nucleotide sequences encoding the light chain and heavy chain are separated by IRES elements or 2A cleavage sites to create a bicistronic vector. See FIG. 2B for amino acid sequence of a transgene product. The vector additionally includes a constitutive promoter, such as CB7 or an inducible promoter, such as a hypoxia-inducible promoter.

### 6.3 EXAMPLE 3: Gantenerumab Fab cDNA-Based Vector

A gantenerumab Fab cDNA-based vector is constructed comprising a transgene comprising nucleotide sequences encoding the Fab portion of the heavy and light chain sequences of gantenerumab (amino acid sequences being SEQ ID NOs. 5 and 6, respectively). The nucleotide sequence coding for the Fab portion of the heavy and light chain is codon optimized for expression in human CNS cells and may be the nucleotide sequence of SEQ ID NOS: 105 and 106, respectively. The transgene also comprises nucleotide sequences that encodes a signal peptide, particularly, MYRMQLLLLIALSLALVTNS (SEQ ID NO: 161). The nucleotide sequences encoding the light chain and heavy chain are separated by IRES elements or 2A cleavage sites to create a bicistronic vector. See FIG. 2C for amino acid sequence of a transgene product. The vector additionally includes a constitutive promoter, such as CB7 or an inducible promoter, such as a hypoxia-inducible promoter.

### 6.4 EXAMPLE 4: Dupilumab Fab cDNA-Based Vector

An dupilumab Fab cDNA-based vector is constructed comprising a transgene comprising nucleotide sequences encoding the Fab portion of the heavy and light chain sequences of dupilumab (amino acid sequences being SEQ ID NOs. 7 and 8, respectively). The nucleotide sequence coding for the Fab portion of the heavy and light chain is codon optimized for expression in human liver or muscle cells and may be the nucleotide sequence of SEQ ID NOS: 107 and 108, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 3A for amino acid sequence of a transgene product. The vector additionally includes a constitutive promoter, such as CB7 or an inducible promoter, such as a hypoxia-inducible promoter.

### 6.5 EXAMPLE 5: Ixekizumab Fab cDNA-Based Vector

An ixekizumab Fab cDNA-based vector is constructed comprising a transgene comprising nucleotide sequences encoding the Fab portion of the heavy and light chain sequences of ixekizumab (amino acid sequences being SEQ ID NOs. 9 and 10, respectively). The nucleotide sequence coding for the Fab portion of the heavy and light chain is codon optimized for expression in human muscle or liver cells and may be the nucleotide sequence of SEQ ID NOS: 109 and 110, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 3B for amino acid sequence of a transgene product. Optionally, the vector additionally comprises a hypoxia-inducible promoter.

### 6.6 EXAMPLE 6: Secukinumab Fab cDNA-Based Vector

A secukinumab Fab cDNA-based vector is constructed comprising a transgene comprising nucleotide sequences encoding the Fab portion of the heavy and light chain sequences of secukinumab (amino acid sequences being SEQ ID NOs. 11 and 12, respectively). The nucleotide sequence coding for the Fab portion of the heavy and light chain is codon optimized for expression in human liver or muscle cells and may be the nucleotide sequence of SEQ ID NOS: 111 and 112, respectively. The transgene also comprises nucleotide sequences that encode a signal peptide chosen from the group listed in Table 2 or 3, respectively. The nucleotide sequences encoding the light chain and heavy chain are separated by IRES elements or 2A cleavage sites to create a bicistronic vector. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 3C for amino acid sequence of a transgene product. The vector additionally includes a constitutive promoter, such as CB7 or an inducible promoter, such as a hypoxia-inducible promoter.

### 6.7 EXAMPLE 7: Ustekinumab Fab cDNA-Based Vector

An ustekinumab Fab cDNA-based vector is constructed comprising a transgene comprising nucleotide sequences encoding the Fab portion of the heavy and light chain sequences of ustekinumab (amino acid sequences being SEQ ID NOs. 13 and 14, respectively). The nucleotide sequence coding for the Fab portion of the heavy and light chain is codon optimized for expression in human liver or muscle cells and may be the nucleotide sequence of SEQ ID NOS: 113 and 114, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 3D for amino acid sequence of a transgene product. The vector additionally includes a constitutive promoter, such as CB7 or an inducible promoter, such as a hypoxia-inducible promoter.

### 6.8 EXAMPLE 8: Mepolizumab Fab cDNA-Based Vector

A mepolizumab Fab cDNA-based vector is constructed comprising a transgene comprising nucleotide sequences encoding the Fab portion of the heavy and light chain sequences of mepolizumab (amino acid sequences being SEQ ID NOs. 15 and 16, respectively). The nucleotide sequence coding for the Fab portion of the heavy and light chain is codon optimized for expression in human muscle or liver cells and may be the nucleotide sequence of SEQ ID NOS: 115 and 116, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 3E for amino acid sequence of a transgene product. The vector additionally includes a constitutive promoter, such as CB7 or an inducible promoter, such as a hypoxia-inducible promoter.

### 6.9 EXAMPLE 9: Vedolizumab Fab cDNA-Based Vector

A vedolizumab Fab cDNA-based vector is constructed comprising a transgene comprising nucleotide sequences encoding the Fab portion of the heavy and light chain sequences of vedolizumab (amino acid sequences being SEQ ID NOs. 17 and 18, respectively). The nucleotide sequence coding for the Fab portion of the heavy and light chain is codon optimized for expression in human liver or muscle cells and may be the nucleotide sequence of SEQ ID NOS: 117 and 118, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 4A for amino acid sequence of a transgene product. The vector additionally includes a constitutive promoter, such as CB7 or an inducible promoter, such as a hypoxia-inducible promoter.

### 6.10 EXAMPLE 10: Natalizumab Fab cDNA-Based Vector

A natalizumab Fab cDNA-based vector is constructed comprising a transgene comprising nucleotide sequences encoding the Fab portion of the heavy and light chain sequences of natalizumab (amino acid sequences being SEQ ID NOs. 19 and 20, respectively). The nucleotide sequence coding for the Fab portion of the heavy and light chain is codon optimized for expression in human liver or muscle cells and may be the nucleotide sequence of SEQ ID NOS: 119 and 120, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 4B for amino acid sequence of a transgene product. The vector additionally includes a constitutive promoter, such as CB7 or an inducible promoter, such as a hypoxia-inducible promoter.

### 6.11 EXAMPLE 11: Alirocumab Fab cDNA-Based Vector

An alirocumab Fab cDNA-based vector is constructed comprising a transgene comprising nucleotide sequences encoding the Fab portion of the heavy and light chain sequences of alirocumab (amino acid sequences being SEQ ID NOs. 21 and 22, respectively). The nucleotide sequence coding for the Fab portion of the heavy and light chain is codon optimized for expression in human liver or muscle cells and may be the nucleotide sequence of SEQ ID NOS: 121 and 122, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 5A for amino acid sequence of a transgene product. The vector additionally includes a constitutive promoter, such as CB7 or an inducible promoter, such as a hypoxia-inducible promoter.

### 6.12 EXAMPLE 12: Evolocumab Fab cDNA-Based Vector

An evolocumab Fab cDNA-based vector is constructed comprising a transgene comprising nucleotide sequences encoding the Fab portion of the heavy and light chain sequences of evolocumab (amino acid sequences being SEQ ID NOs. 23 and 24, respectively). The nucleotide sequence coding for the Fab portion of the heavy and light chain is codon optimized for expression in human liver or muscle cells and may be the nucleotide sequence of SEQ ID NOS: 123 and 124, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 5B for amino acid sequence of a transgene product. The vector additionally includes a constitutive promoter, such as CB7 or an inducible promoter, such as a hypoxia-inducible promoter.

### 6.13 EXAMPLE 13: Evinacumab Fab cDNA-Based Vector

An evinacumab Fab cDNA-based vector is constructed comprising a transgene comprising nucleotide sequences encoding the Fab portion of the heavy and light chain sequences of evinacumab (amino acid sequences being SEQ ID NOs. 25 and 26, respectively). The nucleotide sequence coding for the Fab portion of the heavy and light chain is codon optimized for expression in human liver or muscle cells and may be the nucleotide sequence of SEQ ID NOS: 125 and 126, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 5C for amino acid sequence of a transgene product. The vector additionally includes a constitutive promoter, such as CB7 or an inducible promoter, such as a hypoxia-inducible promoter.

### 6.14 EXAMPLE 14: Denosumab Fab cDNA-Based Vector

A denosumab Fab cDNA-based vector is constructed comprising a transgene comprising nucleotide sequences encoding the Fab portion of the heavy and light chain sequences of denosumab (amino acid sequences being SEQ ID NOs. 27 and 28, respectively). The nucleotide sequence coding for the Fab portion of the heavy and light chain is codon optimized for expression in human liver or muscle cells and may be the nucleotide sequence of SEQ ID NOS: 127 and 128, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 6 for amino acid sequence of a transgene product. The vector additionally includes a constitutive promoter, such as CB7 or an inducible promoter, such as a hypoxia-inducible promoter.

### 6.15 EXAMPLE 15: Nivolumab Fab cDNA-Based Vector

A nivolumab Fab cDNA-based vector is constructed comprising a transgene comprising nucleotide sequences encoding the Fab portion of the heavy and light chain sequences of nivolumab (amino acid sequences being SEQ ID NOs. 29 and 30 respectively). The nucleotide sequence coding for the Fab portion of the heavy and light chain is codon optimized for expression in human liver or muscle cells and may be the nucleotide sequence of SEQ ID NOS: 129 and 130, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 7A for amino acid sequence of a transgene product. The vector additionally includes a constitutive promoter, such as CB7 or an inducible promoter, such as a hypoxia-inducible promoter.

### 6.16 EXAMPLE 16: Pembrolizumab Fab cDNA-Based Vector

A pembrolizumab Fab cDNA-based vector is constructed comprising a transgene comprising nucleotide sequences encoding the Fab portion of the heavy and light chain sequences of pembrolizumab (amino acid sequences being SEQ ID NOs. 31 and 32, respectively). The nucleotide sequence coding for the Fab portion of the heavy and light chain is codon optimized for expression in human liver or muscle cells and may be the nucleotide sequence of SEQ ID NOS: 131 and 132, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by IRES elements or 2A cleavage sites to create a bicistronic vector. See FIG. 7B for amino acid sequence of a transgene product. The vector additionally includes a constitutive promoter, such as CB7 or an inducible promoter, such as a hypoxia-inducible promoter.

### 6.17 EXAMPLE 17: Ranibizumab Fab cDNA-Based Vector

A ranibizumab Fab cDNA-based vector is constructed comprising a transgene comprising nucleotide sequences encoding the Fab portion of the heavy and light chain sequences of ranibizumab (amino acid sequences being SEQ ID NOs. 33 and 34, respectively). The nucleotide sequence coding for the Fab portion of the heavy and light chain is codon optimized for expression in human retinal or liver cells and may be the nucleotide sequence of SEQ ID NOS: 133 and 134, respectively. The transgene also comprises nucleotide sequences that encode a signal peptide that may be MYRMQLLLLIALSLALVTNS (SEQ ID NO: 161) or is a retina specific signal sequence from Table 1 or a liver specific signal sequence from Table 3. The nucleotide sequences encoding the light chain and heavy chain are separated by IRES elements or 2A cleavage sites to create a bicistronic vector. See FIG. 8A for amino acid sequence of a transgene product. The vector additionally includes a constitutive promoter, such as CB7 or an inducible promoter, such as a hypoxia-inducible promoter.

### 6.18 EXAMPLE 18: Bevacizumab Fab cDNA-Based Vector

A bevacizumab Fab cDNA-based vector is constructed comprising a transgene comprising nucleotide sequences encoding the Fab portion of the heavy and light chain sequences of bevacizumab (amino acid sequences being SEQ ID NOs. 35 and 36, respectively). The nucleotide sequence coding for the Fab portion of the heavy and light chain is codon optimized for expression in human retinal or liver cells and may be the nucleotide sequence of SEQ ID NOS: 135 and 136, respectively. The transgene also comprises nucleotide sequences that encode a signal peptide that may be MYRMQLLLLIALSLALVTNS (SEQ ID NO: 161) or is a retina specific signal sequence from Table 1 or a liver specific signal sequence from Table 3. The nucleotide sequences encoding the light chain and heavy chain are separated by IRES elements or 2A cleavage sites to create a bicistronic vector. See FIG. 8B for amino acid sequence of a transgene product. The vector additionally includes a constitutive promoter, such as CB7 or an inducible promoter, such as a hypoxia-inducible promoter.

### 6.19 EXAMPLE 19: Lampalizumab Fab cDNA-Based Vector

A lampalizumab Fab cDNA-based vector is constructed comprising a transgene comprising nucleotide sequences encoding the Fab portion of the heavy and light chain sequences of lampalizumab (amino acid sequences being SEQ ID NOs. 37 and 38, respectively). The nucleotide sequence coding for the Fab portion of the heavy and light chain is codon optimized for expression in human retinal cells and may be the nucleotide sequence of SEQ ID NOS: 137 and 138, respectively. The transgene also comprises nucleotide sequences that encode a signal peptide that may be MYRMQLLLLIALSLALVTNS (SEQ ID NO: 161) or is a retina specific signal sequence from Table 1. The nucleotide sequences encoding the light chain and heavy chain are separated by IRES elements or 2A cleavage sites to create a bicistronic vector. See FIG. 8C for amino acid sequence of a transgene product. The vector additionally includes a constitutive promoter, such as CB7 or an inducible promoter, such as a hypoxia-inducible promoter.

### 6.20 EXAMPLE 20: Brolucizumab scFv cDNA-Based Vector

A brolucizumab scFv cDNA-based vector is constructed comprising a transgene comprising nucleotide sequences encoding the variable domain of the heavy and light chain sequences of brolucizumab (amino acid sequences being SEQ ID NOs. 39 and 40, respectively). The nucleotide sequence coding for the variable domains of the heavy and light chain is codon optimized for expression in human retinal or liver cells and may be the nucleotide sequence of SEQ ID NOS: 139 and 140, respectively. The transgene also comprises nucleotide sequences that encode a signal peptide that may be MYRMQLLLLIALSLALVTNS (SEQ ID NO: 161) or is a retina specific signal sequence from Table 1 or a liver specific signal sequence from Table 3. The nucleotide sequences encoding the light chain and heavy chain are separated by a flexible peptide linker. See FIG. 8D for amino acid sequence of a transgene product. The vector additionally includes a constitutive promoter, such as CB7 or an inducible promoter, such as a hypoxia-inducible promoter.

### 6.21 EXAMPLE 21: Belimumab Fab cDNA-Based Vector

A belimumab Fab cDNA-based vector is constructed comprising a transgene comprising nucleotide sequences encoding the Fab portion of the heavy and light chain sequences of belimumab (amino acid sequences being SEQ ID NOs. 41 and 42, respectively). The nucleotide sequence coding for the Fab portion of the heavy and light chain is codon optimized for expression in human liver cells and may be the nucleotide sequence of SEQ ID NOS: 141 and 142, respectively. The transgene also comprises nucleotide sequences that encode a signal peptide that may be MYRMQLLLLIALSLALVTNS (SEQ ID NO: 161) or is a liver specific signal sequence from Table 3. The nucleotide sequences encoding the light chain and heavy chain are separated by IRES elements or 2A cleavage sites to create a bicistronic vector. See FIG. 8E for amino acid sequence of a transgene product. The vector additionally includes a constitutive promoter, such as CB7 or an inducible promoter, such as a hypoxia-inducible promoter.

### 6.22 EXAMPLE 22: Eculizumab Fab cDNA-Based Vector

An eculizumab Fab cDNA-based vector is constructed comprising a transgene comprising nucleotide sequences encoding the Fab portion of the heavy and light chain sequences of eculizumab (amino acid sequences being SEQ ID NOs. 43 and 44, respectively). The nucleotide sequence coding for the Fab portion of the heavy and light chain is codon optimized for expression in human liver cells and may be the nucleotide sequence of SEQ ID NOS: 143 and 144, respectively. The transgene also comprises nucleotide sequences that encode a signal peptide that may be MYRMQLLLLIALSLALVTNS (SEQ ID NO: 161) or is a liver specific signal sequence from Table 3. The nucleotide sequences encoding the light chain and heavy chain are separated by IRES elements or 2A cleavage sites to create a bicistronic vector. See FIG. 8F for amino acid sequence of a transgene product. The vector additionally includes a constitutive promoter, such as CB7 promoter.

### 6.23 EXAMPLE 23: Andecaliximab Fab cDNA-Based Vector

An andecaliximab Fab cDNA-based vector is constructed comprising a transgene comprising nucleotide sequences encoding the Fab portion of the heavy and light chain sequences of andecaliximab (amino acid sequences being SEQ ID NOs. 45 and 46, respectively). The nucleotide sequence coding for the Fab portion of the heavy and light chain is codon optimized for expression in human liver cells and may be the nucleotide sequence of SEQ ID NOS: 145 and 146, respectively. The transgene also comprises nucleotide sequences that encode a signal peptide that may be MYRMQLLLLIALSLALVTNS (SEQ ID NO: 161) or is a liver specific signal sequence from Table 3. The nucleotide sequences encoding the light chain and heavy chain are separated by IRES elements or 2A cleavage sites to create a bicistronic vector. See FIG. 8G for amino acid sequence of a transgene product. The vector additionally includes a constitutive promoter, such as CB7 or an inducible promoter, such as a hypoxia-inducible promoter.

### 6.24 EXAMPLE 24: Lanadelumab Fab cDNA-Based Vector

A lanadelumab Fab cDNA-based vector is constructed comprising a transgene comprising nucleotide sequences encoding the Fab portion of the heavy and light chain sequences of lanadelumab (amino acid sequences being SEQ ID NOs. 47 and 48, respectively). The nucleotide sequence coding for the Fab portion of the heavy and light chain is codon optimized for expression in human liver cells and may be the nucleotide sequence of SEQ ID NOS: 147 and 148, respectively. The transgene also comprises nucleotide sequences that encode a signal peptide that may be MYRMQLLLLIALSLALVTNS (SEQ ID NO: 161) or is a liver specific signal sequence from Table 3. The nucleotide sequences encoding the light chain and heavy chain are separated by IRES elements or 2A cleavage sites to create a bicistronic vector. See FIG. 8H for amino acid sequence of a transgene product. The vector additionally includes a constitutive promoter, such as CB7 or an inducible promoter, such as a hypoxia-inducible promoter.

### 6.25 EXAMPLE 25: Adalimumab Fab cDNA-Based Vector

An adalimumab Fab cDNA-based vector is constructed comprising a transgene comprising nucleotide sequences encoding the Fab portion of the heavy and light chain sequences of adalimumab (amino acid sequences being SEQ ID NOs. 49 and 50, respectively). The nucleotide sequence coding for the Fab portion of the heavy and light chain is codon optimized for expression in human liver or muscle cells and may be the nucleotide sequence of SEQ ID NOS: 149 and 150, respectively. The transgene also comprises nucleotide sequences that encode a signal peptide that may be MYRMQLLLLIALSLALVTNS (SEQ ID NO: 161) or is a liver or muscle specific signal sequence from Table 2 or 3. The nucleotide sequences encoding the light chain and heavy chain are separated by IRES elements or 2A cleavage sites to create a bicistronic vector. See FIG. 9A for amino acid sequence of transgene product. The vector additionally includes an inducible promoter, such as a hypoxia-inducible promoter.

### 6.26 EXAMPLE 26: Infliximab Fab cDNA-Based Vector

An infliximab Fab cDNA-based vector is constructed comprising a transgene comprising nucleotide sequences encoding the Fab portion of the heavy and light chain sequences of infliximab (amino acid sequences being SEQ ID NOs. 51 and 52, respectively). The nucleotide sequence coding for the Fab portion of the heavy and light chain is codon optimized for expression in human liver cells and may be the nucleotide sequence of SEQ ID NOS: 151 and 152, respectively. The transgene also comprises nucleotide sequences that encode a signal peptide that may be MYRMQLLLLIALSLALVTNS (SEQ ID NO: 161) or is a liver specific signal sequence from Table 3. The nucleotide sequences encoding the light chain and heavy chain are separated by IRES elements or 2A cleavage sites to create a bicistronic vector. See FIG. 9B for amino acid sequence of a transgene product. The vector additionally includes a constitutive promoter, such as CB7 promoter.

### 6.27 EXAMPLE 27: aTAU Fab cDNA-Based Vector

An aTAU Fab cDNA-based vector is constructed comprising a transgene comprising nucleotide sequences encoding the Fab portion of the heavy and light chain sequences of aTAU (amino acid sequences being SEQ ID NOs. 53 and 54, respectively). The nucleotide sequence coding for the Fab portion of the heavy and light chain is codon optimized for expression in human CNS cells and may be the nucleotide sequence of SEQ ID NOS: 153 and 154, respectively. The transgene also comprises nucleotide sequences that encodes a signal peptide, particularly, MYRMQLLLLIALSLALVTNS (SEQ ID NO: 161). The nucleotide sequences encoding the light chain and heavy chain are separated by IRES elements or 2A cleavage sites to create a bicistronic vector. See FIG. 2D for amino acid sequence of a transgene product. The vector additionally includes a constitutive promoter, such as CB7 or an inducible promoter, such as a hypoxia-inducible promoter.

### 6.28 EXAMPLE 28: Erenumab Fab cDNA-Based Vector

An erenumab Fab cDNA-based vector is constructed comprising a transgene comprising nucleotide sequences encoding the Fab portion of the heavy and light chain sequences of erenumab (amino acid sequences being SEQ ID NOs. 55 and 56, respectively). The nucleotide sequence coding for the Fab portion of the heavy and light chain is codon optimized for expression in human CNS cells and may be the nucleotide sequence of SEQ ID NOS: 155 and 156, respectively. The transgene also comprises nucleotide sequences that encodes a signal peptide, particularly, MYRMQLLLLIALSLALVTNS (SEQ ID NO: 161). The nucleotide sequences encoding the light chain and heavy chain are separated by IRES elements or 2A cleavage sites to create a bicistronic vector. See FIG. 2E for amino acid sequence of a transgene product. The vector additionally includes a constitutive promoter, such as CB7 or an inducible promoter, such as a hypoxia-inducible promoter.

### 6.29 EXAMPLE 29: BAN2401 Fab cDNA-Based Vector

An BAN2401 Fab cDNA-based vector is constructed comprising a transgene comprising nucleotide sequences encoding the Fab portion of the heavy and light chain sequences of BAN2401 (amino acid sequences being SEQ ID NOs. 57 and 58, respectively). The nucleotide sequence coding for the Fab portion of the heavy and light chain is codon optimized for expression in human CNS cells and may be the nucleotide sequence of SEQ ID NOS: 157 and 158, respectively. The transgene also comprises nucleotide sequences that encodes a signal peptide, particularly, MYRMQLLLLIALSLALVTNS (SEQ ID NO: 161). The nucleotide sequences encoding the light chain and heavy chain are separated by IRES elements or 2A cleavage sites to create a bicistronic vector. See FIG. 2F for amino acid sequence of a transgene product. The vector additionally includes a constitutive promoter, such as CB7 or an inducible promoter, such as a hypoxia-inducible promoter.

### 6.30 EXAMPLE 30: E06-scFv Fab cDNA-Based Vector

An E06-scFv Fab cDNA-based vector is constructed comprising a transgene comprising nucleotide sequences encoding the heavy and light chain variable domain sequences of E06-scFv (amino acid sequences being SEQ ID NOs. 59 and 60, respectively). The nucleotide sequence coding for the heavy and light chain variable domains is codon optimized for expression in human liver or muscle cells and may be the nucleotide sequence of SEQ ID NOS: 159 and 160, respectively. The transgene also comprises nucleotide sequences that encode a signal peptide that may be MYRMQLLLLIALSLALVTNS (SEQ ID NO: 161) or is a liver specific signal sequence from Table 2 or a muscle specific signal sequence from Table 3. The nucleotide sequences encoding the light chain and heavy chain are separated by a flexible peptide linker. See FIG. 5D for amino acid sequence of a transgene product. The vector additionally includes a constitutive promoter, such as CB7 or an inducible promoter, such as a hypoxia-inducible promoter.

**TABLE 4. Table of Fab Fragment Amino Acid Sequences**

| mAb | Chain/ SEQ ID NO. | Sequence |
|---|---|---|
| Aducanumab | Heavy/ SEQ ID NO:1 | XVQLVESGGG VVQPGRSLRL SCAASGFAFS SYGMHWVRQA PGKGLEWVAV IWFDGTKKYY TDSVKGRFTI SRDNSKNTLY LQMNTLRAED TAVYYCARDR GIGARRGPYY MDVWGKGTTV TVSSASTKGP SVFPLAPSSK STSGGTAALG CLVKDYFPEP VTVSWNSGAL TSGVHTFPAV LQSSGLYSLS SVVTVPSSSL GTQTYICNVN HKPSNTKVDK RVEPKSCD +/- KTHT (or KTHL) +/- CPPCPA +/-PELLGGPSVFL |
| Aducanumab | Light! SEQ ID NO: 2 | DIQMTQSPSS LSASVGDRVT ITCRASQSIS SYLNWYQQKP GKAPKLLIYA ASSLQSGVPS RFSGSGSGTD FTLTISSLQP EDFATYYCQQ SYSTPLTFGG GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| Crenezumab | Heavy/ SEQ ID NO: 3 | EVQLVESGGG LVQPGGSLRL SCAASGFTFS SYGMSWVRQA PGKGLELVAS INSNGGSTYY PDSVKGRFTI SRDNAKNSLY LQMNSLRAED TAVYYCASGD YWGQGTTVTV SSASTKGPSV FPLAPCSRST SESTAALGCL VKDYFPEPVT VSWNSGALTS GVHTFPAVLQ SSGLYSLSSV VTVPSSSLGT KTYTCNVDHK PSNTKVDKRV ESKY +/- GPPCPPCPA +/- PEFLGGPSVFL |
| Crenezumab | Light! SEQ ID NO: 4 | DIVMTQSPLS LPVTPGEPAS ISCRSSQSLV YSNGDTYLHW YLQKPGQSPQ LLIYKVSNRF SGVPDRFSGS GSGTDFTLKI SRVEAEDVGV YYCSQSTHVP WTFGQGTKVE IKRTVAAPSV FIFPPSDEQL KSGTASVVCL LNNFYPREAK VQWKVDNALQ SGNSQESVTE QDSKDSTYSL SSTLTLSKAD YEKHKVYACE VTHQGLSSPV TKSFNRGEC |
| Gantenerumab | Heavy/ SEQ ID NO: 5 | QVELVESGGG LVQPGGSLRL SCAASGFTFS SYAMSWVRQA PGKGLEWVSA INASGTRTYY ADSVKGRFTI SRDNSKNTLY LQMNSLRAED TAVYYCARGK GNTHKPYGYV RYFDVWGQGT LVTVSSASTK GPSVFPLAPS SKSTSGGTAA LGCLVKDYFP EPVTVSWNSG ALTSGVHTFP AVLQSSGLYS LSSVVTVPSS SLGTQTYICN VNHKPSNTKV DKKVEPKSCD +/- KTHT (or KTHL) +/- CPPCPA +/- PELLGGPSVFL |
| Gantenerumab | Light/ SEQ ID NO: 6 | DIVLTQSPAT LSLSPGERAT LSCRASQSVS SSYLAWYQQK PGQAPRLLIY GASSRATGVP ARFSGSGSGT DFTLTISSLE PEDFATYYCL QIYNMPITFG QGTKVEIKRT VAAPSVFIFP PSDEQLKSGT ASVVCLLNNF YPREAKVQWK VDNALQSGNS QESVTEQDSK DSTYSLSSTL TLSKADYEKH KVYACEVTHQ GLSSPVTKSF NRGEC |
| Dupilumab | Heavy/ SEQ ID NO: 7 | EVQLVESGGG LEQPGGSLRL SCAGSGFTFR DYAMTWVRQA PGKGLEWVSS ISGSGGNTYY ADSVKGRFTI SRDNSKNTLY LQMNSLRAED TAVYYCAKDR LSITIRPRYY GLDVWGQGTT VTVSSASTKG PSVFPLAPCS RSTSESTAAL GCLVKDYFPE PVTVSWNSGA LTSGVHTFPA VLQSSGLYSL SSVVTVPSSS LGTKTYTCNV DHKPSNTKVD KRVESKY +/- GPPCPPCPA +/- PEFLGGPSVFL |
| Dupilumab | Light/ SEQ ID NO: 8 | DIVMTQSPLS LPVTPGEPAS ISCRSSQSLL YSIGYNYLDW YLQKSGQSPQ LLIYLGSNRA SGVPDRFSGS GSGTDFTLKI SRVEAEDVGF YYCMQALQTP YTFGQGTKLE IKRTVAAPSV FIFPPSDEQL KSGTASVVCL LNNFYPREAK VQWKVDNALQ SGNSQESVTE QDSKDSTYSL SSTLTLSKAD YEKHKVYACE VTHQGLSSPV TKSFNRGEC |
| Ixekizumab | Heavy/ SEQ ID NO: 9 | QVQLVQSGAE VKKPGSSVKV SCKASGYSFT DYHIHWVRQA PGQGLEWMGV INPMYGTTDY NQRFKGRVTI TADESTSTAY MELSSLRSED TAVYYCARYD YFTGTGVYWG QGTLVTVSSA STKGPSVFPL APCSRSTSES TAALGCLVKD YFPEPVTVSW NSGALTSGVH TFPAVLQSSG LYSLSSVVTV PSSSLGTKTY TCNVDHKPSN TKVDKRVESK Y +/- GPPCPPCPA +/- PEFLGGPSVFL |
| Ixekizumab | Light/ SEQ ID NO: 10 | DIVMTQTPLS LSVTPGQPAS ISCRSSRSLV HSRGNTYLHW YLQKPGQSPQ LLIYKVSNRF IGVPDRFSGS GSGTDFTLKI SRVEAEDVGV YYCSQSTHLP FTFGQGTKLE IKRTVAAPSV FIFPPSDEQL KSGTASVVCL LNNFYPREAK VQWKVDNALQ SGNSQESVTE QDSKDSTYSL SSTLTLSKAD YEKHKVYACE VTHQGLSSPV TKSFNRGEC |
| Secukinumab | Heavy/ SEQ ID NO: 11 | EVQLVESGGG LVQPGGSLRL SCAASGFTFS NYWMNWVRQA PGKGLEWVAA INQDGSEKYY VGSVKGRFTI SRDNAKNSLY LQMNSLRVED TAVYYCVRDY YDILTDYYIH YWYFDLWGRG TLVTVSSAST KGPSVFPLAP SSKSTSGGTA ALGCLVKDYF PEPVTVSWNS GALTSGVHTF PAVLQSSGLY SLSSVVTVPS SSLGTQTYIC NVNHKPSNTK VDKRVEPKSC D +/- KTHT (or KTHL) +/- CPPCPA +/- PELLGGPSVFL |
| Secukinumab | Light/ SEQ ID NO: 12 | EIVLTQSPGT LSLSPGERAT LSCRASQSVS SSYLAWYQQK PGQAPRLLIY GASSRATGIP DRFSGSGSGT DFTLTISRLE PEDFAVYYCQ QYGSSPCTFG QGTRLEIKRT VAAPSVFIFP |
| | | PSDEQLKSGT ASVVCLLNNF YPREAKVQWK VDNALQSGNS QESVTEQDSK DSTYSLSSTL TLSKADYEKH KVYACEVTHQ GLSSPVTKSF NRGEC |
| Ustekinumab | Heavy/ SEQ ID NO: 13 | EVQLVQSGAE VKKPGESLKI SCKGSGYSFT TYWLGWVRQM PGKGLDWIGI MSPVDSDIRY SPSFQGQVTM SVDKSITTAY LQWNSLKASD TAMYYCARRR PGQGYFDFWG QGTLVTVSSS STKGPSVFPL APSSKSTSGG TAALGCLVKD YFPEPVTVSW NSGALTSGVH TFPAVLQSSG LYSLSSVVTV PSSSLGTQTY ICNVNHKPSN TKVDKRVEPK SCD +/- KTHT (or KTHL)+/- CPPCPA +/-PELLGGPSVFL |
| Ustekinumab | Light/ SEQ ID NO: 14 | DIQMTQSPSS LSASVGDRVT ITCRASQGIS SWLAWYQQKP EKAPKSLIYA ASSLQSGVPS RFSGSGSGTD FTLTISSLQP EDFATYYCQQ YNIYPYTFGQ GTKLEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| Mepolizumab | Heavy/ SEQ ID NO: 15 | QVTLRESGPA LVKPTQTLTL TCTVSGFSLT SYSVHWVRQP PGKGLEWLGV IWASGGTDYN SALMSRLSIS KDTSRNQVVL TMTNMDPVDT ATYYCARDPP SSLLRLDYWG RGTPVTVSSA STKGPSVFPL APSSKSTSGG TAALGCLVKD YFPEPVTVSW NSGALTSGVH TFPAVLQSSG LYSLSSVVTV PSSSLGTQTY ICNVNHKPSN TKVDKRVEPK SCD +/- KTHT (or KTHL) +/- CPPCPA +/-PELLGGPSVFL |
| Mepolizumab | Light/ SEQ ID NO: 16 | DIVMTQSPDS LAVSLGERAT INCKSSQSLL NSGNQKNYLA WXQQKPGQPP KLLIYGASTR ESGVPDRFSG SGSGTDFTLT ISSLQAEDVA VYYCQNVHSF PFTFGGGTKL EIKRTVAAPS VFIFPPSDEQ LKSGTASVVC LLNNFYPREA KVQWKVDNAL QSGNSQESVT EQDSKDSTYS LSSTLTLSKA DYEKHKVYAC EVTHQGLSSP VTKSFNRGEC |
| Vedolizumab | Heavy/ SEQ ID NO: 17 | QVQLVQSGAE VKKPGASVKV SCKGSGYTFT SYWMHWVRQA PGQRLEWIGE IDPSESNTNY NQKFKGRVTL TVDISASTAY MELSSLRSED TAVYYCARGG YDGWDYAIDY WGQGTLVTVS SASTKGPSVF PLAPSSKSTS GGTAALGCLV KDYFPEPVTV SWNSGALTSG VHTFPAVLQS SGLYSLSSVV TVPSSSLGTQ TYICNVNHKP SNTKVDKKVE PKSCD +/- KTHT (or KTHL) +/- CPPCPA +/- PELAGAPSVFL |
| Vedolizumab | Light/ SEQ ID NO: 18 | DVVMTQSPLS LPVTPGEPAS ISCRSSQSLA KSYGNTYLSW YLQKPGQSPQ LLIYGISNRF SGVPDRFSGS GSGTDFTLKI SRVEAEDVGV YYCLQGTHQP YTFGQGTKVE IKRTVAAPSV FIFPPSDEQL KSGTASVVCL LNNFYPREAK VQWKVDNALQ SGNSQESVTE QDSKDSTYSL SSTLTLSKAD YEKHKVYACE VTHQGLSSPV TKSFNRGEC |
| Natalizumab | Heavy /SEQ ID NO: 19 | QVQLVQSGAE VKKPGASVKV SCKASGFNIK DTYIHWVRQA PGQRLEWMGR IDPANGYTKY DPKFQGRVTI TADTSASTAY MELSSLRSED TAVYYCAREG YYGNYGVYAM DYWGQGTLVT VSSASTKGPS VFPLAPCSRS TSESTAALGC LVKDYFPEPV TVSWNSGALT SGVHTFPAVL QSSGLYSLSS VVTVPSSSLG TKTYTCNVDH KPSNTKVDKR VESKY +/- GPPCPPCPA +/- PEFLGGPSVFL |
| Natalizumab | Light/ SEQ ID NO: 20 | DIQMTQSPSS LSASVGDRVT ITCKTSQDIN KYMAWYQQTP GKAPRLLIHY TSALQPGIPS RFSGSGSGRD YTFTISSLQP EDIATYYCLQ YDNLWTFGQG TKVEIKRTVA APSVFIFPPS DEQLKSGTAS VVCLLNNFYP REAKVQWKVD NALQSGNSQE SVTEQDSKDS TYSLSSTLTL SKADYEKHKV YACEVTHQGL SSPVTKSFNR GEC |
| Alirocumab | Heavy/ SEQ ID NO: 21 | EVQLVESGGG LVQPGGSLRL SCAASGFTFN NYAMNWVRQA PGKGLDWVST ISGSGGTTNY ADSVKGRFII SRDSSKHTLY LQMNSLRAED TAVYYCAKDS NWGNFDLWGR GTLVTVSSAS TKGPSVFPLA PSSKSTSGGT AALGCLVKDY FPEPVTVSWN SGALTSGVHT FPAVLQSSGL YSLSSVVTVP SSSLGTQTYI CNVNHKPSNT KVDKKVEPKS CD +/- KTHT (or KTHL) +/- CPPCPA +/- PELLGGPSVFL |
| Alirocumab | Light/ SEQ ID NO: 22 | DIVMTQSPDS LAVSLGERAT INCKSSQSVL YRSNNRNFLG WYQQKPGQPP NLLIYWASTR ESGVPDRFSG SGSGTDFTLT ISSLQAEDVA VYYCQQYYTT PYTFGQGTKL EIKRTVAAPS VFIFPPSDEQ LKSGTASVVC LLNNFYPREA KVQWKVDNAL QSGNSQESVT EQDSKDSTYS LSSTLTLSKA DYEKHKVYAC EVTHQGLSSP VTKSFNRGEC |
| Evolocumab | Heavy/ SEQ ID NO: 23 | EVQLVQSGAE VKKPGASVKV SCKASGYTLT SYGISWVRQA PGQGLEWMGW VSFYNGNTNY AQKLQGRGTM TTDPSTSTAY MELRSLRSDD TAVYYCARGY GMDVWGQGTT VTVSSASTKG PSVFPLAPCS RSTSESTAAL GCLVKDYFPE PVTVSWNSGA LTSGVHTFPA VLQSSGLYSL SSVVTVPSSN FGTQTYTCNV DHKPSNTKVD KTVERKCCVE +/- CPPCPA +/- PPVAG |
| Evolocumab | Light/ SEQ ID NO: 24 | ESALTQPASV SGSPGQSITI SCTGTSSDVG GYNSVSWYQQ HPGKAPKLMI YEVSNRPSGV SNRFSGSKSG NTASLTISGL QAEDEADYYC NSYTSTSMVF GGGTKLTVLG QPKAAPSVTL FPPSSEELQA NKATLVCLIS DFYPGAVTVA WKADSSPVKA GVETTTPSKQ SNNKYAASSY LSLTPEQWKS HRSYSCQVTH EGSTVEKTVA PTECS |
| Evinacumab | Heavy/ SEQ ID NO: 25 | EVQLVESGGG VIQPGGSLRL SCAASGFTFD DYAMNWVRQG PGKGLEWVSA ISGDGGSTYY ADSVKGRFTI SRDNSKNSLY LQMNSLRAED TAFFYCAKDL RNTIFGVVIP DAFDIWGQGT MVTVSSASTK GPSVFPLAPC SRSTSESTAA LGCLVKDYFP EPVTVSWNSG ALTSGVHTFP AVLQSSGLYS LSSVVTVPSS |
| | | SLGTKTYTCN VDHKPSNTKV DKRVESKYGP P +/- CPPCPA +/- PEFLGGPSVFL |
| Evinacumab | Light/ SEQ ID NO: 26 | DIQMTQSPST LSASVGDRVT ITCRASQSIR SWLAWYQQKP GKAPKLLIYK ASSLESGVPS RFSGSGSGTE FTLTISSLQP DDFATYYCQQ YNSYSYTFGQ GTKLEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| Denosumab | Heavy/ SEQ ID NO: 27 | EVQLLESGGG LVQPGGSLRL SCAASGFTFS SYAMSWVRQA PGKGLEWVSG ITGSGGSTYY ADSVKGRFTI SRDNSKNTLY LQMNSLRAED TAVYYCAKDP GTTVIMSWFD PWGQGTLVTV SSASTKGPSV FPLAPSSKST SGGTAALGCL VKDYFPEPVT VSWNSGALTS GVHTFPAVLQ SSGLYSLSSV VTVPSSSLGT QTYICNVNHK PSNTKVDKKV EPKSCD +/- KTHT (or KTHL) +/- CPPCPA +/- PELLGGPSVFL |
| Denosumab | Light/ SEQ ID NO: 28 | EIVLTQSPGT LSLSPGERAT LSCRASQSVR GRYLAWYQQK PGQAPRLLIY GASSRATGIP DRFSGSGSGT DFTLTISRLE PEDFAVFYCQ QYGSSPRTFG QGTKVEIKRT VAAPSVFIFP PSDEQLKSGT ASVVCLLNNF YPREAKVQWK VDNALQSGNS QESVTEQDSK DSTYSLSSTL TLSKADYEKH KVYACEVTHQ GLSSPVTKSF NRGEC |
| Nivolumab | Heavy/ SEQ ID NO: 29 | QVQLVESGGG VVQPGRSLRL DCKASGITFS NSGMHWVRQA PGKGLEWVAV IWYDGSKRYY ADSVKGRFTI SRDNSKNTLF LQMNSLRAED TAVYYCATND DYWGQGTLVT VSSASTKGPS VFPLAPCSRS TSESTAALGC LVKDYFPEPV TVSWNSGALT SGVHTFPAVL QSSGLYSLSS VVTVPSSSLG TKTYTCNVDH KPSNTKVDKR VESKY +/- GPPCPPCPA +/- PEFLGGPSVFL |
| Nivolumab | Light/ SEQ ID NO: 30 | EIVLTQSPAT LSLSPGERAT LSCRASQSVS SYLAWYQQKP GQAPRLLIYD ASNRATGIPA RFSGSGSGTD FTLTISSLEP EDFAVYYCQQ SSNWPRTFGQ GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| Pembrolizumab | Heavy/ SEQ ID NO: 31 | QVQLVQSGVE VKKPGASVKV SCKASGYTFT NYYMYWVRQA PGQGLEWMGG INPSNGGTNF NEKFKNRVTL TTDSSTTTAY MELKSLQFDD TAVYYCARRD YRFDMGFDYW GQGTTVTVSS ASTKGPSVFP LAPCSRSTSE STAALGCLVK DYFPEPVTVS WNSGALTSGV HTFPAVLQSS GLYSLSSVVT VPSSSLGTKT YTCNVDHKPS NTKVDKRVES KY +/- GPPCPPCPA +/- PEFLGGPSVFL |
| Pembrolizumab | Light/ SEQ ID NO: 32 | EIVLTQSPAT LSLSPGERAT LSCRASKGVS TSGYSYLHWY QQKPGQAPRL LIYLASYLES GVPARFSGSG SGTDFTLTIS SLEPEDFAVY YCQHSRDLPL TFGGGTKVEI KRTVAAPSVF |
| | | IFPPSDEQLK SGTASVVCLL NNFYPREAKV QWKVDNALQS GNSQESVTEQ DSKDSTYSLS STLTLSKADY EKHKVYACEV THQGLSSPVT KSFNRGEC |
| Ranibizumab | Heavy/ SEQ ID NO: 33 | EVQLVESGGG LVQPGGSLRL SCAASGYDFT HYGMNWVRQA PGKGLEWVGW INTYTGEPTY AADFKRRFTF SLDTSKSTAY LQMNSLRAED TAVYYCAKYP YYYGTSHWYF DVWGQGTLVT VSSASTKGPS VFPLAPSSKS TSGGTAALGC LVKDYFPEPV TVSWNSGALT SGVHTFPAVL QSSGLYSLSS VVTVPSSSLG TQTYICNVNH KPSNTKVDKK VEPKSCD +/- KTHT (or KTHL) +/- CPPCPA +/- PELLGGPSVFL |
| Ranibizumab | Light/ SEQ ID NO: 34 | DIQLTQSPSS LSASVGDRVT ITCSASQDIS NYLNWYQQKP GKAPKVLIYF TSSLHSGVPS RFSGSGSGTD FTLTISSLQP EDFATYYCQQ YSTVPWTFGQ GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| Bevacizumab | Heavy/ SEQ ID NO: 35 | EVQLVESGGG LVQPGGSLRL SCAASGYTFT NYGMNWVRQA PGKGLEWVGW INTYTGEPTY AADFKRRFTF SLDTSKSTAY LQMNSLRAED TAVYYCAKYP HYYGSSHWYF DVWGQGTLVT VSSASTKGPS VFPLAPSSKS TSGGTAALGC LVKDYFPEPV TVSWNSGALT SGVHTFPAVL QSSGLYSLSS VVTVPSSSLG TQTYICNVNH KPSNTKVDKK VEPKSCD +/- KTHT (KTHL) +/- CPPCPA +/- PELLGGPSVFL |
| Bevacizumab | Light/ SEQ ID NO: 36 | DIQMTQSPSS LSASVGDRVT ITCSASQDIS NYLNWYQQKP GKAPKVLIYF TSSLHSGVPS RFSGSGSGTD FTLTISSLQP EDFATYYCQQ YSTVPWTFGQ GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| Lampalizumab | Heavy/ SEQ ID NO: 37 | EVQLVQSGPE LKKPGASVKV SCKASGYTFT NYGMNWVRQA PGQGLEWMGW INTYTGETTY ADDFKGRFVF SLDTSVSTAY LQISSLKAED TAVYYCEREG GVNNWGQGTL VTVSSASTKG PSVFPLAPSS KSTSGGTAAL GCLVKDYFPE PVTVSWNSGA LTSGVHTFPA VLQSSGLYSL SSVVTVPSSS LGTQTYICNV NHKPSNTKVD KKVEPKSCD +/- KTHT (or KTHL) +/- CPPCPA +/- PELLGGPSVFL |
| Lampalizumab | Light/ SEQ ID NO: 38 | DIQVTQSPSS LSASVGDRVT ITCITSTDID DDMNWYQQKP GKVPKLLISG GNTLRPGVPS RFSGSGSGTD FTLTISSLQP EDVATYYCLQ SDSLPYTFGQ GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| Brolucizumab | Heavy/ SEQ ID NO: 39 | EVQLVESGGG LVQPGGSLRL SCTASGFSLT DYYYMTWVRQ APGKGLEWVG FIDPDDDPYY ATWAKGRFTI SRDNSKNTLY LQMNSLRAED TAVYYCAGGD HNSGWGLDIW GQGTLVTVSS |
| Brolucizumab | Light/ SEQ ID NO: 40 | EIVMTQSPST LSASVGDRVI ITCQASEIIH SWLAWYQQKP GKAPKLLIYL ASTLASGVPS RFSGSGSGAE FTLTISSLQP DDFATYYCQN VYLASTNGAN FGQGTKLTVL G |
| Belimumab | Heavy/ SEQ ID NO: 41 | QVQLQQSGAE VKKPGSSVRV SCKASGGTFN NNAINWVRQA PGQGLEWMGG IIPMFGTAKY SQNFQGRVAI TADESTGTAS MELSSLRSED TAVYYCARSR DLLLFPHHAL SPWGRGTMVT VSSASTKGPS VFPLAPSSKS TSGGTAALGC LVKDYFPEPV TVSWNSGALT SGVHTFPAVL QSSGLYSLSS VVTVPSSSLG TQTYICNVNH KPSNTKVDKK VEPKSCD +/- KTHT (or KTHL) +/- CPPCPA +/-PELLGGPSVFL |
| Belimumab | Light/ SEQ ID NO: 42 | SSELTQDPAV SVALGQTVRV TCQGDSLRSY YASWYQQKPG QAPVLVIYGK NNRPSGIPDR FSGSSSGNTA SLTITGAQAE DEADYYCSSR DSSGNHWVFG GGTELTVLGQ PKAAPSVTLF PPSSEELQAN KATLVCLISD FYPGAVTVAW KADSSPVKAG VETTTPSKQS NNKYAASSYL SLTPEQWKSH RSYSCQVTHE GSTVEKTVAP TECS |
| Eculizumab | Heavy/ SEQ ID NO: 43 | QVQLVQSGAE VKKPGASVKV SCKASGYIFS NYWIQWVRQA PGQGLEWMGE ILPGSGSTEY TENFKDRVTM TRDTSTSTVY MELSSLRSED TAVYYCARYF FGSSPNWYFD VWGQGTLVTV SSASTKGPSV FPLAPCSRST SESTAALGCL VKDYFPEPVT VSWNSGALTS GVHTFPAVLQ SSGLYSLSSV VTVPSSNFGT QTYTCNVDHK PSNTKVDKTV ERKCCVE +/- CPPCPA +/- PPVAG |
| Eculizumab | Light/ SEQ ID NO: 44 | |
| Andecaliximab | Heavy/ SEQ ID NO: 45 | QVQLQESGPG LVKPSETLSL TCTVSGFSLL SYGVHWVRQP PGKGLEWLGV IWTGGTTNYN SALMSRFTIS KDDSKNTVYL KMNSLKTEDT AIYYCARYYY GMDYWGQGTL VTVSSASTKG PSVFPLAPCS RSTSESTAAL GCLVKDYFPE PVTVSWNSGA LTSGVHTFPA VLQSSGLYSL SSVVTVPSSS LGTKTYTCNV DHKPSNTKVD KRVESKY +/- GPPCPPCPA +/- PEFLGGPSVFL |
| Andecaliximab | Light/ SEQ ID NO: 46 | DIQMTQSPSS LSASVGDRVT ITCKASQDVR NTVAWYQQKP GKAPKLLIYS SSYRNTGVPD RFSGSGSGTD FTLTISSLQA EDVAVYYCQQ HYITPYTFGG GTKVEIKRTV AAPSVFIFPP SDEQLKSGTA SVVCLLNNFY PREAKVQWKV DNALQSGNSQ |
| | | ESVTEQDSKD STYSLSSTLT LSKADYEKHK VYACEVTHQG LSSPVTKSFN RGEC |
| Lanadelumab | Heavy/ SEQ ID NO: 47 | EVQLLESGGG LVQPGGSLRL SCAASGFTFS HYIMMWVRQA PGKGLEWVSG IYSSGGITVY ADSVKGRFTI SRDNSKNTLY LQMNSLRAED TAVYYCAYRR IGVPRRDEFD IWGQGTMVTV SSASTKGPSV FPLAPSSKST SGGTAALGCL VKDYFPEPVT VSWNSGALTS GVHTFPAVLQ SSGLYSLSSV VTVPSSSLGT QTYICNVNHK PSNTKVDKRV EPKSCD +/- KTHT (or KTHL) +/- CPPCPA +/- PELLGGPSVFL |
| Lanadelumab | Light/ SEQ ID NO: 48 | DIQMTQSPST LSASVGDRVT ITCRASQSIS SWLAWYQQKP GKAPKLLIYK ASTLESGVPS RFSGSGSGTE FTLTISSLQP DDFATYYCQQ YNTYWTFGQG TKVEIKRTVA APSVFIFPPS DEQLKSGTAS VVCLLNNFYP REAKVQWKVD NALQSGNSQE SVTEQDSKDS TYSLSSTLTL SKADYEKHKV YACEVTHQGL SSPVTKSFNR GEC |
| Adalimumab | Heavy/ SEQ ID NO: 49 | EVQLVESGGG LVQPGRSLRL SCAASGFTFD DYAMHWVRQA PGKGLEWVSA ITWNSGHIDY ADSVEGRFTI SRDNAKNSLY LQMNSLRAED TAVYYCAKVS YLSTASSLDY WGQGTLVTVS SASTKGPSVF PLAPSSKSTS GGTAALGCLV KDYFPEPVTV SWNSGALTSG VHTFPAVLQS SGLYSLSSVV TVPSSSLGTQ TYICNVNHKP SNTKVDKKVE PKSCD +/- KTHT (KTHL) +/- CPPCPA +/-PELLGGPSVFL |
| Adalimumab | Light/ SEQ ID NO: 50 | RFSGSGSGTD FTLTISSLQP EDVATYYCQR YNRAPYTFGQ GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| Infliximab | Heavy/ SEQ ID NO: 51 | EVKLEESGGG LVQPGGSMKL SCVASGFIFS NHWMNWVRQS PEKGLEWVAE IRSKSINSAT HYAESVKGRF TISRDDSKSA VYLQMTDLRT EDTGVYYCSR NYYGSTYDYW GQGTTLTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKKVEP KSCD +/- KTHT (KTHL) +/- CPPCPA +/-PELLGGPSVFL |
| Infliximab | Light/ SEQ ID NO: 52 | DILLTQSPAI LSVSPGERVS FSCRASQFVG SSIHWYQQRT NGSPRLLIKY ASESMSGIPS RFSGSGSGTD FTLSINTVES EDIADYYCQQ SHSWPFTFGS GTNLEVKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| aTAU | Heavy/ SEQ ID NO: 53 | EVKVVESGGG LVQPGGSMKL SCVVSGFTFS NYWVNWVRQA PGKGLEWVAQ IRLKSDNYAT HYEESVKGRF TISRDDSKSS VYLQMNNLRA EDSGIYYCTN WEDYWGQGTT VTVSSASTKG PSVFPLAPCS RSTSESTAAL GCLVKDYFPE PVTVSWNSGA LTSGVHTFPA VLQSSGLYSL SSVVTVPSSS LGTKTYTCNV |
| | | DHKPSNTKVD KRVESKY +/- GPPCPPCPA +/- PEFLGGPSVFL |
| aTAU | Light/ SEQ ID NO: 54 | DIVLTQSPDS LAVSLGERAT ISCRASQSVS TSRYSYIHWY QQKPGQPPKL LIKYASNLES GVPSRFSGSG SGTDFTLNIH PLEPEDFATY YCHHSWEIPL TFGQGTKLEI KRTVAAPSVF IFPPSDEQLK SGTASVVCLL NNFYPREAKV QWKVDNALQS GNSQESVTEQ DSKDSTYSLS STLTLSKADY EKHKVYACEV THQGLSSPVT KSFNRGEC |
| Erenumab | Heavy/ SEQ ID NO: 55 | QVQLVESGGG VVQPGRSLRL SCAASGFTFS SFGMHWVRQA PGKGLEWVAV ISFDGSIKYS VDSVKGRFTI SRDNSKNTLF LQMNSLRAED TAVYYCARDR LNYYDSSGYY HYKYYGMAVW GQGTTVTVSS ASTKGPSVFP LAPCSRSTSE STAALGCLVK DYFPEPVTVS WNSGALTSGV HTFPAVLQSS GLYSLSSVVT VPSSNFGTQT YTCNVDHKPS NTKVDKTVER KCCVE +/- CPPCPA +/-PPVAG |
| Erenumab | Light/ SEQ ID NO: 56 | QSVLTQPPSV SAAPGQKVTI SCSGSSSNIG NNYVSWYQQL PGTAPKLLIY DNNKRPSGIP DRFSGSKSGT STTLGITGLQ TGDEADYYCG TWDSRLSAVV FGGGTKLTVL GQPKANPTVT LFPPSSEELQ ANKATLVCLI SDFYPGAVTV AWKADGSPVK AGVETTKPSK QSNNKYAASS YLSLTPEQWK SHRSYSCQVT HEGSTVEKTV APTECS |
| BAN2401 | Heavy/ SEQ ID NO: 57 | EVQLVESGGG LVQPGGSLRL SCSASGFTFS SFGMHWVRQA PGKGLEWVAY ISSGSSTIYY GDTVKGRFTI SRDNAKNSLF LQMSSLRAED TAVYYCAREG GYYYGRSYYT MDYWGQGTTV TVSSASTKGP SVFPLAPSSK STSGGTAALG CLVKDYFPEP VTVSWNSGAL TSGVHTFPAV LQSSGLYSLS SVVTVPSSSL GTQTYICNVN HKPSNTKVDK KVEPKSCD +/- KTHT (KTHL) +/- CPPCPA +/- PELLGG |
| BAN2401 | Light/ SEQ ID NO: 58 | DVVMTQSPLS LPVTPGAPAS ISCRSSQSIV HSNGNTYLEW YLQKPGQSPK LLIYKVSNRF SGVPDRFSGS GSGTDFTLRI SRVEAEDVGI YYCFQGSHVP PTFGPGTKLE IKRTVAAPSV FIFPPSDEQL KSGTASVVCL LNNFYPREAK VQWKVDNALQ SGNSQESVTE QDSKDSTYSL SSTLTLSKAD YEKHKVYACE VTHQGLSSPV TKSFNRGEC |
| E06-scFV | Heavy/ SEQ ID NO: 59 | EVKLVESGGG LVQPGGSLRL SCATSGFTFS DFYMEWVRQA PGKRLEWIAA SRNKANDYTT EYADSVKGRF IVSRDTSQSI LYLQMNALRA EDTAIYYCAR DYYGSSYWYF DVWGAGTTVT VSS |
| E06-scFV | Light/ SEQ ID NO: 60 | DIVMTQSPSS LSVSAGKKVT ISCTASESLY SSKHKVHYLA WYQKKPEQSP KLLIYGASNR YIGVPDRFTG SGSGTDFTLT ISSVQVEDLT HYYCAQFYSY PLTFGAGTKL EIK |
| AAVrh10 | SEQ ID NO:80 | MAADGYLPDW LEDNLSEGIR EWWDLKPGAP KPKANQQKQD DGRGLVLPGY KYLGPFNGLD KGEPVNAADA AALEHDKAYD |
| | | QQLKAGDNPY LRYNHADAEF QERLQEDTSF GGNLGRAVFQ AKKRVLEPLG LVEEGAKTAP GKKRPVEPSP QRSPDSSTGI GKKGQQPAKK RLNFGQTGDS ESVPDPQPIG EPPAGPSGLG SGTMAAGGGA PMADNNEGAD GVGSSSGNWH CDSTWLGDRV ITTSTRTWAL PTYNNHLYKQ ISNGTSGGST NDNTYFGYST PWGYFDFNRF HCHFSPRDWQ RLINNNWGFR PKRLNFKLFN IQVKEVTQNE GTKTIANNLT STIQVFTDSE YQLPYVLGSA HQGCLPPFPA DVFMIPQYGY LTLNNGSQAV GRSSFYCLEY FPSQMLRTGN NFEFSYQFED VPFHSSYAHS QSLDRLMNPL IDQYLYYLSR TQSTGGTAGT QQLLFSQAGP NNMSAQAKNW LPGPCYRQQR VSTTLSQNNN SNFAWTGATK YHLNGRDSLV NPGVAMATHK DDEERFFPSS GVLMFGKQGA GKDNVDYSSV MLTSEEEIKT TNPVATEQYG VVADNLQQQN AAPIVGAVNS QGALPGMVWQ NRDVYLQGPI WAKIPHTDGN FHPSPLMGGF GLKHPPPQIL IKNTPVPADP PTTFSQAKLA SFITQYSTGQ VSVEIEWELQ KENSKRWNPE IQYTSNYYKS TNVDFAVNTD GTYSEPRPIG TRYLTRNL |

**TABLE 5. Table of Fab Fragment Nucleic Acid Sequences**

| mAb | Chain/ SEQ ID NO. | Sequence |
|---|---|---|
| Aducanumab | Heavy/ SEQ ID NO:101 | gtgcagctgg tggagagcgg cggcggcgtg gtgcagcccg gcagaagcct gagactgagc tgcgccgcca gcggcttcgc cttcagcagc tacggcatgc actgggtgag acaggccccc ggcaagggcc tggagtgggt ggccgtgatc tggttcgacg gcaccaagaa gtactacacc gacagcgtga agggcagatt caccatcagc agagacaaca gcaagaacac cctgtacctg cagatgaaca ccctgagagc cgaggacacc gccgtgtact actgcgccag agacagaggc atcggcgcca gaagaggccc ctactacatg gacgtgtggg gcaagggcac caccgtgacc gtgagcagcg ccagcaccaa gggccccagc gtgttccccc tggcccccag cagcaagagc accagcggcg gcaccgccgc cctgggctgc ctggtgaagg actacttccc cgagcccgtg accgtgagct ggaacagcgg cgccctgacc agcggcgtgc acaccttccc cgccgtgctg cagagcagcg gcctgtacag cctgagcagc gtggtgaccg tgcccagcag cagcctgggc acccagacct acatctgcaa cgtgaaccac aagcccagca acaccaaggt ggacaagaga gtggagccca agagctgcga c +/- aagacccacacc (or aagacccacctg) +/- tgcccccctgccccgcc +/- cccgagctgctgggcggccccagcgtgttcctg |
| Aducanumab | Light/ SEQ ID NO: 102 | gacatccaga tgacccagag ccccagcagc ctgagcgcca gcgtgggcga cagagtgacc atcacctgca gagccagcca gagcatcagc agctacctga actggtacca gcagaagccc ggcaaggccc ccaagctgct gatctacgcc gccagcagcc tgcagagcgg cgtgcccagc agattcagcg gcagcggcag cggcaccgac ttcaccctga ccatcagcag cctgcagccc gaggacttcg ccacctacta ctgccagcag agctacagca cccccctgac cttcggcggc ggcaccaagg tggagatcaa gagaaccgtg gccgccccca gcgtgttcat cttccccccc agcgacgagc agctgaagag cggcaccgcc agcgtggtgt gcctgctgaa caacttctac cccagagagg ccaaggtgca gtggaaggtg gacaacgccc tgcagagcgg caacagccag gagagcgtga ccgagcagga cagcaaggac agcacctaca gcctgagcag caccctgacc ctgagcaagg ccgactacga gaagcacaag gtgtacgcct gcgaggtgac ccaccagggc ctgagcagcc ccgtgaccaa gagcttcaac agaggcgagt gc |
| Crenezumab | Heavy/ SEQ ID NO: 103 | gaggtgcagc tggtggagag cggcggcggc ctggtgcagc ccggcggcag cctgagactg agctgcgccg ccagcggctt caccttcagc agctacggca tgagctgggt gagacaggcc cccggcaagg gcctggagct ggtggccagc atcaacagca acggcggcag cacctactac cccgacagcg tgaagggcag attcaccatc agcagagaca acgccaagaa cagcctgtac ctgcagatga acagcctgag agccgaggac accgccgtgt actactgcgc cagcggcgac tactggggcc agggcaccac cgtgaccgtg agcagcgcca gcaccaaggg ccccagcgtg ttccccctgg cccccctgcag cagaagcacc agcgagagca ccgccgccct gggctgcctg gtgaaggact acttccccga gcccgtgacc gtgagctgga acagcggcgc cctgaccagc ggcgtgcaca ccttccccgc cgtgctgcag agcagcggcc tgtacagcct gagcagcgtg gtgaccgtgc ccagcagcag cctgggcacc aagacctaca cctgcaacgt ggaccacaag cccagcaaca ccaaggtgga caagagagtg gagagcaagt ac +/- ggccccccctgcccccccctgccccgcc +/- cccgagttcctgggcggccccagcgtgttcctg |
| Crenezumab | Light/ SEQ ID NO: 104 | gacatcgtga tgacccagag ccccctgagc ctgcccgtga cccccggcga gcccgccagc atcagctgca gaagcagcca gagcctggtg tacagcaacg gcgacaccta cctgcactgg tacctgcaga agcccggcca gagcccccag ctgctgatct acaaggtgag caacagattc agcggcgtgc ccgacagatt cagcggcagc ggcagcggca ccgacttcac cctgaagatc agcagagtgg aggccgagga cgtgggcgtg tactactgca gccagagcac ccacgtgccc tggaccttcg gccagggcac caaggtggag atcaagagaa ccgtggccgc ccccagcgtg |
| | | ttcatcttcc cccccagcga cgagcagctg aagagcggca ccgccagcgt ggtgtgcctg ctgaacaact tctaccccag agaggccaag gtgcagtgga aggtggacaa cgccctgcag agcggcaaca gccaggagag cgtgaccgag caggacagca aggacagcac ctacagcctg agcagcaccc tgaccctgag caaggccgac tacgagaagc acaaggtgta cgcctgcgag gtgacccacc agggcctgag cagccccgtg accaagagct tcaacagagg cgagtgc |
| Gantenerumab | Heavy SEQ ID NO: 105 | caggtggagc tggtggagag cggcggcggc ctggtgcagc ccggcggcag cctgagactg agctgcgccg ccagcggctt caccttcagc agctacgcca tgagctgggt gagacaggcc cccggcaagg gcctggagtg ggtgagcgcc atcaacgcca gcggcaccag aacctactac gccgacagcg tgaagggcag attcaccatc agcagagaca acagcaagaa caccctgtac ctgcagatga acagcctgag agccgaggac accgccgtgt actactgcgc cagaggcaag ggcaacaccc acaagcccta cggctacgtg agatacttcg acgtgtgggg ccagggcacc ctggtgaccg tgagcagcgc cagcaccaag ggccccagcg tgttccccct ggcccccagc agcaagagca ccagcggcgg caccgccgcc ctgggctgcc tggtgaagga ctacttcccc gagcccgtga ccgtgagctg gaacagcggc gccctgacca gcggcgtgca caccttcccc gccgtgctgc agagcagcgg cctgtacagc ctgagcagcg tggtgaccgt gcccagcagc agcctgggca cccagaccta catctgcaac gtgaaccaca agcccagcaa caccaaggtg gacaagaagg tggagcccaa gagctgcgac +/- aagacccacacc (or aagacccacctg) +/- tgcccccctgccccgcc +/ ccgagctgctgggcggccccagcgtgttcctg |
| Gantenerumab | Light/ SEQ ID NO: 106 | gacatcgtgc tgacccagag ccccgccacc ctgagcctga gccccggcga gagagccacc ctgagctgca gagccagcca gagcgtgagc agcagctacc tggcctggta ccagcagaag cccggccagg cccccagact gctgatctac ggcgccagca gcagagccac cggcgtgccc gccagattca gcggcagcgg cagcggcacc gacttcaccc tgaccatcag cagcctggag cccgaggact tcgccaccta ctactgcctg cagatctaca acatgcccat caccttcggc cagggcacca aggtggagat caagagaacc gtggccgccc ccagcgtgtt catcttcccc cccagcgacg agcagctgaa gagcggcacc gccagcgtgg tgtgcctgct gaacaacttc taccccagag aggccaaggt gcagtggaag gtggacaacg ccctgcagag cggcaacagc caggagagcg tgaccgagca ggacagcaag gacagcacct acagcctgag cagcaccctg accctgagca aggccgacta cgagaagcac aaggtgtacg cctgcgaggt gacccaccag |
| | | ggcctgagca gccccgtgac caagagcttc aacagaggcg agtgc |
| Dupilumab | Heavy/ SEQ ID NO: 107 | gaggtgcagc tggtggagag cggcggcggc ctggagcagc ccggcggcag cctgagactg agctgcgccg gcagcggctt caccttcaga gactacgcca tgacctgggt gagacaggcc cccggcaagg gcctggagtg ggtgagcagc atcagcggca gcggcggcaa cacctactac gccgacagcg tgaagggcag attcaccatc agcagagaca acagcaagaa caccctgtac ctgcagatga acagcctgag agccgaggac accgccgtgt actactgcgc caaggacaga ctgagcatca ccatcagacc cagatactac ggcctggacg tgtggggcca gggcaccacc gtgaccgtga gcagcgccag caccaagggc cccagcgtgt tccccctggc cccctgcagc agaagcacca gcgagagcac cgccgccctg ggctgcctgg tgaaggacta cttccccgag cccgtgaccg tgagctggaa cagcggcgcc ctgaccagcg gcgtgcacac cttccccgcc gtgctgcaga gcagcggcct gtacagcctg agcagcgtgg tgaccgtgcc cagcagcagc ctgggcacca agacctacac ctgcaacgtg gaccacaagc ccagcaacac caaggtggac aagagagtgg agagcaagtac +/- ggccccccctgccccccctgccccgcc +/- cccgagttcctgggcggccccagcgtgttcctg |
| Dupilumab | Light/ SEQ ID NO: 108 | gacatcgtga tgacccagag ccccctgagc ctgcccgtga cccccggcga gcccgccagc atcagctgca gaagcagcca gagcctgctg tacagcatcg gctacaacta cctggactgg tacctgcaga agagcggcca gagcccccag ctgctgatct acctgggcag caacagagcc agcggcgtgc ccgacagatt cagcggcagc ggcagcggca ccgacttcac cctgaagatc agcagagtgg aggccgagga cgtgggcttc tactactgca tgcaggccct gcagaccccc tacaccttcg gccagggcac caagctggag atcaagagaa ccgtggccgc ccccagcgtg ttcatcttcc cccccagcga cgagcagctg aagagcggca ccgccagcgt ggtgtgcctg ctgaacaact tctaccccag agaggccaag gtgcagtgga aggtggacaa cgccctgcag agcggcaaca gccaggagag cgtgaccgag caggacagca aggacagcac ctacagcctg agcagcaccc tgaccctgag caaggccgac tacgagaagc acaaggtgta cgcctgcgag gtgacccacc agggcctgag cagccccgtg accaagagct tcaacagagg cgagtgc |
| Ixekizumab | Heavy/ SEQ ID NO: 109 | caggtgcagc tggtgcagag cggcgccgag gtgaagaagc ccggcagcag cgtgaaggtg agctgcaagg ccagcggcta cagcttcacc gactaccaca tccactgggt gagacaggcc cccggccagg gcctggagtg gatgggcgtg atcaacccca tgtacggcac caccgactac aaccagagat tcaagggcag |
| | | agtgaccatc accgccgacg agagcaccag caccgcctac atggagctga gcagcctgag aagcgaggac accgccgtgt actactgcgc cagatacgac tacttcaccg gcaccggcgt gtactggggc cagggcaccc tggtgaccgt gagcagcgcc agcaccaagg gccccagcgt gttccccctg gcccccctgca gcagaagcac cagcgagagc accgccgccc tgggctgcct ggtgaaggac tacttccccg agcccgtgac cgtgagctgg aacagcggcg ccctgaccag cggcgtgcac accttccccg ccgtgctgca gagcagcggc ctgtacagcc tgagcagcgt ggtgaccgtg cccagcagca gcctgggcac caagacctac acctgcaacg tggaccacaa gcccagcaac accaaggtgg acaagagagt ggagagcaag tac +/- ggccccccctgccccccctgccccgcc +/- cccgagttcctgggcggccccagcgtgttcctg |
| Ixekizumab | Light/ SEQ ID NO:110 | gacatcgtga tgacccagac ccccctgagc ctgagcgtga cccccggcca gcccgccagc atcagctgca gaagcagcag aagcctggtg cacagcagag gcaacaccta cctgcactgg tacctgcaga agcccggcca gagcccccag ctgctgatct acaaggtgag caacagattc atcggcgtgc ccgacagatt cagcggcagc ggcagcggca ccgacttcac cctgaagatc agcagagtgg aggccgagga cgtgggcgtg tactactgca gccagagcac ccacctgccc ttcaccttcg gccagggcac caagctggag atcaagagaa ccgtggccgc ccccagcgtg ttcatcttcc cccccagcga cgagcagctg aagagcggca ccgccagcgt ggtgtgcctg ctgaacaact tctaccccag agaggccaag gtgcagtgga aggtggacaa cgccctgcag agcggcaaca gccaggagag cgtgaccgag caggacagca aggacagcac ctacagcctg agcagcaccc tgaccctgag caaggccgac tacgagaagc acaaggtgta cgcctgcgag gtgacccacc agggcctgag cagccccgtg accaagagct tcaacagagg cgagtgc |
| Secukinumab | Heavy/ SEQ ID NO: 111 | gaggtgcagc tggtggagag cggcggcggc ctggtgcagc ccggcggcag cctgagactg agctgcgccg ccagcggctt caccttcagc aactactgga tgaactgggt gagacaggcc cccggcaagg gcctggagtg ggtggccgcc atcaaccagg acggcagcga gaagtactac gtgggcagcg tgaagggcag attcaccatc agcagagaca acgccaagaa cagcctgtac ctgcagatga acagcctgag agtggaggac accgccgtgt actactgcgt gagagactac tacgacatcc tgaccgacta ctacatccac tactggtact tcgacctgtg gggcagaggc accctggtga ccgtgagcag cgccagcacc aagggcccca gcgtgttccc cctggccccc agcagcaaga gcaccagcgg cggcaccgcc gccctgggct gcctggtgaa ggactacttc cccgagcccg tgaccgtgag ctggaacagc ggcgccctga |
| | | ccagcggcgt gcacaccttc cccgccgtgc tgcagagcag cggcctgtac agcctgagca gcgtggtgac cgtgcccagc agcagcctgg gcacccagac ctacatctgc aacgtgaacc acaagcccag caacaccaag gtggacaaga gagtggagcc caagagctgc gac +/- aagacccacacc (or aagacccacctg) +/- tgccccccctgccccgcc +/ ccgagctgctgggcggccccagcgtgttcctg |
| Secukinumab | Light/ SEQ ID NO: 112 | gagatcgtgc tgacccagag ccccggcacc ctgagcctga gccccggcga gagagccacc ctgagctgca gagccagcca gagcgtgagc agcagctacc tggcctggta ccagcagaag cccggccagg cccccagact gctgatctac ggcgccagca gcagagccac cggcatcccc gacagattca gcggcagcgg cagcggcacc gacttcaccc tgaccatcag cagactggag cccgaggact tcgccgtgta ctactgccag cagtacggca gcagcccctg caccttcggc cagggcacca gactggagat caagagaacc gtggccgccc ccagcgtgtt catcttcccc cccagcgacg agcagctgaa gagcggcacc gccagcgtgg tgtgcctgct gaacaacttc taccccagag aggccaaggt gcagtggaag gtggacaacg ccctgcagag cggcaacagc caggagagcg tgaccgagca ggacagcaag gacagcacct acagcctgag cagcaccctg accctgagca aggccgacta cgagaagcac aaggtgtacg cctgcgaggt gacccaccag ggcctgagca gccccgtgac caagagcttc aacagaggcg agtgc |
| Ustekinumab | Heavy/ SEQ ID NO: 113 | gaggtgcagc tggtgcagag cggcgccgag gtgaagaagc ccggcgagag cctgaagatc agctgcaagg gcagcggcta cagcttcacc acctactggc tgggctgggt gagacagatg cccggcaagg gcctggactg gatcggcatc atgagccccg tggacagcga catcagatac agccccagct tccagggcca ggtgaccatg agcgtggaca agagcatcac caccgcctac ctgcagtgga acagcctgaa ggccagcgac accgccatgt actactgcgc cagaagaaga cccggccagg gctacttcga cttctggggc cagggcaccc tggtgaccgt gagcagcagc agcaccaagg gccccagcgt gttccccctg gcccccagca gcaagagcac cagcggcggc accgccgccc tgggctgcct ggtgaaggac tacttccccg agcccgtgac cgtgagctgg aacagcggcg ccctgaccag cggcgtgcac accttccccg ccgtgctgca gagcagcggc ctgtacagcc tgagcagcgt ggtgaccgtg cccagcagca gcctgggcac ccagacctac atctgcaacg tgaaccacaa gcccagcaac accaaggtgg acaagagagt ggagcccaag agctgcgac +/- aagacccaca cc (or aagacccacctg)+/- tgcccccccctgccccgcc +/- cccgagctgctgggcggccccagcgtgttcctg |
| Ustekinumab | Light/ SEQ ID NO: 114 | gacatccaga tgacccagag ccccagcagc ctgagcgcca gcgtgggcga cagagtgacc atcacctgca gagccagcca gggcatcagc agctggctgg cctggtacca gcagaagccc gagaaggccc ccaagagcct gatctacgcc gccagcagcc tgcagagcgg cgtgcccagc agattcagcg gcagcggcag cggcaccgac ttcaccctga ccatcagcag cctgcagccc gaggacttcg ccacctacta ctgccagcag tacaacatct acccctacac cttcggccag ggcaccaagc tggagatcaa gagaaccgtg gccgccccca gcgtgttcat cttccccccc agcgacgagc agctgaagag cggcaccgcc agcgtggtgt gcctgctgaa caacttctac cccagagagg ccaaggtgca gtggaaggtg gacaacgccc tgcagagcgg caacagccag gagagcgtga ccgagcagga cagcaaggac agcacctaca gcctgagcag caccctgacc ctgagcaagg ccgactacga gaagcacaag gtgtacgcct gcgaggtgac ccaccagggc ctgagcagcc ccgtgaccaa gagcttcaac agaggcgagt gc |
| Mepolizumab | Heavy/ SEQ ID NO: 115 | caggtgaccc tgagagagag cggccccgcc ctggtgaagc ccacccagac cctgaccctg acctgcaccg tgagcggctt cagcctgacc agctacagcg tgcactgggt gagacagccc cccggcaagg gcctggagtg gctgggcgtg atctggccca gcggcggcac cgactacaac agcgccctga tgagcagact gagcatcagc aaggacacca gcagaaacca ggtggtgctg accatgacca acatggaccc cgtggacacc gccacctact actgcgccag agaccccccc agcagcctgc tgagactgga ctactggggc agaggcaccc ccgtgaccgt gagcagcgcc agcaccaagg gccccagcgt gttccccctg gcccccagca gcaagagcac cagcggcggc accgccgccc tgggctgcct ggtgaaggac tacttccccg agcccgtgac cgtgagctgg aacagcggcg ccctgaccag cggcgtgcac accttccccg ccgtgctgca gagcagcggc ctgtacagcc tgagcagcgt ggtgaccgtg cccagcagca gcctgggcac ccagacctac atctgcaacg tgaaccacaa gcccagcaac accaaggtgg acaagagagt ggagcccaag agctgcgac +/- aagacccacacc (or aagacccacctg) +/- tgccccccctgccccgcc +/- cccgagctgctgggcggccccagcgtgttcctg |
| Mepolizumab | Light/ SEQ ID NO: 116 | gacatcgtga tgacccagag ccccgacagc ctggccgtga gcctgggcga gagagccacc atcaactgca agagcagcca gagcctgctg aacagcggca accagaagaa ctacctggcc tggcagcaga agcccggcca gccccccaag ctgctgatct acggcgccag caccagagag agcggcgtgc ccgacagatt cagcggcagc ggcagcggca ccgacttcac cctgaccatc agcagcctgc aggccgagga cgtggccgtg tactactgcc |
| | | agaacgtgca cagcttcccc ttcaccttcg gcggcggcac caagctggag atcaagagaa ccgtggccgc ccccagcgtg ttcatcttcc cccccagcga cgagcagctg aagagcggca ccgccagcgt ggtgtgcctg ctgaacaact tctaccccag agaggccaag gtgcagtgga aggtggacaa cgccctgcag agcggcaaca gccaggagag cgtgaccgag caggacagca aggacagcac ctacagcctg agcagcaccc tgaccctgag caaggccgac tacgagaagc acaaggtgta cgcctgcgag gtgacccacc agggcctgag cagccccgtg accaagagct tcaacagagg cgagtgc |
| Vedolizumab | Heavy/ SEQ ID NO: 117 | caggtgcagc tggtgcagag cggcgccgag gtgaagaagc ccggcgccag cgtgaaggtg agctgcaagg gcagcggcta caccttcacc agctactgga tgcactgggt gagacaggcc cccggccaga gactggagtg gatcggcgag atcgacccca gcgagagcaa caccaactac aaccagaagt tcaagggcag agtgaccctg accgtggaca tcagcgccag caccgcctac atggagctga gcagcctgag aagcgaggac accgccgtgt actactgcgc cagaggcggc tacgacggct gggactacgc catcgactac tggggccagg gcaccctggt gaccgtgagc agcgccagca ccaagggccc cagcgtgttc cccctggccc ccagcagcaa gagcaccagc ggcggcaccg ccgccctggg ctgcctggtg aaggactact tccccgagcc cgtgaccgtg agctggaaca gcggcgccct gaccagcggc gtgcacacct tccccgccgt gctgcagagc agcggcctgt acagcctgag cagcgtggtg accgtgccca gcagcagcct gggcacccag acctacatct gcaacgtgaa ccacaagccc agcaacacca aggtggacaa gaaggtggag cccaagagct gcgac +/- aagacccacacc (or aagacccacctg) +/- tgcccccctgccccgcc +/- cccgagctggccggcgcccccagcgtgttcctg |
| Vedolizumab | Light/ SEQ ID NO: 118 | gacgtggtga tgacccagag ccccctgagc ctgcccgtga cccccggcga gcccgccagc atcagctgca gaagcagcca gagcctggcc aagagctacg gcaacaccta cctgagctgg tacctgcaga agcccggcca gagcccccag ctgctgatct acggcatcag caacagattc agcggcgtgc ccgacagatt cagcggcagc ggcagcggca ccgacttcac cctgaagatc agcagagtgg aggccgagga cgtgggcgtg tactactgcc tgcagggcac ccaccagccc tacaccttcg gccagggcac caaggtggag atcaagagaa ccgtggccgc ccccagcgtg ttcatcttcc cccccagcga cgagcagctg aagagcggca ccgccagcgt ggtgtgcctg ctgaacaact tctaccccag agaggccaag gtgcagtgga aggtggacaa cgccctgcag agcggcaaca gccaggagag cgtgaccgag caggacagca aggacagcac ctacagcctg agcagcaccc tgaccctgag |
| | | caaggccgac tacgagaagc acaaggtgta cgcctgcgag gtgacccacc agggcctgag cagccccgtg accaagagct tcaacagagg cgagtgc |
| Natalizumab | Heavy/ SEQ ID NO: 119 | caggtgcagc tggtgcagag cggcgccgag gtgaagaagc ccggcgccag cgtgaaggtg agctgcaagg ccagcggctt caacatcaag gacacctaca tccactgggt gagacaggcc cccggccaga gactggagtg gatgggcaga atcgaccccg ccaacggcta caccaagtac gaccccaagt tccagggcag agtgaccatc accgccgaca ccagcgccag caccgcctac atggagctga gcagcctgag aagcgaggac accgccgtgt actactgcgc cagagagggc tactacggca actacggcgt gtacgccatg gactactggg gccagggcac cctggtgacc gtgagcagcg ccagcaccaa gggccccagc gtgttccccc tggccccctg cagcagaagc accagcgaga gcaccgccgc cctgggctgc ctggtgaagg actacttccc cgagcccgtg accgtgagct ggaacagcgg cgccctgacc agcggcgtgc acaccttccc cgccgtgctg cagagcagcg gcctgtacag cctgagcagc gtggtgaccg tgcccagcag cagcctgggc accaagacct acacctgcaa cgtggaccac aagcccagca acaccaaggt ggacaagaga gtggagagca agtac +/- ggccccccctgccccccctgccccgcc +/- cccgagttcctgggcggccccagcgtgttcctg |
| Natalizumab | Light\ SEQ ID NO: 120 | gacatccaga tgacccagag ccccagcagc ctgagcgcca gcgtgggcga cagagtgacc atcacctgca agaccagcca ggacatcaac aagtacatgg cctggtacca gcagaccccc ggcaaggccc ccagactgct gatccactac accagcgccc tgcagcccgg catccccagc agattcagcg gcagcggcag cggcagagac tacaccttca ccatcagcag cctgcagccc gaggacatcg ccacctacta ctgcctgcag tacgacaacc tgtggacctt cggccagggc accaaggtgg agatcaagag aaccgtggcc gcccccagcg tgttcatctt cccccccagc gacgagcagc tgaagagcgg caccgccagc gtggtgtgcc tgctgaacaa cttctacccc agagaggcca aggtgcagtg gaaggtggac aacgccctgc agagcggcaa cagccaggag agcgtgaccg agcaggacag caaggacagc acctacagcc tgagcagcac cctgaccctg agcaaggccg actacgagaa gcacaaggtg tacgcctgcg aggtgaccca ccagggcctg agcagccccg tgaccaagag cttcaacaga ggcgagtgc |
| Alirocumab | Heavy/ SEQ ID NO: 121 | gaggtgcagc tggtggagag cggcggcggc ctggtgcagc ccggcggcag cctgagactg agctgcgccg ccagcggctt caccttcaac aactacgcca tgaactgggt gagacaggcc cccggcaagg gcctggactg ggtgagcacc atcagcggca gcggcggcac caccaactac gccgacagcg tgaagggcag attcatcatc agcagagaca gcagcaagca caccctgtac |
| | | ctgcagatga acagcctgag agccgaggac accgccgtgt actactgcgc caaggacagc aactggggca acttcgacct gtggggcaga ggcaccctgg tgaccgtgag cagcgccagc accaagggcc ccagcgtgtt ccccctggcc cccagcagca agagcaccag cggcggcacc gccgccctgg gctgcctggt gaaggactac ttccccgagc ccgtgaccgt gagctggaac agcggcgccc tgaccagcgg cgtgcacacc ttccccgccg tgctgcagag cagcggcctg tacagcctga gcagcgtggt gaccgtgccc agcagcagcc tgggcaccca gacctacatc tgcaacgtga accacaagcc cagcaacacc aaggtggaca agaaggtgga gcccaagagc tgcgac +/- aagacccacacc (or aagacccacctg) +/- tgccccccctgccccgcc +/- cccgagctgctgggcggccccagcgtgttcctg |
| Alirocumab | Light/ SEQ ID NO: 122 | gacatcgtga tgacccagag ccccgacagc ctggccgtga gcctgggcga gagagccacc atcaactgca agagcagcca gagcgtgctg tacagaagca acaacagaaa cttcctgggc tggtaccagc agaagcccgg ccagcccccc aacctgctga tctactgggc cagcaccaga gagagcggcg tgcccgacag attcagcggc agcggcagcg gcaccgactt caccctgacc atcagcagcc tgcaggccga ggacgtggcc gtgtactact gccagcagta ctacaccacc ccctacacct tcggccaggg caccaagctg gagatcaaga gaaccgtggc cgcccccagc gtgttcatct tcccccccag cgacgagcag ctgaagagcg gcaccgccag cgtggtgtgc ctgctgaaca acttctaccc cagagaggcc aaggtgcagt ggaaggtgga caacgccctg cagagcggca acagccagga gagcgtgacc gagcaggaca gcaaggacag cacctacagc ctgagcagca ccctgaccct gagcaaggcc gactacgaga agcacaaggt gtacgcctgc gaggtgaccc accagggcct gagcagcccc gtgaccaaga gcttcaacag aggcgagtgc |
| Evolocumab | Heavy/ SEQ ID NO: 123 | gaggtgcagc tggtgcagag cggcgccgag gtgaagaagc ccggcgccag cgtgaaggtg agctgcaagg ccagcggcta caccctgacc agctacggca tcagctgggt gagacaggcc cccggccagg gcctggagtg gatgggctgg gtgagcttct acaacggcaa caccaactac gcccagaagc tgcagggcag aggcaccatg accaccgacc ccagcaccag caccgcctac atggagctga gaagcctgag aagcgacgac accgccgtgt actactgcgc cagaggctac ggcatggacg tgtgggccca gggcaccacc gtgaccgtga gcagcgccag caccaagggc cccagcgtgt tccccctggc cccctgcagc agaagcacca gcgagagcac cgccgccctg ggctgcctgg tgaaggacta cttccccgag cccgtgaccg tgagctggaa cagcggcgcc ctgaccagcg gcgtgcacac cttccccgcc gtgctgcaga |
| | | gcagcggcct gtacagcctg agcagcgtgg tgaccgtgcc cagcagcaac ttcggcaccc agacctacac ctgcaacgtg gaccacaagc ccagcaacac caaggtggac aagaccgtgg agagaaagtg ctgcgtggag +/- tgccccccctgccccgcc +/- ccccccgtggccggc |
| Evolocumab | Light/ SEQ ID NO: 124 | gagagcgccc tgacccagcc cgccagcgtg agcggcagcc ccggccagag catcaccatc agctgcaccg gcaccagcag cgacgtgggc ggctacaaca gcgtgagctg gtaccagcag caccccggca aggcccccaa gctgatgatc tacgaggtga gcaacagacc cagcggcgtg agcaacagat tcagcggcag caagagcggc aacaccgcca gcctgaccat cagcggcctg caggccgagg acgaggccga ctactactgc aacagctaca ccagcaccag catggtgttc ggcggcggca ccaagctgac cgtgctgggc cagcccaagg ccgcccccag cgtgaccctg ttccccccca gcagcgagga gctgcaggcc aacaaggcca ccctggtgtg cctgatcagc gacttctacc ccggcgccgt gaccgtggcc tggaaggccg acagcagccc cgtgaaggcc ggcgtggaga ccaccacccc cagcaagcag agcaacaaca agtacgccgc cagcagctac ctgagcctga cccccgagca gtggaagagc cacagaagct acagctgcca ggtgacccac gagggcagca ccgtggagaa gaccgtggcc cccaccgagt gcagc |
| Evinacumab | Heavy/ SEQ ID NO: 125 | gaggtgcagc tggtggagag cggcggcggc gtgatccagc ccggcggcag cctgagactg agctgcgccg ccagcggctt caccttcgac gactacgcca tgaactgggt gagacagggc cccggcaagg gcctggagtg ggtgagcgcc atcagcggcg acggcggcag cacctactac gccgacagcg tgaagggcag attcaccatc agcagagaca acagcaagaa cagcctgtac ctgcagatga acagcctgag agccgaggac accgccttct tctactgcgc caaggacctg agaaacacca tcttcggcgt ggtgatcccc gacgccttcg acatctgggg ccagggcacc atggtgaccg tgagcagcgc cagcaccaag ggccccagcg tgttccccct ggccccctgc agcagaagca ccagcgagag caccgccgcc ctgggctgcc tggtgaagga ctacttcccc gagcccgtga ccgtgagctg gaacagcggc gccctgacca gcggcgtgca caccttcccc gccgtgctgc agagcagcgg cctgtacagc ctgagcagcg tggtgaccgt gcccagcagc agcctgggca ccaagaccta cacctgcaac gtggaccaca agcccagcaa caccaaggtg gacaagagag tggagagcaa gtacggcccc ccc +/- tgccccccctgccccgcc +/- cccgagttcctgggcggccccagcgtgttcctg |
| Evinacumab | Light/ SEQ ID NO: 126 | gacatccaga tgacccagag ccccagcacc ctgagcgcca gcgtgggcga cagagtgacc atcacctgca gagccagcca gagcatcaga agctggctgg cctggtacca gcagaagccc |
| | | ggcaaggccc ccaagctgct gatctacaag gccagcagcc tggagagcgg cgtgcccagc agattcagcg gcagcggcag cggcaccgag ttcaccctga ccatcagcag cctgcagccc gacgacttcg ccacctacta ctgccagcag tacaacagct acagctacac cttcggccag ggcaccaagc tggagatcaa gagaaccgtg gccgccccca gcgtgttcat cttccccccc agcgacgagc agctgaagag cggcaccgcc agcgtggtgt gcctgctgaa caacttctac cccagagagg ccaaggtgca gtggaaggtg gacaacgccc tgcagagcgg caacagccag gagagcgtga ccgagcagga cagcaaggac agcacctaca gcctgagcag caccctgacc ctgagcaagg ccgactacga gaagcacaag gtgtacgcct gcgaggtgac ccaccagggc ctgagcagcc ccgtgaccaa gagcttcaac agaggcgagt gc |
| Denosumab | Heavy/ SEQ ID NO: 127 | gaggtgcagc tgctggagag cggcggcggc ctggtgcagc ccggcggcag cctgagactg agctgcgccg ccagcggctt caccttcagc agctacgcca tgagctgggt gagacaggcc cccggcaagg gcctggagtg ggtgagcggc atcaccggca gcggcggcag cacctactac gccgacagcg tgaagggcag attcaccatc agcagagaca acagcaagaa caccctgtac ctgcagatga acagcctgag agccgaggac accgccgtgt actactgcgc caaggacccc ggcaccaccg tgatcatgag ctggttcgac ccctggggcc agggcaccct ggtgaccgtg agcagcgcca gcaccaaggg ccccagcgtg ttccccctgg cccccagcag caagagcacc agcggcggca ccgccgccct gggctgcctg gtgaaggact acttccccga gcccgtgacc gtgagctgga acagcggcgc cctgaccagc ggcgtgcaca ccttccccgc cgtgctgcag agcagcggcc tgtacagcct gagcagcgtg gtgaccgtgc ccagcagcag cctgggcacc cagacctaca tctgcaacgt gaaccacaag cccagcaaca ccaaggtgga caagaaggtg gagcccaaga gctgcgac +/- aagacccacacc (aagacccacctg) +/- tgcccccccctgccccgcc +/- cccgagctgctgggcggccccagcgtgttcctg |
| Denosumab | Light/ SEQ ID NO: 128 | gagatcgtgc tgacccagag ccccggcacc ctgagcctga gccccggcga gagagccacc ctgagctgca gagccagcca gagcgtgaga ggcagatacc tggcctggta ccagcagaag cccggccagg cccccagact gctgatctac ggcgccagca gcagagccac cggcatcccc gacagattca gcggcagcgg cagcggcacc gacttcaccc tgaccatcag cagactggag cccgaggact tcgccgtgtt ctactgccag cagtacggca gcagccccag aaccttcggc cagggcacca aggtggagat caagagaacc gtggccgccc ccagcgtgtt catcttcccc cccagcgacg agcagctgaa gagcggcacc gccagcgtgg |
| | | tgtgcctgct gaacaacttc taccccagag aggccaaggt gcagtggaag gtggacaacg ccctgcagag cggcaacagc caggagagcg tgaccgagca ggacagcaag gacagcacct acagcctgag cagcaccctg accctgagca aggccgacta cgagaagcac aaggtgtacg cctgcgaggt gacccaccag ggcctgagca gccccgtgac caagagcttc aacagaggcg agtgc |
| Nivolumab | Heavy/ SEQ ID NO: 129 | caggtgcagc tggtggagag cggcggcggc gtggtgcagc ccggcagaag cctgagactg gactgcaagg ccagcggcat caccttcagc aacagcggca tgcactgggt gagacaggcc cccggcaagg gcctggagtg ggtggccgtg atctggtacg acggcagcaa gagatactac gccgacagcg tgaagggcag attcaccatc agcagagaca acagcaagaa caccctgttc ctgcagatga acagcctgag agccgaggac accgccgtgt actactgcgc caccaacgac gactactggg gccagggcac cctggtgacc gtgagcagcg ccagcaccaa gggccccagc gtgttccccc tggccccctg cagcagaagc accagcgaga gcaccgccgc cctgggctgc ctggtgaagg actacttccc cgagcccgtg accgtgagct ggaacagcgg cgccctgacc agcggcgtgc acaccttccc cgccgtgctg cagagcagcg gcctgtacag cctgagcagc gtggtgaccg tgcccagcag cagcctgggc accaagacct acacctgcaa cgtggaccac aagcccagca acaccaaggt ggacaagaga gtggagagca agtac +/- ggccccccctgccccccctgccccgcc +/- cccgagttcctgggcggccccagcgtgttcctg |
| Nivolumab | Light/ SEQ ID NO: 130 | gagatcgtgc tgacccagag ccccgccacc ctgagcctga gccccggcga gagagccacc ctgagctgca gagccagcca gagcgtgagc agctacctgg cctggtacca gcagaagccc ggccaggccc ccagactgct gatctacgac gccagcaaca gagccaccgg catccccgcc agattcagcg gcagcggcag cggcaccgac ttcaccctga ccatcagcag cctggagccc gaggacttcg ccgtgtacta ctgccagcag agcagcaact ggcccagaac cttcggccag ggcaccaagg tggagatcaa gagaaccgtg gccgccccca gcgtgttcat cttccccccc agcgacgagc agctgaagag cggcaccgcc agcgtggtgt gcctgctgaa caacttctac cccagagagg ccaaggtgca gtggaaggtg gacaacgccc tgcagagcgg caacagccag gagagcgtga ccgagcagga cagcaaggac agcacctaca gcctgagcag caccctgacc ctgagcaagg ccgactacga gaagcacaag gtgtacgcct gcgaggtgac ccaccagggc ctgagcagcc ccgtgaccaa gagcttcaac agaggcgagt gc |
| Pembrolizumab | Heavy /SEQ ID NO: 131 | caggtgcagc tggtgcagag cggcgtggag gtgaagaagc ccggcgccag cgtgaaggtg agctgcaagg ccagcggcta caccttcacc aactactaca tgtactgggt gagacaggcc cccggccagg gcctggagtg gatgggcggc atcaacccca gcaacggcgg caccaacttc aacgagaagt tcaagaacag agtgaccctg accaccgaca gcagcaccac caccgcctac atggagctga agagcctgca gttcgacgac accgccgtgt actactgcgc cagaagagac tacagattcg acatgggctt cgactactgg ggccagggca ccaccgtgac cgtgagcagc gccagcacca agggccccag cgtgttcccc ctggccccct gcagcagaag caccagcgag agcaccgccg ccctgggctg cctggtgaag gactacttcc ccgagcccgt gaccgtgagc tggaacagcg gcgccctgac cagcggcgtg cacaccttcc ccgccgtgct gcagagcagc ggcctgtaca gcctgagcag cgtggtgacc gtgcccagca gcagcctggg caccaagacc tacacctgca acgtggacca caagcccagc aacaccaagg tggacaagag agtggagagc aagtac +/- ggccccccctgccccccctgccccgcc +/- cccgagttcctgggcggccccagcgtgttcctg |
| Pembrolizumab | Light/ SEQ ID NO: 132 | gagatcgtgc tgacccagag ccccgccacc ctgagcctga gccccggcga gagagccacc ctgagctgca gagccagcaa gggcgtgagc accagcggct acagctacct gcactggtac cagcagaagc ccggccaggc ccccagactg ctgatctacc tggccagcta cctggagagc ggcgtgcccg ccagattcag cggcagcggc agcggcaccg acttcaccct gaccatcagc agcctggagc ccgaggactt cgccgtgtac tactgccagc acagcagaga cctgcccctg accttcggcg gcggcaccaa ggtggagatc aagagaaccg tggccgcccc cagcgtgttc atcttccccc ccagcgacga gcagctgaag agcggcaccg ccagcgtggt gtgcctgctg aacaacttct accccagaga ggccaaggtg cagtggaagg tggacaacgc cctgcagagc ggcaacagcc aggagagcgt gaccgagcag gacagcaagg acagcaccta cagcctgagc agcaccctga ccctgagcaa ggccgactac gagaagcaca aggtgtacgc ctgcgaggtg acccaccagg gcctgagcag ccccgtgacc aagagcttca acagaggcga gtgc |
| Ranibizumab | Heavy /SEQ ID NO: 133 | gaggtgcagc tggtggagag cggcggcggc ctggtgcagc ccggcggcag cctgagactg agctgcgccg ccagcggcta cgacttcacc cactacggca tgaactgggt gagacaggcc cccggcaagg gcctggagtg ggtgggctgg atcaacacct acaccggcga gcccacctac gccgccgact tcaagagaag attcaccttc agcctggaca ccagcaagag caccgcctac ctgcagatga acagcctgag agccgaggac accgccgtgt actactgcgc caagtacccc tactactacg gcaccagcca |
| | | ctggtacttc gacgtgtggg gccagggcac cctggtgacc gtgagcagcg ccagcaccaa gggccccagc gtgttccccc tggcccccag cagcaagagc accagcggcg gcaccgccgc cctgggctgc ctggtgaagg actacttccc cgagcccgtg accgtgagct ggaacagcgg cgccctgacc agcggcgtgc acaccttccc cgccgtgctg cagagcagcg gcctgtacag cctgagcagc gtggtgaccg tgcccagcag cagcctgggc acccagacct acatctgcaa cgtgaaccac aagcccagca acaccaaggt ggacaagaaggtggagcccaagagctgcgac +/- aagacccacacc (or aagacccacctg) +/- tgcccccctgccccgcc +/- cccgagctgctgggcggccccagcgtgttcctg |
| Ranibizumab | Light/ SEQ ID NO: 134 | gacatccagc tgacccagag ccccagcagc ctgagcgcca gcgtgggcga cagagtgacc atcacctgca gcgccagcca ggacatcagc aactacctga actggtacca gcagaagccc ggcaaggccc ccaaggtgct gatctacttc accagcagcc tgcacagcgg cgtgcccagc agattcagcg gcagcggcag cggcaccgac ttcaccctga ccatcagcag cctgcagccc gaggacttcg ccacctacta ctgccagcag tacagcaccg tgccctggac cttcggccag ggcaccaagg tggagatcaa gagaaccgtg gccgccccca gcgtgttcat cttccccccc agcgacgagc agctgaagag cggcaccgcc agcgtggtgt gcctgctgaa caacttctac cccagagagg ccaaggtgca gtggaaggtg gacaacgccc tgcagagcgg caacagccag gagagcgtga ccgagcagga cagcaaggac agcacctaca gcctgagcag caccctgacc ctgagcaagg ccgactacga gaagcacaag gtgtacgcct gcgaggtgac ccaccagggc ctgagcagcc ccgtgaccaa gagcttcaac agaggcgagt gc |
| Bevacizumab | Heavy/ SEQ ID NO: 135 | gaggtgcagc tggtggagag cggcggcggc ctggtgcagc ccggcggcag cctgagactg agctgcgccg ccagcggcta caccttcacc aactacggca tgaactgggt gagacaggcc cccggcaagg gcctggagtg ggtgggctgg atcaacacct acaccggcga gcccacctac gccgccgact tcaagagaag attcaccttc agcctggaca ccagcaagag caccgcctac ctgcagatga acagcctgag agccgaggac accgccgtgt actactgcgc caagtacccc cactactacg gcagcagcca ctggtacttc gacgtgtggg gccagggcac cctggtgacc gtgagcagcg ccagcaccaa gggccccagc gtgttccccc tggcccccag cagcaagagc accagcggcg gcaccgccgc cctgggctgc ctggtgaagg actacttccc cgagcccgtg accgtgagct ggaacagcgg cgccctgacc agcggcgtgc acaccttccc cgccgtgctg cagagcagcg gcctgtacag cctgagcagc gtggtgaccg tgcccagcag cagcctgggc |
| | | acccagacct acatctgcaa cgtgaaccac aagcccagca acaccaaggt ggacaagaag gtggagccca agagctgcga c +/- aagacccacacc (aagacccacctg) +/- tgcccccctgccccgcc +/- cccgagctgctgggcggccccagcgtgttcctg |
| Bevacizumab | Light/ SEQ ID NO: 136 | gacatccaga tgacccagag ccccagcagc ctgagcgcca gcgtgggcga cagagtgacc atcacctgca gcgccagcca ggacatcagc aactacctga actggtacca gcagaagccc ggcaaggccc ccaaggtgct gatctacttc accagcagcc tgcacagcgg cgtgcccagc agattcagcg gcagcggcag cggcaccgac ttcaccctga ccatcagcag cctgcagccc gaggacttcg ccacctacta ctgccagcag tacagcaccg tgccctggac cttcggccag ggcaccaagg tggagatcaa gagaaccgtg gccgccccca gcgtgttcat cttccccccc agcgacgagc agctgaagag cggcaccgcc agcgtggtgt gcctgctgaa caacttctac cccagagagg ccaaggtgca gtggaaggtg gacaacgccc tgcagagcgg caacagccag gagagcgtga ccgagcagga cagcaaggac agcacctaca gcctgagcag caccctgacc ctgagcaagg ccgactacga gaagcacaag gtgtacgcct gcgaggtgac ccaccagggc ctgagcagcc ccgtgaccaa gagcttcaac agaggcgagt gc |
| Lampalizumab | Heavy/ SEQ ID NO: 137 | gaggtgcagc tggtgcagag cggccccgag ctgaagaagc ccggcgccag cgtgaaggtg agctgcaagg ccagcggcta caccttcacc aactacggca tgaactgggt gagacaggcc cccggccagg gcctggagtg gatgggctgg atcaacacct acaccggcga gaccacctac gccgacgact tcaagggcag attcgtgttc agcctggaca ccagcgtgag caccgcctac ctgcagatca gcagcctgaa ggccgaggac accgccgtgt actactgcga gagagagggc ggcgtgaaca actgggccca gggcaccctg gtgaccgtga gcagcgccag caccaagggc cccagcgtgt tccccctggc ccccagcagc aagagcacca gcggcggcac cgccgccctg ggctgcctgg tgaaggacta cttccccgag cccgtgaccg tgagctggaa cagcggcgcc ctgaccagcg gcgtgcacac cttccccgcc gtgctgcaga gcagcggcct gtacagcctg agcagcgtgg tgaccgtgcc cagcagcagc ctgggcaccc agacctacat ctgcaacgtg aaccacaagc ccagcaacac caaggtggac aagaaggtgg agcccaagag ctgcgac +/- aagacccacacc (or aagacccacctg) +/- tgccccccctgccccgcc +/- cccgagctgctgggcggccccagcgtgttcctg |
| Lampalizumab | Light/ SEQ ID NO: 138 | gacatccagg tgacccagag ccccagcagc ctgagcgcca gcgtgggcga cagagtgacc atcacctgca tcaccagcac cgacatcgac gacgacatga actggtacca gcagaagccc |
| | | ggcaaggtgc ccaagctgct gatcagcggc ggcaacaccc tgagacccgg cgtgcccagc agattcagcg gcagcggcag cggcaccgac ttcaccctga ccatcagcag cctgcagccc gaggacgtgg ccacctacta ctgcctgcag agcgacagcc tgccctacac cttcggccag ggcaccaagg tggagatcaa gagaaccgtg gccgccccca gcgtgttcat cttccccccc agcgacgagc agctgaagag cggcaccgcc agcgtggtgt gcctgctgaa caacttctac cccagagagg ccaaggtgca gtggaaggtg gacaacgccc tgcagagcgg caacagccag gagagcgtga ccgagcagga cagcaaggac agcacctaca gcctgagcag caccctgacc ctgagcaagg ccgactacga gaagcacaag gtgtacgcct gcgaggtgac ccaccagggc ctgagcagcc ccgtgaccaa gagcttcaac agaggcgagt gc |
| Brolucizumab | Light/ SEQ ID NO: 139 | gaggtgcagc tggtggagag cggcggcggc ctggtgcagc ccggcggcag cctgagactg agctgcaccg ccagcggctt cagcctgacc gactactact acatgacctg ggtgagacag gcccccggca agggcctgga gtgggtgggc ttcatcgacc ccgacgacga cccctactac gccacctggg ccaagggcag attcaccatc agcagagaca acagcaagaa caccctgtac ctgcagatga acagcctgag agccgaggac accgccgtgt actactgcgc cggcggcgac cacaacagcg gctggggcct ggacatctgg ggccagggca ccctggtgac cgtgagcagc |
| Brolucizumab | Heavy/ SEQ ID NO: 140 | gagatcgtga tgacccagag ccccagcacc ctgagcgcca gcgtgggcga cagagtgatc atcacctgcc aggccagcga gatcatccac agctggctgg cctggtacca gcagaagccc ggcaaggccc ccaagctgct gatctacctg gccagcaccc tggccagcgg cgtgcccagc agattcagcg gcagcggcag cggcgccgag ttcaccctga ccatcagcag cctgcagccc gacgacttcg ccacctacta ctgccagaac gtgtacctgg ccagcaccaa cggcgccaac ttcggccagg gcaccaagct gaccgtgctg ggc |
| Belimumab | Heavy/ SEQ ID NO: 141 | caggtgcagc tgcagcagag cggcgcggaa gtgaaaaaac cgggcagcag cgtgcgcgtg agctgcaaag cgagcggcgg cacctttaac aacaacgcga ttaactgggt gcgccaggcg ccgggccagg gcctggaatg gatgggcggc attattccga tgtttggcac cgcgaaatat agccagaact ttcagggccg cgtggcgatt accgcggatg aaagcaccgg caccgcgagc atggaactga gcagcctgcg cagcgaagat accgcggtgt attattgcgc gcgcagccgc gatctgctgc tgtttccgca tcatgcgctg agcccgtggg gccgcggcac catggtgacc gtgagcagcg cgagcaccaa aggcccgagc gtgtttccgc tggcgccgag cagcaaaagc accagcggcg gcaccgcggc gctgggctgc ctggtgaaag attattttcc ggaaccggtg |
| | | accgtgagca acagcggcgc gctgaccagc ggcgtgcata cctttccggc ggtgctgcag agcagcggcc tgtatagcct gagcagcgtg gtgaccgtgc cgagcagcag cctgggcacc cagacctata tttgcaacgt gaaccataaa ccgagcaaca ccaaagtgga taaaaaagtg  gaaccgaaaagctgcgat+/- aaaacccatacc (or aaaacccatctg) +/- tgcccgccgtgcccggcg +/- ccggaactgctgggcggcccgagcgtgtttctg |
| Belimumab | Light/ SEQ ID NO: 142 | agcagcgaac tgacccagga tccggcggtg agcgtggcgc tgggccagac cgtgcgcgtg acctgccagg gcgatagcct gcgcagctat tatgcgagct ggtatcagca gaaaccgggc caggcgccgg tgctggtgat ttatggcaaa aacaaccgcc cgagcggcat tccggatcgc tttagcggca gcagcagcgg caacaccgcg agcctgacca ttaccggcgc gcaggcggaa gatgaagcgg attattattg cagcagccgc gatagcagcg gcaaccattg ggtgtttggc ggcggcaccg aactgaccgt gctgggccag ccgaaagcgg cgccgagcgt gaccctgttt ccgccgagca gcgaagaact gcaggcgaac aaagcgaccc tggtgtgcct gattagcgat ttttatccgg gcgcggtgac cgtggcgtgg aaagcggata gcagcccggt ggcgggcgtg gaaaccacca ccccgagcaa acagagcaac aacaaatatg cggcgagcag ctatctgagc ctgaccccgg aacagtggaa aagccatcgc agctatagct gccaggtgac ccatgaaggc agcaccgtgg aaaaaaccgt ggcgccgacc gaatgcagc |
| Eculizumab | Heavy/ SEQ ID NO: 143 | caggtgcagc tggtgcagag cggcgccgag gtgaagaagc ccggcgccag cgtgaaggtg agctgcaagg ccagcggcta catcttcagc aactactgga tccagtgggt gagacaggcc cccggccagg gcctggagtg gatgggcgag atcctgcccg gcagcggcag caccgagtac accgagaact tcaaggacag agtgaccatg accagagaca ccagcaccag caccgtgtac atggagctga gcagcctgag aagcgaggac accgccgtgt actactgcgc cagatacttc ttcggcagca gccccaactg gtacttcgac gtgtggggcc agggcaccct ggtgaccgtg agcagcgcca gcaccaaggg ccccagcgtg ttccccctgg ccccctgcag cagaagcacc agcgagagca ccgccgccct gggctgcctg gtgaaggact acttccccga gcccgtgacc gtgagctgga acagcggcgc cctgaccagc ggcgtgcaca ccttccccgc cgtgctgcag agcagcggcc tgtacagcct gagcagcgtg gtgaccgtgc ccagcagcaa cttcggcacc cagacctaca cctgcaacgt ggaccacaag cccagcaaca ccaaggtgga caagaccgtg gagagaaagt gctgcgtgga g +/- tgccccccctgccccgcc +/- ccccccgtggccggc |
| Eculizumab | Light/ SEQ ID NO: 144 | gacatccaga tgacccagag ccccagcagc ctgagcgcca gcgtgggcga cagagtgacc atcacctgcg gcgccagcga gaacatctac ggcgccctga actggtacca gcagaagccc ggcaaggccc ccaagctgct gatctacggc gccaccaacc tggccgacgg cgtgcccagc agattcagcg gcagcggcag cggcaccgac ttcaccctga ccatcagcag cctgcagccc gaggacttcg ccacctacta ctgccagaac gtgctgaaca cccccctgac cttcggccag ggcaccaagg tggagatcaa gagaaccgtg gccgccccca gcgtgttcat cttccccccc agcgacgagc agctgaagag cggcaccgcc agcgtggtgt gcctgctgaa caacttctac cccagagagg ccaaggtgca gtggaaggtg gacaacgccc tgcagagcgg caacagccag gagagcgtga ccgagcagga cagcaaggac agcacctaca gcctgagcag caccctgacc ctgagcaagg ccgactacga gaagcacaag gtgtacgcct gcgaggtgac ccaccagggc ctgagcagcc ccgtgaccaa gagcttcaac agaggcgagt gc |
| Andecaliximab | Heavy/ SEQ ID NO: 145 | caggtgcagc tgcaggagag cggccccggc ctggtgaagc ccagcgagac cctgagcctg acctgcaccg tgagcggctt cagcctgctg agctacggcg tgcactgggt gagacagccc cccggcaagg gcctggagtg gctgggcgtg atctggaccg gcggcaccac caactacaac agcgccctga tgagcagatt caccatcagc aaggacgaca gcaagaacac cgtgtacctg aagatgaaca gcctgaagac cgaggacacc gccatctact actgcgccag atactactac ggcatggact actggggcca gggcaccctg gtgaccgtga gcagcgccag caccaagggc cccagcgtgt tccccctggc cccctgcagc agaagcacca gcgagagcac cgccgccctg ggctgcctgg tgaaggacta cttccccgag cccgtgaccg tgagctggaa cagcggcgcc ctgaccagcg gcgtgcacac cttccccgcc gtgctgcaga gcagcggcct gtacagcctg agcagcgtgg tgaccgtgcc cagcagcagc ctgggcacca agacctacac ctgcaacgtg gaccacaagc ccagcaacac caaggtggac aagagagtgg agagcaagta c +/- ggccccccctgccccccctgccccgcc +/- cccgagttcctgggcggccccagcgtgttcctg |
| Andecaliximab | Light/ SEQ ID NO: 146 | gacatccaga tgacccagag ccccagcagc ctgagcgcca gcgtgggcga cagagtgacc atcacctgca aggccagcca ggacgtgaga aacaccgtgg cctggtacca gcagaagccc ggcaaggccc ccaagctgct gatctacagc agcagctaca gaaacaccgg cgtgcccgac agattcagcg gcagcggcag cggcaccgac ttcaccctga ccatcagcag cctgcaggcc gaggacgtgg ccgtgtacta ctgccagcag cactacatca ccccctacac cttcggcggc ggcaccaagg tggagatcaa gagaaccgtg gccgccccca gcgtgttcat cttccccccc |
| | | agcgacgagc agctgaagag cggcaccgcc agcgtggtgt gcctgctgaa caacttctac cccagagagg ccaaggtgca gtggaaggtg gacaacgccc tgcagagcgg caacagccag gagagcgtga ccgagcagga cagcaaggac agcacctaca gcctgagcag caccctgacc ctgagcaagg ccgactacga gaagcacaag gtgtacgcct gcgaggtgac ccaccagggc ctgagcagcc ccgtgaccaa gagcttcaac agaggcgagt gc |
| Lanadelumab | Heavy/ SEQ ID NO: 147 | gaggtgcagc tgctggagag cggcggcggc ctggtgcagc ccggcggcag cctgagactg agctgcgccg ccagcggctt caccttcagc cactacatca tgatgtgggt gagacaggcc cccggcaagg gcctggagtg ggtgagcggc atctacagca gcggcggcat caccgtgtac gccgacagcg tgaagggcag attcaccatc agcagagaca acagcaagaa caccctgtac ctgcagatga acagcctgag agccgaggac accgccgtgt actactgcgc ctacagaaga atcggcgtgc ccagaagaga cgagttcgac atctggggcc agggcaccat ggtgaccgtg agcagcgcca gcaccaaggg ccccagcgtg ttccccctgg cccccagcag caagagcacc agcggcggca ccgccgccct gggctgcctg gtgaaggact acttccccga gcccgtgacc gtgagctgga acagcggcgc cctgaccagc ggcgtgcaca ccttccccgc cgtgctgcag agcagcggcc tgtacagcct gagcagcgtg gtgaccgtgc ccagcagcag cctgggcacc cagacctaca tctgcaacgt gaaccacaag cccagcaaca ccaaggtgga caagagagtg gagcccaaga gctgcgac +/- aagacccacacc (or aagacccacctg) +/- tgcccccctgccccgcc +/- cccgagctgctgggcggccccagcgtgttcctg |
| Lanadelumab | Light/ SEQ ID NO: 148 | gacatccaga tgacccagag ccccagcacc ctgagcgcca gcgtgggcga cagagtgacc atcacctgca gagccagcca gagcatcagc agctggctgg cctggtacca gcagaagccc ggcaaggccc ccaagctgct gatctacaag gccagcaccc tggagagcgg cgtgcccagc agattcagcg gcagcggcag cggcaccgag ttcaccctga ccatcagcag cctgcagccc gacgacttcg ccacctacta ctgccagcag tacaacacct actggacctt cggccagggc accaaggtgg agatcaagag aaccgtggcc gcccccagcg tgttcatctt cccccccagc gacgagcagc tgaagagcgg caccgccagc gtggtgtgcc tgctgaacaa cttctacccc agagaggcca aggtgcagtg gaaggtggac aacgccctgc agagcggcaa cagccaggag agcgtgaccg agcaggacag caaggacagc acctacagcc tgagcagcac cctgaccctg agcaaggccg actacgagaa gcacaaggtg tacgcctgcg aggtgaccca ccagggcctg agcagccccg tgaccaagag cttcaacaga ggcgagtgc |
| Adalimumab | Heavy/ SEQ ID NO: 149 | gaggtgcagc tggtggagag cggcggcggc ctggtgcagc ccggcagaag cctgagactg agctgcgccg ccagcggctt caccttcgac gactacgcca tgcactgggt gagacaggcc cccggcaagg gcctggagtg ggtgagcgcc atcacctgga acagcggcca catcgactac gccgacagcg tggagggcag attcaccatc agcagagaca acgccaagaa cagcctgtac ctgcagatga acagcctgag agccgaggac accgccgtgt actactgcgc caaggtgagc tacctgagca ccgccagcag cctggactac tggggccagg gcaccctggt gaccgtgagc agcgccagca ccaagggccc cagcgtgttc cccctggccc ccagcagcaa gagcaccagc ggcggcaccg ccgccctggg ctgcctggtg aaggactact tccccgagcc cgtgaccgtg agctggaaca gcggcgccct gaccagcggc gtgcacacct tccccgccgt gctgcagagc agcggcctgt acagcctgag cagcgtggtg accgtgccca gcagcagcct gggcacccag acctacatct gcaacgtgaa ccacaagccc agcaacacca aggtggacaa gaaggtggag cccaagagct gcgac +/- aagacccacacc (aagacccacctg) +/- tgccccccctgccccgcc +/- ccgagctgctgggcggccccagcgtgttcctg |
| Adalimumab | Light/ SEQ ID NO: 150 | agattcagcg gcagcggcag cggcaccgac ttcaccctga ccatcagcag cctgcagccc gaggacgtgg ccacctacta ctgccagaga tacaacagag ccccctacac cttcggccag ggcaccaagg tggagatcaa gagaaccgtg gccgccccca gcgtgttcat cttccccccc agcgacgagc agctgaagag cggcaccgcc agcgtggtgt gcctgctgaa caacttctac cccagagagg ccaaggtgca gtggaaggtg gacaacgccc tgcagagcgg caacagccag gagagcgtga ccgagcagga cagcaaggac agcacctaca gcctgagcag caccctgacc ctgagcaagg ccgactacga gaagcacaag gtgtacgcct gcgaggtgac ccaccagggc ctgagcagcc ccgtgaccaa gagcttcaac agaggcgagt gc |
| Infliximab | Heavy/ SEQ ID NO: 151 | gaggtgaagc tggaggagag cggcggcggc ctggtgcagc ccggcggcag catgaagctg agctgcgtgg ccagcggctt catcttcagc aaccactgga tgaactgggt gagacagagc cccgagaagg gcctggagtg ggtggccgag atcagaagca agagcatcaa cagcgccacc cactacgccg agagcgtgaa gggcagattc accatcagca gagacgacag caagagcgcc gtgtacctgc agatgaccga cctgagaacc gaggacaccg gcgtgtacta ctgcagcaga aactactacg gcagcaccta cgactactgg ggccagggca ccaccctgac cgtgagcagc gccagcacca agggccccag cgtgttcccc ctggccccca gcagcaagag caccagcggc ggcaccgccg ccctgggctg cctggtgaag gactacttcc ccgagcccgt gaccgtgagc |
| | | tggaacagcg gcgccctgac cagcggcgtg cacaccttcc ccgccgtgct gcagagcagc ggcctgtaca gcctgagcag cgtggtgacc gtgcccagca gcagcctggg cacccagacc tacatctgca acgtgaacca caagcccagc aacaccaagg tggacaagaa ggtggagccc aagagctgcg ac +/- aagacccacacc (aagacccacctg) +/- tgcccccctgccccgcc +/- cccgagctgctgggcggccccagcgtgttcctg |
| Infliximab | Light/ SEQ ID NO: 152 | gacatcctgc tgacccagag ccccgccatc ctgagcgtga gccccggcga gagagtgagc ttcagctgca gagccagcca gttcgtgggc agcagcatcc actggtacca gcagagaacc aacggcagcc ccagactgct gatcaagtac gccagcgaga gcatgagcgg catccccagc agattcagcg gcagcggcag cggcaccgac ttcaccctga gcatcaacac cgtggagagc gaggacatcg ccgactacta ctgccagcag agccacagct ggcccttcac cttcggcagc ggcaccaacc tggaggtgaa gagaaccgtg gccgccccca gcgtgttcat cttccccccc agcgacgagc agctgaagag cggcaccgcc agcgtggtgt gcctgctgaa caacttctac cccagagagg ccaaggtgca gtggaaggtg gacaacgccc tgcagagcgg caacagccag gagagcgtga ccgagcagga cagcaaggac agcacctaca gcctgagcag caccctgacc ctgagcaagg ccgactacga gaagcacaag gtgtacgcct gcgaggtgac ccaccagggc ctgagcagcc ccgtgaccaa gagcttcaac agaggcgagt gc |
| aTAU | Heavy/ SEQ ID NO: 153 | gaggtgaagg tggtggagag cggcggcggc ctggtgcagc ccggcggcag catgaagctg agctgcgtgg tgagcggctt caccttcagc aactactggg tgaactgggt gaggcaggcc cccggcaagg gcctggagtg ggtggcccag atcaggctga agagcgacaa ctacgccacc cactacgagg agagcgtgaa gggcaggttc accatcagca gggacgacag caagagcagc gtgtacctgc agatgaacaa cctgagggcc gaggacagcg gcatctacta ctgcaccaac tgggaggact actgggccca gggcaccacc gtgaccgtga gcagcgccag caccaagggc cccagcgtgt tccccctggc cccctgcagc aggagcacca gcgagagcac cgccgccctg ggctgcctgg tgaaggacta cttccccgag cccgtgaccg tgagctggaa cagcggcgcc ctgaccagcg gcgtgcacac cttccccgcc gtgctgcaga gcagcggcct gtacagcctg agcagcgtgg tgaccgtgcc cagcagcagc ctgggcacca agacctacac ctgcaacgtg gaccacaagc ccagcaacac caaggtggac aagagggtgg agagcaagta c +/- ggccccccctgccccccctgccccgcc +/- cccgagttcctgggcggccccagcgtgttcctg |
| aTAU | Light/ SEQ ID NO: 154 | gacatcgtgc tgacccagag ccccgacagc ctggccgtga gcctgggcga gagggccacc atcagctgca gggccagcca gagcgtgagc accagcaggt acagctacat ccactggtac cagcagaagc ccggccagcc ccccaagctg ctgatcaagt acgccagcaa cctggagagc ggcgtgccca gcaggttcag cggcagcggc agcggcaccg acttcaccct gaacatccac cccctggagc ccgaggactt cgccacctac tactgccacc acagctggga gatccccctg accttcggcc agggcaccaa gctggagatc aagaggaccg tggccgcccc cagcgtgttc atcttccccc ccagcgacga gcagctgaag agcggcaccg ccagcgtggt gtgcctgctg aacaacttct accccaggga ggccaaggtg cagtggaagg tggacaacgc cctgcagagc ggcaacagcc aggagagcgt gaccgagcag gacagcaagg acagcaccta cagcctgagc agcaccctga ccctgagcaa ggccgactac gagaagcaca aggtgtacgc ctgcgaggtg acccaccagg gcctgagcag ccccgtgacc aagagcttca acaggggcga gtgc |
| Erenumab | Heavy/ SEQ ID NO: 155 | caggtgcagc tggtggagag cggcggcggc gtggtgcagc ccggcagaag cctgagactg agctgcgccg ccagcggctt caccttcagc agcttcggca tgcactgggt gagacaggcc cccggcaagg gcctggagtg ggtggccgtg atcagcttcg acggcagcat caagtacagc gtggacagcg tgaagggcag attcaccatc agcagagaca acagcaagaa caccctgttc ctgcagatga acagcctgag agccgaggac accgccgtgt actactgcgc cagagacaga ctgaactact acgacagcag cggctactac cactacaagt actacggcat ggccgtgtgg ggccagggca ccaccgtgac cgtgagcagc gccagcacca agggccccag cgtgttcccc ctggccccct gcagcagaag caccagcgag agcaccgccg ccctgggctg cctggtgaag gactacttcc ccgagcccgt gaccgtgagc tggaacagcg gcgccctgac cagcggcgtg cacaccttcc ccgccgtgct gcagagcagc ggcctgtaca gcctgagcag cgtggtgacc gtgcccagca gcaacttcgg cacccagacc tacacctgca acgtggacca caagcccagc aacaccaagg tggacaagac cgtggagaga aagtgctgcgt ggagtgcccc ccctgccccc gcccccccg tggccggc |
| Erenumab | Light/ SEQ ID NO: 156 | cagagcgtgc tgacccagcc ccccagcgtg agcgccgccc ccggccagaa ggtgaccatc agctgcagcg gcagcagcag caacatcggc aacaactacg tgagctggta ccagcagctg cccggcaccg cccccaagct gctgatctac gacaacaaca agagacccag cggcatcccc gacagattca gcggcagcaa gagcggcacc agcaccaccc tgggcatcac cggcctgcag accggcgacg aggccgacta ctactgcggc acctgggaca gcagactgag cgccgtggtg ttcggcggcg gcaccaagct |
| | | gaccgtgctg ggccagccca aggccaaccc caccgtgacc ctgttccccc ccagcagcga ggagctgcag gccaacaagg ccaccctggt gtgcctgatc agcgacttct accccggcgc cgtgaccgtg gcctggaagg ccgacggcag ccccgtgaag gccggcgtgg agaccaccaa gcccagcaag cagagcaaca acaagtacgc cgccagcagc tacctgagcc tgacccccga gcagtggaag agccacagaa gctacagctg ccaggtgacc cacgagggca gcaccgtgga gaagaccgtg gcccccaccg agtgcagc |
| BAN2401 | Heavy/ SEQ ID NO: 157 | gaggtgcagc tggtggagag cggcggcggc ctggtgcagc ccggcggcag cctgaggctg agctgcagcg ccagcggctt caccttcagc agcttcggca tgcactgggt gaggcaggcc cccggcaagg gcctggagtg ggtggcctac atcagcagcg gcagcagcac catctactac ggcgacaccg tgaagggcag gttcaccatc agcagggaca acgccaagaa cagcctgttc ctgcagatga gcagcctgag ggccgaggac accgccgtgt actactgcgc cagggagggc ggctactact acggcaggag ctactacacc atggactact ggggccaggg caccaccgtg accgtgagca gcgccagcac caagggcccc agcgtgttcc ccctggcccc cagcagcaag agcaccagcg gcggcaccgc cgccctgggc tgcctggtga aggactactt ccccgagccc gtgaccgtga gctggaacag cggcgccctg accagcggcg tgcacacctt ccccgccgtg ctgcagagca gcggcctgta cagcctgagc agcgtggtga ccgtgcccag cagcagcctg ggcacccaga cctacatctg caacgtgaac cacaagccca gcaacaccaa ggtggacaag aaggtggagcccaagagctgcgac +/- aagacccacacc (or aagacccacctg) +/- tgcccccctgccccgcc +/ ccgagctgctgggcggc |
| BAN2401 | Light/ SEQ ID NO: 158 | gacgtggtga tgacccagag ccccctgagc ctgcccgtga cccccggcgc ccccgccagc atcagctgca ggagcagcca gagcatcgtg cacagcaacg gcaacaccta cctggagtgg tacctgcaga agcccggcca gagccccaag ctgctgatct acaaggtgag caacaggttc agcggcgtgc ccgacaggtt cagcggcagc ggcagcggca ccgacttcac cctgaggatc agcagggtgg aggccgagga cgtgggcatc tactactgct tccagggcag ccacgtgccc cccaccttcg gccccggcac caagctggag atcaagagga ccgtggccgc ccccagcgtg ttcatcttcc cccccagcga cgagcagctg aagagcggca ccgccagcgt ggtgtgcctg ctgaacaact tctaccccag ggaggccaag gtgcagtgga aggtggacaa cgccctgcag agcggcaaca gccaggagag cgtgaccgag caggacagca aggacagcac ctacagcctg agcagcaccc tgaccctgag caaggccgac tacgagaagc acaaggtgta cgcctgcgag |
| | | gtgacccacc agggcctgag cagccccgtg accaagagct tcaacagggg cgagtgc |
| E06-scFV | Heavy/ SEQ ID NO: 159 | gaggtgaagc tggtggagag cggcggcggc ctggtgcagc ccggcggcag cctgaggctg agctgcgcca ccagcggctt caccttcagc gacttctaca tggagtgggt gaggcaggcc cccggcaaga ggctggagtg gatcgccgcc agcaggaaca aggccaacga ctacaccacc gagtacgccg acagcgtgaa gggcaggttc atcgtgagca gggacaccag ccagagcatc ctgtacctgc agatgaacgc cctgagggcc gaggacaccg ccatctacta ctgcgccagg gactactacg gcagcagcta ctggtacttc gacgtgtggg gcgccggcac caccgtgacc gtgagcagc |
| E06-scFV | Light/ SEQ ID NO: 160 | gacatcgtga tgacccagag ccccagcagc ctgagcgtga gcgccggcaa gaaggtgacc atcagctgca ccgccagcga gagcctgtac agcagcaagc acaaggtgca ctacctggcc tggtaccaga agaagcccga gcagagcccc aagctgctga tctacggcgc cagcaacagg tacatcggcg tgcccgacag gttcaccggc agcggcagcg gcaccgactt caccctgacc atcagcagcg tgcaggtgga ggacctgacc cactactact gcgcccagtt ctacagctac cccctgacct tcggcgccgg caccaagctg gagatcaag |

### EQUIVALENTS

Although the invention is described in detail with reference to specific embodiments thereof, it will be understood that variations which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claims. Those skilled in the art will recognize, or be able to ascertain using no more than routine experimentation, many equivalents to the specific embodiments of the invention described herein. Such equivalents are intended to be encompassed by the following claims.

All publications, patents and patent applications mentioned in this specification are herein incorporated by reference into the specification to the same extent as if each individual publication, patent or patent application was specifically and individually indicated to be incorporated herein by reference in their entireties.

## Claims

1. A pharmaceutical composition for use in treating hereditary angioedema in a human subject in need thereof, comprising an AAV vector comprising:
(a) a viral capsid that is at least 95% identical to the amino acid sequence of an AAV8 capsid (SEQ ID NO: 78) or an AAV9 capsid (SEQ ID NO: 79); and
(b) an artificial genome comprising an expression cassette flanked by AAV ITRs wherein the expression cassette comprises a transgene encoding a lanadelumab antibody or an antigen-binding fragment thereof, operably linked to one or more regulatory sequences that control expression of the transgene in human muscle cells or human liver cells, and
wherein the transgene comprises a nucleotide sequence encoding a heavy chain comprising heavy chain CDRs i) GFTFSHYIMM (amino acids 26-35 of SEQ ID NO: 47); ii) GIYSSGGITVYADSVKG (amino acids 50-66 of SEQ ID NO: 47); and iii) YRRIGVPRRDEFDI (amino acids 98-111 of SEQ ID NO: 47);
and wherein the transgene comprises a nucleotide sequence encoding a light chain comprising light chain CDRs i) SASVGDRVTITCRASQSISSWLA (amino acids 12-34 of SEQ ID NO: 48); ii) KASTLES (amino acids 50-56 of SEQ ID NO: 48); and iii) QQYNTYWT (amino acids 89-96 of SEQ ID NO: 48);
and wherein the nucleotide sequence encoding the heavy chain and the nucleotide sequence encoding the light chain are separated by a nucleotide sequence encoding a cleavable linker or an IRES; and
wherein said AAV vector is formulated for intravenous administration to the liver or muscle of said subject such that the lanadelumab antibody or antigen binding fragment thereof is expressed in said subject.

2. The pharmaceutical composition for use according to claim 1, wherein the lanadelumab antibody or antigen-binding fragment thereof is a Fab, a F(ab')₂, or a full-length antibody.

3. The pharmaceutical composition for use according to claim 1 or claim 2, wherein the lanadelumab antibody or antigen-binding fragment thereof comprises a heavy chain with an amino acid sequence of SEQ ID NO: 47 and a light chain with an amino acid sequence of SEQ ID NO: 48.

4. The pharmaceutical composition for use according to any one of claims 1 to 3, wherein the cleavable linker is a Furin/F2A linker, Furin/P2A linker, Furin/T2A linker, or Furin/E2A linker.

5. The pharmaceutical composition for use according to any one of claims 1 to 4, wherein the nucleotide sequence encoding the heavy chain comprises the nucleotide sequence of SEQ ID NO: 147 and the nucleotide sequence encoding the light chain comprises the nucleotide sequence of SEQ ID NO: 148.

6. The pharmaceutical composition for use according to any one of claims 1 to 5, wherein the transgene comprises all or a portion of a hinge domain sequence, at the C-terminus of the heavy chain.

7. The pharmaceutical composition for use according to any one claims 1 to 6, wherein the lanadelumab antibody is a full length antibody.

8. The pharmaceutical composition for use according to any one of claims 1 to 7, wherein the transgene encodes a signal sequence at the N-terminus of each of the heavy chain and the light chain of said lanadelumab antibody or antigen-binding fragment thereof, and wherein said signal sequence directs secretion and post translational modification in said human liver cells or human muscle cells.

9. The pharmaceutical composition for use according to claim 8, wherein said signal sequence is MYRMQLLLLIALSLALVTNS (SEQ ID NO: 161) or a signal sequence from Table 3.

10. The pharmaceutical composition for use according to any one of claims 1 to 9, wherein the AAV capsid is an AAV8 capsid.

11. A host cell comprising:
an artificial genome comprising an expression cassette flanked by AAV inverted terminal repeats (ITRs), wherein the expression cassette comprises a transgene encoding a lanadelumab antibody or antigen-binding fragment thereof, which transgene comprises a nucleotide sequence encoding a heavy chain comprising an amino acid sequence of SEQ ID NO: 47 and a nucleotide sequence encoding a light chain comprising an amino acid sequence of SEQ ID NO: 48; and wherein the nucleotide sequence encoding SEQ ID NO: 47 and the nucleotide sequence encoding SEQ ID NO: 48 are separated by a nucleotide sequence encoding a cleavable linker or an IRES; and
wherein each of the nucleotide sequence encoding the heavy chain and the nucleotide sequence encoding the light chain are operably linked to one or more regulatory sequences that control expression of the heavy chain and the light chain in human liver cells or human muscle cells.

12. A method of producing recombinant AAVs comprising:
(a) culturing a host cell containing:
(i) an artificial genome comprising an expression cassette flanked by AAV inverted terminal repeats (ITRs), wherein the expression cassette comprises a transgene encoding a lanadelumab antibody or antigen-binding fragment thereof, which transgene comprises a nucleotide sequence encoding a heavy chain comprising an amino acid sequence of SEQ ID NO: 47 and a nucleotide sequence encoding a light chain comprising an amino acid sequence of SEQ ID NO: 48; and wherein the nucleotide sequence encoding SEQ ID NO: 47 and the nucleotide sequence encoding SEQ ID NO: 48 are separated by a nucleotide sequence encoding a cleavable linker or an IRES; and
wherein each of the nucleotide sequence encoding the heavy chain and the nucleotide sequence encoding the light chain are operably linked to one or more regulatory sequences that control expression of the heavy chain and the light chain in human liver cells or human muscle cells;
(ii) a trans expression cassette lacking AAV ITRs, wherein the trans expression cassette encodes an AAV rep protein and an AAV capsid protein operably linked to expression control elements that drive expression of the AAV rep protein and AAV capsid protein in the host cell in culture and supply the rep and cap proteins in trans;
(iii) sufficient adenovirus helper functions to permit replication and packaging of the artificial genome by the AAV capsid proteins; and
(b) recovering recombinant AAV encapsidating the artificial genome from the cell culture.

13. The method of claim 14, wherein the AAV capsid protein is an AAV8 capsid protein or an AAV9 capsid protein.

14. A recombinant AAV, produced by the method comprising:
(a) culturing a host cell containing:
(i) an artificial genome comprising an expression cassette flanked by AAV inverted terminal repeats (ITRs), wherein the expression cassette comprises a transgene encoding a lanadelumab antibody or antigen-binding fragment thereof, which transgene comprises a nucleotide sequence encoding a heavy chain comprising an amino acid sequence of SEQ ID NO: 47 and a nucleotide sequence encoding a light chain comprising an amino acid sequence of SEQ ID NO:48; and wherein the nucleotide sequence encoding SEQ ID NO: 47 and the nucleotide sequence encoding SEQ ID NO:48 are separated by a nucleotide sequence encoding a cleavable linker or an IRES; and
wherein each of the nucleotide sequence encoding the heavy chain and the nucleotide sequence encoding the light chain are operably linked to one or more regulatory sequences that control expression of the heavy chain and the light chain in human liver cells or human muscle cells;
(ii) a trans expression cassette lacking AAV ITRs, wherein the trans expression cassette encodes an AAV rep protein and an AAV capsid protein that is at least 95% identical to the amino acid sequence of an AAV8 capsid (SEQ ID NO: 78) or AAV9 capsid (SEQ ID NO: 79) operably linked to expression control elements that drive expression of the AAV rep protein and AAV capsid protein in the host cell in culture and supply the rep and cap proteins in trans;
(iii) sufficient adenovirus helper functions to permit replication and packaging of the artificial genome by the AAV capsid proteins; and
(b) recovering recombinant AAV encapsidating the artificial genome from the cell culture.

15. The recombinant AAV according to claim 14, wherein the AAV capsid protein is an AAV8 capsid protein or an AAV9 capsid protein.
